(19) Europäisches Patentamt
European Patent Office
Office européen des brevets

(11) Publication number: **0 401 522 B1**

(12) **EUROPEAN PATENT SPECIFICATION**

(45) Date of publication of patent specification: **06.12.95**

(51) Int. Cl.[6]: **C07D 239/26**, C07D 239/34, C09K 19/34, C09K 19/42

(21) Application number: **90108594.4**

(22) Date of filing: **07.05.90**

The file contains technical information submitted after the application was filed and not included in this specification

# (54) Liquid crystal composition and liquid crystal device using same.

(30) Priority: **08.05.89 JP 115682/89**
**26.01.90 JP 16557/90**

(43) Date of publication of application:
**12.12.90 Bulletin 90/50**

(45) Publication of the grant of the patent:
**06.12.95 Bulletin 95/49**

(84) Designated Contracting States:
**AT BE CH DE DK ES FR GB GR IT LI LU NL SE**

(56) References cited:

| | |
|---|---|
| **EP-A- 0 268 198** | **EP-A- 0 293 910** |
| **EP-A- 0 307 880** | **EP-A- 0 308 794** |
| **WO-A-86/04060** | **DE-A- 3 518 734** |

(73) Proprietor: **CANON KABUSHIKI KAISHA**
**30-2, 3-chome, Shimomaruko,**
**Ohta-ku**
**Tokyo (JP)**

(72) Inventor: **Mori, Shosei**
**c/o Canon Kabushiki Kaisha,**
**3-30-2, Shimomaruko**
**Ohta-ku,**
**Tokyo (JP)**

Inventor: **Takiguchi, Takao**
**c/o Canon Kabushiki Kaisha,**
**3-30-2, Shimomaruko**
**Ohta-ku,**
**Tokyo (JP)**

Inventor: **Iwaki, Takashi**
**c/o Canon Kabushiki Kaisha,**
**3-30-2, Shimomaruko**
**Ohta-ku,**
**Tokyo (JP)**

Inventor: **Yamada, Yoko**
**c/o Canon Kabushiki Kaisha,**
**3-30-2, Shimomaruko**
**Ohta-ku,**
**Tokyo (JP)**

Inventor: **Togano, Takeshi**
**c/o Canon Kabushiki Kaisha,**
**3-30-2, Shimomaruko**
**Ohta-ku,**
**Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 401 522 B1

Inventor: **Yamashita, Masataka**
**c/o Canon Kabushiki Kaisha,**
**3-30-2, Shimomaruko**
**Ohta-ku,**
**Tokyo (JP)**
Inventor: **Terada, Masahiro**
**c/o Canon Kabushiki Kaisha,**
**3-30-2, Shimomaruko**
**Ohta-ku,**
**Tokyo (JP)**
Inventor: **Katagiri, Kazuharu**
**c/o Canon Kabushiki Kaisha,**
**3-30-2, Shimomaruko**
**Ohta-ku,**
**Tokyo (JP)**

(74) Representative: **Bühling, Gerhard, Dipl.-Chem. et al**
**Patentanwaltsbüro**
**Tiedtke-Bühling-Kinne & Partner**
**Bavariaring 4**
**D-80336 München (DE)**

## Description

FIELD OF THE INVENTION AND RELATED ART

The present invention relates to a novel liquid crystal composition and liquid crystal device using the composition, and more particularly to a novel liquid crystal composition with improved responsiveness to an electric field and a liquid crystal device using the liquid crystal composition for use in a liquid crystal display apparatus, a liquid crystal-optical shutter, etc.

Hitherto, liquid crystal devices have been used as an electro-optical device in various fields. Most liquid crystal devices which have been put into practice use TN (twisted nematic) type liquid crystals, as shown in "Voltage-Dependent Optical Activity of a Twisted Nematic Liquid Crystal" by M. Schadt and W. Helfrich "Applied Physics Letters" Vol. 18, No. 4 (Feb. 15, 1971) pp. 127-128.

These devices are based on the dielectric alignment effect of a liquid crystal and utilize an effect that the average molecular axis direction is directed to a specific direction in response to an applied electric field because of the dielectric anisotropy of liquid crystal molecules. It is said that the limit of response speed is on the order of milli-seconds, which is too slow for many uses. On the other hand, a simple matrix system of driving is most promising for application to a large-area flat display in view of cost, productivity, etc., in combination. In the simple matrix system, an electrode arrangement wherein scanning electrodes and signal electrodes are arranged in a matrix, and for driving, a multiplex driving scheme is adopted wherein an address signal is sequentially, periodically and selectively applied to the scanning electrodes and prescribed data signals are selectively applied in parallel to the signal electrodes in synchronism with the address signal.

When the above-mentioned TN-type liquid crystal is used in a device of such a driving system, a certain electric field is applied to regions where a scanning electrode is selected and signal electrodes are not selected or regions where a scanning electrode is not selected and a signal electrode is selected (which regions are so called "half-selected points"). If the difference between a voltage applied to the selected points and a voltage applied to the half-selected points is sufficiently large, and a voltage threshold level required for allowing liquid crystal molecules to be aligned or oriented perpendicular to an electric field is set to a value therebetween, display devices normally operate. However, in fact, as the number (N) of scanning lines increases, a time (duty ratio) during which an effective electric field is applied to one selected point when a whole image area (corresponding to one frame) is scanned decreases with a ratio of 1/N. Accordingly, the larger the number of scanning lines are, the smaller is the voltage difference of an effective value applied to a selected point and non-selected points when scanning is repeatedly effected. As a result, this leads to unavoidable drawbacks of lowering of image contrast or occurrence of interference or crosstalk. These phenomena are regarded as essentially unavoidable problems appearing when a liquid crystal having no bistability (i.e. liquid crystal molecules are horizontally oriented with respect to the electrode surface as stable state and are vertically oriented with respect to the electrode surface only when an electric field is effectively applied) is driven (i.e. repeatedly scanned) by using a time storage effect. To overcome these drawbacks, the voltage averaging method, the two-frequency driving method, the multiple matrix method, etc. has been already proposed. However, any method is not sufficient to overcome the above-mentioned drawbacks. As a result, it is the present state that the development of large image area or high packaging density with respect to display elements is delayed because it is difficult to sufficiently increase the number of scanning lines.

To overcome drawbacks with such prior art liquid crystal devices, the use of liquid crystal devices having bistability has been proposed by Clark and Lagerwall (e.g. Japanese Laid-Open Patent Appln. No. 56-107216, U.S.P. No. 4367924, etc.). In this instance, as the liquid crystals having bistability, ferroelectric liquid crystals having chiral smectic C-phase (SmC*) or H-phase (SmH*) are generally used. These liquid crystals have bistable states of first and second optically stable states with respect to an electric field applied thereto. Accordingly, as different from optical modulation devices in which the above-mentioned TN-type liquid crystals are used, the bistable liquid crystal molecules are oriented to first and second optically stable states with respect to one and the other electric field vectors, respectively. Further, this type of liquid crystal has a property (bistability) of assuming either one of the two stable states in response to an applied electric field and retaining the resultant state in the absence of an electric field.

In addition to the above-described characteristic of showing bistability, such a ferroelectric liquid crystal (hereinafter sometimes abbreviated as "FLC") has an excellent property, i.e., a high-speed responsiveness. This is because the spontaneous polarization of the ferroelectric liquid crystal and an applied electric field directly interact with each other to induce transition of orientation states. The resultant response speed is faster than the response speed due to the interaction between dielectric anisotropy and an electric field by

3 to 4 digits.

Thus, a ferroelectric liquid crystal potentially has very excellent characteristics, and by making use of these properties, it is possible to provide essential improvements to many of the above-mentioned problems with the conventional TN-type devices. Particularly, the application to a high-speed optical shutter and a display of a high density and a large picture is expected. For this reason, there has been made extensive research with respect to liquid crystal materials showing ferroelectricity. However, ferroelectric liquid crystal materials developed heretofore cannot be said to satisfy sufficient characteristics required for a liquid crystal device including low-temperature operation characteristic, high-speed responsiveness, etc. Among a response time $\tau$, the magnitude of spontaneous polarization Ps and viscosity $\eta$, the following relationship exists: $\tau = \eta/(Ps \cdot E)$, where E is an applied voltage. Accordingly, a high response speed can be obtained by (a) increasing the spontaneous polarization Ps, (b) lowering the viscosity $\eta$, or (c) increasing the applied voltage E. However, the driving voltage has a certain upper limit in view of driving with IC, etc., and should desirably be as low as possible. Accordingly, it is actually necessary to lower the viscosity $\eta$ or increase the spontaneous polarization Ps.

A ferroelectric chiral smectic liquid crystal having a large spontaneous polarization generally provides a large internal electric field in a cell given by the spontaneous polarization and is liable to pose many constraints on the device construction giving bistability. Further, an excessively large spontaneous polarization is liable to accompany an increase in viscosity, so that remarkable increase in response speed may not be attained as a result.

Further, if it is assumed that the operation temperature of an actual display device is 5 - 40 °C, the response speed changes by a factor of about 20, so that it actually exceeds the range controllable by driving voltage and frequency.

As described hereinabove, commercialization of a ferroelectric liquid crystal device requires a ferroelectric chiral smectic liquid crystal composition having a low viscosity, a high-speed responsiveness and a small temperature-dependence of response speed.

In DE-A-3 518 734 and EP-A-0 293 910 ferroelectric liquid crystal compositions are disclosed comprising a mesomorphic compound having a cyclohexyl group and a phenyl pyridine moiety in its structural core. According to DE-A-3 518 734 the phenyl ring may have a fluoro substituent.

## SUMMARY OF THE INVENTION

An object of the present invention is to provide a liquid crystal composition, particularly a ferroelectric chiral smectic liquid crystal composition for providing a practical ferroelectric liquid crystal device, and a liquid crystal device using the liquid crystal composition and having a high response speed-and a smaller temperature-dependence of the response speed.

According to the present invention, there is provided a liquid crystal composition, comprising: at least one mesomorphic compound represented by the below formula (I)

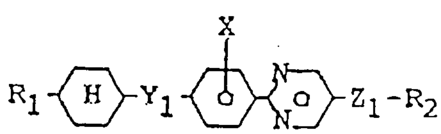

(I),

wherein $R_1$ denotes an n-alkyl group having 1-16 carbon atoms; $R_2$ denotes an optically active or inactive group selected from the following groups (i) to (iv):

(i) n-alkyl group having 1-16 carbon atoms;

(ii)

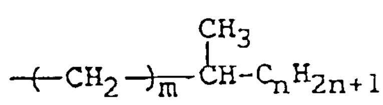

wherein m is 1-7 and n is 2-9 with proviso that $3 \leq m + n \leq 14$;

(iii)

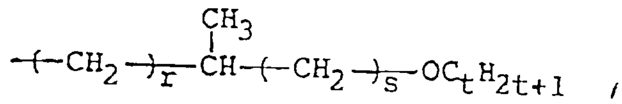

wherein r is 0-7 s is 0 or 1 and t is 1-14 with proviso that $1 \leq r+s+t \leq 14$; and

(iv)

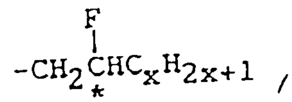

wherein x is 1-14 and * denotes an optical active center;

$Y_1$ denotes -COO-, -OCO-, $-CH_2O-$ or $-OCH_2-$; $Z_1$ denotes a single bond, -O-, -COO-, -OCO- or -OCOO-; and X denotes a halogen, cyano group or methyl group; and

at least one mesomorphic compound represented by the following formula (III):

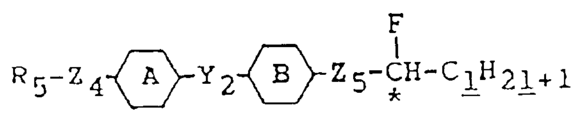

(III),

wherein $R_5$ denotes a linear or branched alkyl group having 1-18 carbon atoms; $y_2$ denotes a single bond, -COO-, -OCO-, -COS-, SCO-, $CH_2O-$, $-OCH_2-$ or -CH=CH-COO-; $Z_4$ denotes a single bond, -O-, -COO- or -OCO-; $Z_5$ denotes $-OCH_2-$, $-COOCH_2-$, -OCO- or

$-O-(CH_2)_k-O-CH_2$;

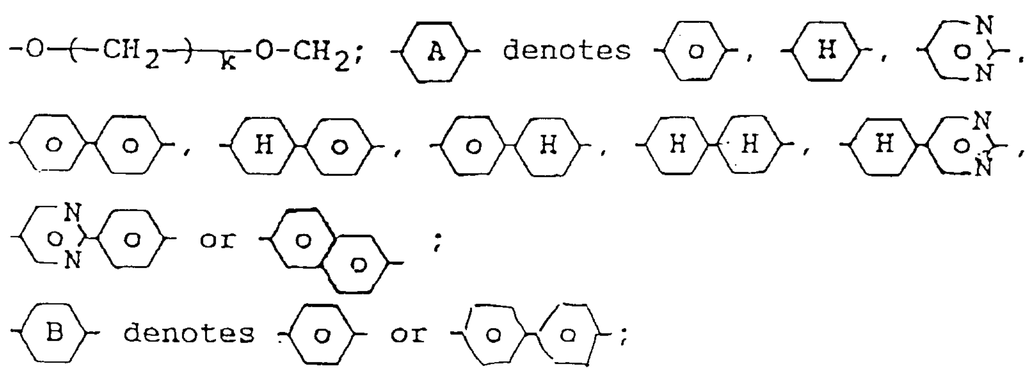

denotes , , , , , , , , or ;

denotes or ;

l is 1 - 12 and k is 1 - 4.

Further, according to the present invention there is provided a liquid crystal composition comprising at least one mesomorphic compound represented by the following formula (I):

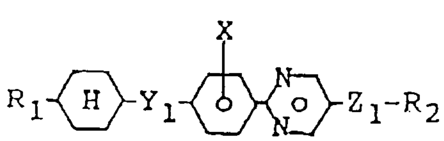

(I),

wherein $R_1$ denotes an n-alkyl group having 1-16 carbon atoms; $R_2$ denotes an optically active or inactive group selected from the following groups (i) to (iv):

(i) n-alkyl group having 1-16 carbon atoms;

(ii)

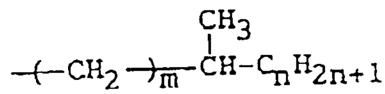

wherein m is 1-7 and n is 2-9 with proviso that $3 \leqq m + n \leqq 14$;

(iii)

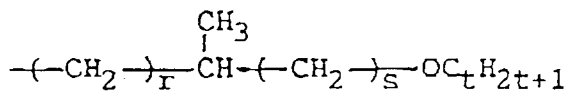

wherein r is 0-7, s is 0 or 1 and t is 1-14 with proviso that $1 \leqq r + s + t \leqq 14$; and

(iv)

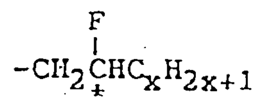

wherein x is 1-14; and * denotes an optically active center; $Y_1$ denotes -COO-, -OCO-, $-CH_2O-$ or $-OCH_2-$; $Z_1$ denotes a single bond, -O-, -COO-, -OCO- or -OCOO-; and X denotes a halogen, cyano group or methyl group;

at least one mesomorphic compound represented by the following formula (II):

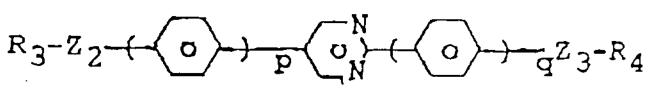

(II),

wherein $R_3$ and $R_4$ respectively denote a linear or branched optically inactive alkyl group having 1-18 carbon atoms capable of having $C_1$ - $C_{12}$ alkoxy group; $Z_2$ and $Z_3$ respectively denote a single bond, -O-, -OCO-, -COO- or -OCOO-; and p and q are respectively O, 1 or 2 and (III):

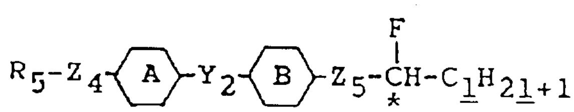

(III),

wherein $R_5$ denotes a linear or branched alkyl group having 1-18 carbon atoms; $y_2$ denotes a single bond, -COO-, -OCO-, -COS-, SCO-, $CH_2O$-, $-OCH_2-$ or - CH=CH-COO-; $Z_4$ denotes a single bond, -O-, -COO- or -OCO-; $Z_5$ denotes $-OCH_2-$, $-COOCH_2-$, -OCO- or

$-O-(-CH_2-)_k-O-CH_2$; 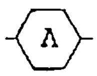 denotes 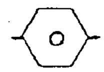, 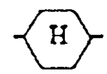, 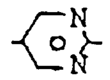,
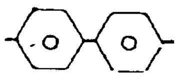, 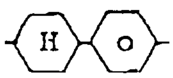, 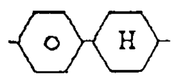, 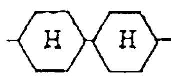, 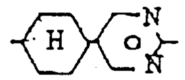,
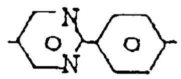 or 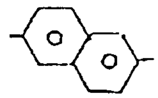 ;
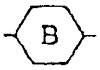 denotes 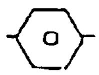 or 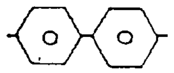;

l is 1 - 12 and k is 1 - 4.

The present invention further provides liquid crystal devices comprising a pair of substrates and any one of the liquid crystal compositions as described above disposed between the electrode plates.

These and other objects, features and advantages of the present invention will become more apparent upon a consideration of the following description of the preferred embodiments of the present invention taken in conjunction with the accompanying drawings.

## BRIEF DESCRIPTION OF THE DRAWINGS

Figure 1 is a schematic sectional view of a liquid crystal display device using a ferroelectric liquid crystal; and

Figures 2 and 3 are schematic perspective views of a device cell embodiment for illustrating the operation principle of a ferroelectric liquid crystal device.

## DETAILED DESCRIPTION OF THE INVENTION

Preferred examples of the compounds represented by the above-mentioned general formula (I) may include those represented by the following formulas (I-a) and (I-b):

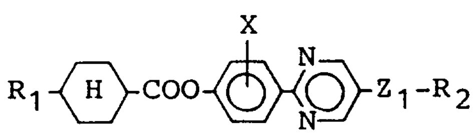 (I-a)

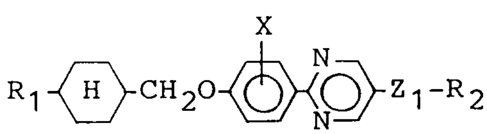 (I-b).

In the above formulas (I-a) and (I-b), $R_1$, $R_2$, X and $Z_1$ are the same as in the general formula (I). A particularly preferred class of compounds among the compounds represented by the above formulas (I-a) and (I-b) may include those represented by the following formula (I-c):

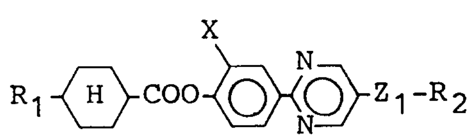 (I-c).

In the above-mentioned formula (I), $R_1$ is a group of (i) below and $R_2$ is a group selected from the following groups (i) to (iv).

(i) n-alkyl group having 1 - 16 carbon atoms;
(ii)

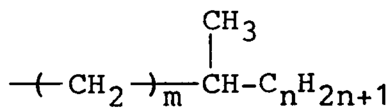

wherein m is 1 - 7 and n is 2 - 9 with proviso that $3 \leqq m+n \leqq 14$ (optically active or inactive);
(iii)

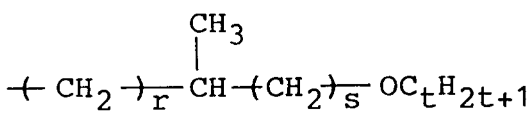

wherein r is 0 - 7, s is 0 or 1 and t is 1 - 14 with proviso that $1 \leqq r+s+t \leqq 14$ (optically active or inactive); and
(iv)

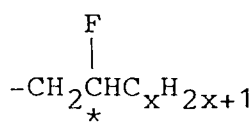

wherein x is 1 - 14. Herein * denotes an optically active center.

Further, in the above-mentioned formula (I), preferred examples of X may include halogens such as fluorine, chlorine and bromine, among which fluorine is particularly preferred.

Still further, in the above-mentioned formula (I), preferred examples of $Z_1$ may include a single bond, -O-, -COO- and -OCO-, among which a single bond or -O- is particularly preferred.

Preferred examples of the compounds represented by the above-mentioned formula (II) may include those represented by the following formulas (II-a) to (II-e):

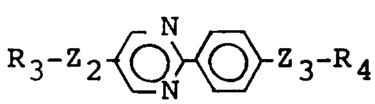 (II-a)

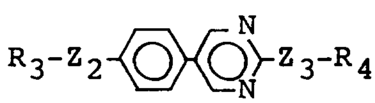 (II-b)

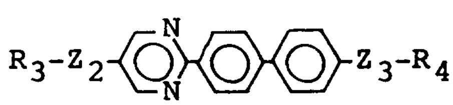 (II-c)

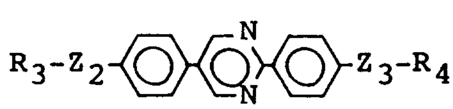 (II-d)

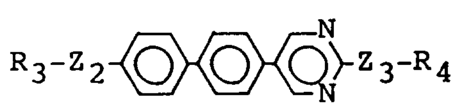 (II-e)

In the above formulas (II-a) to (II-e), $R_3$, $R_4$, $Z_2$ and $Z_3$ are the same as in the general formula (II). Preferred examples of $Z_2$ and $Z_3$ may include those of the following combinations (II-i) to (II-viii):

(II-i) $Z_2$ is a single bond and $Z_3$ is a single bond,
(II-ii) $Z_2$ is a single bond and $Z_3$ is -O-,
(II-iii) $Z_2$ is a single bond and $Z_3$ is -OCO-,
(II-iv) $Z_2$ is a single bond and $Z_3$ is -COO-,
(II-v) $Z_2$ is -O- and $Z_3$ is a single bond,
(II-vi) $Z_2$ is -O- and $Z_3$ is -O-,
(II-vii) $Z_2$ is -O- and $Z_3$ is -OCO-, and
(II-Viii) $Z_2$ is -O- and $Z_3$ is -COO-.

Further, in the above formulas (II-a) to (II-e), each of $R_3$ and $R_4$ may preferably be a linear or branched alkyl group having 4 - 14 carbon atoms capable of having a $C_1$ - $C_{12}$ alkyoxy substituent. Particularly preferred examples of $R_3$ and $R_4$ may include those of the following combinations (II-ix) to (II-xi):

(II-ix) $R_3$ is an n-alkyl group having 4 - 14 carbon atoms and $R_4$ is an n-alkyl group having 4 - 14 carbon atoms,

(II-x) $R_3$ is an n-alkyl group having 4 - 14 carbon atoms and $R_4$ is

$$-(CH_2)_u-\overset{\displaystyle CH_3}{\overset{|}{C}H}-R_6 ,$$

wherein u is 0 - 7 and $R_6$ denotes a linear or branched alkyl group,

(II-xi) $R_3$ is an n-alkyl group and $R_4$ is

$$-(CH_2)_y-\overset{\displaystyle CH_3}{\overset{|}{C}H}-(CH_2)_u-OR_6$$

wherein y is 0 - 7, u is 0 or 1, and $R_6$ denotes a linear or branched alkyl group.

Preferred examples of the compounds represented by the above-mentioned general formula (III) may include those represented by the following formulas (III-a) to (III-f):

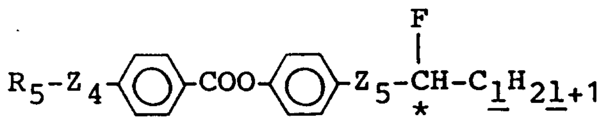

(III-a)

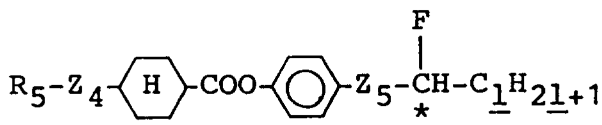

(III-b)

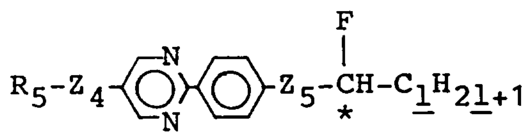

(III-c)

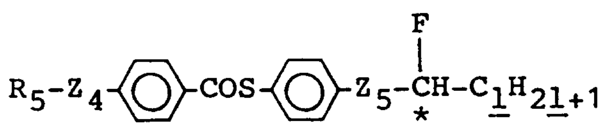

(III-d)

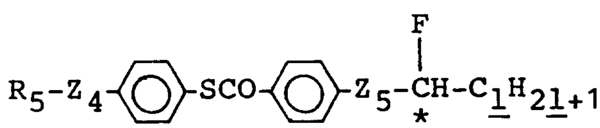

(III-e)

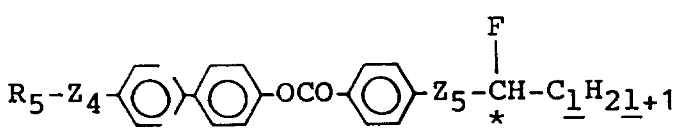

(III-f).

In the above formulas (III-a) to (III-f), $R_5$, $Z_4$, $Z_5$ and $\underline{l}$ are the same as in the general formula (III). Particularly preferred compounds among the compounds represented by the above formulas (III-a) to (III-f) may include those represented by the formulas (III-a) to (III-c).

Further, in the above formulas (III-a) to (III-f), preferred examples of $Z_4$ and $Z_5$ may include those of the following combinations (III-i) to (III-v):

(III-i) $Z_4$ is a single bond and $Z_5$ is $-OCH_2-$,

(III-ii) $Z_4$ is a single bond and $Z_5$ is $-COOCH_2-$,

(III-iii) $Z_4$ is a single bond and $Z_5$ is -OCO-,

(III-iv) $Z_4$ is -O- and $Z_5$ is $-OCH_2-$,

(III-v) $Z_4$ is -O- and $Z_5$ is $-COOCH_2-$.

Representative reaction schemes for producing the mesomorphic compounds represented by the above formula (I) are shown below.

(Case where $Y_1$ is -COO- or $-CH_2O-$)

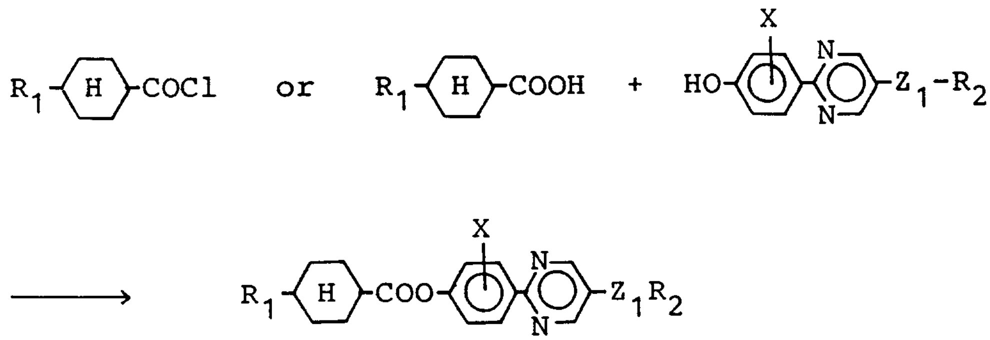


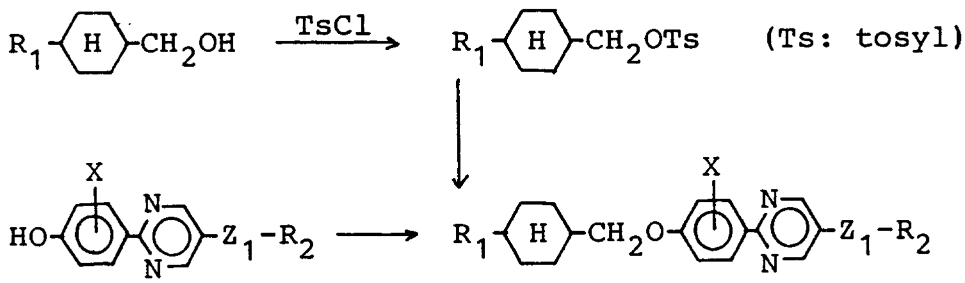


(Case where $Y_1$ is -OCO- or $-OCH_2-$)

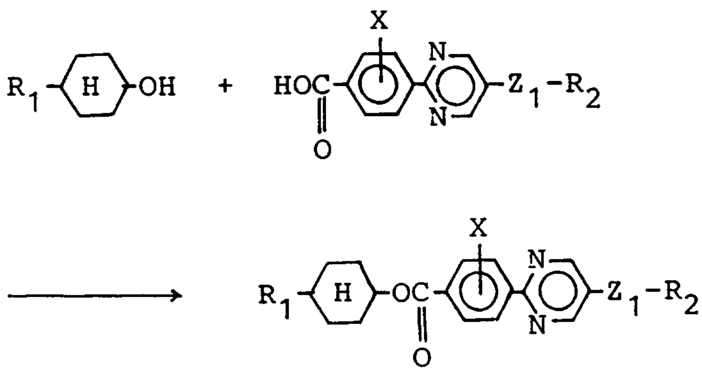

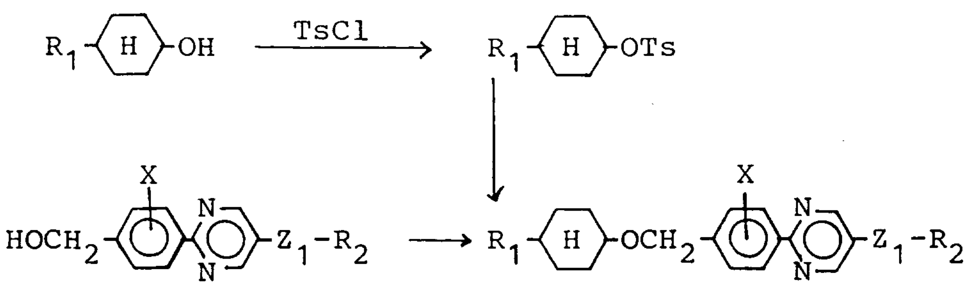


Specific examples of the mesomorphic compounds represented by the above-mentioned general formula (I) may include those shown by the following structural formulas.

1 — 1

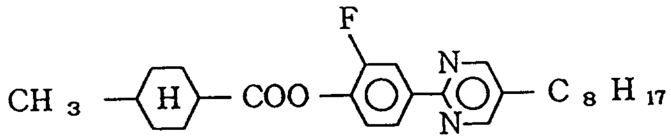

1 — 2

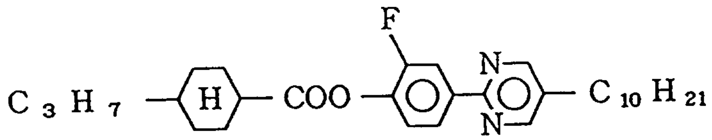

1 — 3

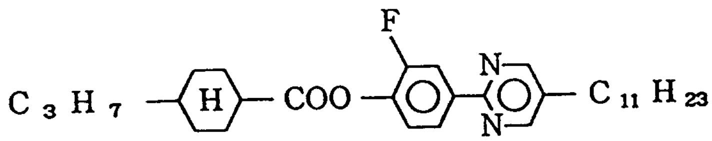

1 — 4

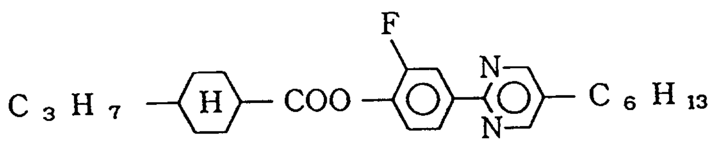

1 - 5

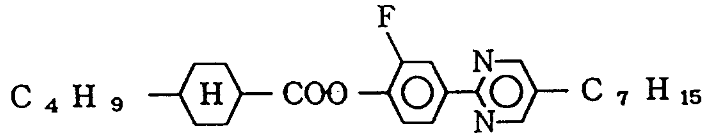

1 − 6

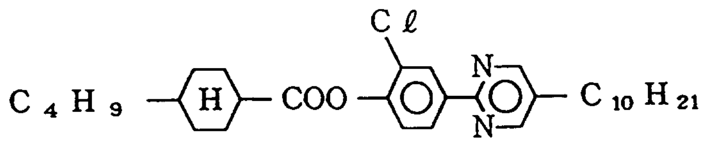

1 − 7

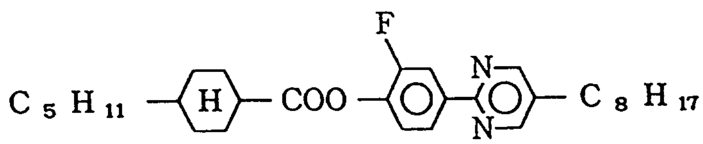

1 − 8

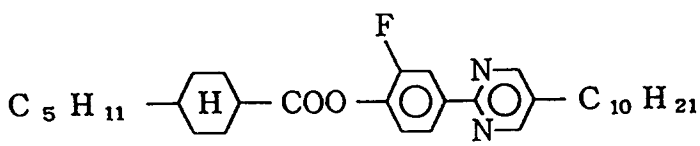

1 − 9

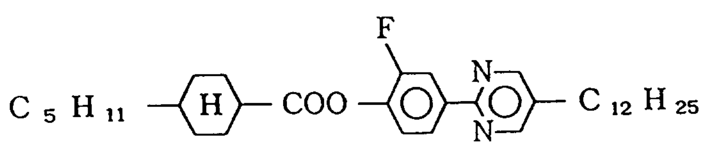

1 − 10

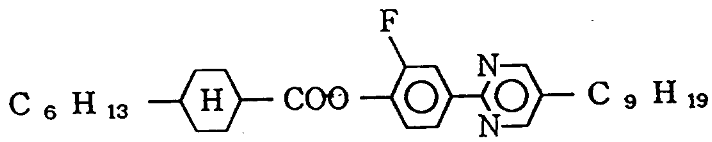

1 − 11

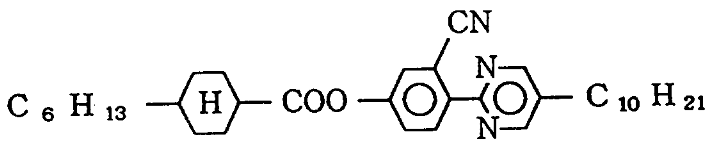

1 − 12

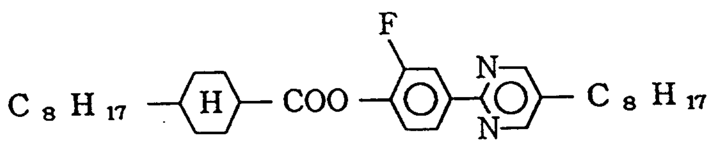

1 − 13

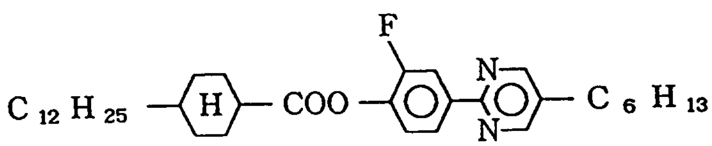

1 − 14

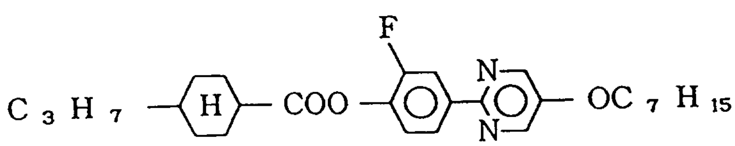

1－15

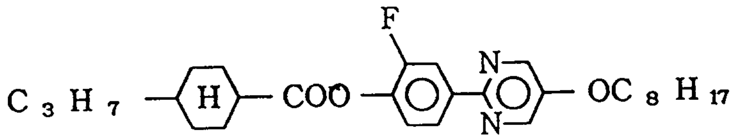

1－16

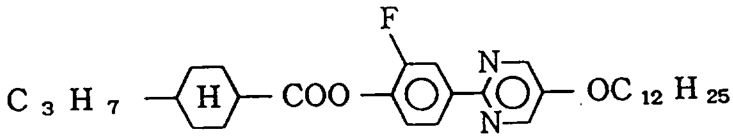

1－17

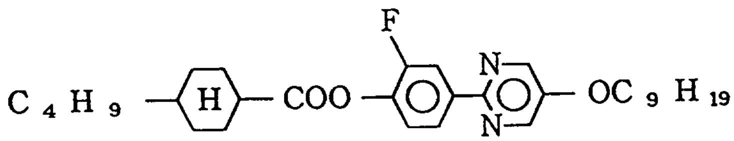

1－18

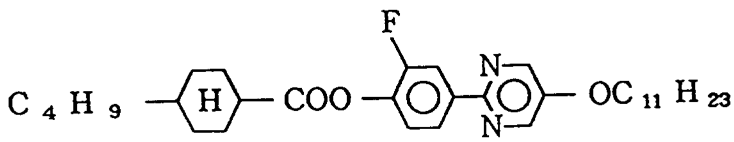

1－19

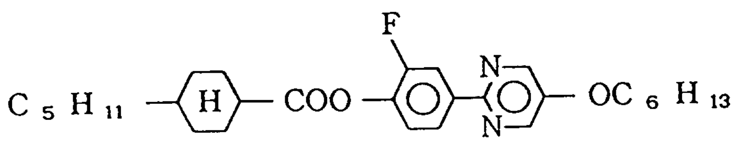

1 − 20

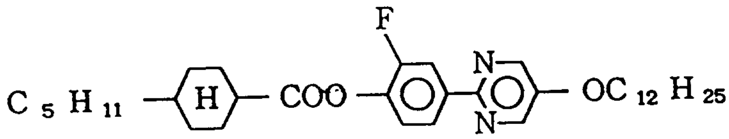

1 − 21

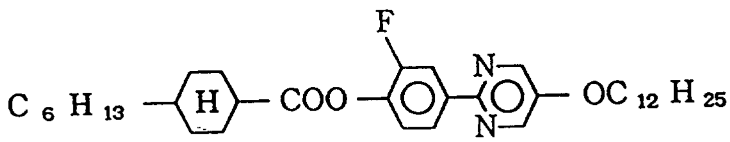

1 − 22

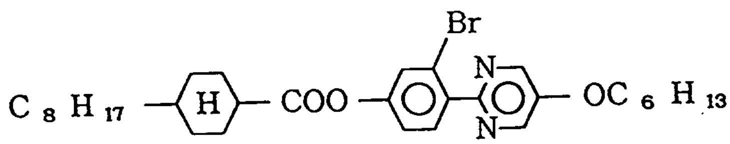

1 − 23

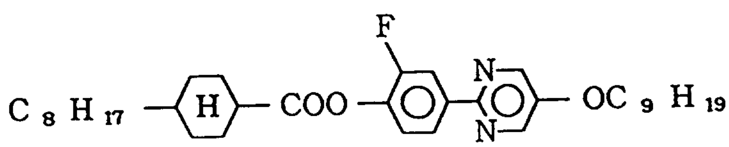

1 − 24

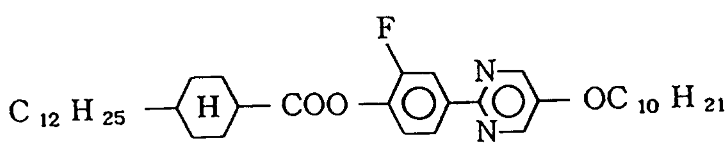

1－25

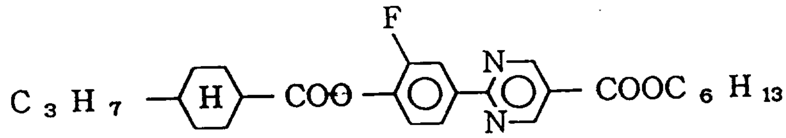

1－26

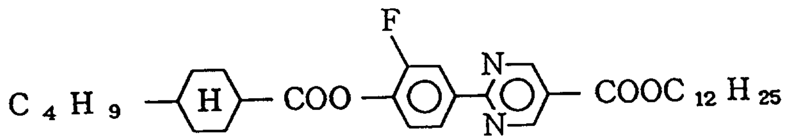

1－27

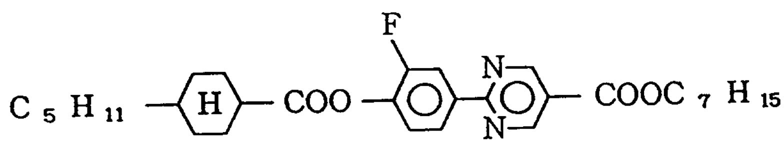

1－28

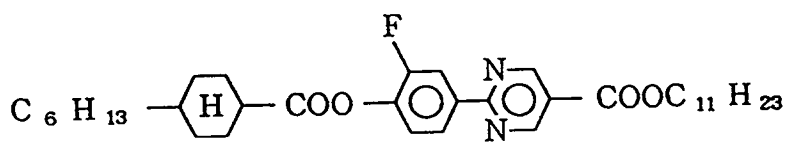

1－29

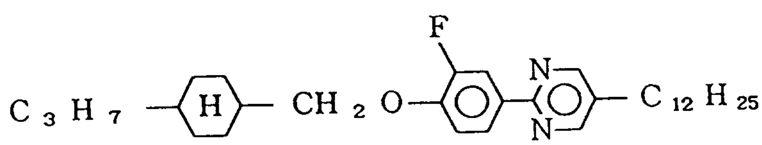

1－30

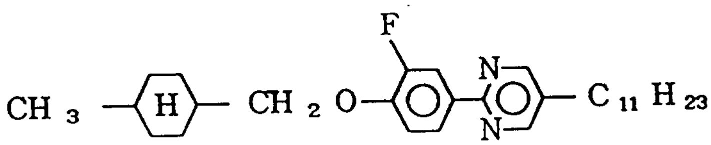

1－31

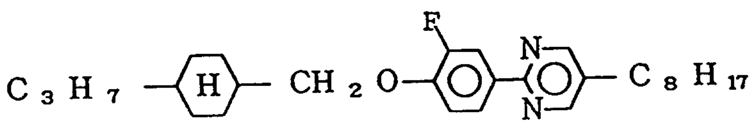

1－32

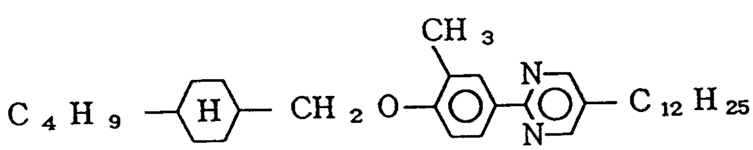

1－33

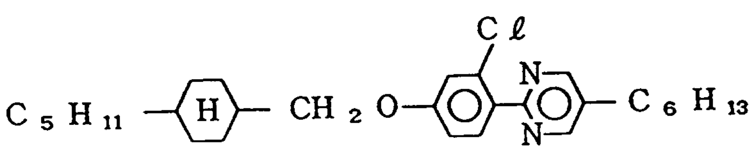

1－34

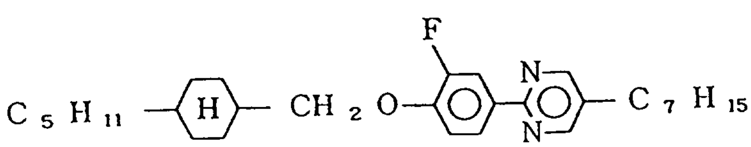

1 − 35

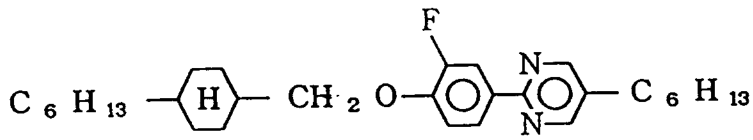

1 − 36

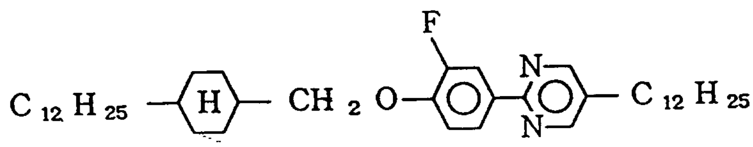

1 − 37

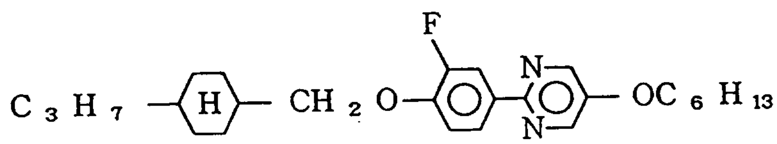

1 − 38

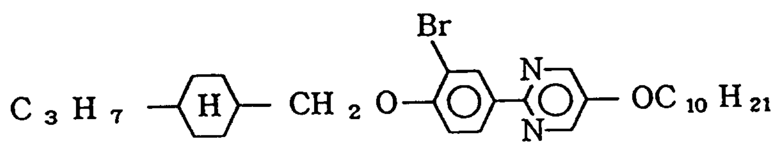

1 − 39

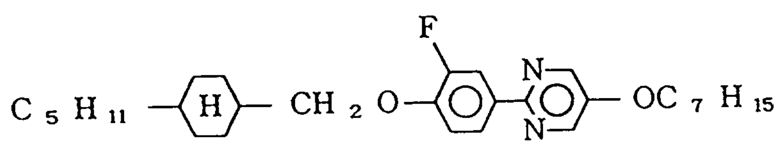

1 − 40

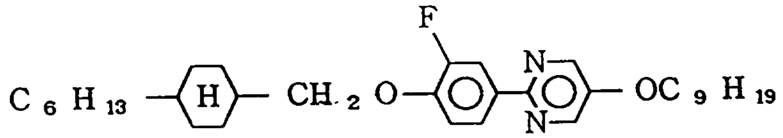

1 − 41

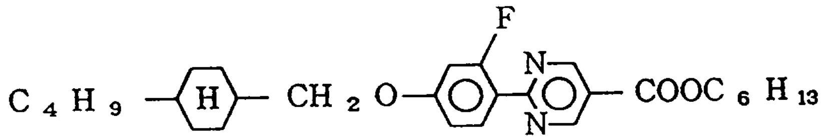

1 − 42

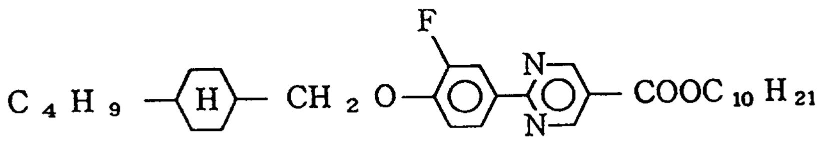

1 − 43

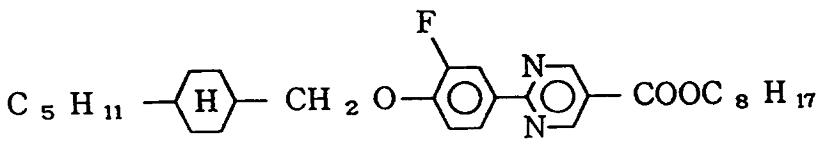

1 − 44

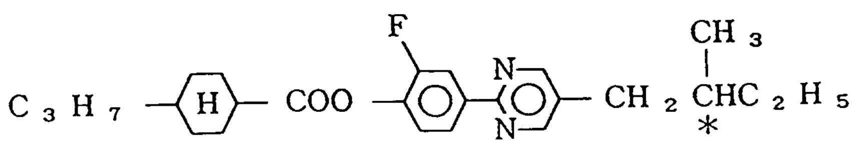

1－45

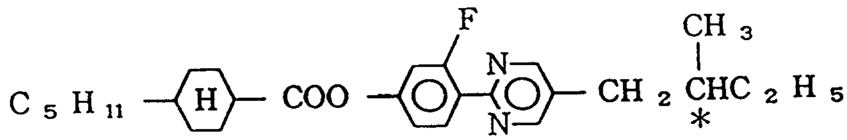

1－46

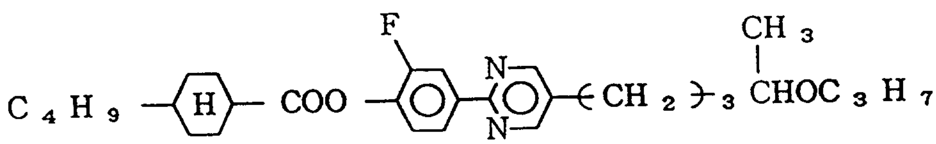

1－47

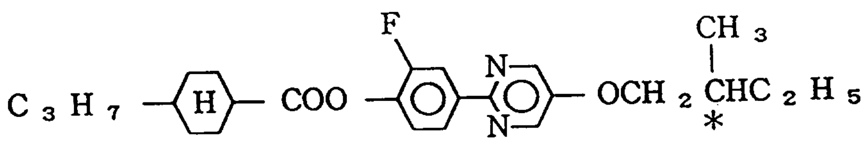

1－48

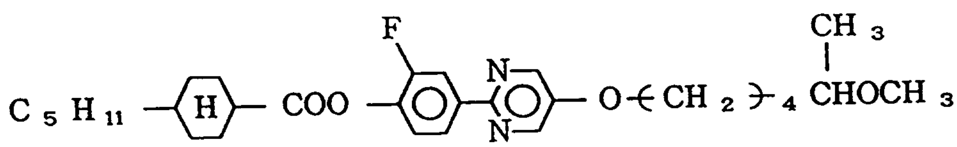

1－49

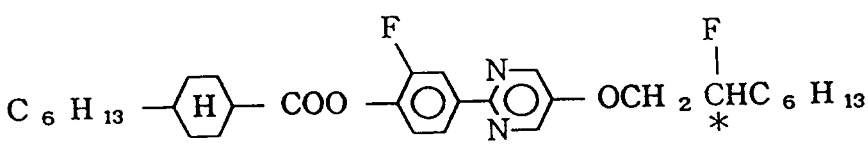

1 − 5 0

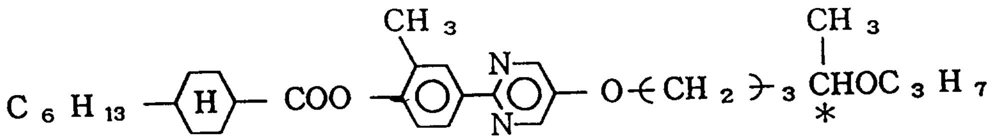

1 − 5 1

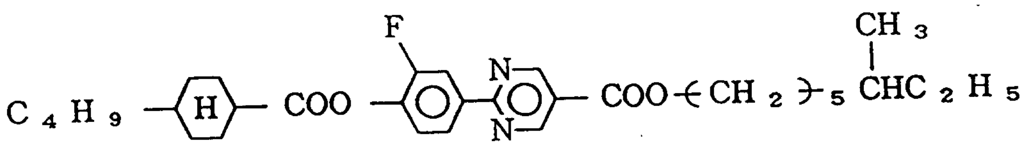

1 − 5 2

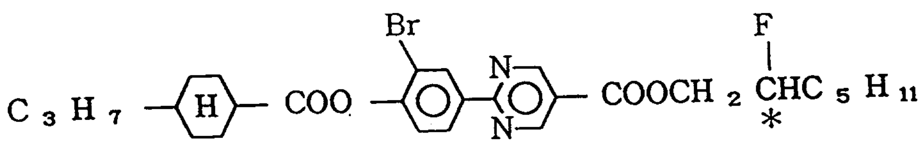

1 − 5 3

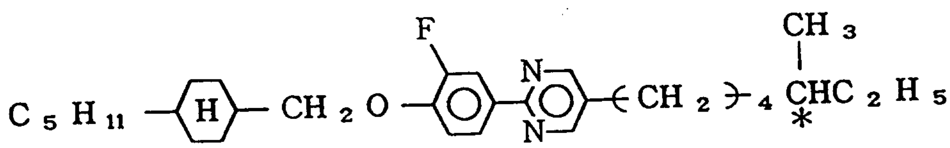

1 − 5 4

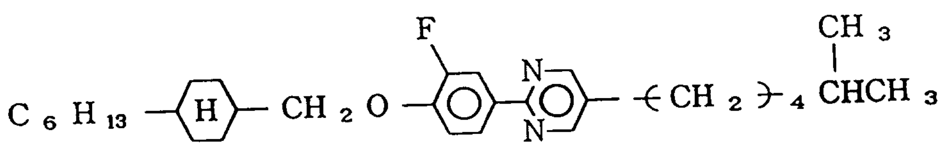

1－55

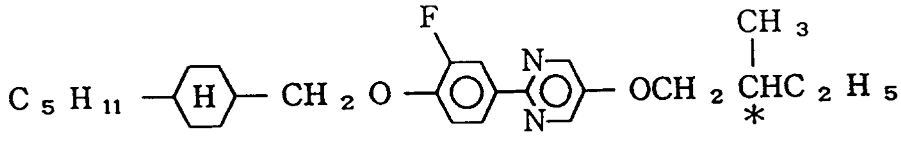

1－56

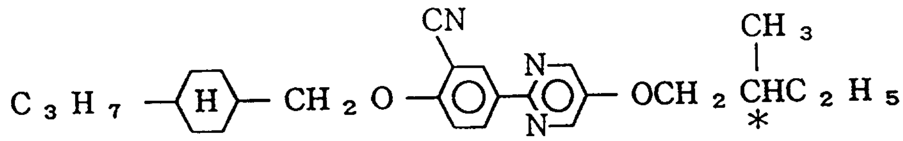

1－57

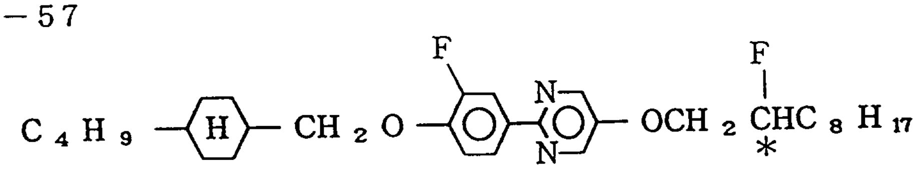

1－58

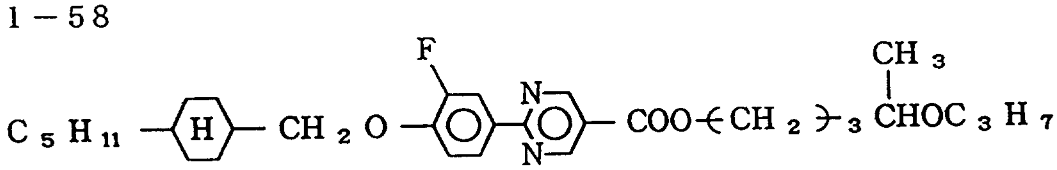

1－59

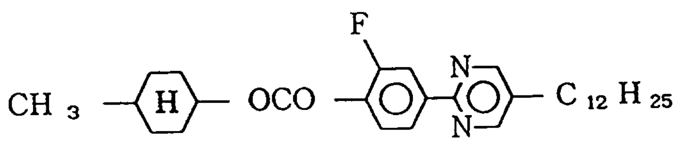

1 − 60

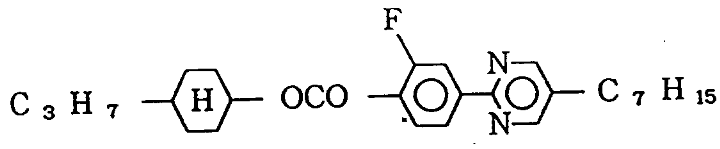

1 − 61

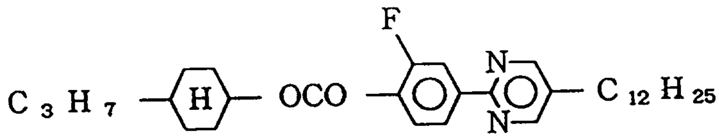

1 − 62

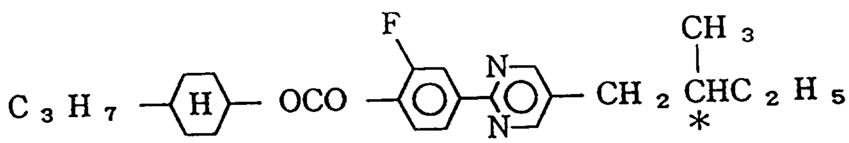

1 − 63

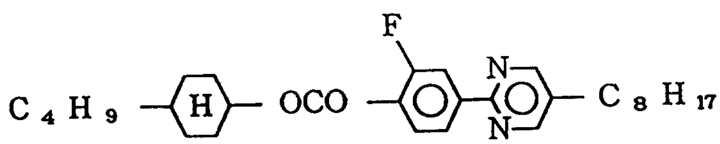

1 − 64

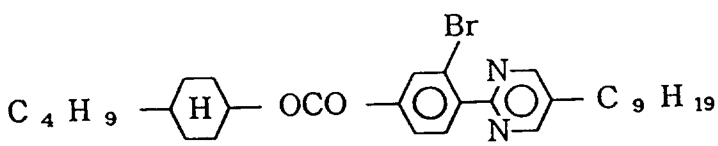

1 − 65

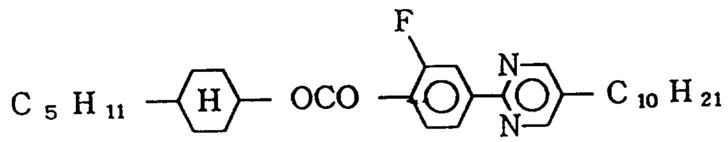

1 − 66

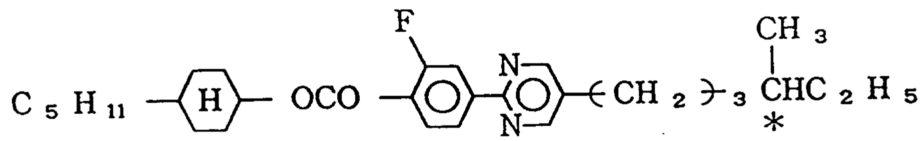

1 − 67

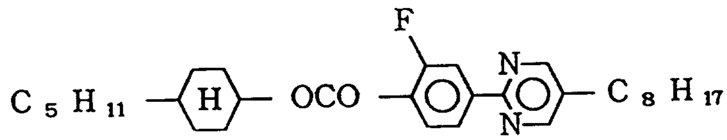

1 − 68

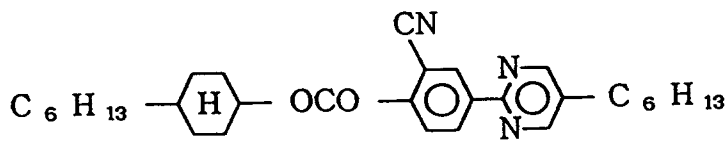

1 − 69

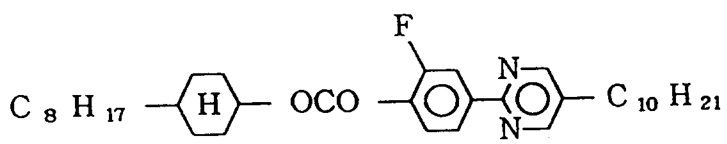

1－70

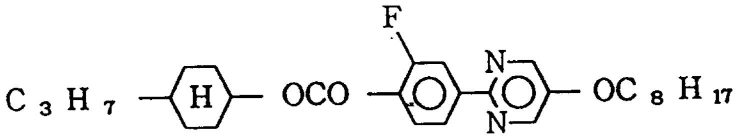

1－71

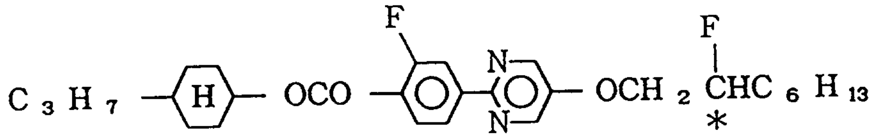

1－72

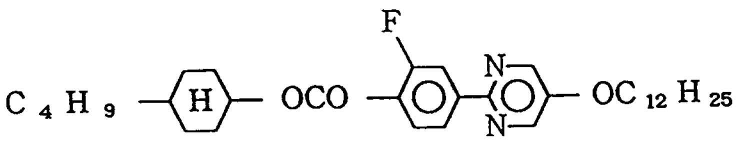

1－73

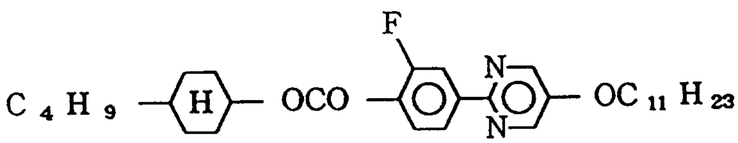

1－74

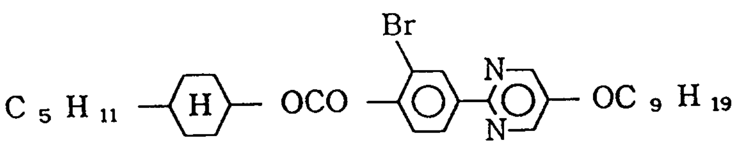

1 − 75

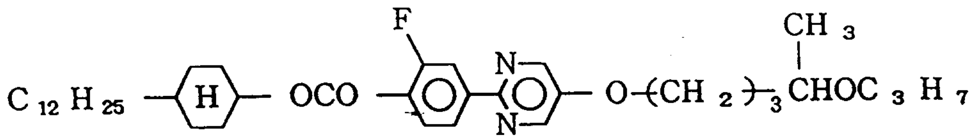

1 − 76

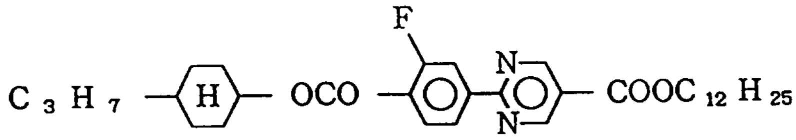

1 − 77

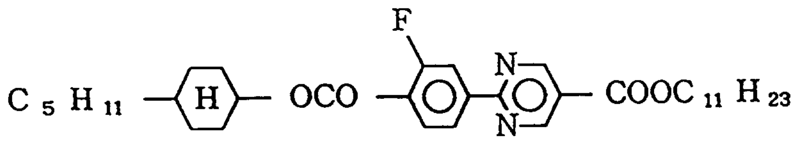

1 − 78

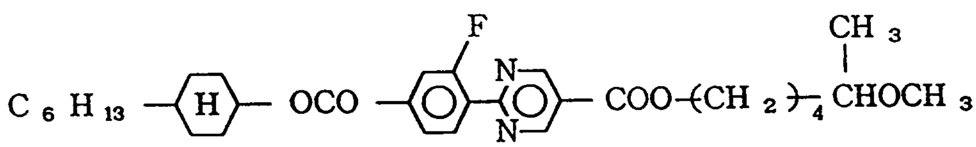

1 − 79

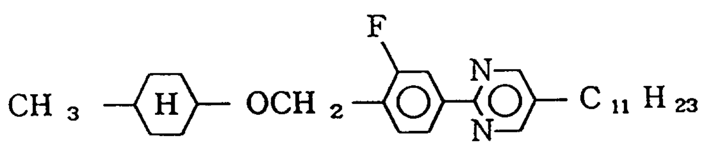

1－80

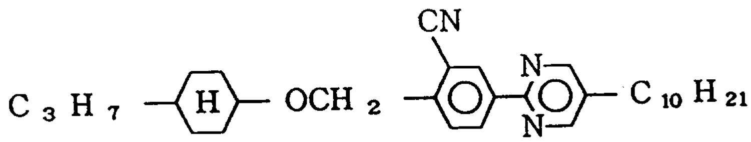

1－81

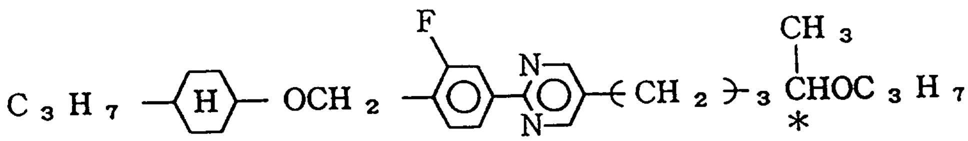

1－82

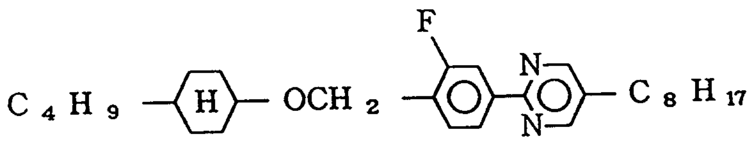

1－83

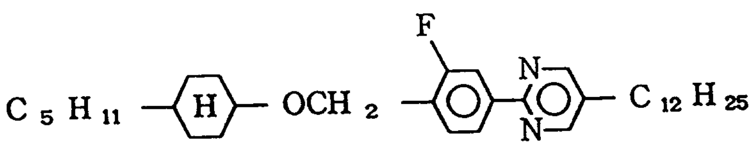

1－84

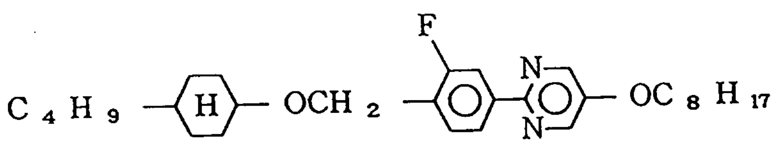

1 − 85

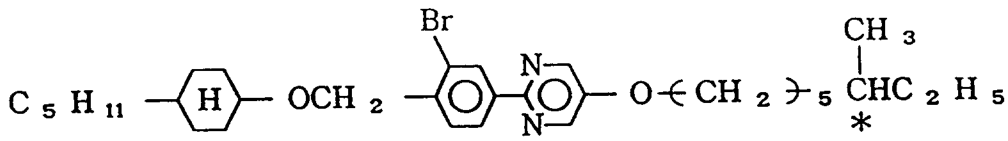

1 − 86

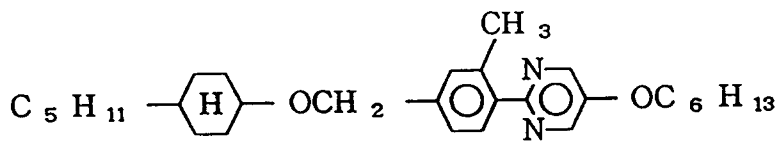

1 − 87

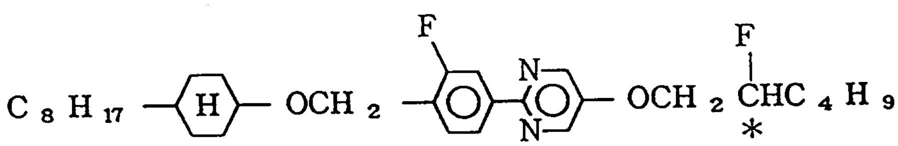

1 − 88

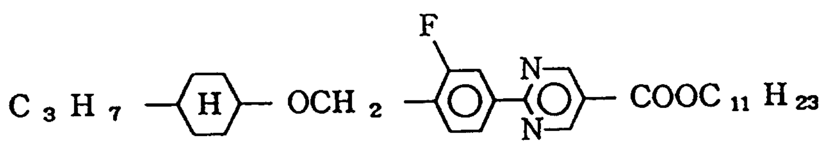

1 − 89

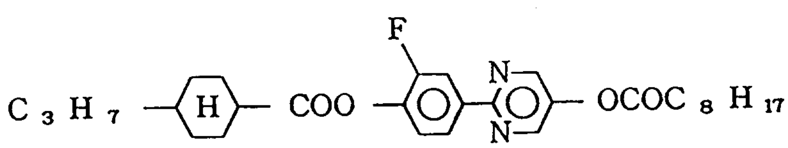

1 − 90

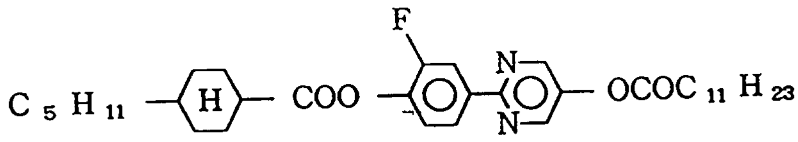

1 − 91

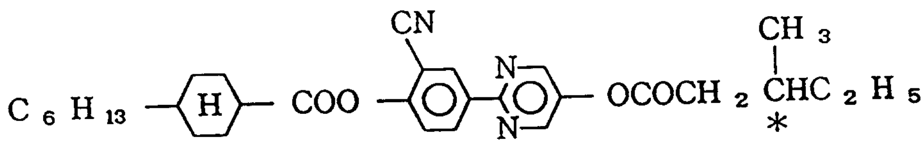

1 − 92

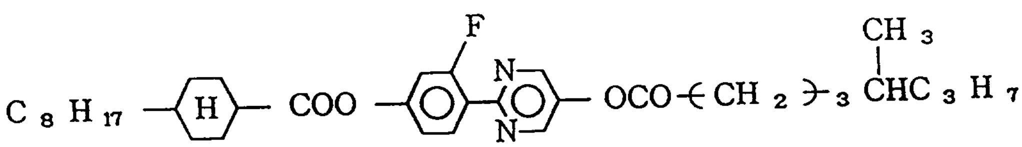

1 − 93

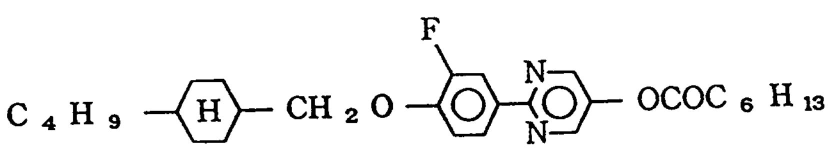

1 − 94

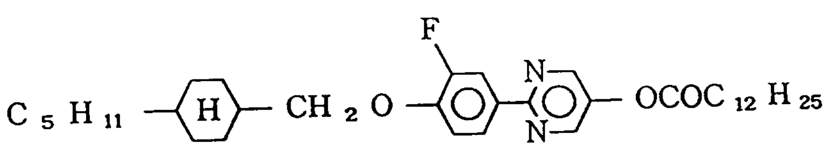

1－95

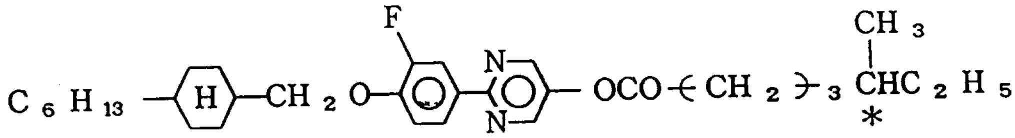

1－96

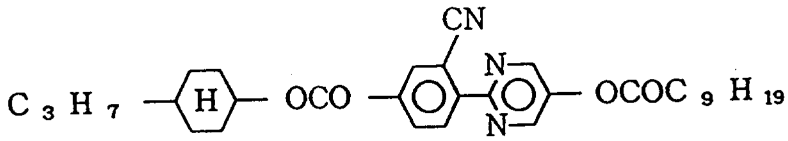

1－97

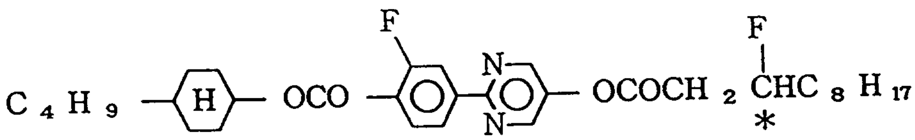

1－98

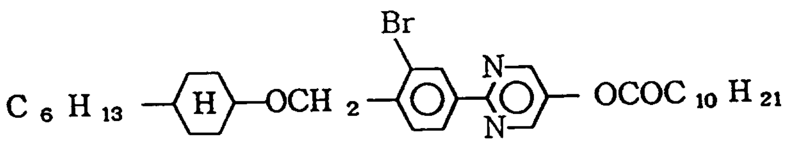

1－99

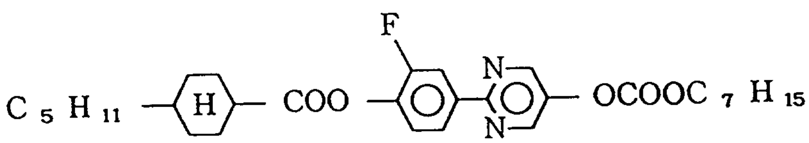

1－100

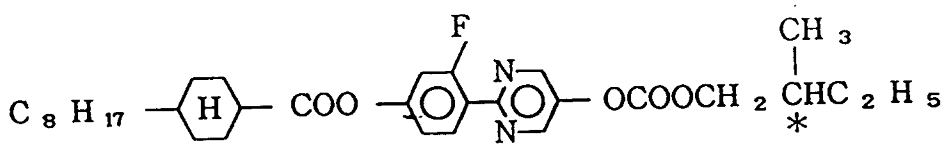

1－101

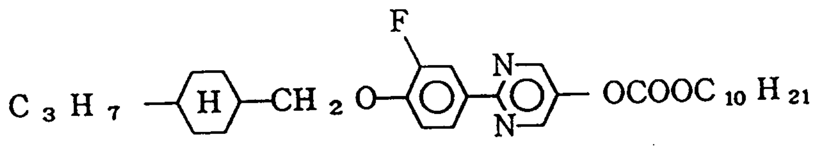

1－102

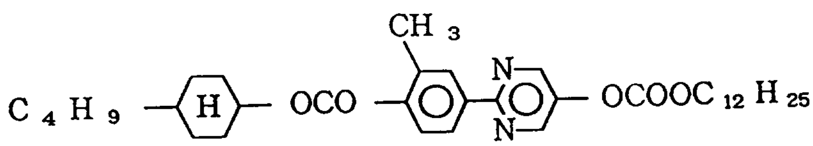

1－103

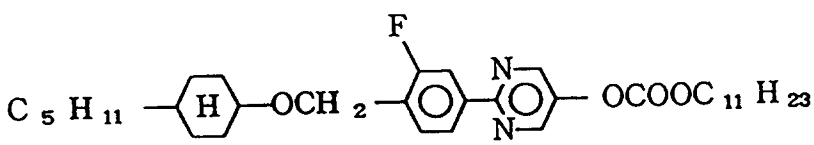

1－104

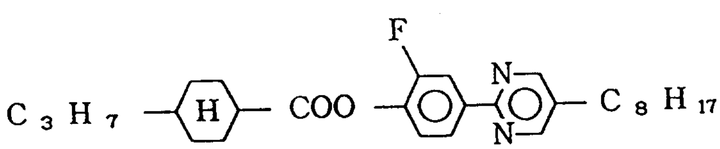

1－105

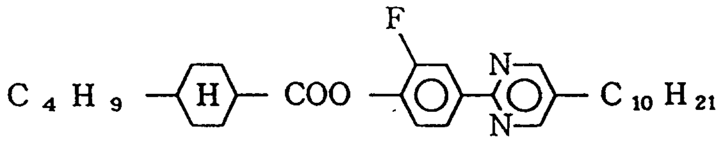

1－106

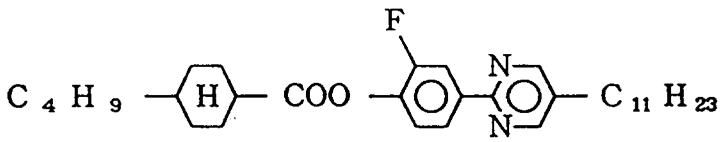

1－107

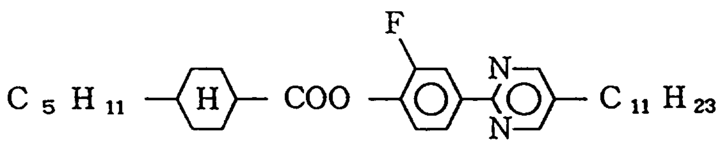

1－108

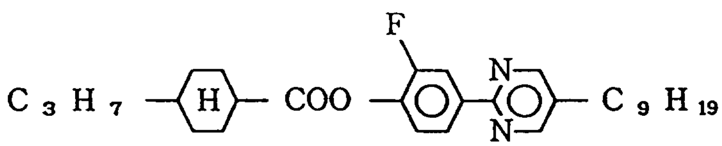

1－109

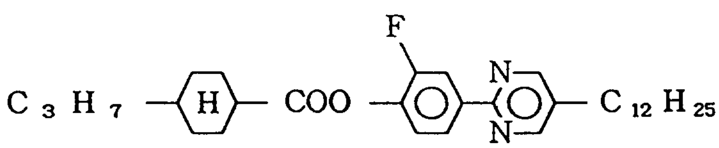

1 − 1 1 0

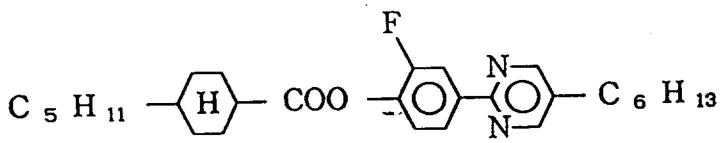

1 − 1 1 1

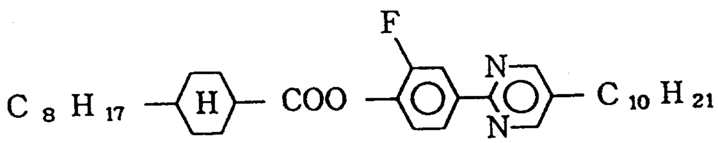

1 − 1 1 2

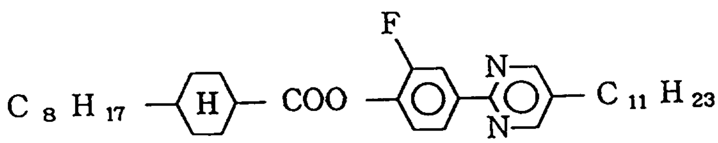

1 − 1 1 3

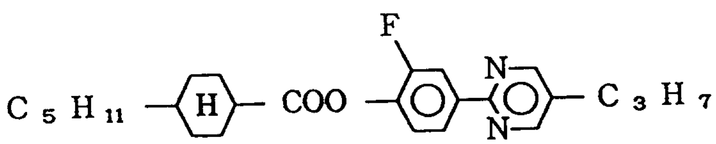

1 − 1 1 4

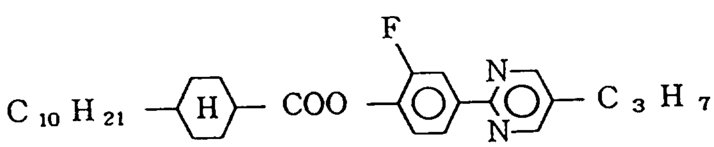

1 − 115

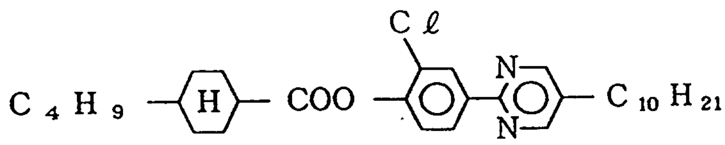

1 − 116

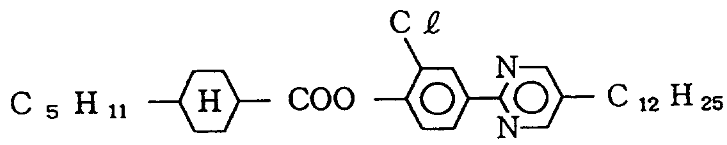

The compounds represented by the formula (II) may be synthesized through processes as disclosed by, e.g., East German Patent 95892 (1973) and Japanese Patent Publication (KOKOKU) 5434/1987. For example, the compound represented by the following formula:

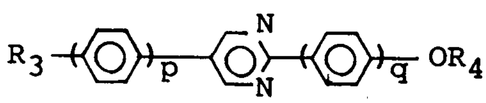

may be synthesized through the following reaction scheme.

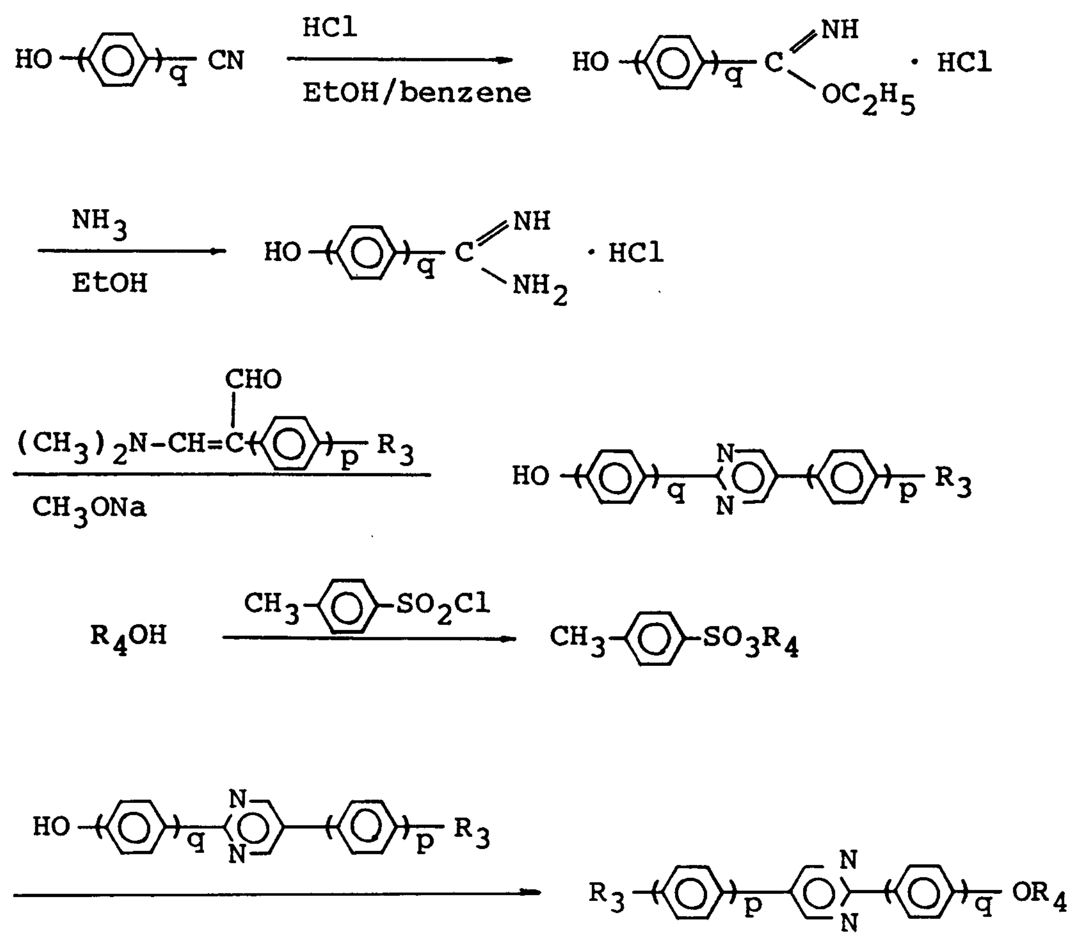


In the above, $R_3$, $R_4$, p and q are the same as defined above.

A representative example of synthesis of a compound represented by the formula (II) is described below.

Synthesis Example 1

(Synthesis of compound Example No. 2-54 shown below)

A solution of 1.83 g (9.6 mmol) of p-toluenesulfonic acid chloride in 5 ml of pyridine was added dropwise to a solution of 1.06 g (8.0 mmol) of 5-methoxyhexanol in 5 ml of pyridine below 5 °C on an iced water bath. After stirring for 6 hours at room temperature, the reaction mixture was injected into 100 ml of cold water and, after being acidified with 6N-hydrochloric acid, was extracted with isopropyl ether. The organic layer was washed with water and dried with anhydrous magnesium sulfate, followed by distilling-off of the solvent to obtain 5-methoxyhexyl-p-toluenesulfonate.

Separately, 2.0 g (6.41 mmol) of 5-decyl-2-(p-hydroxyphenyl)pyrimidine and 0.61 g of potassium hydroxide were added to 10 ml of dimethylformamide, and the mixture was stirred for 40 min. at 100 °C. To the mixture was added the above-prepared 5-methoxyhexyl-p-toluenesulfonate followed by 4 hours of stirring under heating at 100 °C. After the reaction, the reaction mixture was poured into 100 ml of cold water and extracted with benzene, followed by washing with water, drying with anhydrous magnesium sulfate and distilling-off of the solvent, to obtain a pale yellow oily product. The product was purified by column chromatography (silica gel - ethyl acetate/benzene = 1/9) and recrystallized from hexane to obtain 1.35 g of 5-decyl-2-[4-(5'-methoxyhexyloxy)phenyl]pyrimidine.

<u>Phase transition temperature (°C)</u>

$$\text{Cryst.} \underset{0.2}{\overset{3.5}{\rightleftarrows}} \text{SmC} \underset{26.7}{\overset{27.9}{\rightleftarrows}} \text{SmA} \underset{37.6}{\overset{40.3}{\rightleftarrows}} \text{Iso.}$$

Cryst.: crystal phase,
SmC: smectic C phase,
SmA: smectic A phase, and
Iso.: isotropic phase.

Specific examples of the mesomorphic compounds represented by the above-mentioned general formula (II) may include those shown by the following structural formulas.

(2 − 1)

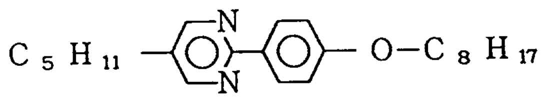

(2 − 2)

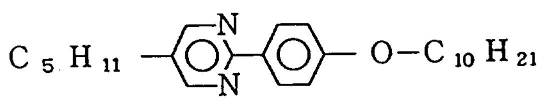

(2 − 3)

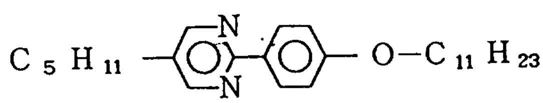

(2 − 4)

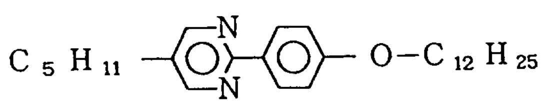

(2－5)

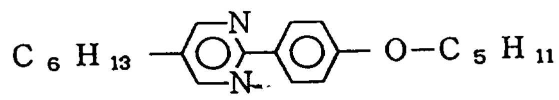

(2－6)

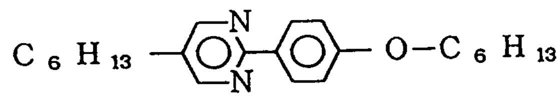

(2－7)

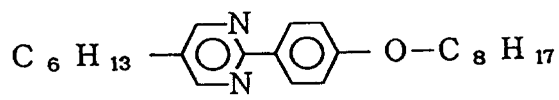

(2－8)

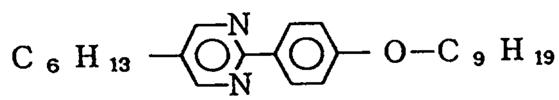

(2－9)

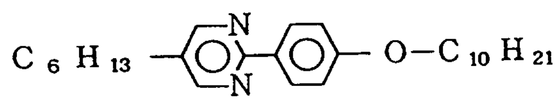

(2－10)

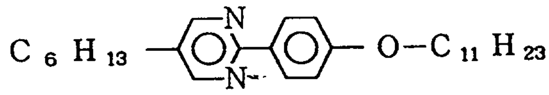

(2－11)

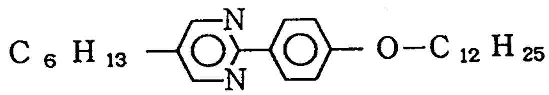

(2－12)

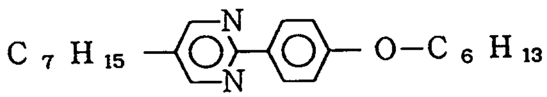

(2－13)

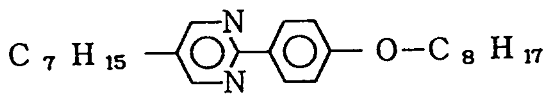

(2－14)

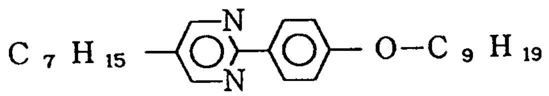

(2 − 15)

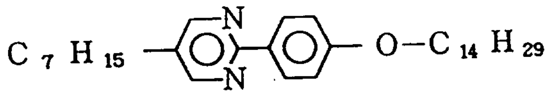

(2 − 16)

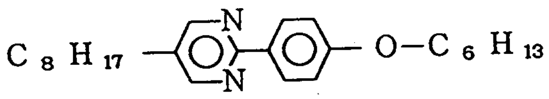

(2 − 17)

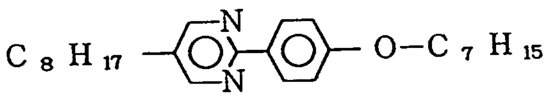

(2 − 18)

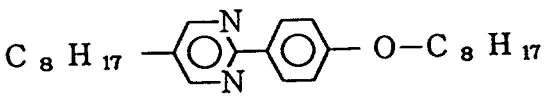

(2 − 19)

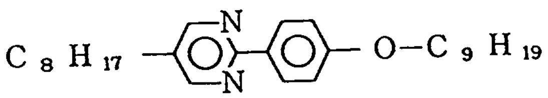

(2－20)

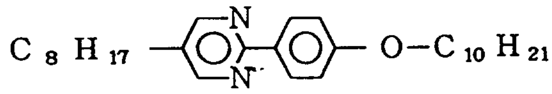

(2－21)

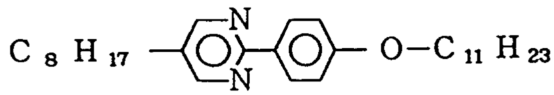

(2－22)

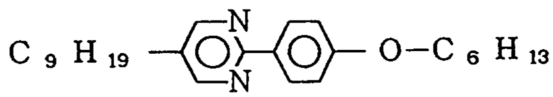

(2－23)

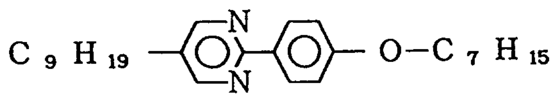

(2－24)

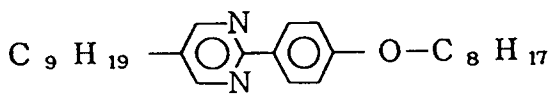

(2 − 25)

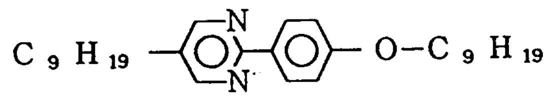

(2 − 26)

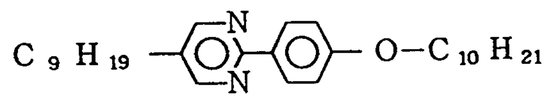

(2 − 27)

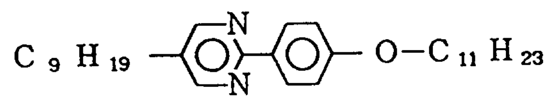

(2 − 28)

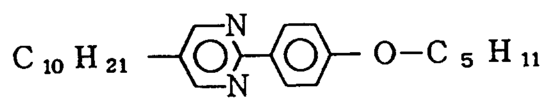

(2 − 29)

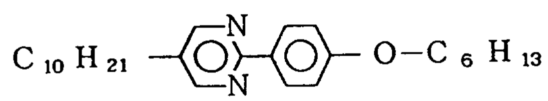

(2－30)

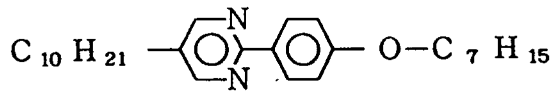

(2－31)

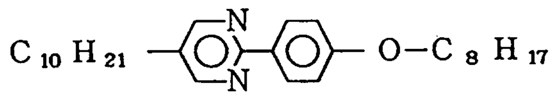

(2－32)

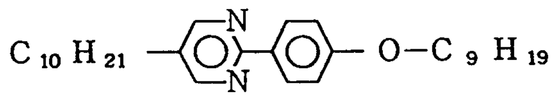

(2－33)

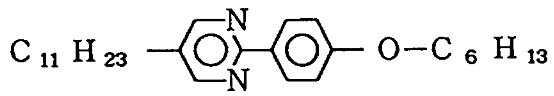

(2－34)

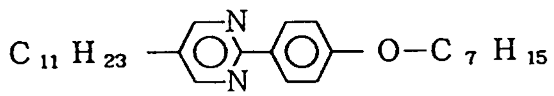

(2－35)

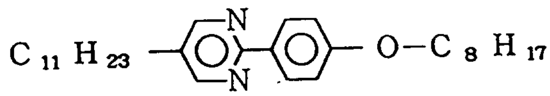

(2－36)

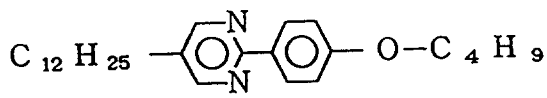

(2－37)

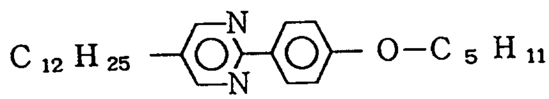

(2－38)

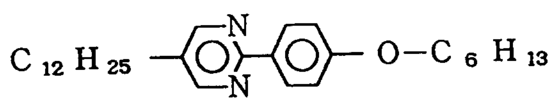

(2－39)

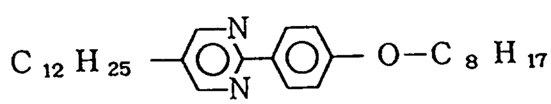

(2−40)

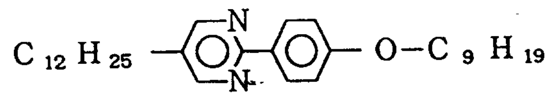

(2−41)

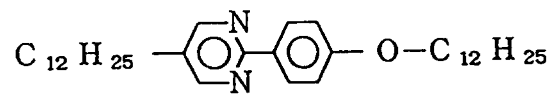

(2−42)

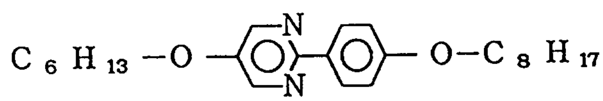

(2−43)

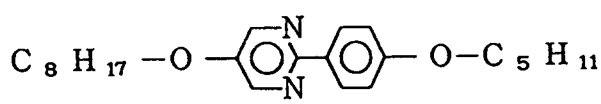

(2−44)

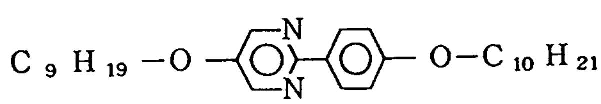

(2 − 45)

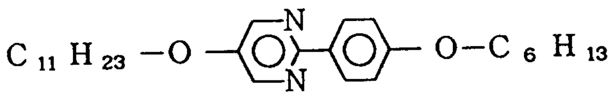

(2 − 46)

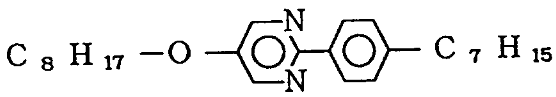

(2 − 47)

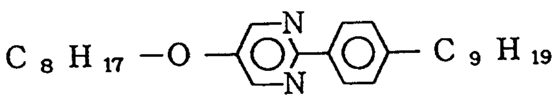

(2 − 48)

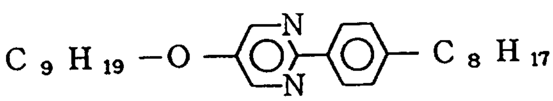

(2 − 49)

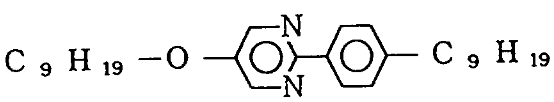

(2－50)

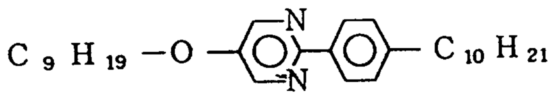

(2－51)

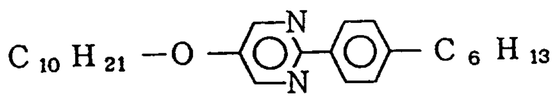

(2－52)

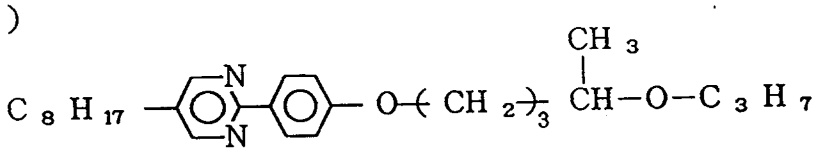

(2－53)

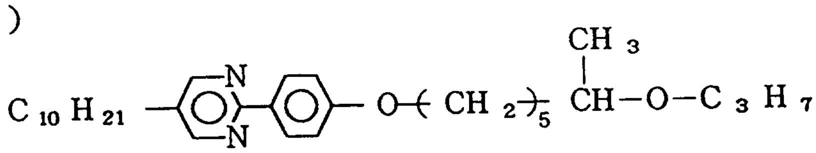

(2－54)

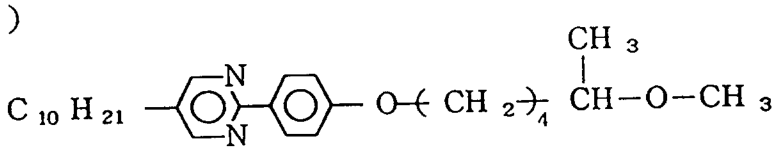

(2－55)

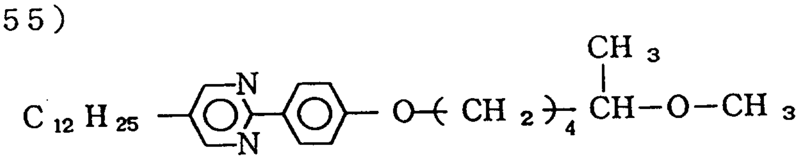

(2－56)

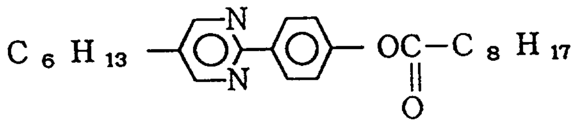

(2－57)

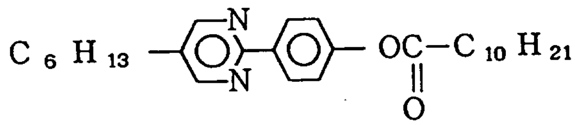

(2－58)

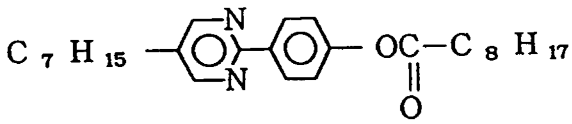

(2－59)

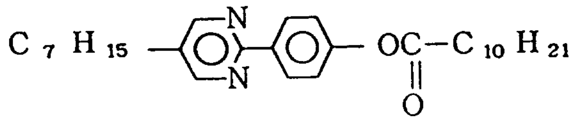

(2－60)

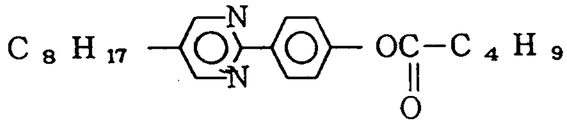

(2－61)

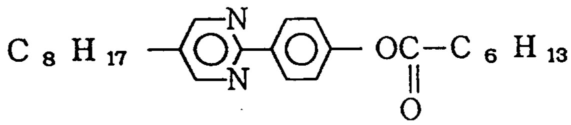

(2－62)

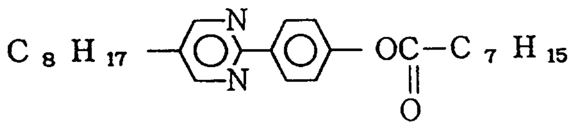

(2－63)

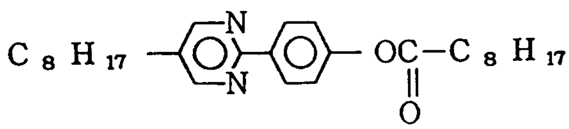

(2－64)

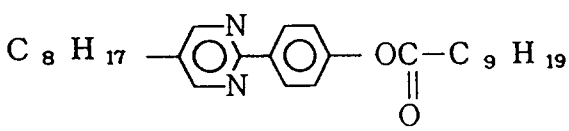

(2－65)

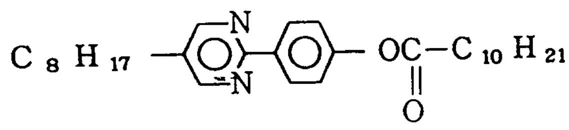

(2－65)

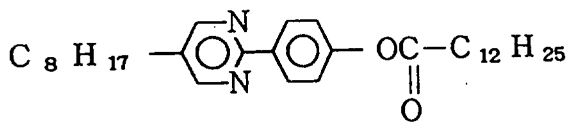

(2－66)

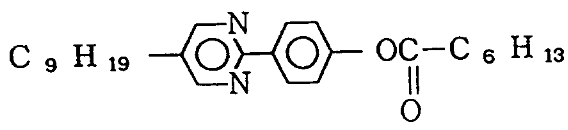

(2－67)

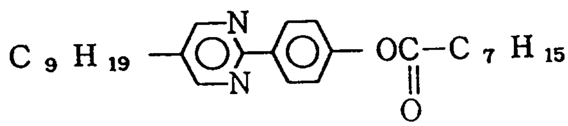

(2－68)

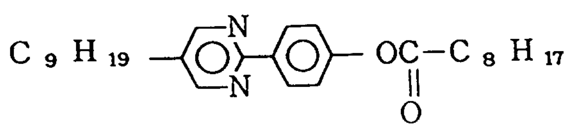

(2－69)

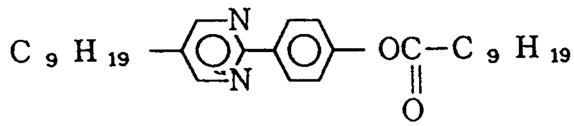

(2－70)

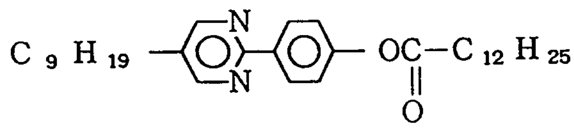

(2－71)

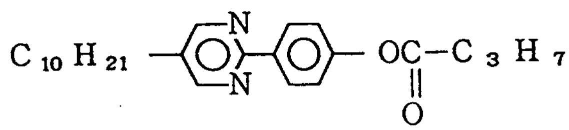

(2－72)

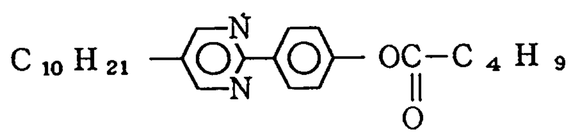

(2－73)

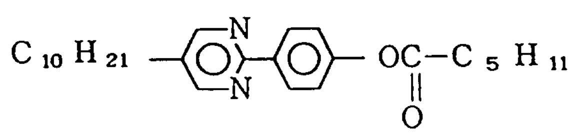

(2 − 74)

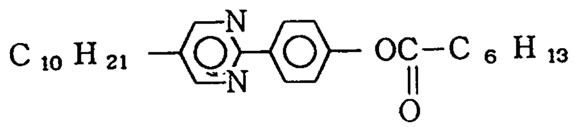

(2 − 75)

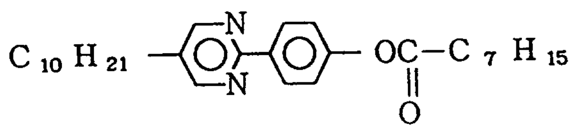

(2 − 76)

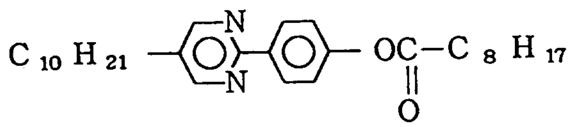

(2 − 77)

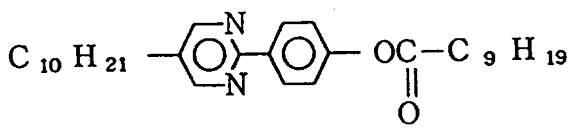

(2 − 78)

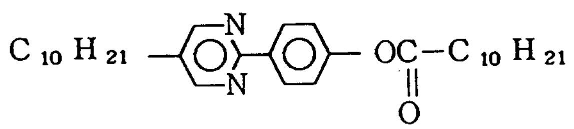

(2－79)

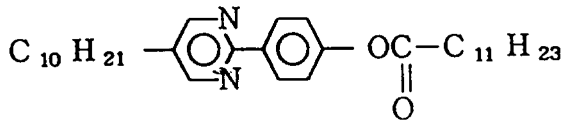

(2－80)

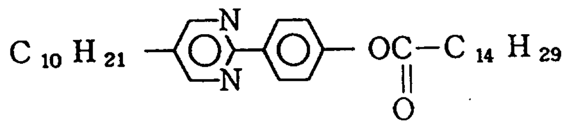

(2－81)

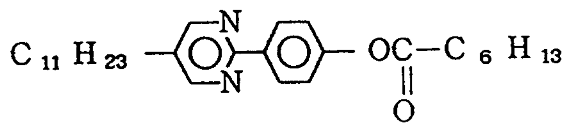

(2－82)

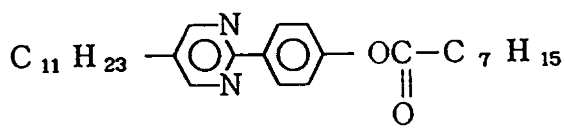

(2－83)

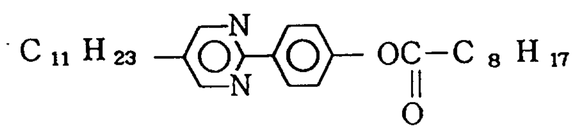

(2－84)

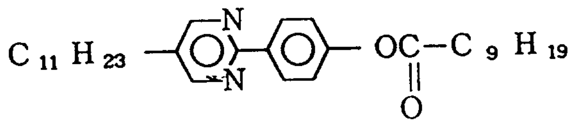

(2－85)

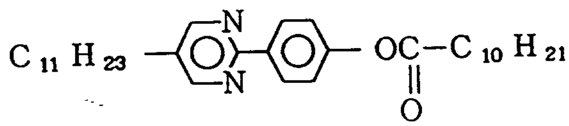

(2－86)

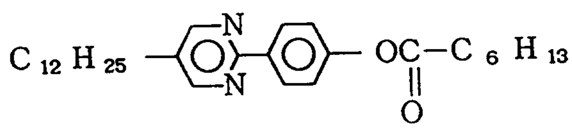

(2－87)

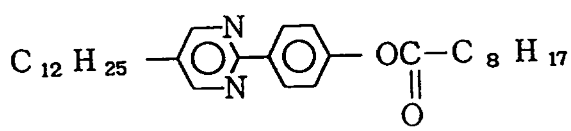

(2－88)

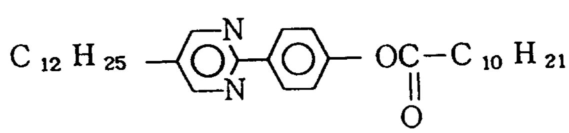

(2 − 89)

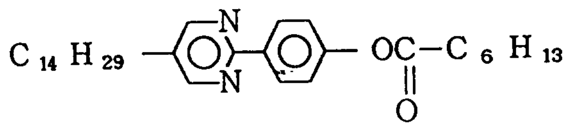

(2 − 90)

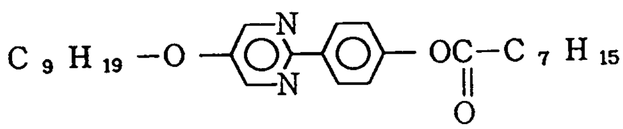

(2 − 91)

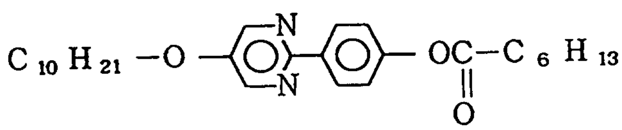

(2 − 92)

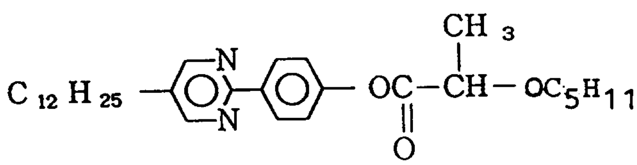

(2 − 93)

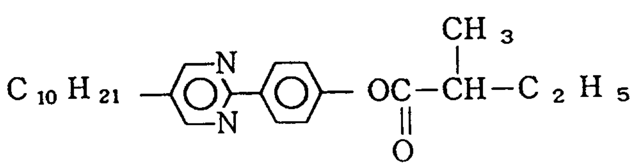

(2－94)

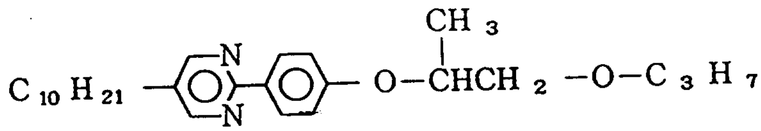

(2－95)

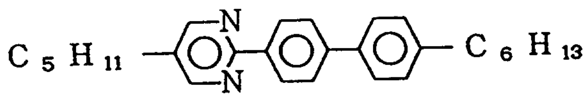

(2－96)

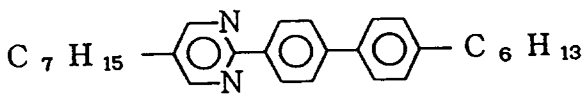

(2－97)

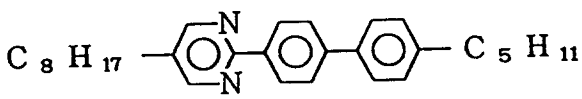

(2－98)

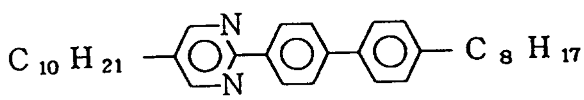

(2－99)

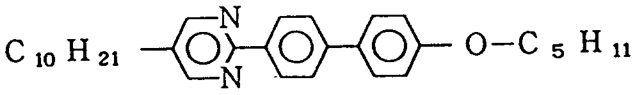

(2－100)

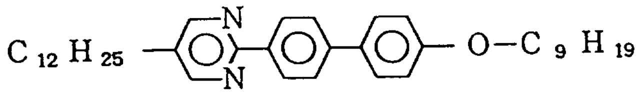

(2－101)

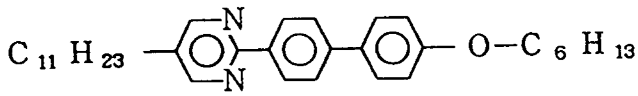

(2－102)

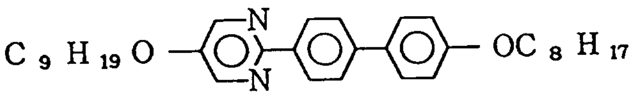

(2－103)

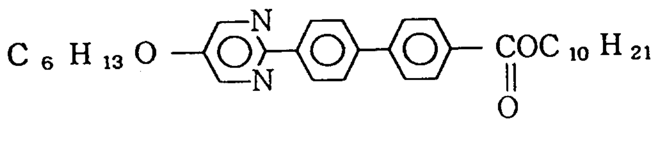

(2 − 104)

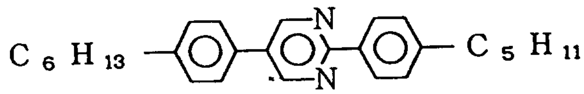

(2 − 105)

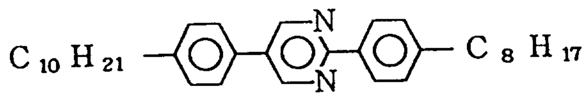

(2 − 106)

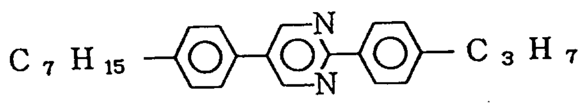

(2 − 107)

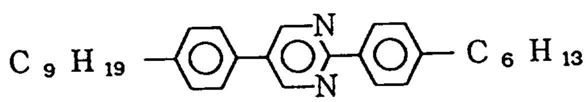

(2 − 108)

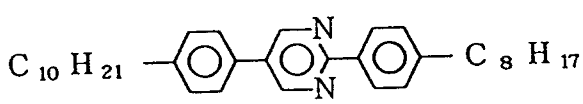

(2－109)

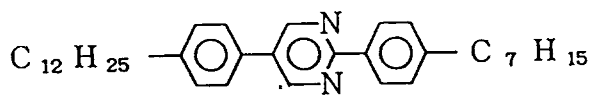

(2－110)

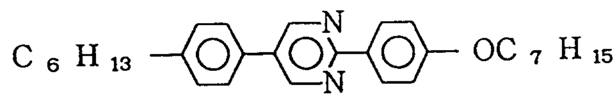

(2－111)

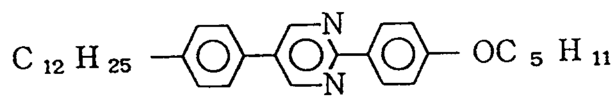

(2－112)

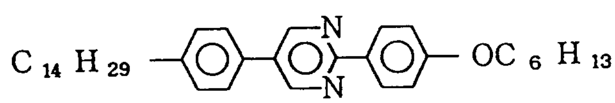

(2－113)

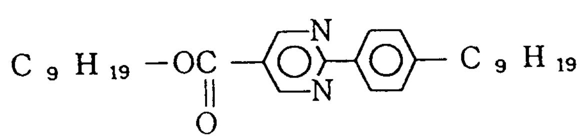

(2-114)

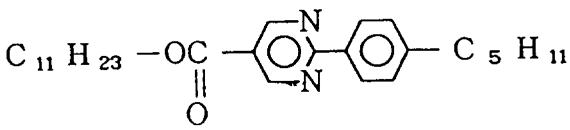

(2-115)

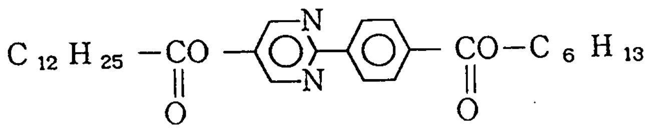

(2-116)

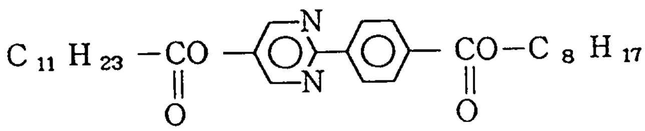

(2-117)

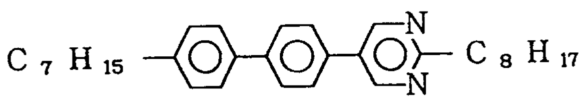

(2-118)

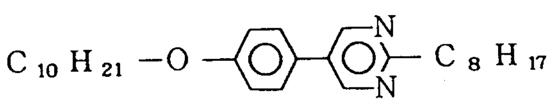

(2-119)

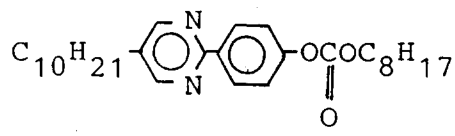

(2-120)

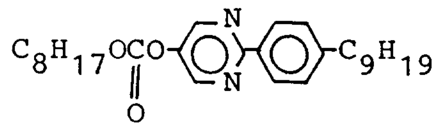

The compounds represented by the formula (III) may be synthesized through processes as disclosed by, e.g., Japanese Laid-Open Patent Applications (KOKAI) 22042/1988 and 122651/1988. Representative examples of synthesis of the compounds are shown hereinbelow.

Synthesis Example 2

(Synthesis of Compound Example 3-28 shown below)

1.00 g (4.16 mM) of p-2-fluorooctyloxyphenol was dissolved in a mixture of 10 ml of pyridine and 5 ml of toluene, and a solution of 1.30 g (6.00 mM) of trans-4-n-pentylcyclohexanecarbonyl chloride was added dropwise thereto in 20 - 40 min. at below 5 °C. After the addition, the mixture was stirred overnight at room temperature to obtain a white precipitate.

After the reaction, the reaction product was extracted with benzene, and the resultant benzene layer was washed with distilled water, followed by drying with magnesium sulfate and distilling-off of the benzene, purification by silica gel column chromatography and recrystallization from ethanol/methanol to obtain 1.20 g (2.85 mM) of trans-4-n-pentylcyclohexanecarboxylic acid-p-2-fluorooctyloxyphenyl-ester.
(Yield: 68.6 %)

NMR data (ppm)
0.83 - 2.83 ppm (34H, m)
4.00 - 4.50 ppm (2H, q)
7.11 ppm (4H, s)
IR data ($cm^{-1}$)
3456, 2928, 2852, 1742, 1508, 1470, 1248, 1200, 1166, 1132, 854.

Phase transition temperature (°C)

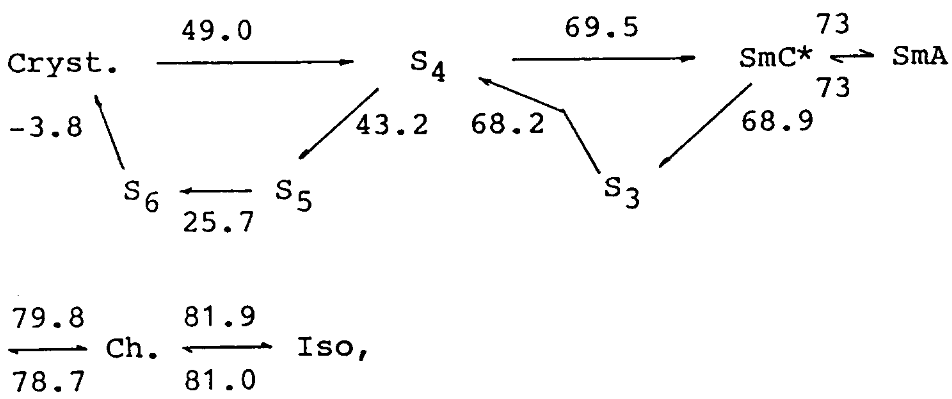


$S_3$ - $S_6$: phases of higher order than SmC*,
SmC*: chiral smectic C phase, and
Ch.: cholesteric phase.

Synthesis Example 3

(Synthesis of Compound Example 3-85)

In a vessel sufficiently replaced with nitrogen, 0.40 g (3.0 mmol) of (-)-2-fluoroheptanol and 1.00 g (13 mmol) of dry pyridine were placed and dried for 30 min. under cooling on an ice bath. Into the solution, 0.69 g (3.6 mmol) of p-toluenesulfonyl chloride was added, and the mixture was stirred for 5 hours. After the reaction, 10 ml of 1N-HCl was added, and the resultant mixture was subjected to two times of extraction with 10 ml of methylene chloride. The extract liquid was washed once with 10 ml of distilled water and dried with an appropriate amount of anhydrous sodium sulfate, followed by distilling-off of the solvent to obtain 0.59 g (2.0 mmol) of (+)-2-fluoroheptyl p-toluenesulfonate.

The yield was 66 %, and the product showed the following optical rotation and IR data.

Optical rotation:

$$[\alpha]_{D}^{26.4} \ +2.59 \text{ degrees} \quad (c = 1, CHCl_3)$$

$$[\alpha]_{435}^{23.6} \ +9.58 \text{ degrees} \quad (c = 1, CHCl_3)$$

IR ($cm^{-1}$):

2900, 2850, 1600, 1450, 1350, 1170, 1090 980, 810, 660, 550

0.43 g (1.5 mmol) of the thus obtained (+)-2-fluoroheptyl p-toluenesulfonate and 0.28 g (1.0 mmol) of 5-octyl-2-(4-hydroxyphenyl)pyrimidine were mixed with 0.2 ml of 1-butanol, followed by sufficient stirring. To the solution was quickly added a previously obtained alkaline solution of 0.048 g (1.2 mmol) of sodium hydroxide in 1.0 ml of 1-butanol, followed by 5.5 hours of heat-refluxing. After the reaction, 10 ml of distilled water was added, and the mixture was extracted respectively once with 10 ml of benzene and 5 ml of benzene, followed by drying with an appropriate amount of anhydrous sodium sulfate, distilling-off of the solvent and purification by silica gel column chromatography (chloroform) to obtain 0.17 g (0.43 mmol) of objective (+)-5-octyl-2-[4-(2-fluoroheptyloxy)phenyl]pyrimidine.

The yield was 43 %, and the product showed the following optical rotation and IR data.

Optical rotation:

$[\alpha]_{D}^{25.6}$ +0.44 degree (c = 1, $CHCl_3$)

$[\alpha]_{435}^{22.4}$ +4.19 degrees (c = 1, $CHCl_3$)

IR ($cm^{-1}$)

2900, 2850, 1600, 1580, 1420, 1250 1160, 800, 720, 650, 550.

Specific examples of the mesomorphic compounds represented by the above-mentioned general formula (III) may include those shown by the following structural formulas.

(3 − 1)

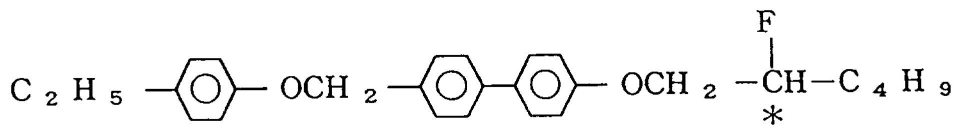

(3 − 2)

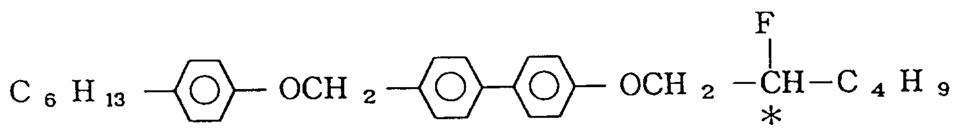

(3 − 3)

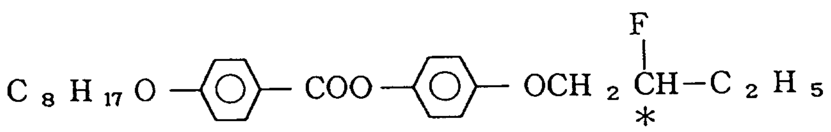

(3 − 4)

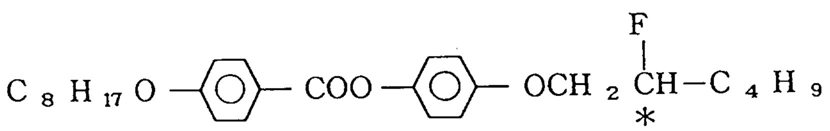

(3 − 5)

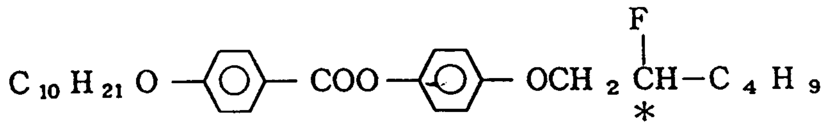

(3 − 6)

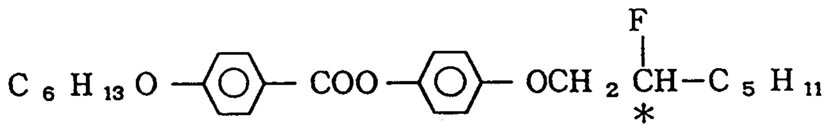

(3 − 7)

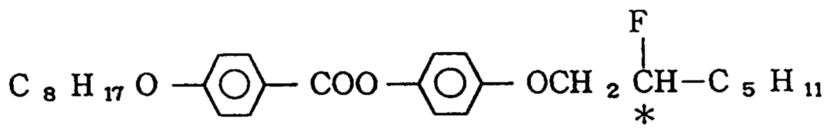

(3 − 8)

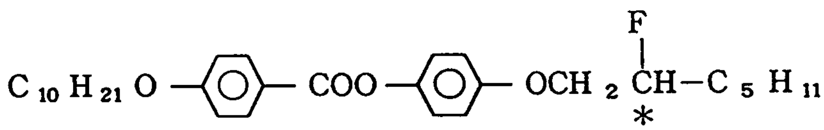

(3 − 9)

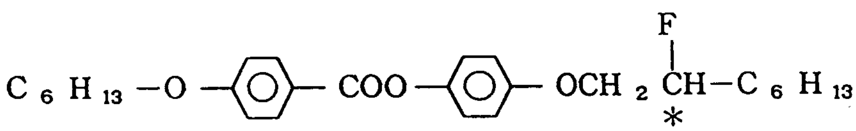

(3－10)

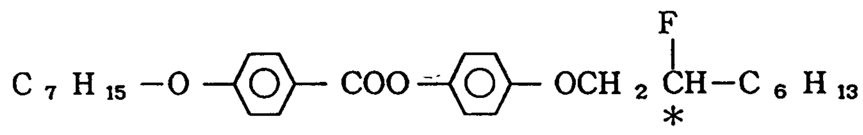

(3－11)

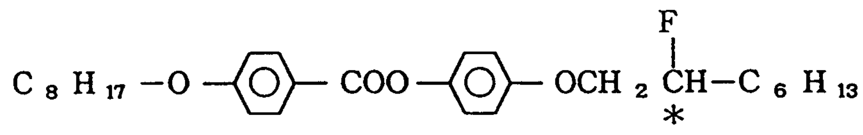

(3－12)

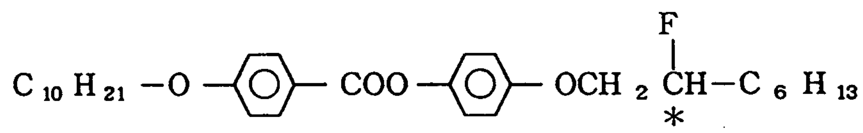

(3－13)

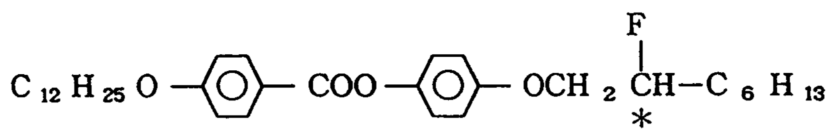

(3－14)

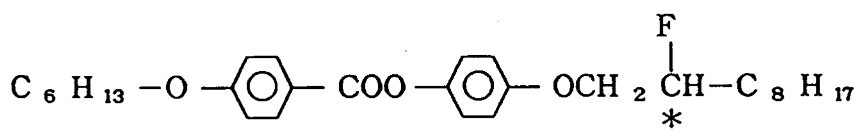

(3 − 15)

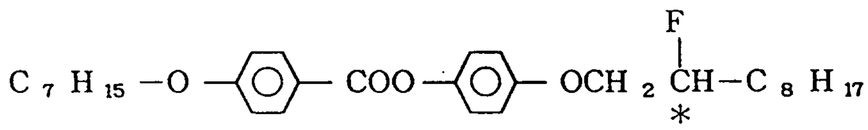

(3 − 16)

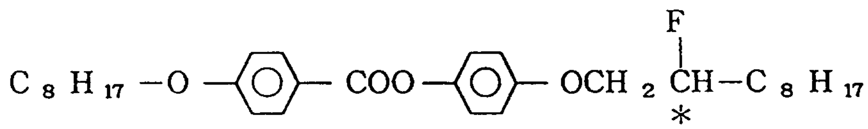

(3 − 17)

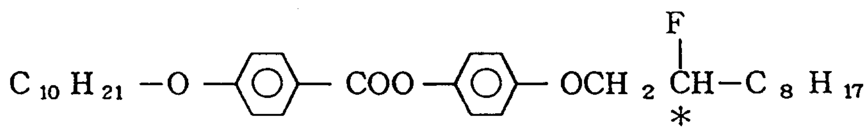

(3 − 18)

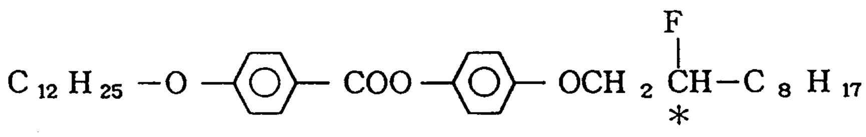

(3 − 19)

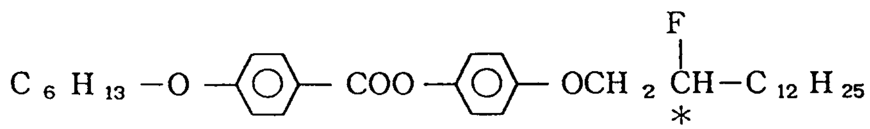

(3－20)

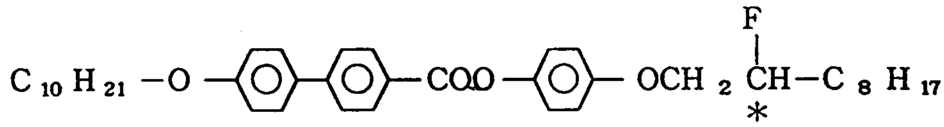

(3－21)

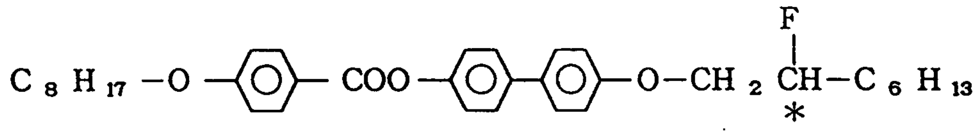

(3－22)

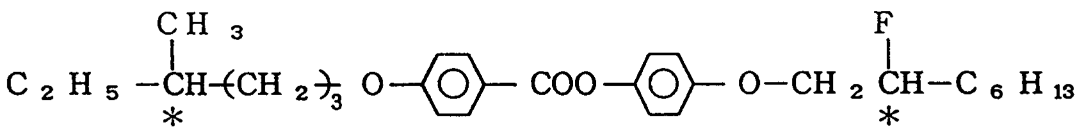

(3－23)

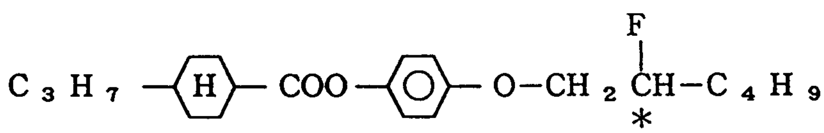

(3－24)

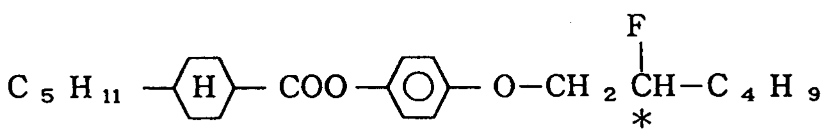

(3－25)

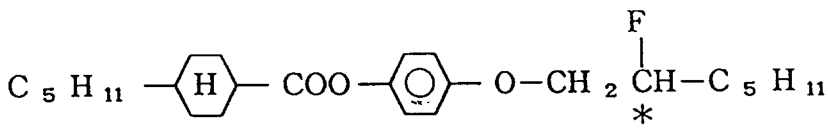

(3－26)

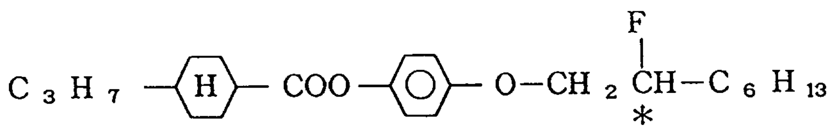

(3－27)

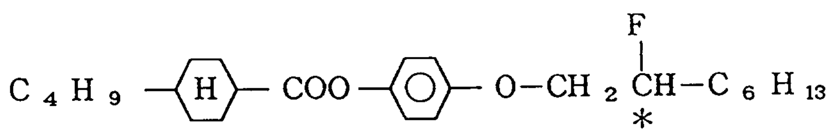

(3－28)

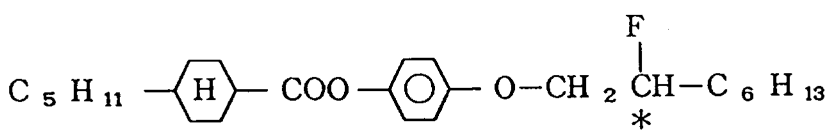

(3－29)

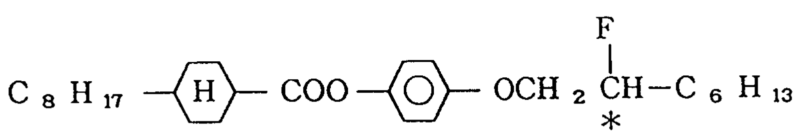

(3－30)

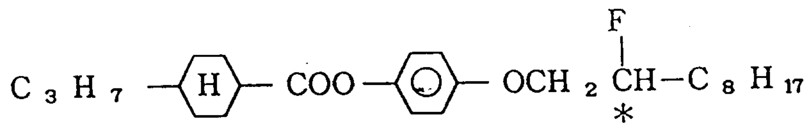

(3－31)

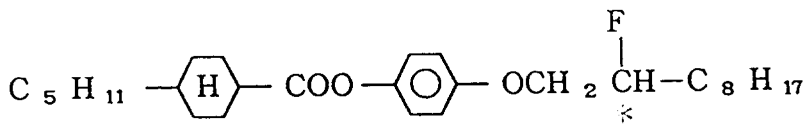

(3－32)

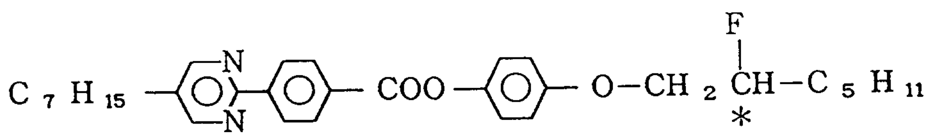

(3－32)

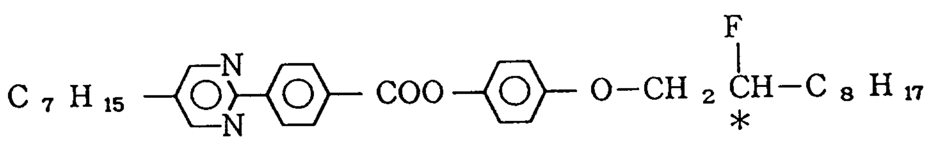

(3－33)

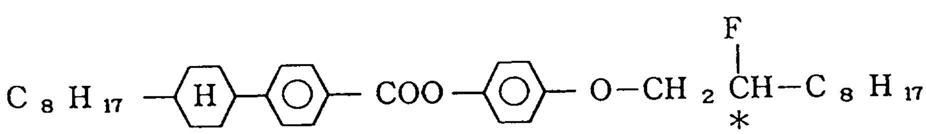

(3－34)

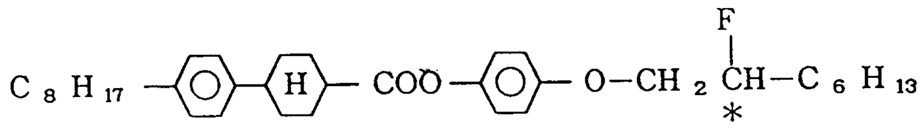

(3－35)

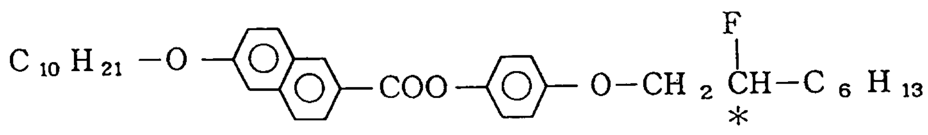

(3－36)

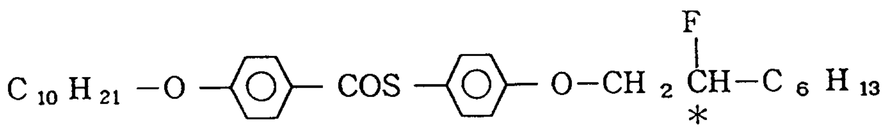

(3－37)

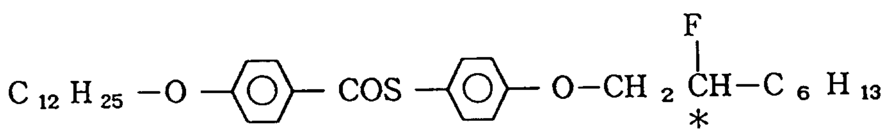

(3－38)

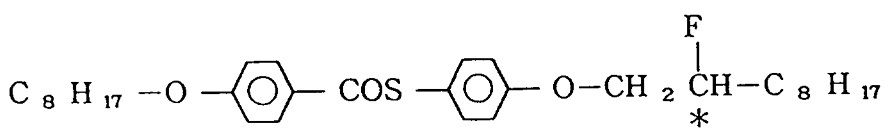

(3－39)

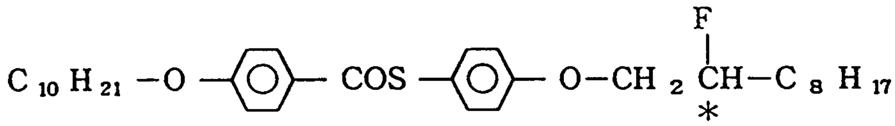

(3－40)

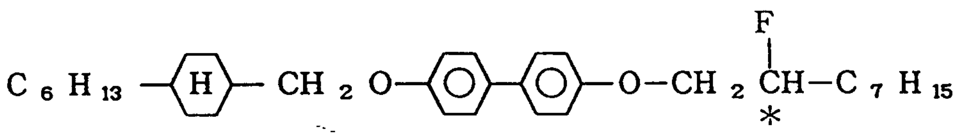

(3－41)

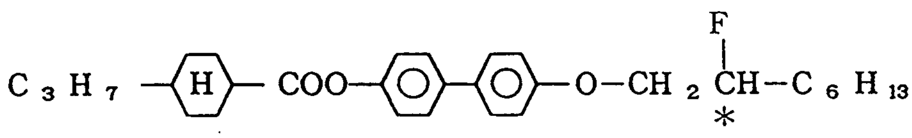

(3－42)

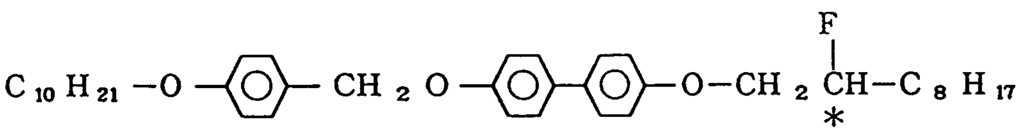

(3－43)

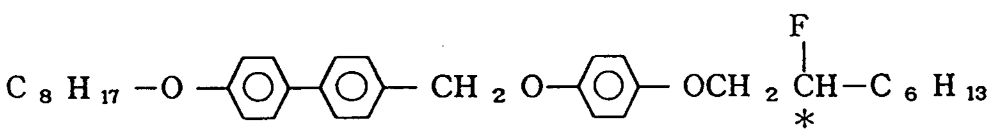

(3 − 44)

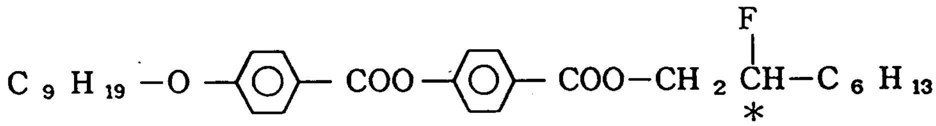

(3 − 45)

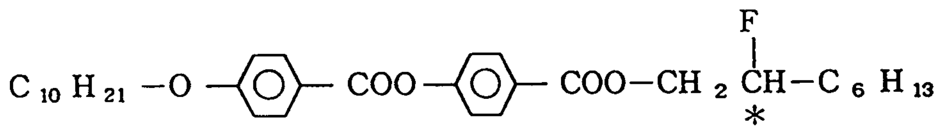

(3 − 46)

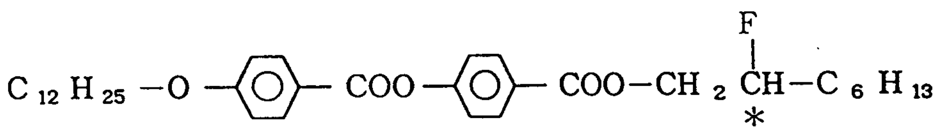

(3 − 47)

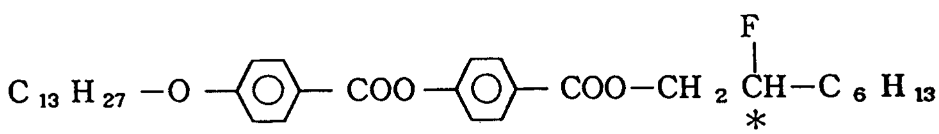

(3 − 48)

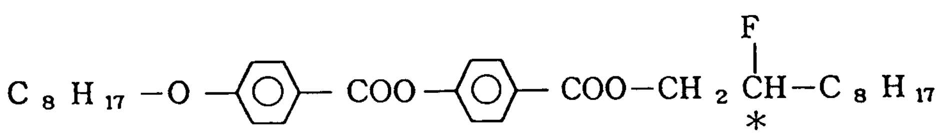

(3－49)

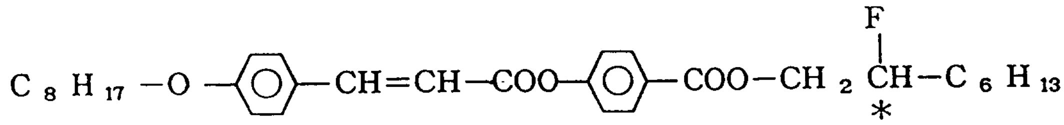

(3－50)

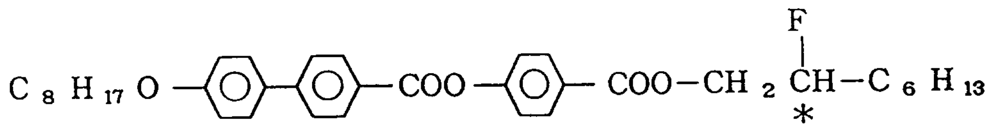

(3－52)

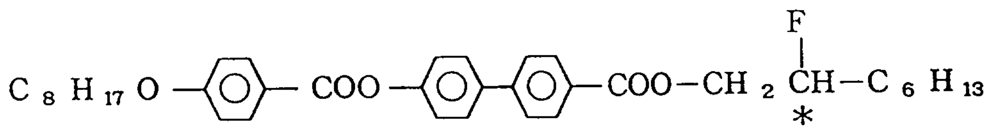

(3－53)

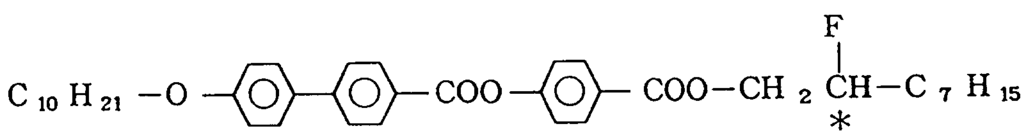

(3－54)

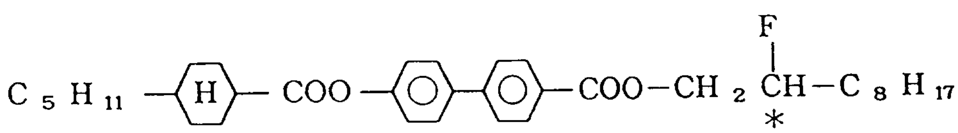

(3－55)

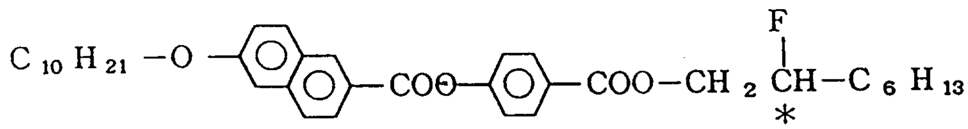

(3－56)

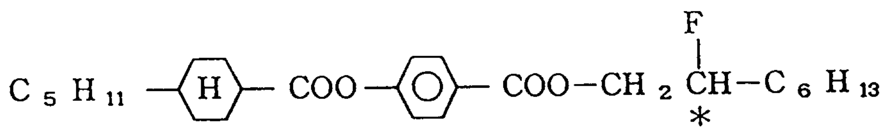

(3－57)

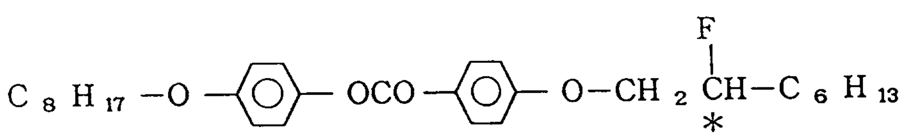

(3－58)

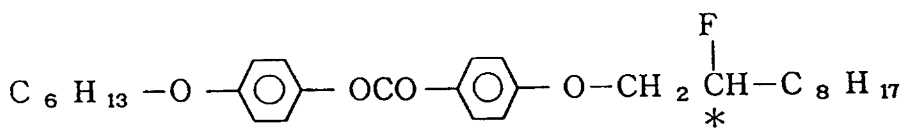

(3－59)

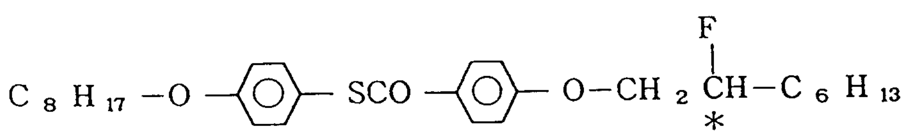

(3－60)

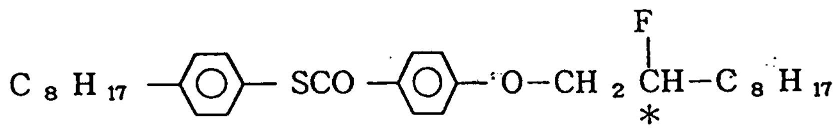

(3－61)

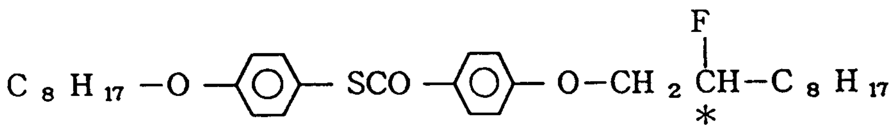

(3－62)

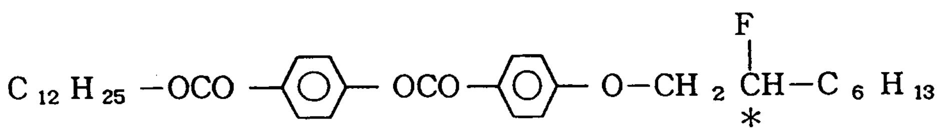

(3－63)

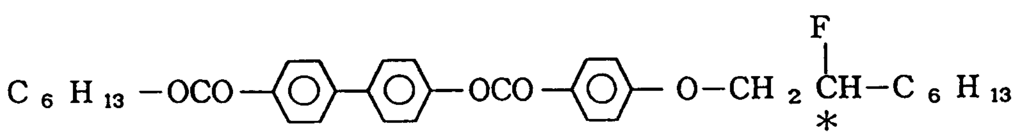

(3－64)

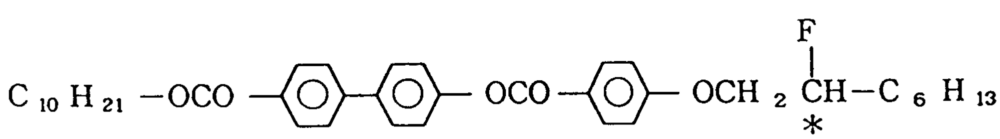

(3 − 65)

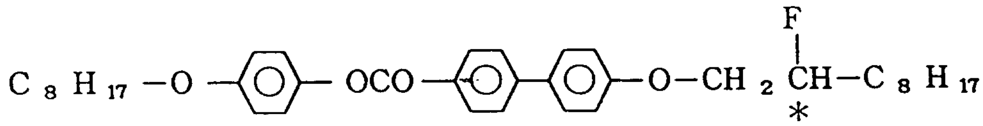

(3 − 66)

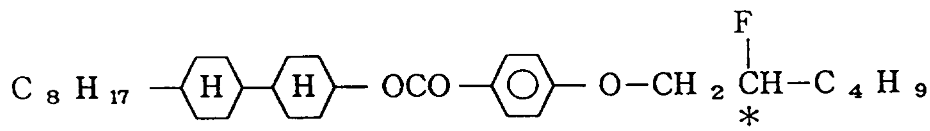

(3 − 67)

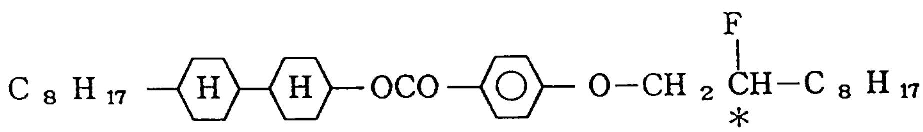

(3 − 68)

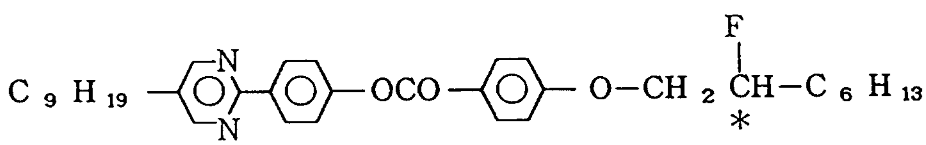

(3 − 69)

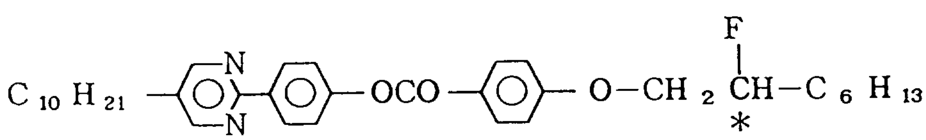

(3－70)

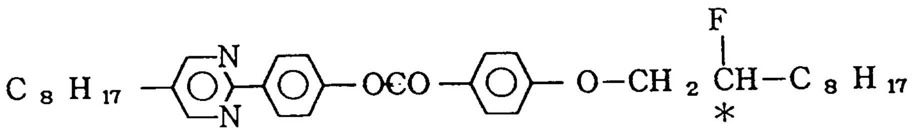

(3－71)

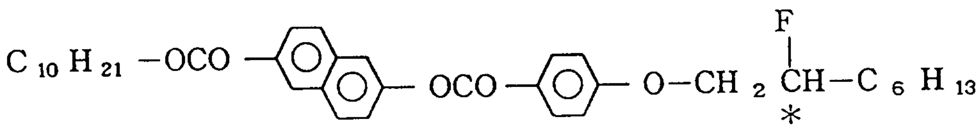

(3－72)

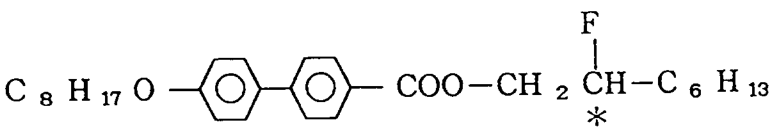

(3－73)

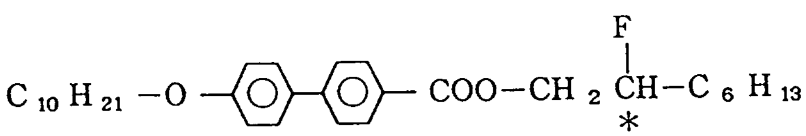

(3－74)

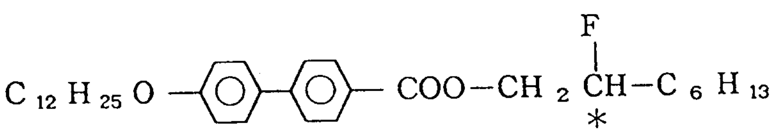

(3－75)

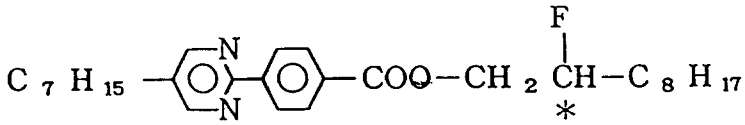

(3－76)

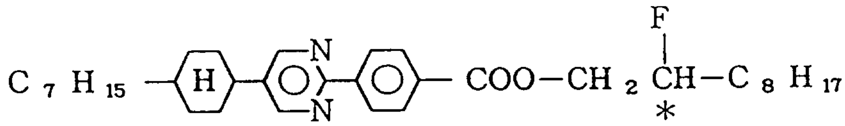

(3－77)

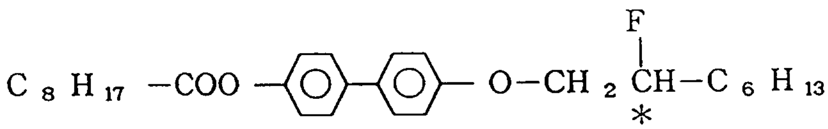

(3－78)

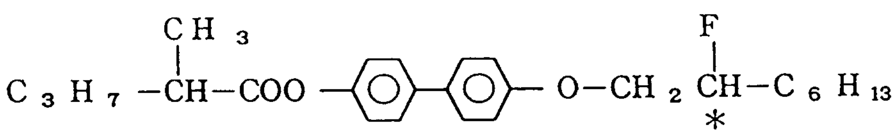

(3－79)

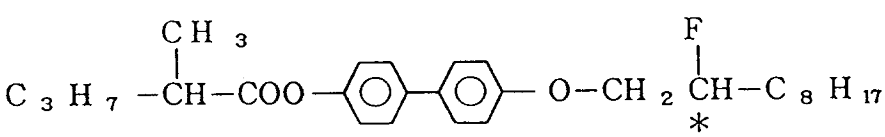

(3－80)

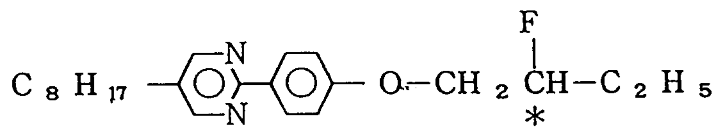

(3－81)

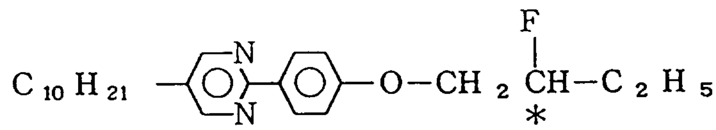

(3－82)

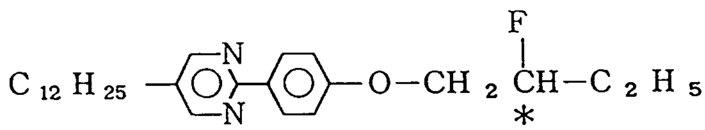

(3－83)

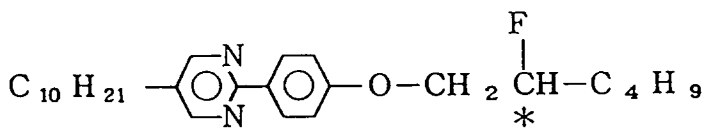

(3－84)

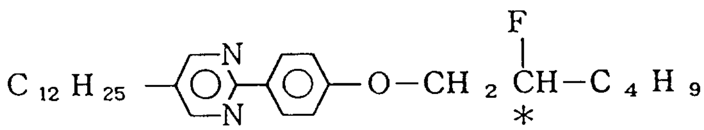

(3－85)

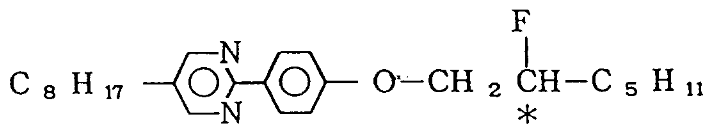

(3－86)

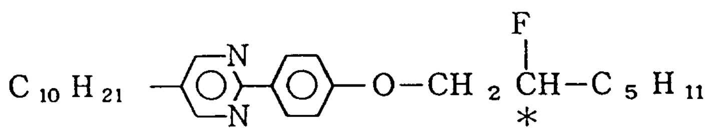

(3－87)

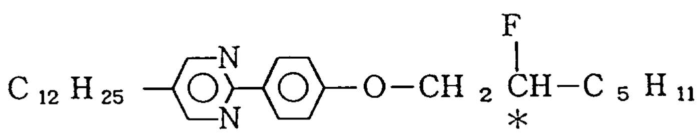

(3－88)

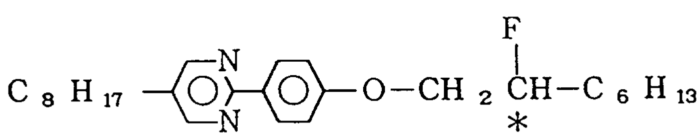

(3－89)

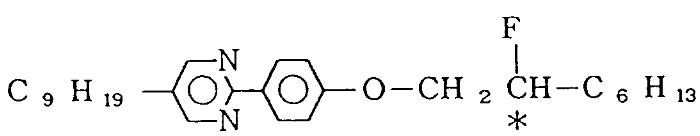

(3－90)

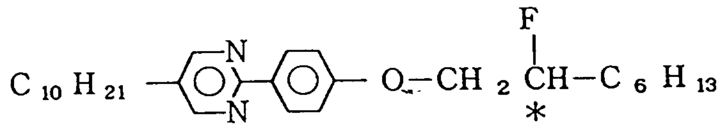

(3－91)

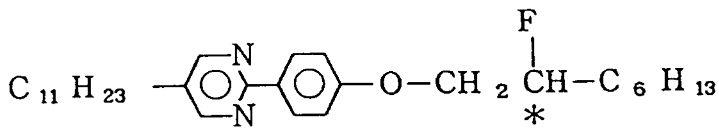

(3－92)

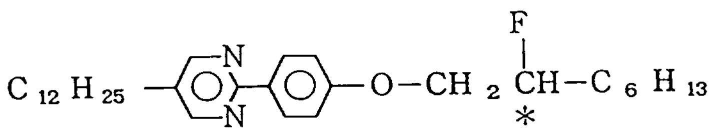

(3－93)

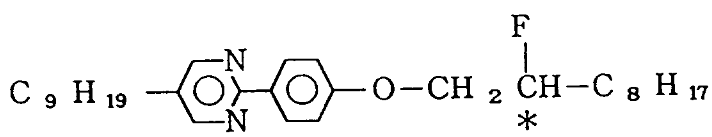

(3－94)

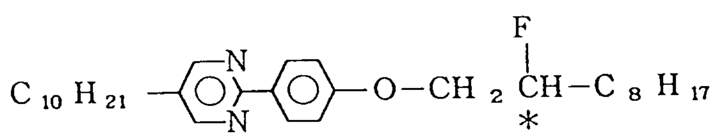

(3－95)

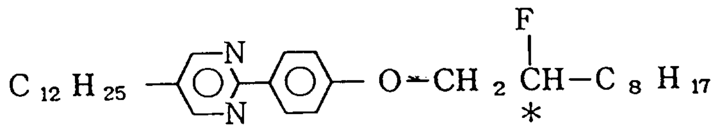

(3－96)

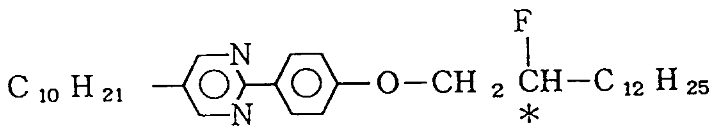

(3－97)

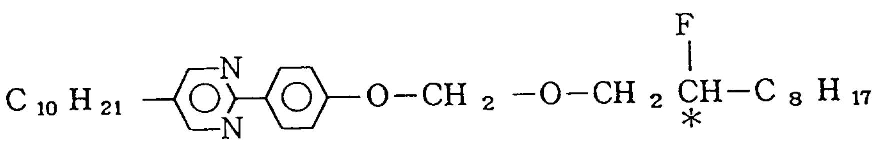

(3－98)

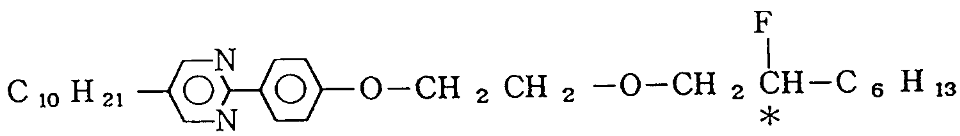

(3－99)

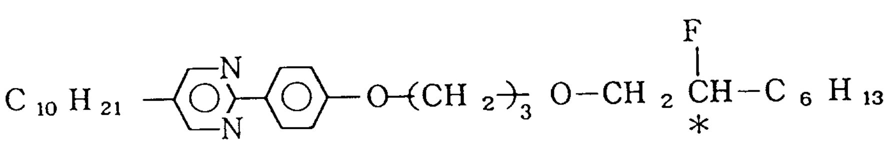

(3−100)

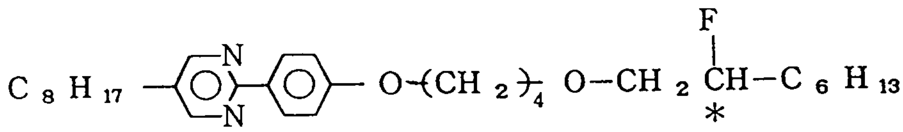

(3−101)

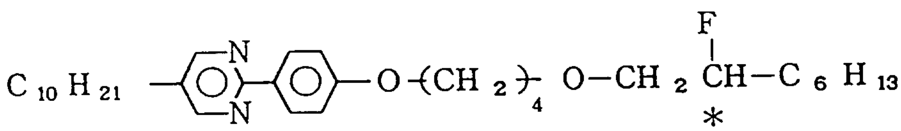

(3−102)

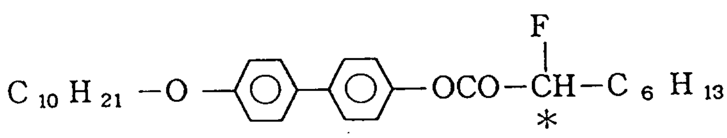

(3−103)

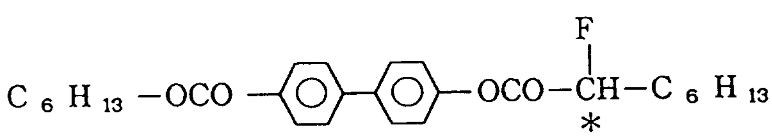

(3−104)

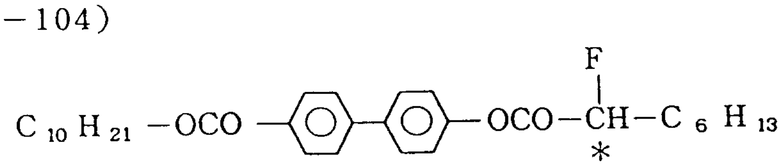

(3−105)

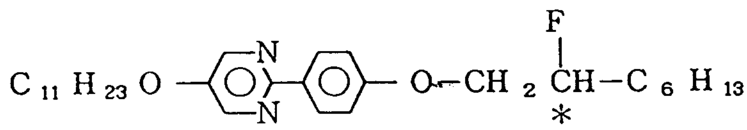

(3−106)

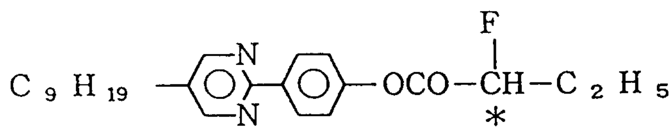

(3−107)

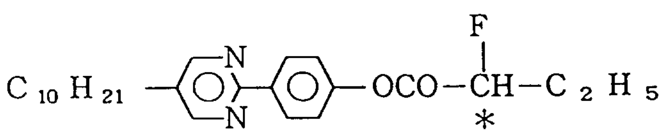

(3−108)

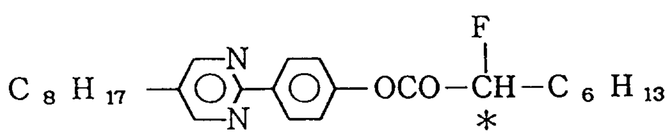

(3−109)

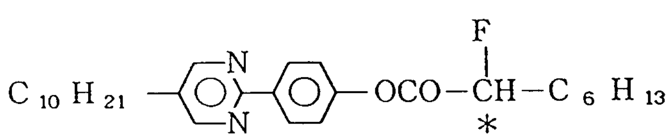

(3−110)

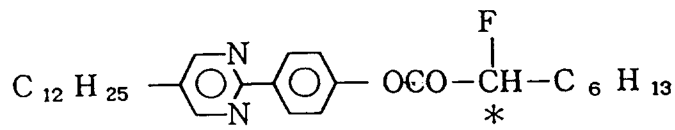

(3−111)

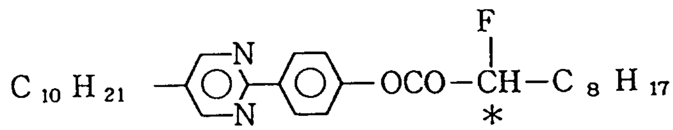

(3−112)

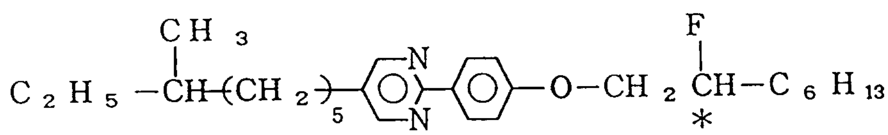

(3−113)

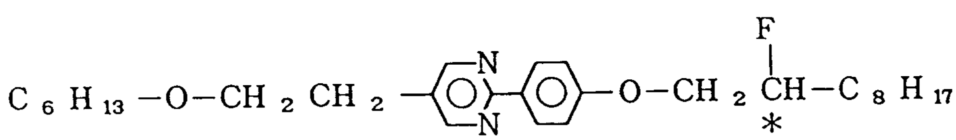

The liquid crystal composition according to the present invention may be obtained by mixing at least one species of the compound represented by the formula (I) and at least one species of another mesomorphic compound in appropriate proportions. The liquid crystal composition according to the present invention may preferably be formulated as a ferroelectric liquid crystal composition, particularly a ferroelectric chiral smectic liquid crystal composition.

Specific examples of another mesomorphic compound as described above other than the compounds represented by the formulas (II) and (III) may include those denoted by the following structural formulas.

(1)

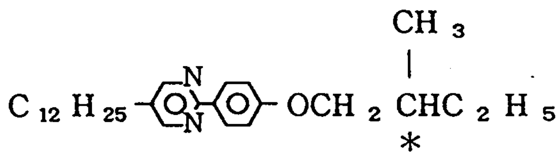

(2)

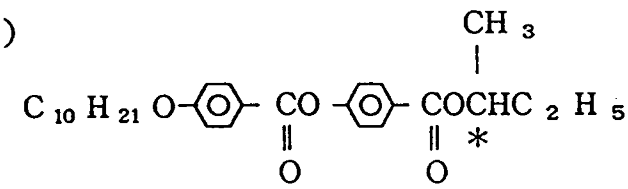

(3)

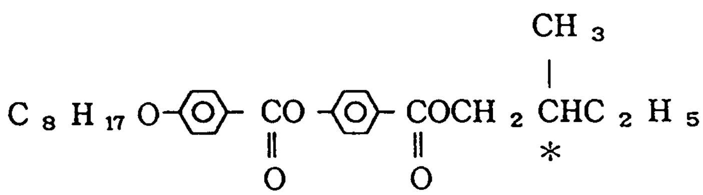

(4)

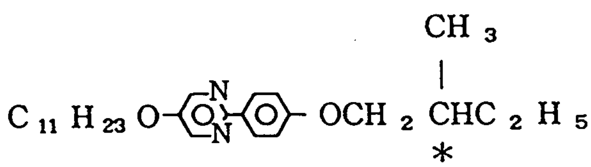

(5)

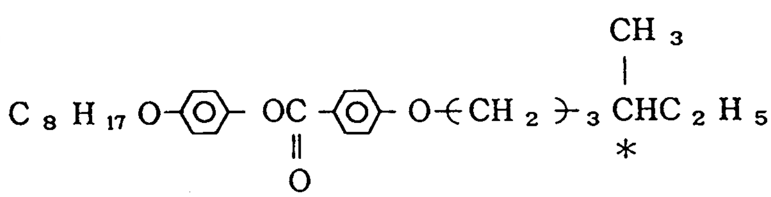

(6)

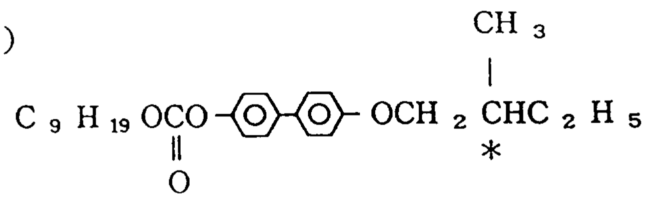

(7)

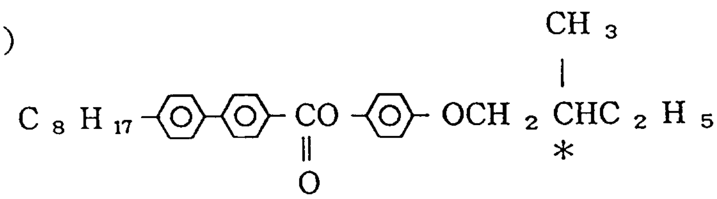

(8)

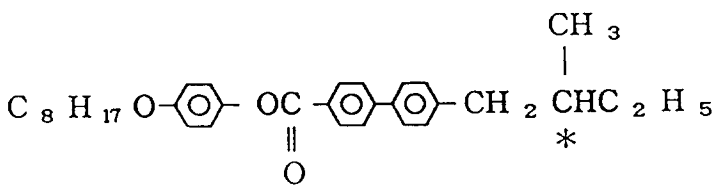

(9)

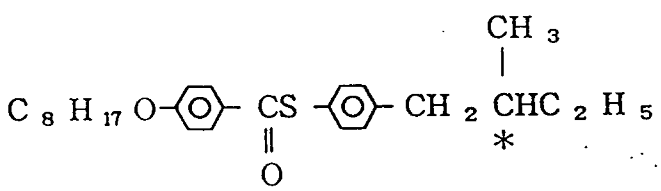

(10)

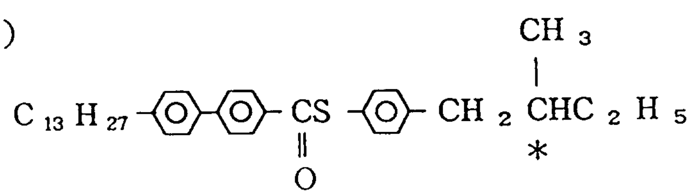

(11)

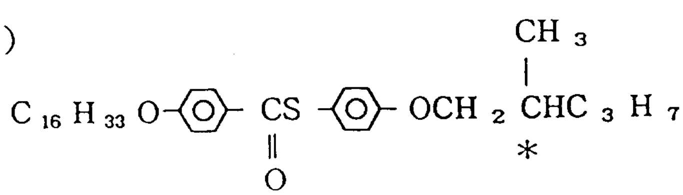

(12)

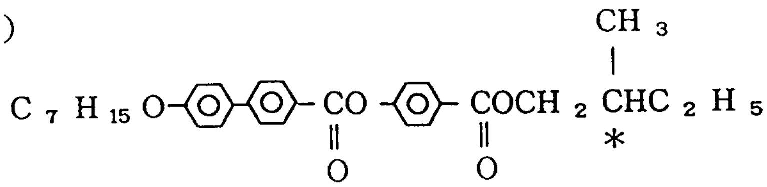

(13)

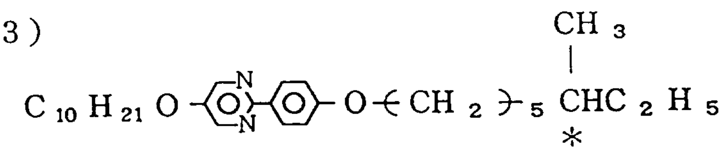

(14)

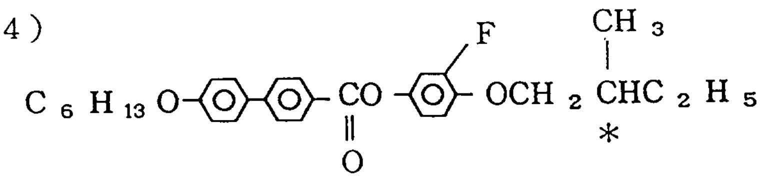

(15)

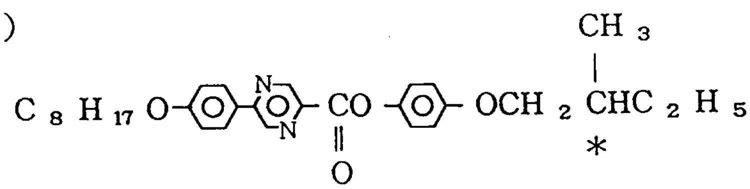

(16)

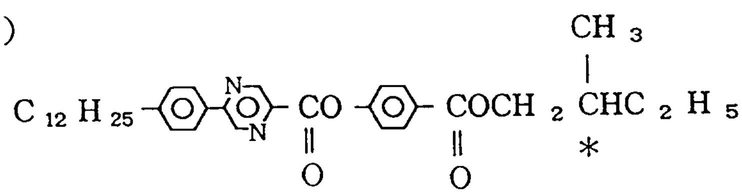

(17)

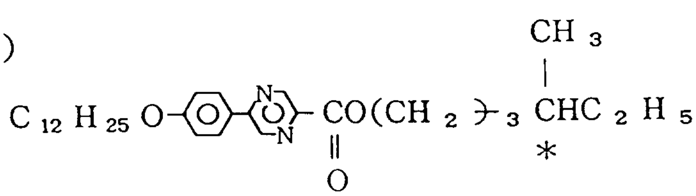

(18)

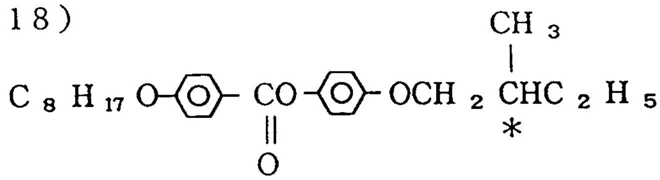

(19)

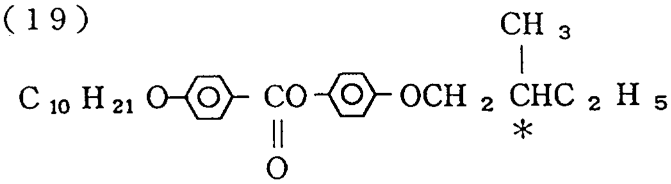

(20)

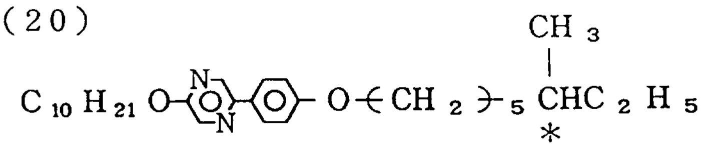

(21)

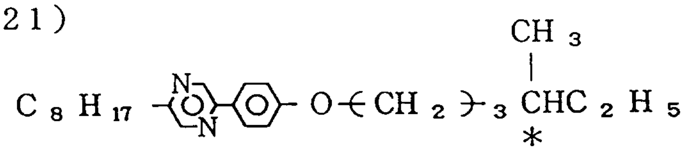

(22)

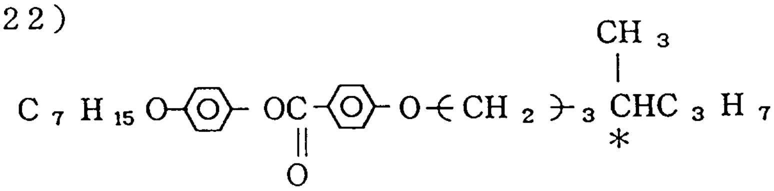

(23)

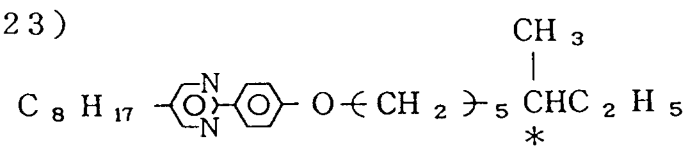

(24)

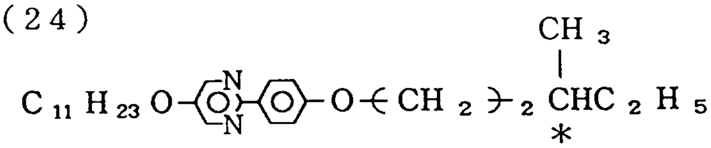

(25)

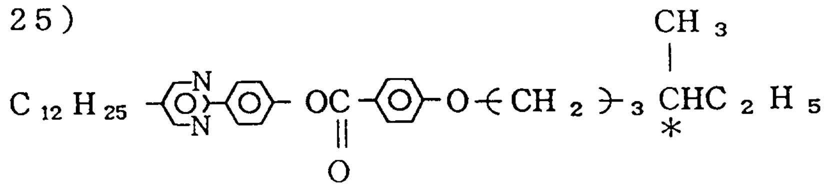

(26)

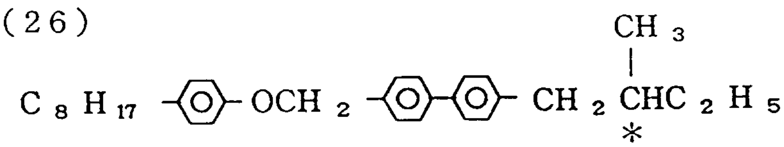

(27)

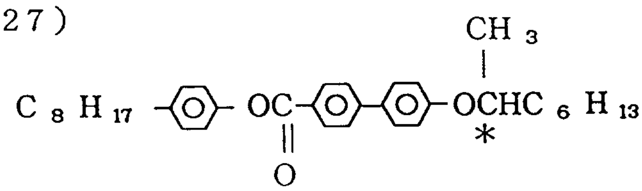

(28)

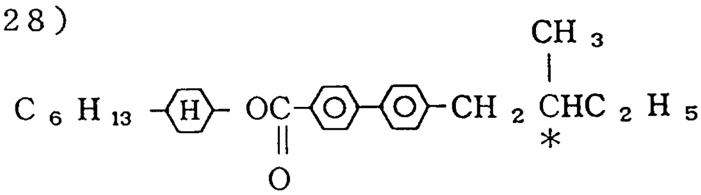

(29)

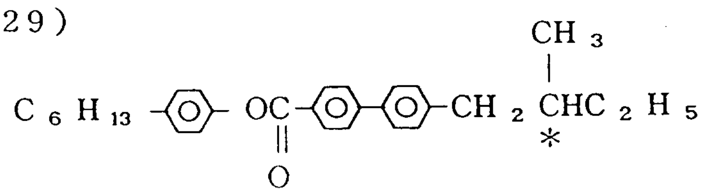

(30)

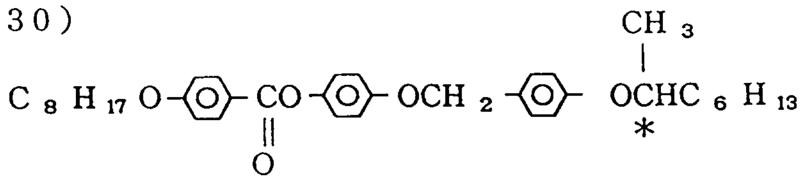

(31)

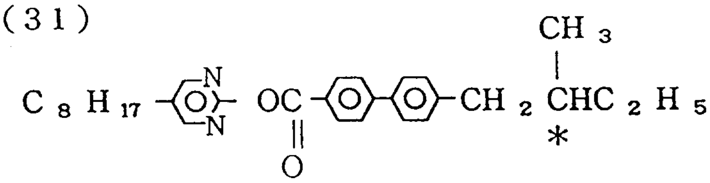

(32)

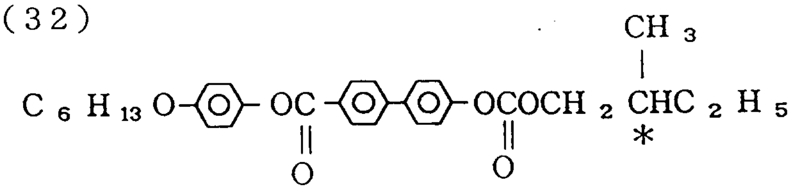

(33)

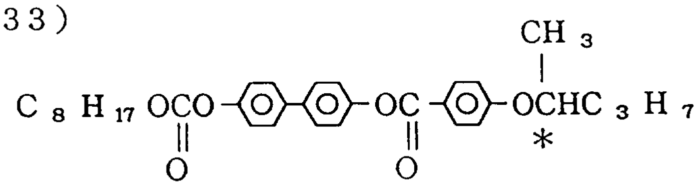

(34)

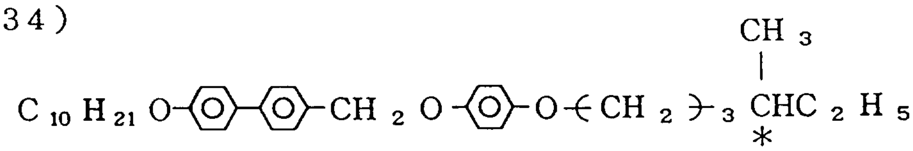

(35)

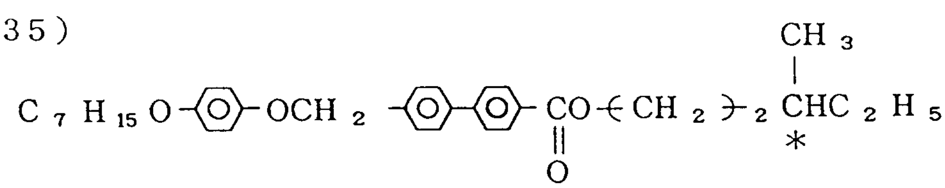

(36)

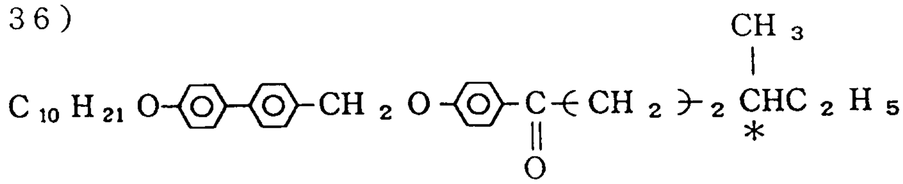

(37)

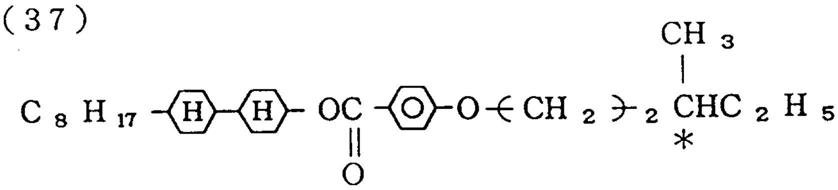

(38)

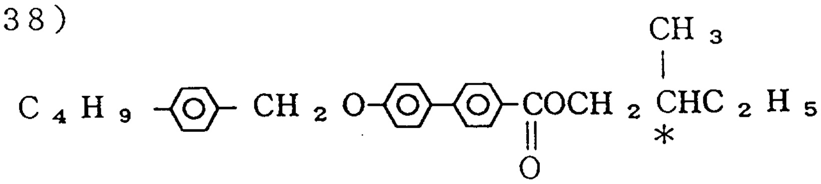

(39)

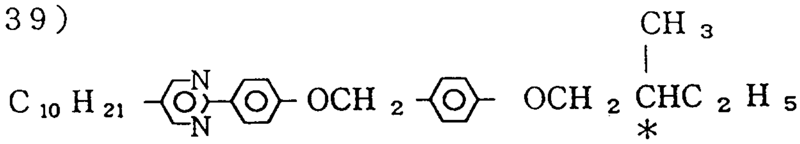

(40)

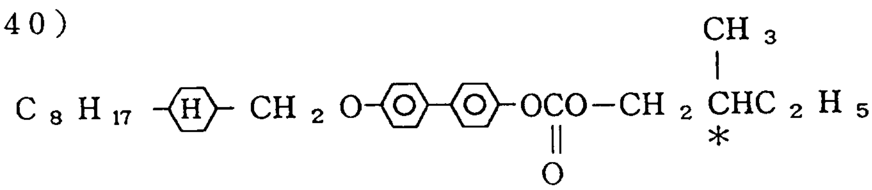

(41)

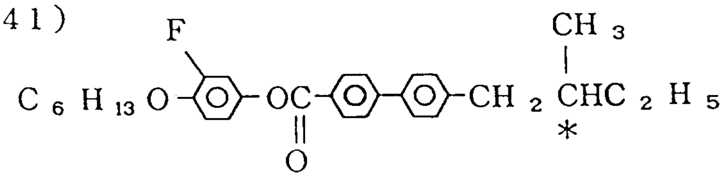

(42)

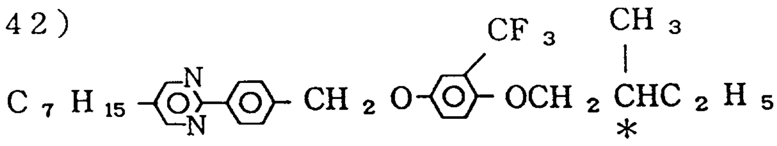

(43)

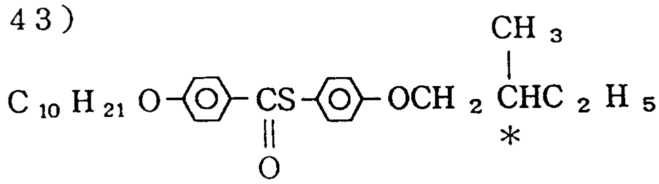

(44)

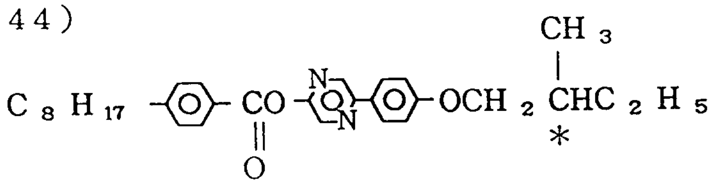

(45)

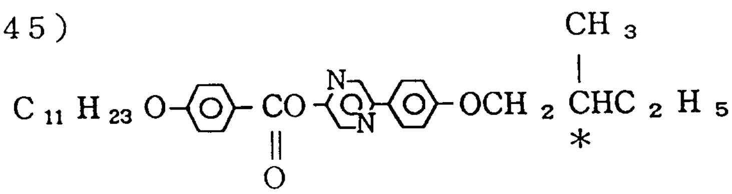

(46)

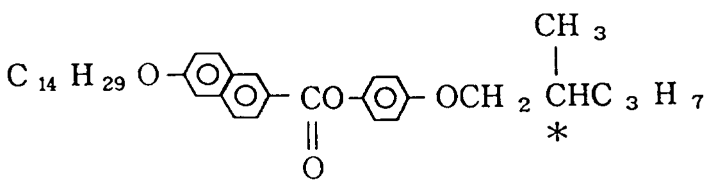

(47)

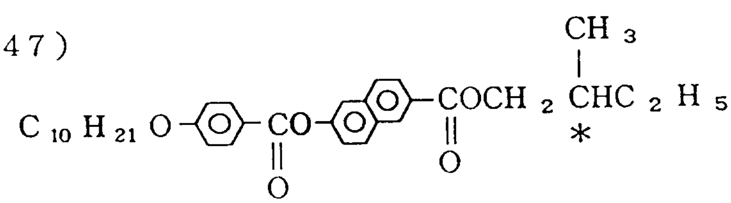

(48)

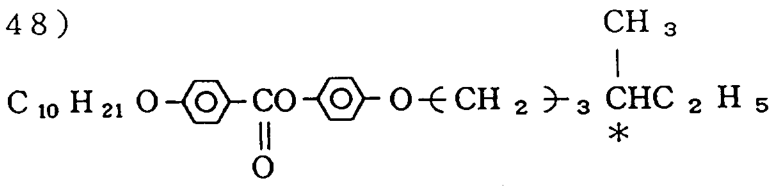

(49)

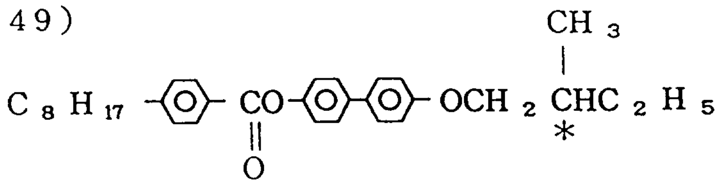

(50)

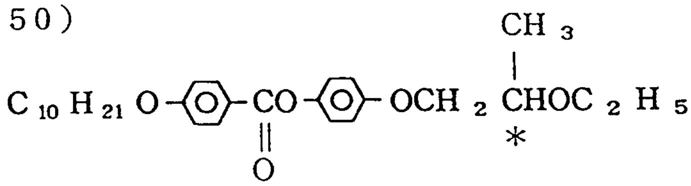

(51)

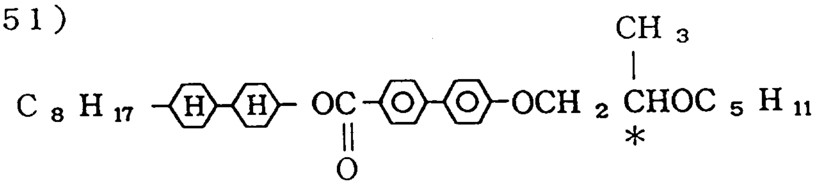

(52)

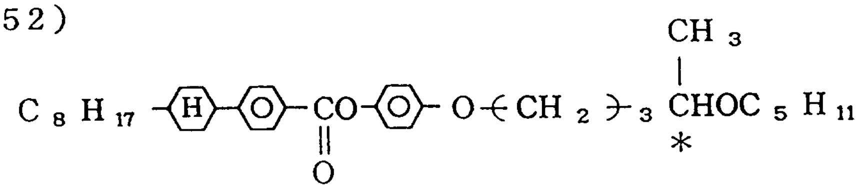

(53)

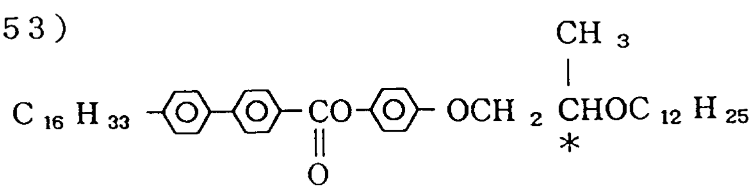

(54)

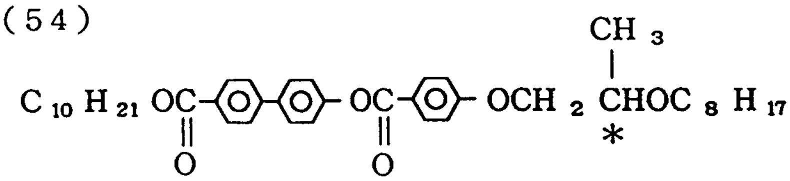

(55)

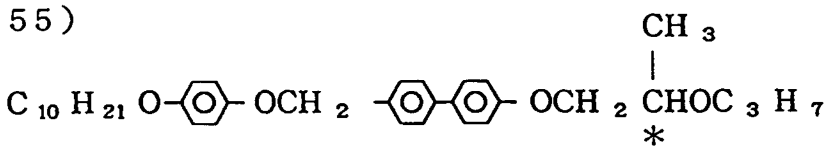

(56)

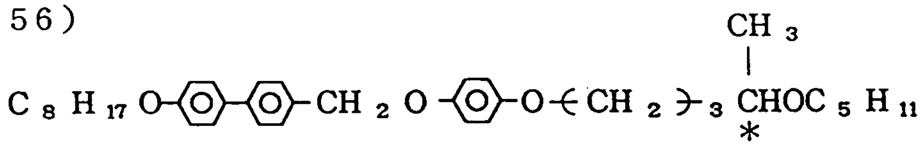

(57)

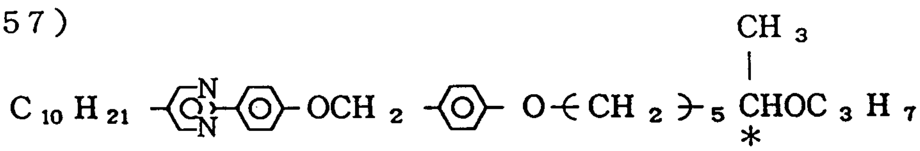

(58)

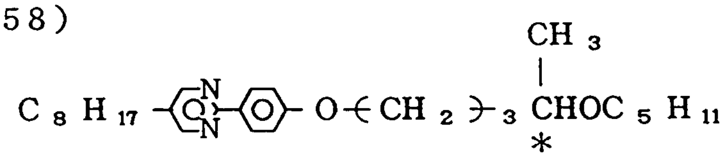

(59)

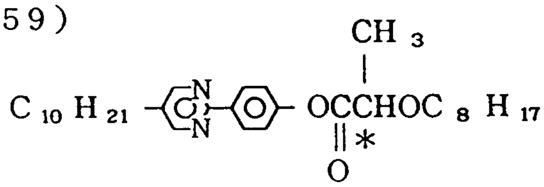

(60)

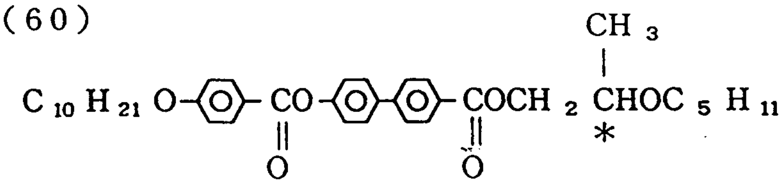

(61)

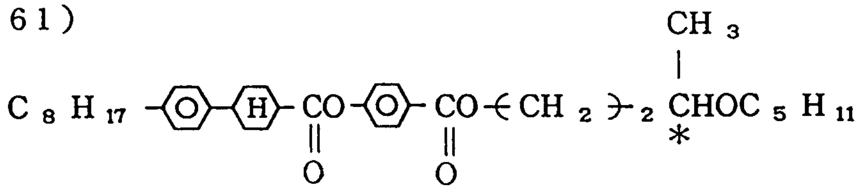

(62)

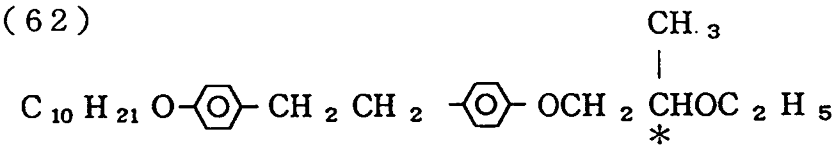

(63)

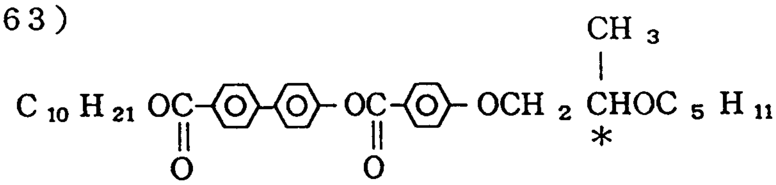

(64)

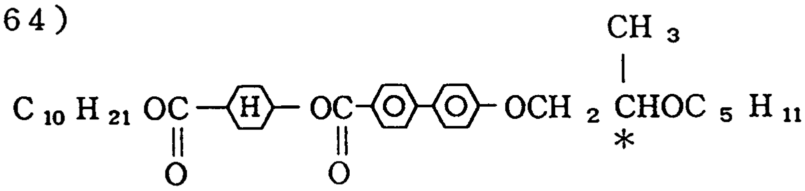

(65)

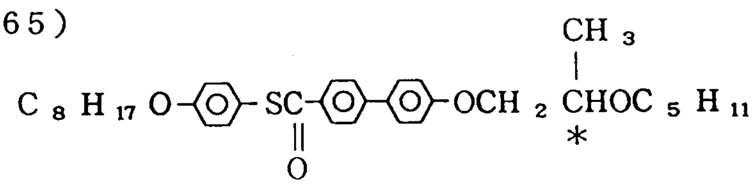

(66)

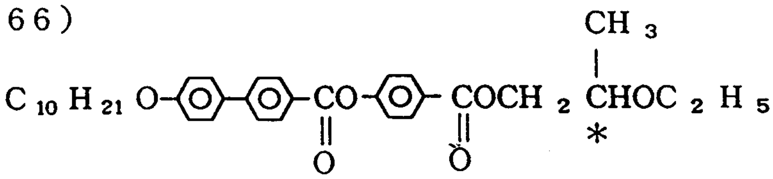

(67)

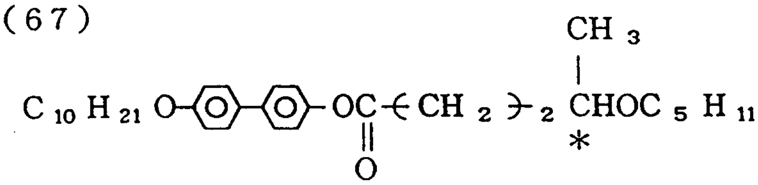

(68)

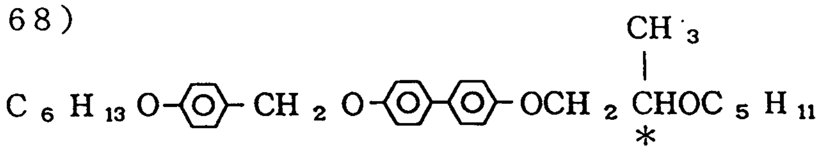

(69)

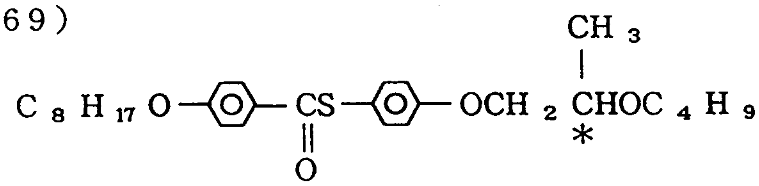

(70)

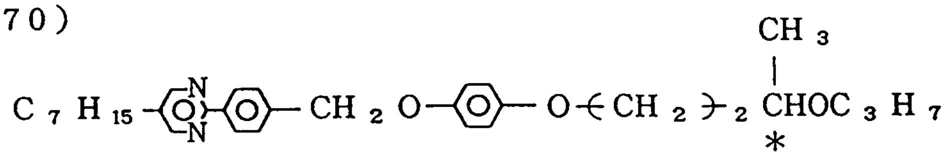

(71)

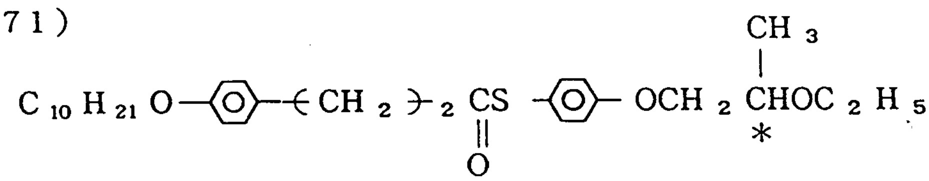

(72)

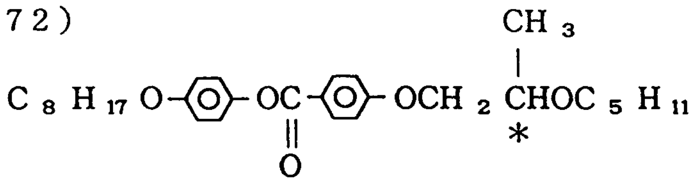

(73)

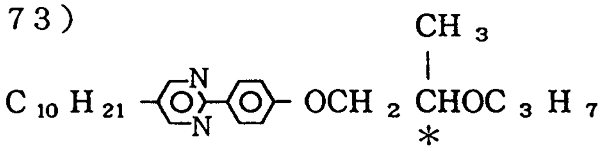

(74)

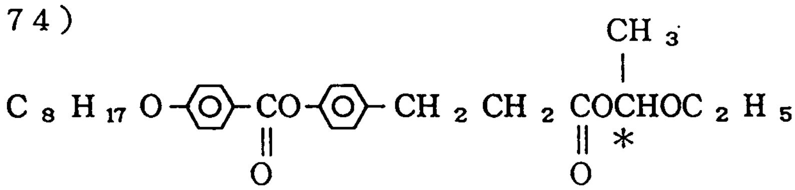

(75)

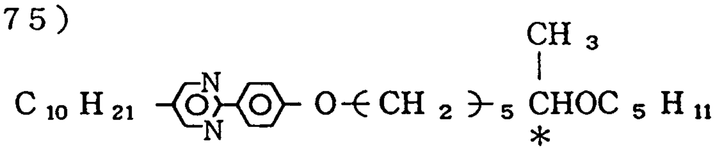

(76)

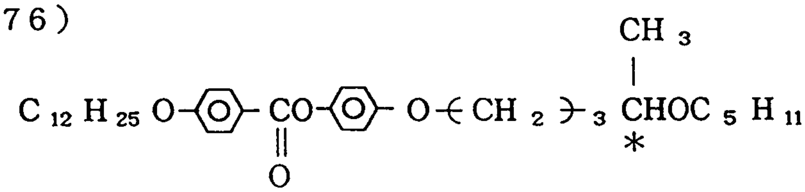

(77)

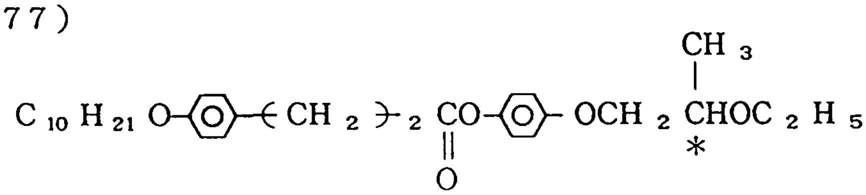

(78)

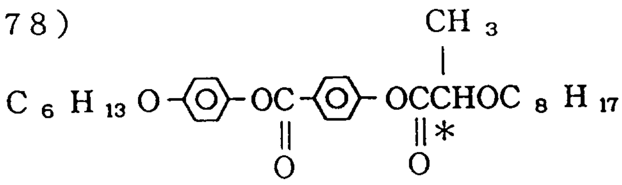

(79)

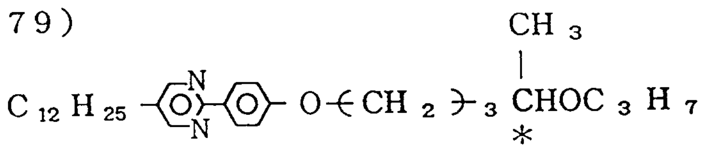

(80)

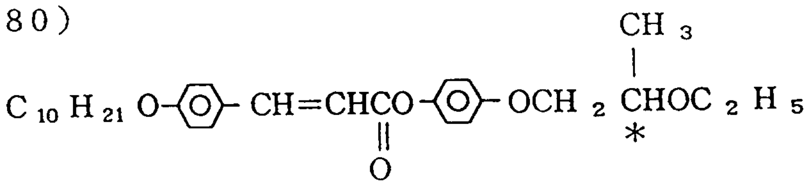

(81)

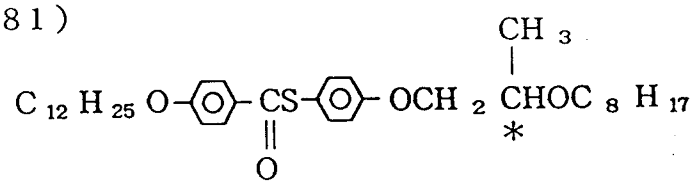

(82)

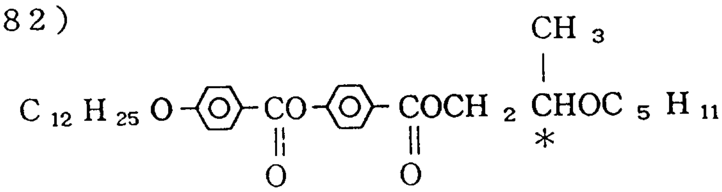

(83)

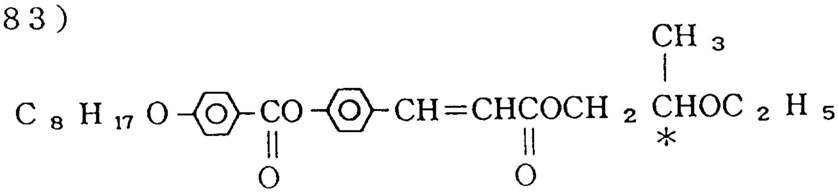

(84)

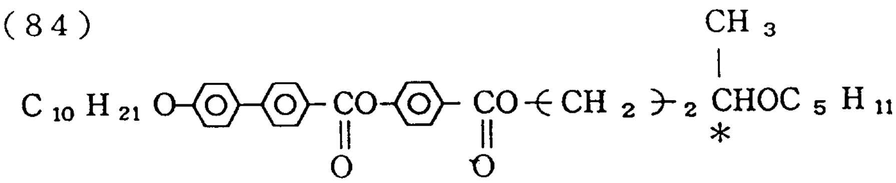

(85)

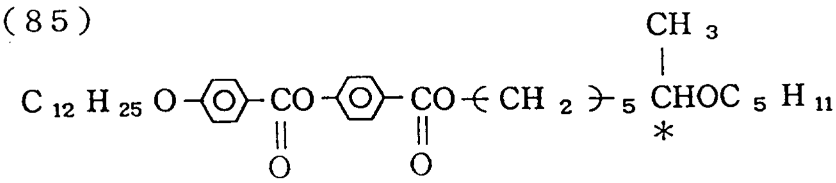

(86)

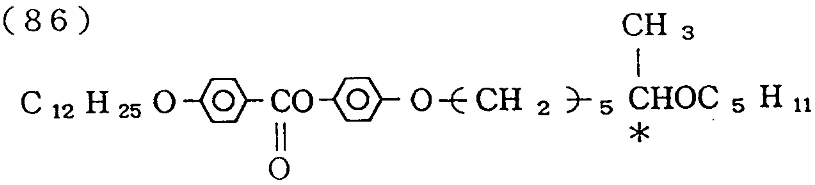

(87)

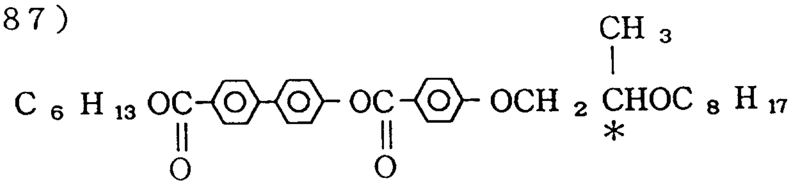

(88)

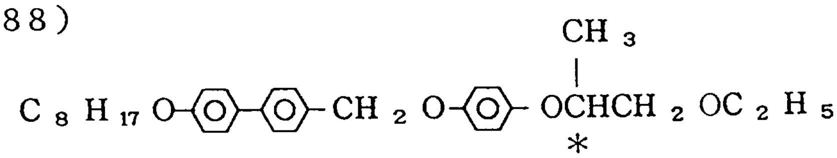

(89)

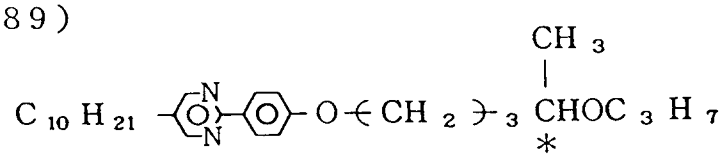

(90)

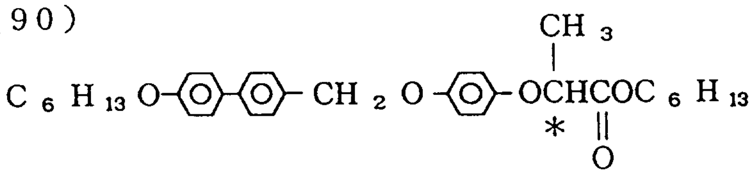

(91)

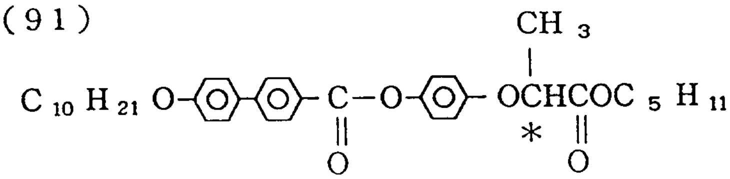

(92)

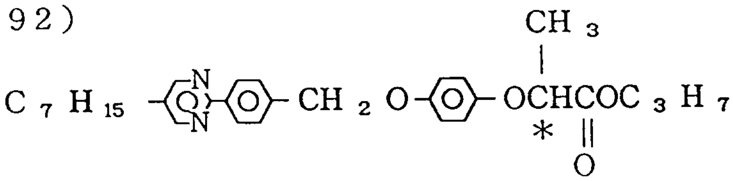

(93)

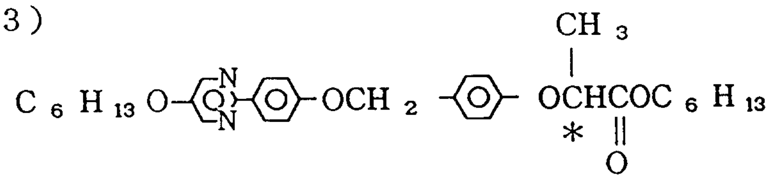

(94)

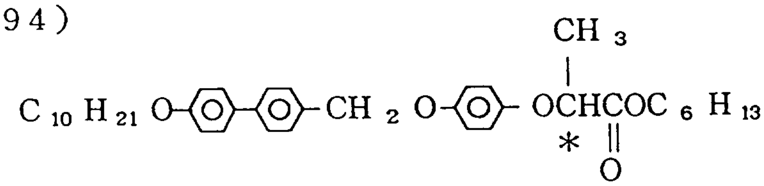

(95)

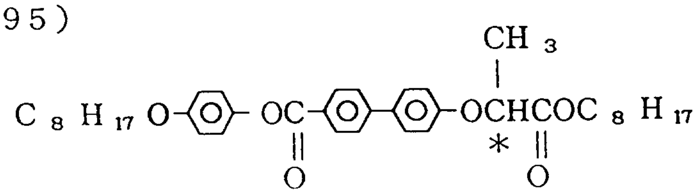

(96)

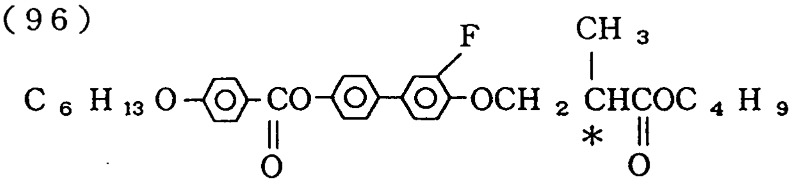

(97)

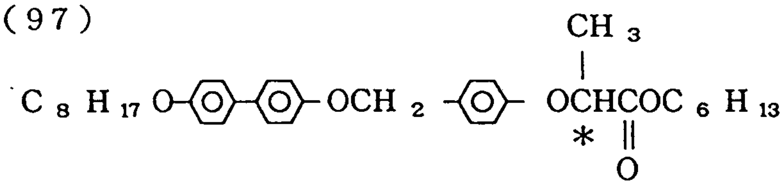

(98)

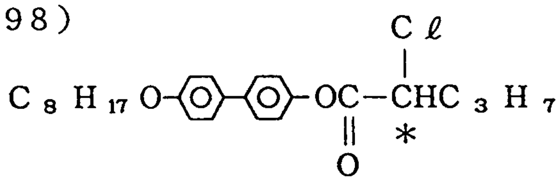

(99)

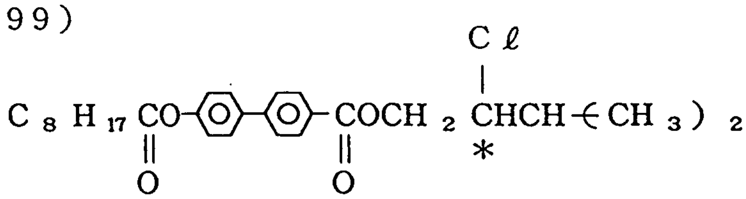

(100)

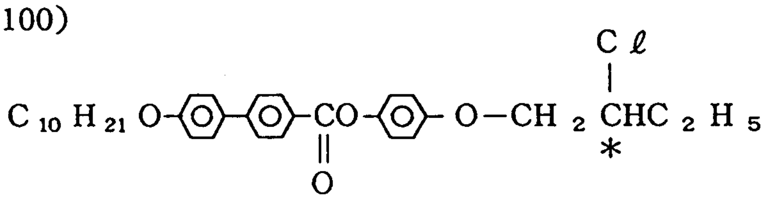

(101)

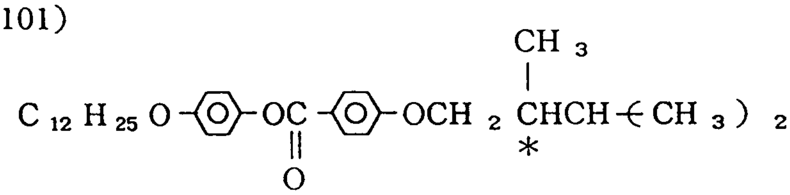

(102)

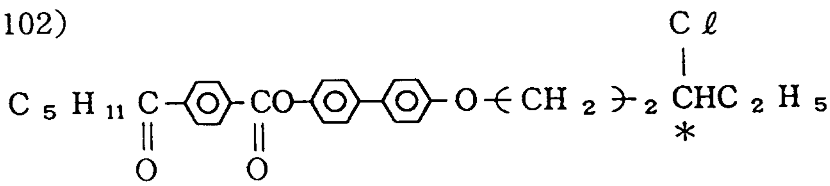

(103)

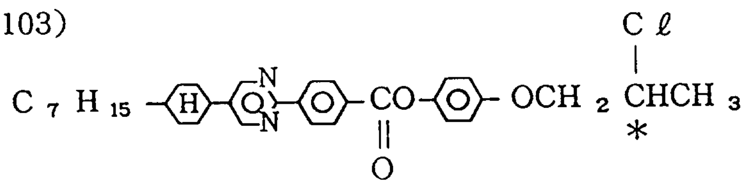

(104)

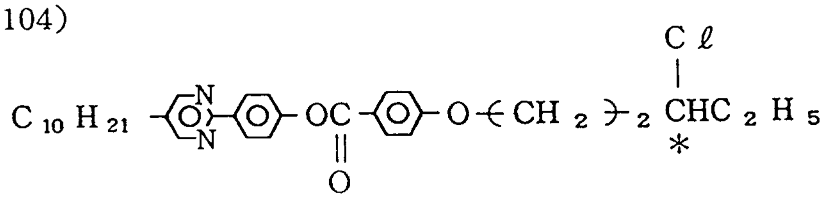

(105)

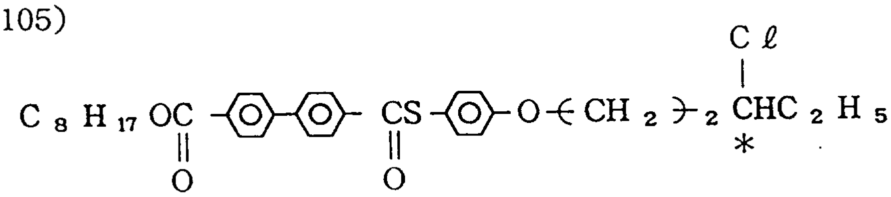

(106)

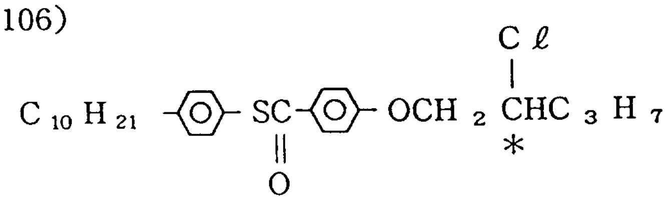

(107)

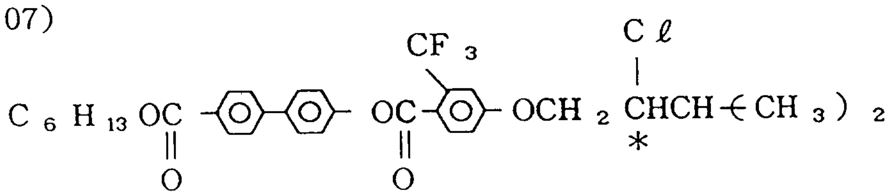

(108)

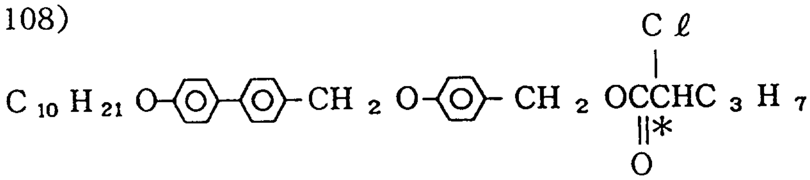

(109)

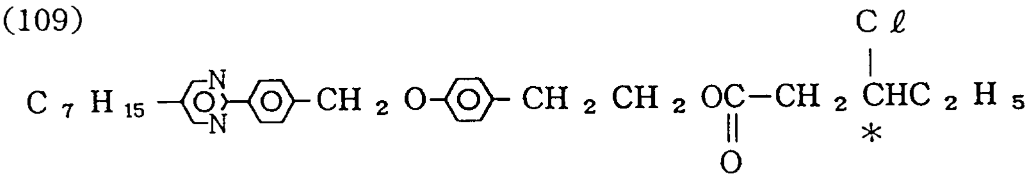

(110)

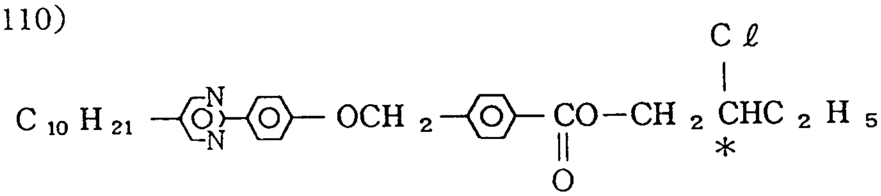

(111)

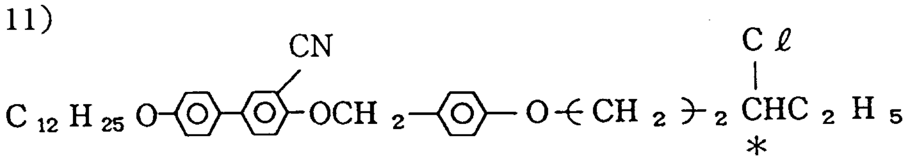

(112)

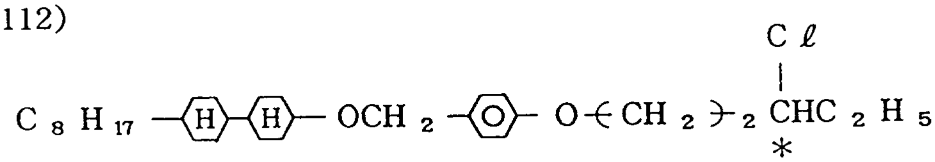

(113)

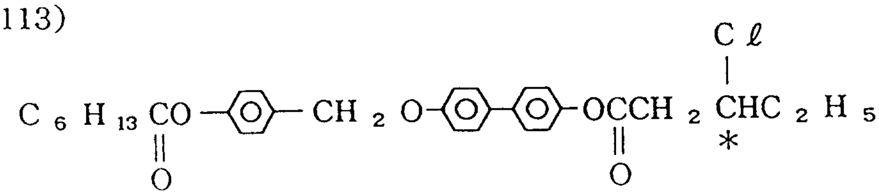

(114)

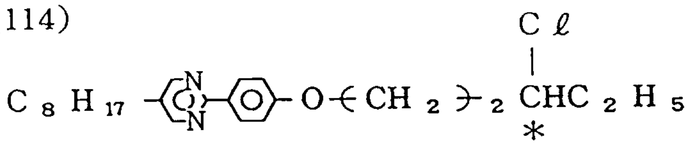

(115)

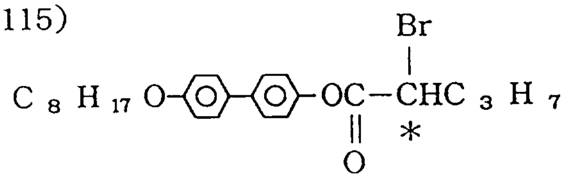

(116)

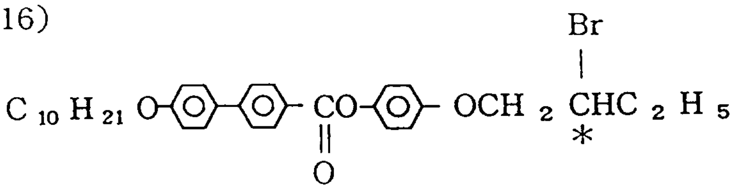

(117)

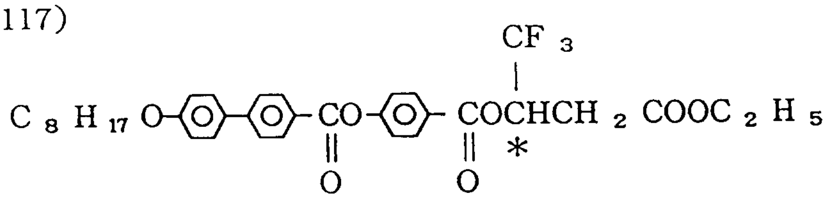

(118)

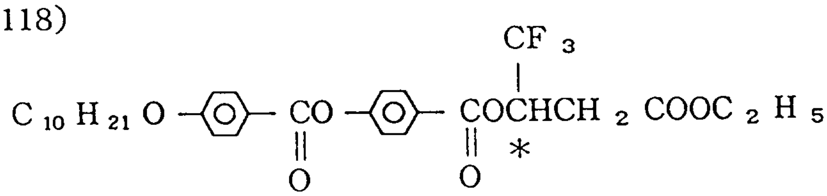

(119)

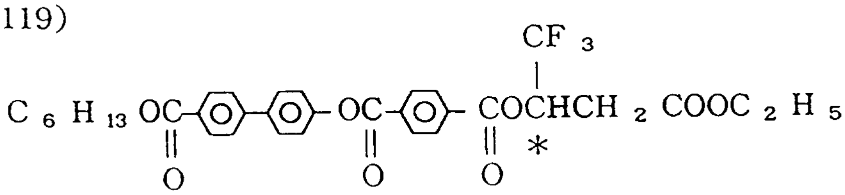

(120)

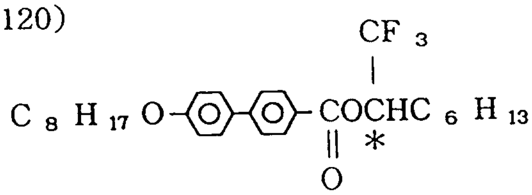

(121)

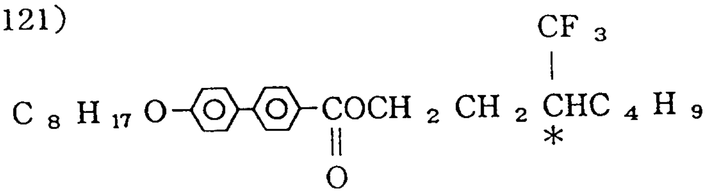

(122)

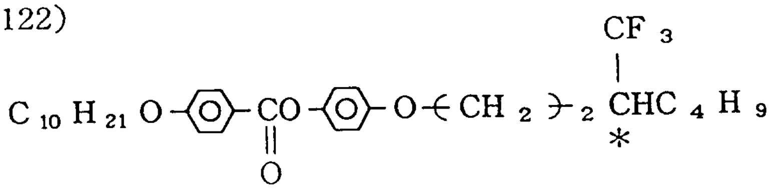

(123)

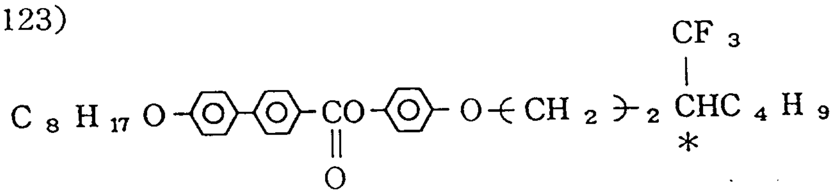

(124)

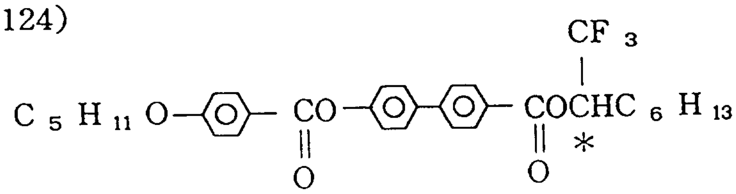

(125)

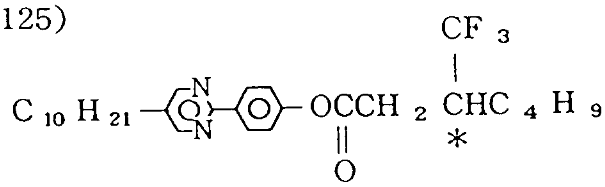

(126)

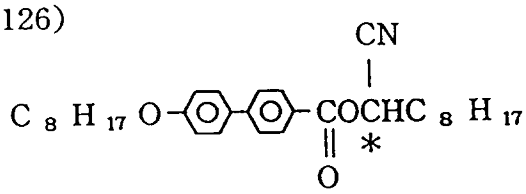

(127)

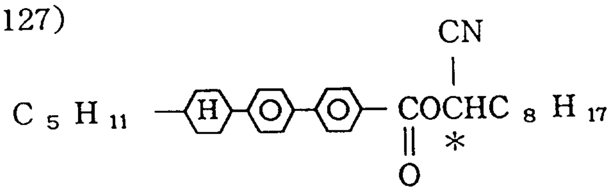

(128)

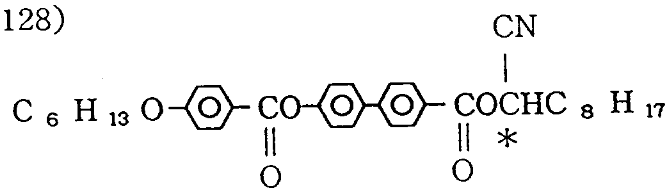

(129)

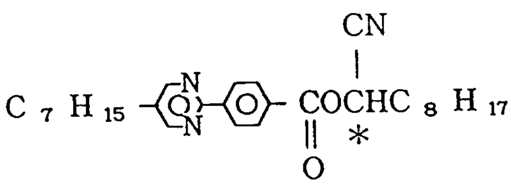

(130)

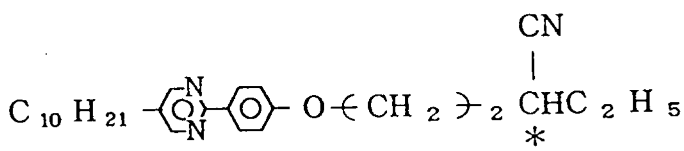

(131)

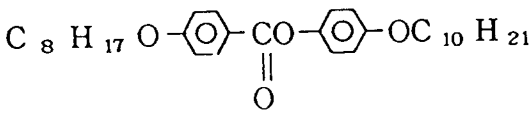

(132)

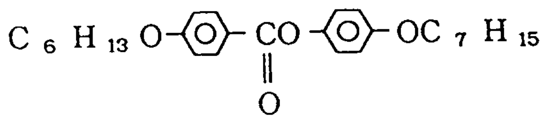

(133)

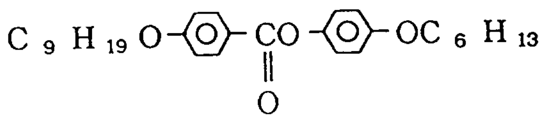

(134)

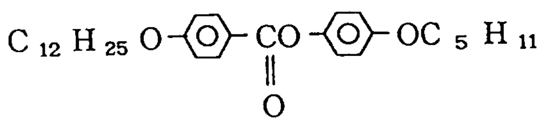

(135)

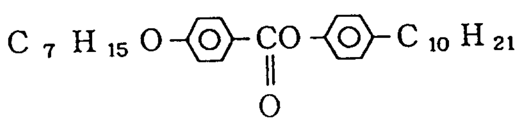

(136)

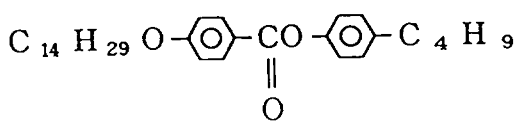

(137)

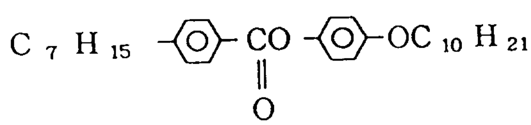

(138)

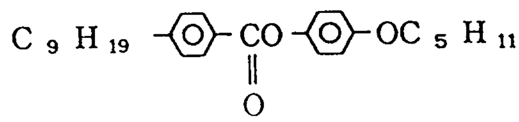

(139)

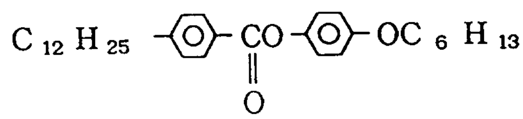

(140)

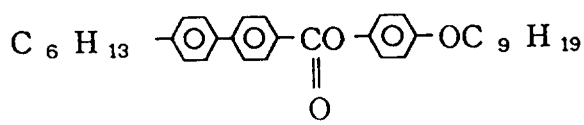

(141)

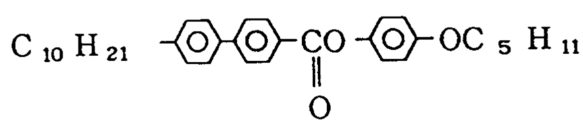

(142)

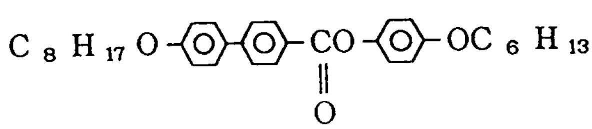

(143)

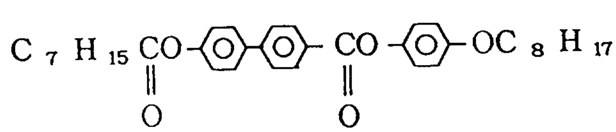

(144)

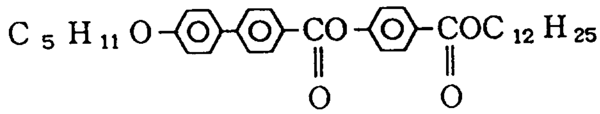

(145)

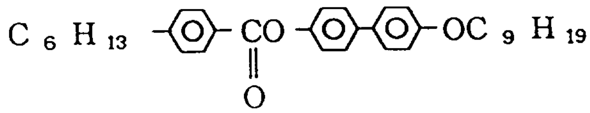

(146)

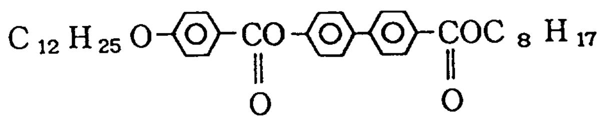

(147)

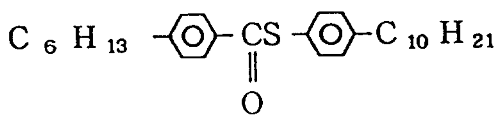

(148)

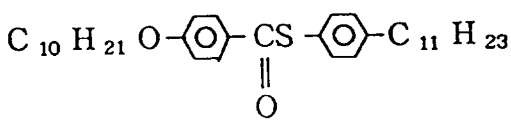

(149)

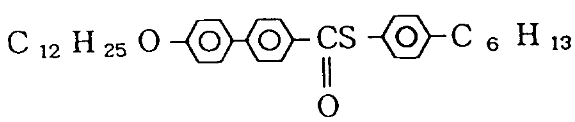

(150)

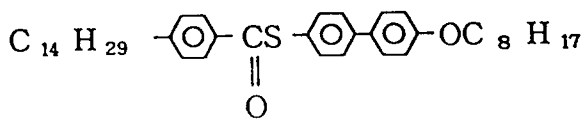

(151)

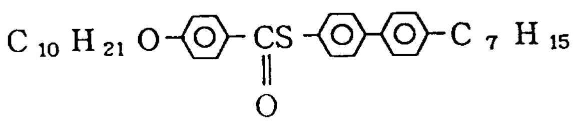

(152)

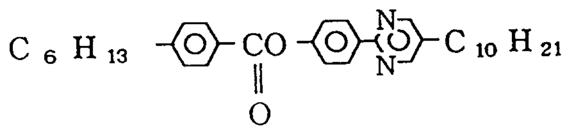

(153)

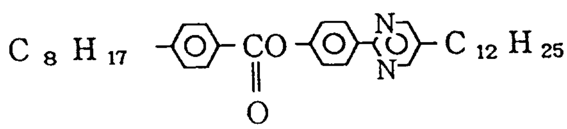

(154)

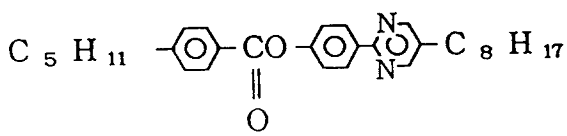

(155)

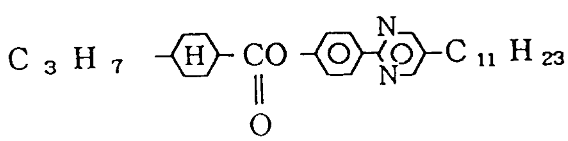

(156)

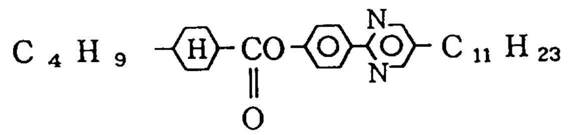

(157)

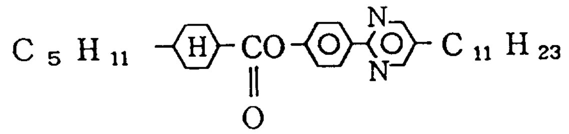

(158)

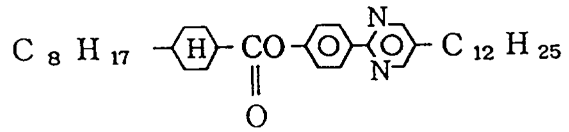

(159)

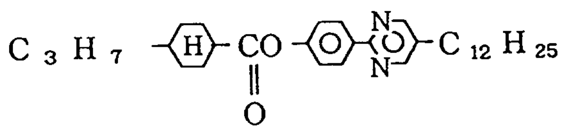

(160)

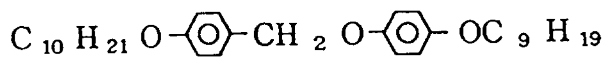

(161)

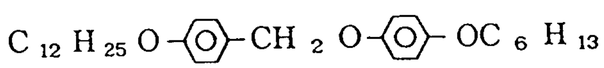

(162)

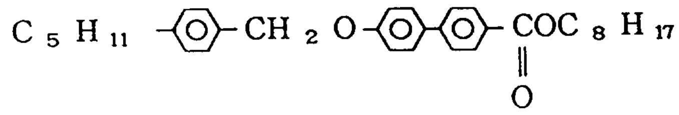

(163)

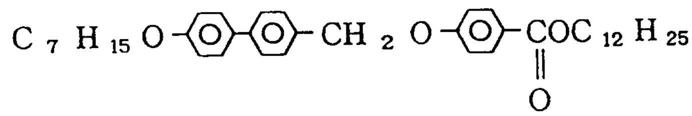

(164)

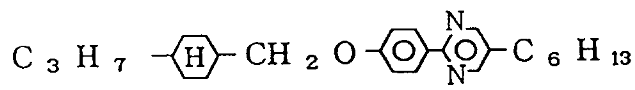

(165)

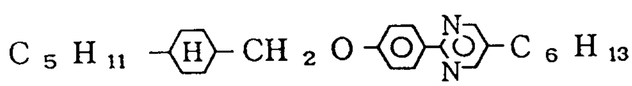

(166)

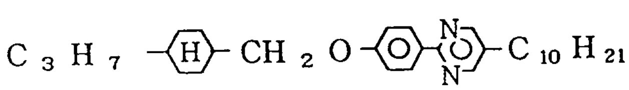

(167)

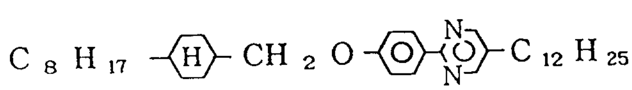

In formulating the liquid crystal composition according to the present invention comprising mesomorphic compounds represented by the formula (I) and (III) together with another mesomorphic compound, it is desirable to mix 1 - 300 wt. Parts each, preferably 2 - 200 wt. parts each, of mesomorphic compound of the formulas (I) and (III) with 100 wt. parts of the remainder of the liquid crystal composition other than the compounds of the formulas (I) and (III).

Further, when two or more species of either one or both of the compounds represented by the formulas (I) and (III) are used, the two or more species of the compounds of the formulas (I) and (III) may be used in a total amount of 1 - 500 wt. parts, preferably 2 - 200 wt. parts, per 100 wt. parts of the remainder of the liquid crystal composition other than the compounds of the formulas (I) and (III).

In formulating the liquid crystal composition according to the present invention comprising mesomorphic compounds represented by the formulas (I), (II) and (III) together with another mesomorphic compound, it is desirable to mix 1 - 300 wt. parts each, preferably 2 - 200 wt. parts each, of mesomorphic

compounds of the formulas (I), (II) and (III) with 100 wt. parts of the remainder of the liquid crystal composition other than the compounds of the formulas (I), (II) and (III).

Further, when two or more species of either one or all of the compounds represented by the formulas (I), (II) and (III) are used, the two or more species of the compounds of the formulas (I), (II) and (III) may be used in a total amount of 1 - 500 wt. parts, preferably 2 - 200 wt. parts, per 100 wt. parts of the remainder of the liquid crystal composition other than the compound of the formulas (I), (II) and (III).

The ferroelectric liquid crystal device according to the present invention may preferably be prepared by heating the liquid crystal composition prepared as described above into an isotropic liquid under vacuum, filling a blank cell comprising a pair of oppositely spaced electrode plates with the composition, gradually cooling the cell to form a liquid crystal layer and restoring the normal pressure.

Figure 1 is a schematic sectional view of an embodiment of the ferroelectric liquid crystal device prepared as described above for explanation of the structure thereof.

Referring to Figure 1, the ferroelectric liquid crystal device includes a ferroelectric liquid crystal layer 1 disposed between a pair of glass substrates 2 each having thereon a transparent electrode 3 and an insulating alignment control layer 4. Lead wires 6 are connected to the electrodes so as to apply a driving voltage to the liquid crystal layer 1 from a power supply 7. Outside the substrates 2, a pair of polarizers 8 are disposed so as to modulate incident light $I_0$ from a light source 9 in cooperation with the liquid crystal 1 to provide modulated light I.

Each of two glass substrates 2 is coated with a transparent electrode 3 comprising a film of $In_2O_3$, $SnO_2$ or ITO (indium-tin-oxide) to form an electrode plate. Further thereon, an insulating alignment control layer 4 is formed by rubbing a film of a polymer such as polyimide with gauze or acetate fiber-planted cloth so as to align the liquid crystal molecules in the rubbing direction. Further, it is also possible to compose the alignment control layer of two layers, e.g., by first forming an insulating layer of an inorganic material, such as silicon nitride, silicon nitride containing hydrogen, silicon carbide, silicon carbide containing hydrogen, silicon oxide, boron nitride, boron nitride containing hydrogen, cerium oxide, aluminum oxide, zirconium oxide, titanium oxide, or magnesium fluoride, and forming thereon an alignment control layer of an organic insulating material, such as polyvinyl alcohol, polyimide, polyamide-imide, polyester-imide, polyparaxylylene, polyester, polycarbonate, polyvinyl acetal, polyvinyl chloride, polyvinyl acetate, polyamide, polystyrene, cellulose resin, melamine resin, urea resin, acrylic resin, or photoresist resin. Alternatively, it is also possible to use a single layer of inorganic insulating alignment control layer or organic insulating alignment control layer. An inorganic insulating alignment control layer may be formed by vapor deposition, while an organic insulating alignment control layer may be formed by applying a selection of an organic insulating material or a precursor thereof in a concentration of 0.1 to 20 wt. %, preferably 0.2 - 10 wt. %, by spinner coating, dip coating, screen printing, spray coating or roller coating, followed by curing or hardening under prescribed hardening condition (e.g., by heating). The insulating alignment control layer may have a thickness of ordinarily 50 Å - 1 micron, preferably 100 - 3000 Å, further preferably 100 - 1000 Å. The two glass substrates 2 with transparent electrodes 3 (which may be inclusively referred to herein as "electrode plates") and further with insulating alignment control layers 4 thereof are held to have a prescribed (but arbitrary) gap with a spacer 5. For example, such a cell structure with a prescribed gap may be formed by sandwiching spacers of silica beads or alumina beads having a prescribed diameter with two glass plates, and then sealing the periphery thereof with, e.g., an epoxy adhesive. Alternatively, a polymer film or glass fiber may also be used as a spacer. Between the two glass plates, a ferroelectric liquid crystal is sealed up to provide a ferroelectric liquid crystal layer 1 in a thickness of generally 0.5 to 20 microns, preferably 1 to 5 microns.

The transparent electrodes 3 are connected to the external power supply 7 through the lead wires 6. Further, outside the glass substrates 2, polarizers 8 are applied. The device shown in Figure 1 is of a transmission type and is provided with a light source 9.

Figure 2 is a schematic illustration of a ferroelectric liquid crystal cell (device) for explaining operation thereof. Reference numerals 21a and 21b denote substrates (glass plates) on which a transparent electrode of, e.g., $In_2O_3$, $SnO_2$, ITO (indium-tin-oxide), etc., is disposed, respectively. A liquid crystal of an SmC*-phase (chiral smectic C phase) or SmH*-phase (chiral smectic H phase) in which liquid crystal molecular layers 22 are aligned perpendicular to surfaces of the glass plates is hermetically disposed therebetween. Full lines 23 show liquid crystal molecules. Each liquid crystal molecule 23 has a dipole moment ($P_\perp$) 24 in a direction perpendicular to the axis thereof. The liquid crystal molecules 23 continuously form a helical structure in the direction of extension of the substrates. When a voltage higher than a certain threshold level is applied between electrodes formed on the substrates 21a and 21b, a helical structure of the liquid crystal molecule 23 is unwound or released to change the alignment direction of respective liquid crystal molecules 23 so that the dipole moments ($P_\perp$) 24 are all directed in the direction of the electric field. The liquid

crystal molecules 23 have an elongated shape and show refractive anisotropy between the long axis and the short axis thereof. Accordingly, it is easily understood that when, for instance, polarizers arranged in a cross nicol relationship, i.e., with their polarizing directions crossing each other, are disposed on the upper and the lower surfaces of the glass plates, the liquid crystal cell thus arranged functions as a liquid crystal optical modulation device of which optical characteristics vary depending upon the polarity of an applied voltage.

Further, when the liquid crystal cell is made sufficiently thin (e.g., less than about 10 microns), the helical structure of the liquid crystal molecules is unwound to provide a non-helical structure even in the absence of an electric field, whereby the dipole moment assumes either of the two states, i.e., Pa in an upper direction 34a or Pb in a lower direction 34b as shown in Figure 3, thus providing a bistable condition. When an electric field Ea or Eb higher than a certain threshold level and different from each other in polarity as shown in Figure 3 is applied to a cell having the above-mentioned characteristics, the dipole moment is directed either in the upper direction 34a or in the lower direction 34b depending on the vector of the electric field Ea or Eb. In correspondence with this, the liquid crystal molecules are oriented in either of a first stable state 33a and a second stable state 33b.

When the above-mentioned ferroelectric liquid crystal is used as an optical modulation element, it is possible to obtain two advantages. First is that the response speed is quite fast. Second is that the orientation of the liquid crystal shows bistability. The second advantage will be further explained, e.g., with reference to Figure 3. When the electric field Ea is applied to the liquid crystal molecules, they are oriented in the first stable state 33a. This state is stably retained even if the electric field is removed. On the other hand, when the electric field Eb of which direction is opposite to that of the electric field Ea is applied thereto, the liquid crystal molecules are oriented to the second stable state 33b, whereby the directions of molecules are changed. This state is similarly stably retained even if the electric field is removed. Further, as long as the magnitude of the electric field Ea or Eb being applied is not above a certain threshold value, the liquid crystal molecules are placed in the respective orientation states.

When such a ferroelectric liquid crystal device comprising a ferroelectric liquid crystal composition as described above between a pair of electrode plates is constituted as a simple matrix display device, the device may be driven by a driving method as disclosed in Japanese Laid-Open Patent Applications (KOKAI) Nos. 193426/1984, 193427/1984, 156046/1985, 156047/1985, etc.

Hereinbelow, the present invention will be explained more specifically with reference to examples. It is however to be understood that the present invention is not restricted to these examples.

Synthesis Example 4

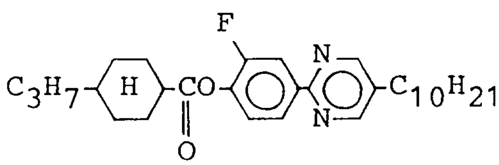

2-fluoro-4-(5-decyl-2-pyrimidinyl)phenyl trans-4-n-propylcyclohexylcarboxylate (Example Compound No. 1-2) represented by the above formula was synthesized in the following manner.

0.50 g (1.52 mM) of 2-fluoro-4-(5-decyl-2-pyrimidinyl)phenol was dissolved in 10 ml of pyridine and stirred on an iced water bath. To the solution, 0.34 g (1.82 mM) of trans-4-n-propylcyclohexanecarbonyl chloride was added dropwise, followed by stirring for 5 hours on the iced water bath. After the reaction, the reaction mixture was poured into water, acidified with conc. hydrochloric acid and extracted with methylene chloride. The organic layer was washed with water, followed by drying with sodium sulfate, distilling-off of the solvent, purification by silica gel column chromatography and recrystallization from ethanol/ethyl acetate mixture solvent to obtain 0.44 g (0.92 mM) of the objective compound (Yield: 60.3 %).

Phase transition temperature (° C)

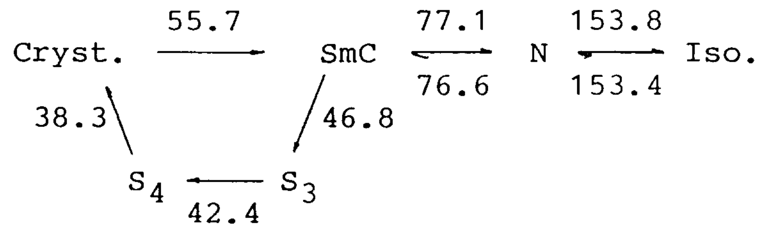


N: nematic phase.

Synthesis Example 5

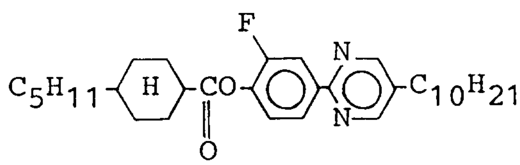

2-fluoro-4-(5-decyl-2-pyrimidinyl)phenyl trans-4-n-pentylcyclohexylcarboxylate (Example Compound No. 1-8) represented by the above formula was synthesized in the following manner.

0.39 g (1.80 mM) of trans-4-n-pentylcyclohexylcarbonyl chloride was added dropwise to 0.50 g (1.52 mM) of 2-fluoro-4-(5-decyl-2-pyrimidinyl)phenyl dissolved in 4 ml of pyridine on an iced bath. After the addition, the iced water bath was removed and the resultant mixture was stirred for 30 min. at room temperature, followed by further stirring for 2 hours at 40 - 50 ° C on a water bath. After the reaction, the reaction mixture was poured into 100 ml of iced water to precipitate a crystal. The crystal was recovered by filtration and dissolved in ethyl acetate, followed by washing with 2N-hydrochloric acid and water, drying with sodium sulfate and distilling-off of the solvent. The resultant crystal was purified by silica gel column chromatography (eluent: toluene) and recrystallized from ethanol in a freezer to obtain 0.45 g (0.88 mM) of the objective compound (Yield: 58.2 %).

Phase transition temperature (° C)

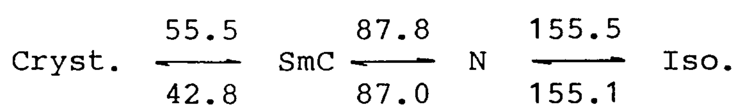


Example 1

A liquid crystal composition A was prepared by mixing the following compounds in respectively indicated proportions.

| Ex.Comp.No. | Structural formula | wt.parts |
| --- | --- | --- |
| 20 | 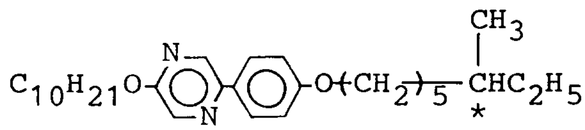 | 15 |
| 21 | 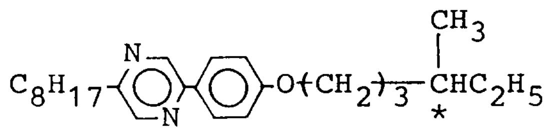 | 15 |
| 58 | 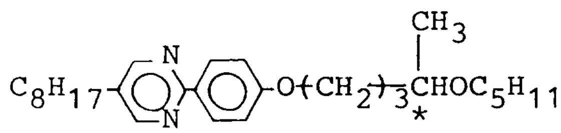 | 10 |
| 89 | 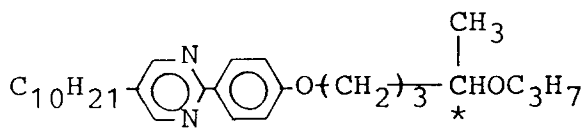 | 20 |
| 159 | 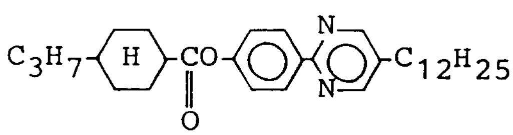 | 15 |
| 165 | 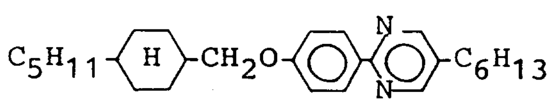 | 5 |
| 3-69 | 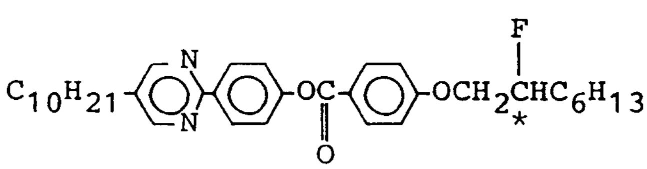 | 13 |
| 3-94 | 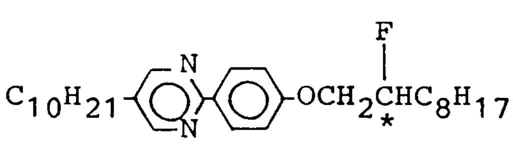 | 7 |

The liquid crystal composition A was further mixed with Example Compound No. 1-2 in the proportions indicated below to provide a liquid crystal composition B.

| Ex.Comp.No. | Structural formula | wt.parts |
| --- | --- | --- |
| 1-2 | 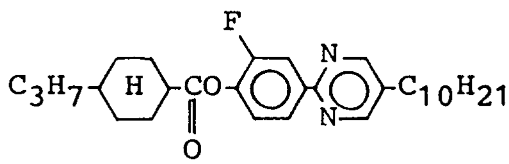 | 5 |
| | Composition A | 90 |

Two 0.7 mm-thick glass plates were provided and respectively coated with an ITO film to form an electrode for voltage application, which was further coated with an insulating layer of vapor-deposited $SiO_2$. On the insulating layer, a 0.2 %-solution of silane coupling agent (KBM-602, available from Shinetsu Kagaku K.K.) in isopropyl alcohol was applied by spinner coating at a speed of 2000 rpm for 15 second and subjected to hot curing treatment at 120 °C for 20 min.

Further, each glass plate provided with an ITO film and treated in the above described manner was coated with a 1.5 %-solution of polyimide resin precursor (SP-510, available from Toray K.K.) in dimethylacetoamide by a spinner coater rotating at 2000 rpm for 15 seconds. Thereafter, the coating film was subjected to heat curing at 300 °C for 60 min. to obtain about 250 Å-thick film. The coating film was rubbed with acetate fiber-planted cloth. The thus treated two glass plates were washed with isopropyl alcohol. After alumina beads with an average particle size of 2.0 microns were dispersed on one of the glass plates, the two glass plates were applied to each other with a bonding sealing agent (Lixon Bond, available from Chisso K.K.) so that their rubbed directions were parallel to each other and heated at 100 °C for 60 min. to form a blank cell. The cell gap was found to be about 2 microns as measured by a Berek compensator.

Then, the liquid crystal composition B was heated into an isotropic liquid, and injected into the above prepared cell under vacuum and, after sealing, was gradually cooled at a rate of 20 °C/hour to 25 °C to prepare a ferroelectric liquid crystal device.

The ferroelectric liquid crystal device was subjected to measurement of an optical response time (time from voltage application until the transmittance change reaches 90 % of the maximum under the application of a peak-to-peak voltage Vpp of 20 V in combination with right-angle-cross-nicol polarizers).

The results are shown below.

| | 15 °C | 25 °C | 35 °C |
| --- | --- | --- | --- |
| Response time (μsec) | 134 | 89 | 74 |

Further, when the device was driven and a clear switching action was observed, and good bistability was shown after the termination of the voltage application.

Comparative Example 1

A ferroelectric liquid crystal device was prepared in the same manner as in Example 1 except that the liquid crystal composition A prepared in Example 1 was injected into a cell. The measured values of the response time of the device were as follows.

| | 15 °C | 25 °C | 35 °C |
| --- | --- | --- | --- |
| Response time (μsec) | 155 | 100 | 80 |

As is understood from the comparison between Example 1 and Comparative Example 1, a ferroelectric liquid crystal device using the liquid crystal composition B containing both the compounds according to general formulas (I) and (III) showed an improved low-temperature operation characteristic, a high response speed and a decreased temperature-dependence of the response speed.

Example 2

A liquid crystal composition C was prepared in the same manner as in Example 1 except that the following Example Compounds were used instead of Example Compound No. 1-2 in respectively indicated proportions.

| Ex.Comp.No. | Structural formula | wt.parts |
| --- | --- | --- |
| 1-8 | 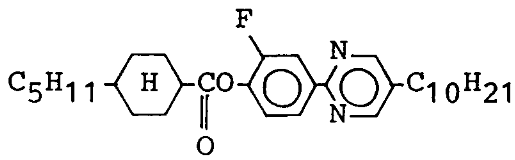 | 5 |
| 1-37 | 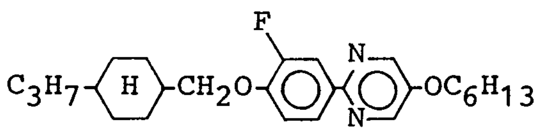 | 5 |
| | Composition A | 90 |

A ferroelectric liquid crystal device was prepared in the same manner as in Example 1 except for using the composition C. The ferroelectric liquid crystal device was subjected to measurement of response time and observation of a switching state, etc. in the same manner as in Example 1, whereby the following results were obtained.

| | 15 °C | 25 °C | 35 °C |
| --- | --- | --- | --- |
| Response time (μsec) | 132 | 88 | 72 |

Further, when the device was driven, a clear switching action was observed, and good bistability was shown after the termination of the voltage application.

As is understood from the comparison between Example 2 and Comparative Example 1, a ferroelectric liquid crystal device using the liquid crystal composition C containing both the compounds according to general formulas (I) and (III) showed an improved low-temperature operation characteristic, a high response speed and a decreased temperature-dependence of the response speed.

Example 3

A liquid crystal composition D was prepared in the same manner as in Example 1 except that the following Example Compounds were used instead of Example Compound No. 1-2 in respectively indicated proportions.

| Ex.Comp.No. | Structural formula | wt.parts |
|---|---|---|
| 1-17 | 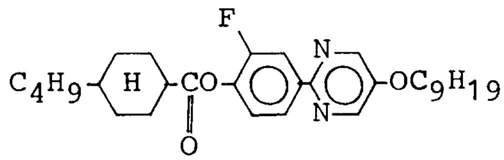 | 3 |
| 1-61 | 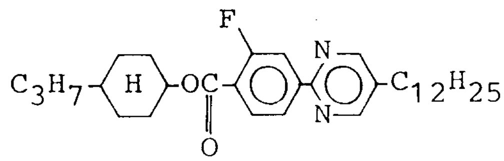 | 3 |
| 1-45 | 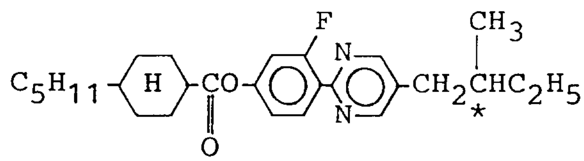 | 4 |
| | Composition A | 90 |

A ferroelectric liquid crystal device was prepared in the same manner as in Example 1 except for using the composition D. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 128 | 86 | 71 |

Further, when the device was driven, a clear switching action was observed, and good bistability was shown after the termination of the voltage application.

As is understood from the comparison between Example 3 and Comparative Example 1, a ferroelectric liquid crystal device using the liquid crystal composition D containing both the compounds according to general formulas (I) and (III) showed an improved low-temperature operation characteristic, a high response speed and a decreased temperature-dependence of the response speed.

Example 4

A liquid crystal composition E was prepared in the same manner as in Example 1 except that the following Example Compounds were used instead of Example Compound No. 1-2 in respectively indicated proportions.

| Ex.Comp.No. | Structural formula | wt.parts |
|---|---|---|
| 1-11 | 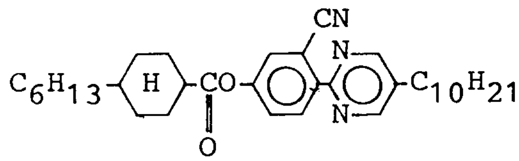 | 4 |
| 1-49 | 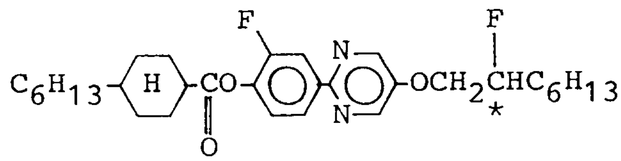 | 3 |
| 1-55 | 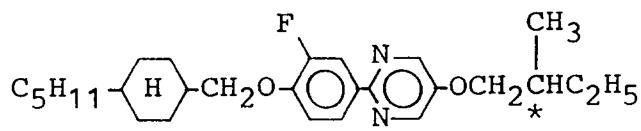 | 3 |
| | Composition A | 90 |

A ferroelectric liquid crystal device was prepared in the same manner as in Example 1 except for using the composition E. The ferroelectric liquid crystal device was subjected to measurement of response time and observation of a switching state, etc. in the same manner as in Example 1, whereby the following results were obtained.

| | 15°C | 25°C | 35°C |
|---|---|---|---|
| Response time (μsec) | 119 | 79 | 65 |

Further, when the device was driven, a clear switching action was observed, and good bistability was shown after the termination of the voltage application.

As is understood from the comparison between Example 4 and Comparative Example 1, a ferroelectric liquid crystal device using the liquid crystal composition E containing both the compounds according to general formulas (I) and (III) showed an improved low-temperature operation characteristic, a high response speed and a decreased temperature-dependence of the response speed.

Example 5

A liquid crystal composition F was prepared in the same manner as in Example 1 except that the following Example Compounds were used instead of Example Compound No. 1-2 in respectively indicated proportions.

| Ex.Comp.No. | Structural formula | wt.parts |
|---|---|---|
| 1-20 | 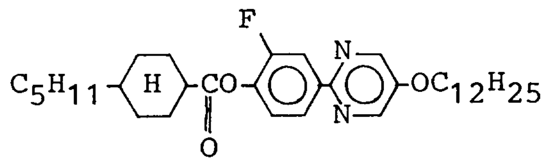 | 4 |
| 1-72 | 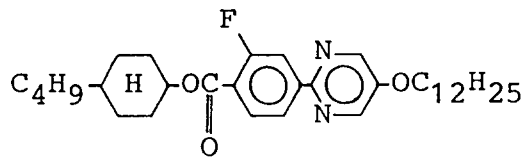 | 4 |
| 1-80 | 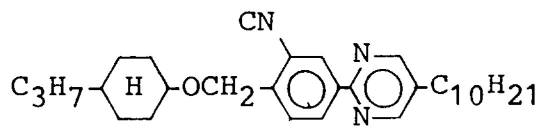 | 2 |
| | Composition A | 90 |

A ferroelectric liquid crystal device was prepared in the same manner as in Example 1 except for using the composition F. The ferroelectric liquid crystal device was subjected to measurement of response time and observation of a switching state, etc. in the same manner as in Example 1, whereby the following results were obtained.

| | 15°C | 25°C | 35°C |
|---|---|---|---|
| Response time (μsec) | 131 | 90 | 71 |

Further, when the device was driven, a clear switching action was observed, and good bistability was shown after the termination of the voltage application.

As is understood from the comparison between Example 5 and Comparative Example 1, a ferroelectric liquid crystal device using the liquid crystal composition F containing both the compounds according to general formulas (I) and (III) showed an improved low-temperature operation characteristic, a high response speed and a decreased temperature-dependence of the response speed.

Example 6

A liquid crystal composition G was prepared in the same manner as in Example 1 except that the following Example Compounds were used instead of Example Compound No. 1-2 in respectively indicated proportions.

| Ex.Comp.No. | Structural formula | wt.parts |
|---|---|---|
| 1-26 | 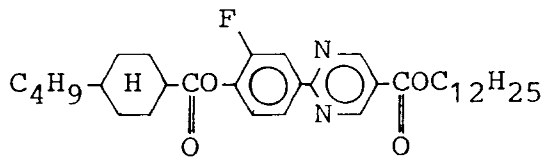 | 3 |
| 1-32 | 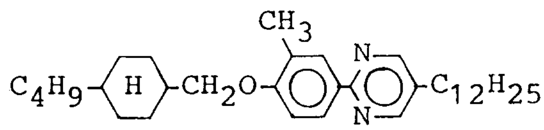 | 3 |
| 1-84 | 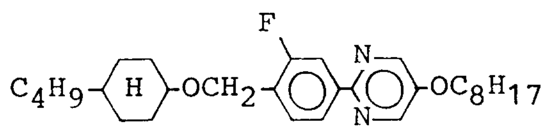 | 3 |
| | Composition A | 91 |

A ferroelectric liquid crystal device was prepared in the same manner as in Example 1 except for using the composition G. The ferroelectric liquid crystal device was subjected to measurement of response time and observation of a switching state, etc. in the same manner as in Example 1, whereby the following results were obtained.

| | 15°C | 25°C | 35°C |
|---|---|---|---|
| Response time (μsec) | 131 | 89 | 70 |

Further, when the device was driven, a clear switching action was observed, and good bistability was shown after the termination of the voltage application.

As is understood from the comparison between Example 6 and Comparative Example 1, a ferroelectric liquid crystal device using the liquid crystal composition G containing both the compounds according to general formulas (I) and (III) showed an improved low-temperature operation characteristic, a high response speed and a decreased temperature-dependence of the response speed.

Example 7

A liquid crystal composition H was prepared by mixing the following compounds in respectively indicated proportions.

| Ex.Comp.No. | Structural formula | wt.parts |
|---|---|---|
| 8 | 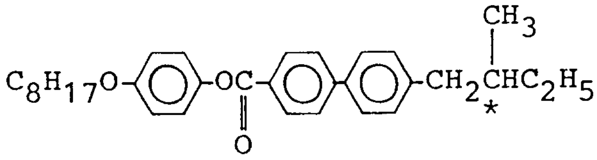 | 16 |
| 9 | 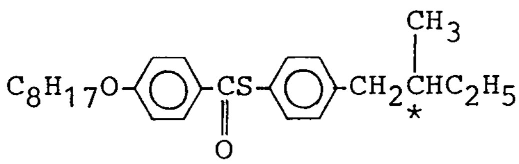 | 22.5 |
| 18 | 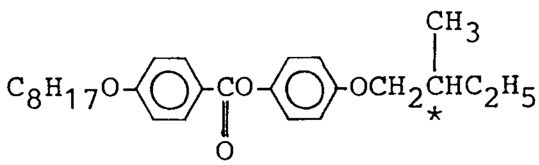 | 64 |
| 23 | 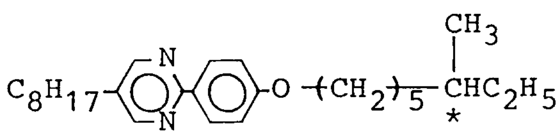 | 10 |
| 24 | 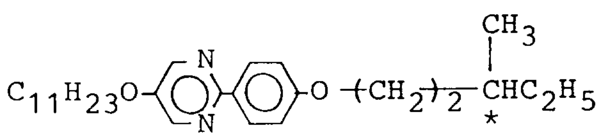 | 10 |

| | | |
|---|---|---|
| 43 | 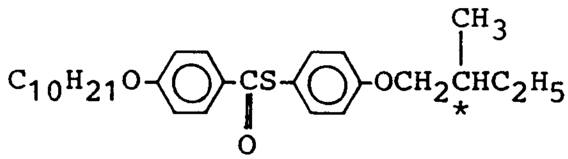 | 22.5 |
| 63 | 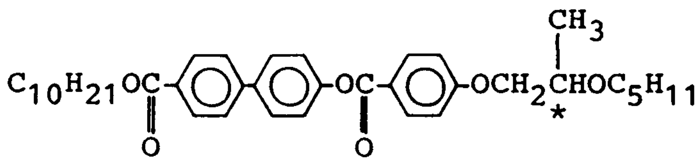 | 15 |
| 87 | 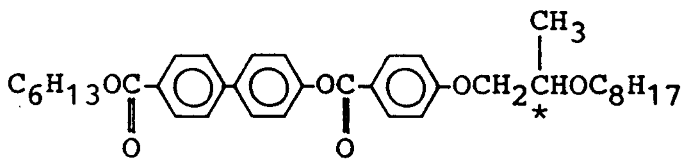 | 15 |
| 159 | 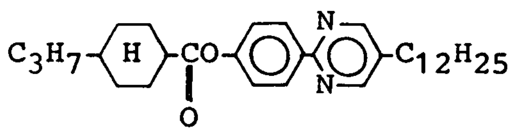 | 20 |
| 3-13 | 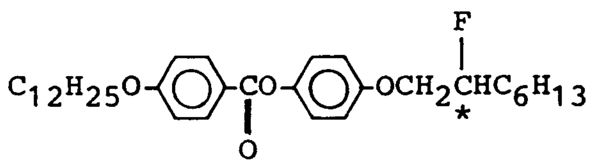 | 6.75 |
| 3-7 | 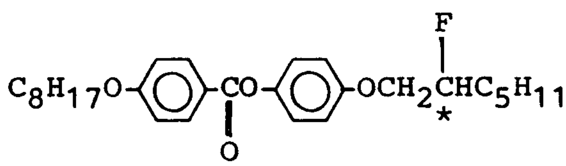 | 18.75 |

The liquid crystal composition H was further mixed with Example Compounds Nos. 1-2, 1-16 and 1-34 in the proportions indicated below to provide a liquid crystal composition I.

| Ex.Comp.No. | Structural formula | wt.parts |
|---|---|---|
| 1-2 | 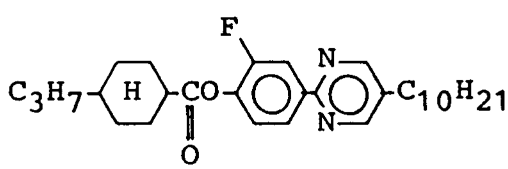 | 4 |

| | | |
|---|---|---|
| 1-16 | 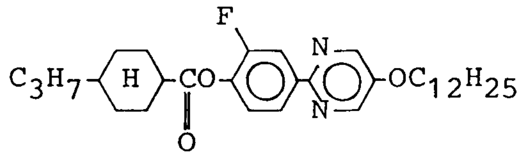 | 3 |
| 1-34 | 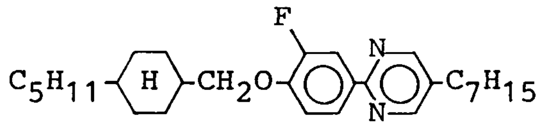 | 3 |
| | Composition H | 90 |

A ferroelectric liquid crystal device was prepared in the same manner as in Example 1 except for using the composition I. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 390 | 251 | 192 |

Comparative Example 2

A ferroelectric liquid crystal device was prepared in the same manner as in Example 1 except that the liquid crystal composition H prepared in Example 7 was injected into a cell. The measured values of the response time of the device were as follows.

| | 15 °C | 25 °C | 35 °C |
|---|---|---|---|
| Response time (μsec) | 450 | 270 | 195 |

As is understood from the comparison between Example 7 and Comparative Example 2, a ferroelectric liquid crystal device using the liquid crystal composition I containing both the compounds according to general formulas (I) and (III) showed an improved low-temperature operation characteristic, a high response speed and a decreased temperature-dependence of the response speed.

Example 8

A liquid crystal composition J was prepared in the same manner as in Example 7 except that the following Example Compounds were used instead of Example Compounds Nos. 1-2, 1-16 and 1-34 prepared in Example 7 in respectively indicated proportions.

| Ex.Comp.No. | Structural formula | wt.parts |
|---|---|---|
| 1-6 | 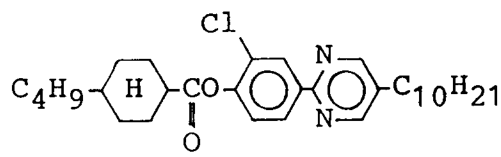 | 3 |
| 1-33 | 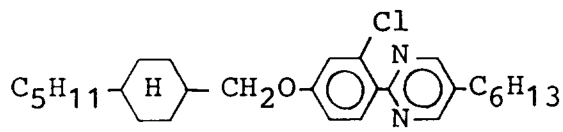 | 4 |
| 1-64 | 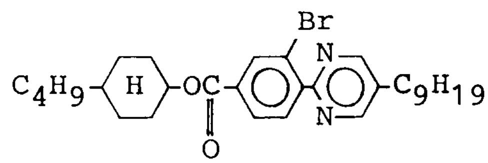 | 3 |
| | Composition H | 90 |

A ferroelectric liquid crystal device was prepared in the same manner as in Example 1 except for using the composition J. In the ferroelectric liquid crystal device, a monodomain with a good and uniform alignment characteristic was observed. The ferroelectric liquid crystal device was subjected to measurement of response time and observation of a switching state, etc. in the same manner as in Example 3, whereby the following results were obtained.

| | 15 °C | 25 °C | 35 °C |
|---|---|---|---|
| Response time (μsec) | 395 | 242 | 183 |

Further, when the device was driven, a clear switching action was observed, and good bistability was shown after the termination of the voltage application.

As is understood from the comparison between Example 8 and Comparative Example 2, a ferroelectric liquid crystal device using the liquid crystal composition J containing both the compounds according to general formulas (I) and (III) showed an improved low-temperature operation characteristic, a high response speed and a decreased temperature-dependence of the response speed.

Example 9

A liquid crystal composition K was prepared in the same manner as in Example 7 except that the following Example Compounds were used instead of Example Compounds Nos. 1-2, 1-16 and 1-34 prepared in Example 7 in respectively indicated proportions.

| Ex.Comp.No. | Structural formula | wt.parts |
|---|---|---|
| 1-48 | 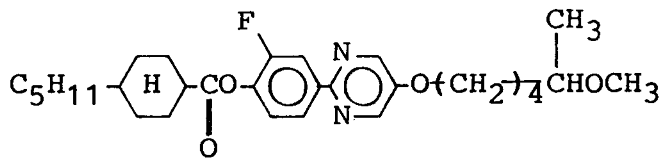 | 3 |
| 1-53 | 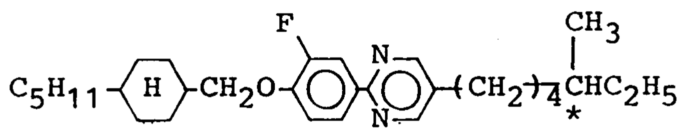 | 3 |
| 1-71 | 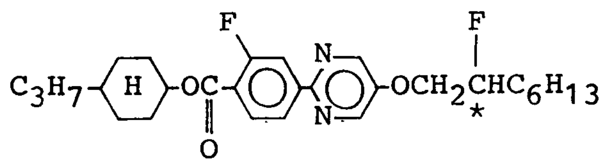 | 4 |
| | Composition H | 90 |

A ferroelectric liquid crystal device was prepared in the same manner as in Example 1 except for using the composition K. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15°C | 25°C | 35°C |
|---|---|---|---|
| Response time (μsec) | 388 | 241 | 185 |

Further, when the device was driven, a clear switching action was observed, and good bistability was shown after the termination of the voltage application.

As is understood from the comparison between Example 9 and Comparative Example 2, a ferroelectric liquid crystal device using the liquid crystal composition K containing both the compounds according to general formulas (I) and (III) showed an improved low-temperature operation characteristic, a high response speed and a decreased temperature-dependence of the response speed.

Example 10

A liquid crystal composition L was prepared in the same manner as in Example 7 except that the following Example Compounds were used instead of Example Compounds Nos. 1-2, 1-16 and 1-34 prepared in Example 7 in respectively indicated proportions.

| Ex.Comp.No. | Structural formula | wt.parts |
|---|---|---|
| 1-4 | 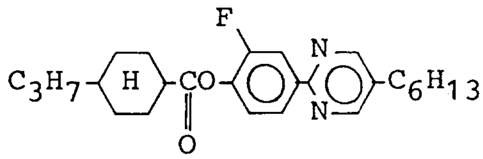 | 2 |
| 1-8 | 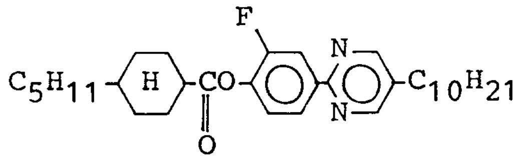 | 2 |
| 1-49 | 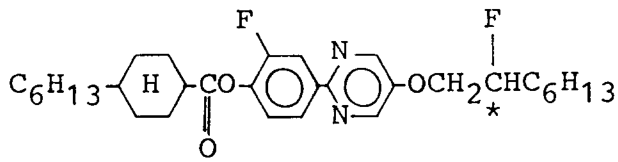 | 3 |
| 1-63 | 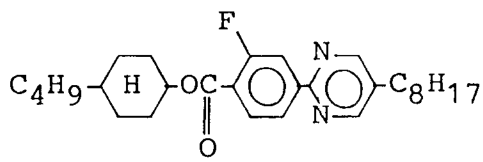 | 2 |
| 1-86 | 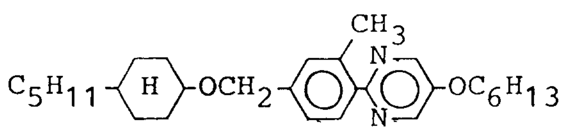 | 2 |
| | Composition H | 89 |

A ferroelectric liquid crystal device was prepared in the same manner as in Example 1 except for using the composition L. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 366 | 244 | 187 |

Further, when the device was driven, a clear switching action was observed, and good bistability was shown after the termination of the voltage application.

As is understood from the comparison between Example 10 and Comparative Example 2, a ferroelectric liquid crystal device using the liquid crystal composition L containing both the compounds according to general formulas (I) and (III) showed an improved low-temperature operation characteristic, a high response speed and a decreased temperature-dependence of the response speed.

Example 11

A liquid crystal composition M was prepared in the same manner as in Example 7 except that the following Example Compounds were used instead of Example Compounds Nos. 1-2, 1-16 and 1-34 prepared in Example 7 in respectively indicated proportions.

| Ex.Comp.No. | Structural formula | wt.parts |
| --- | --- | --- |
| 1-1 | 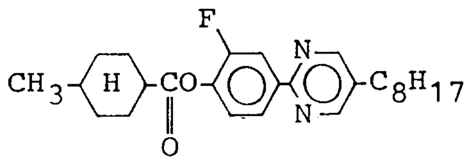 | 4 |
| 1-41 | 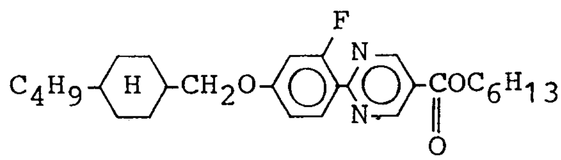 | 2 |
| 1-57 | 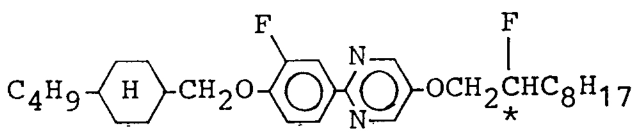 | 3 |
| 1-66 | 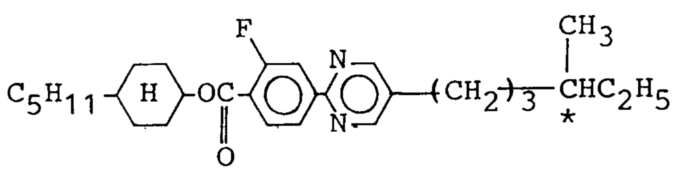 | 2 |
| | Composition H | 89 |

A ferroelectric liquid crystal device was prepared in the same manner as in Example 1 except for using the composition M. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15°C | 25°C | 35°C |
| --- | --- | --- | --- |
| Response time (μsec) | 374 | 247 | 190 |

Further, when the device was driven, a clear switching action was observed, and good bistability was shown after the termination of the voltage application.

As is understood from the comparison between Example 11 and Comparative Example 2, a ferroelectric liquid crystal device using the liquid crystal composition M containing both the compounds according to general formulas (I) and (III) showed an improved low-temperature operation characteristic, a high response speed and a decreased temperature-dependence of the response speed.

Example 12

A liquid crystal composition N was prepared in the same manner as in Example 7 except that the following Example Compounds were used instead of Example Compounds Nos. 1-2, 1-16 and 1-34 prepared in Example 7 in respectively indicated proportions.

| Ex.Comp.No. | Structural formula | wt.parts |
|---|---|---|
| 1-2 | 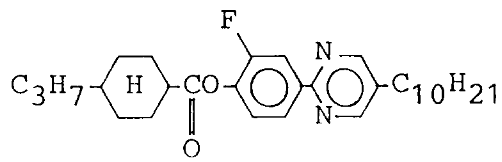 | 4 |
| 1-7 | 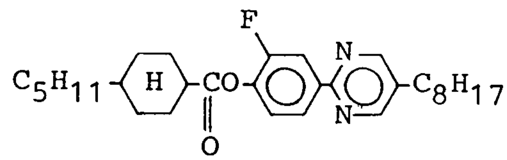 | 2 |
| 1-38 | 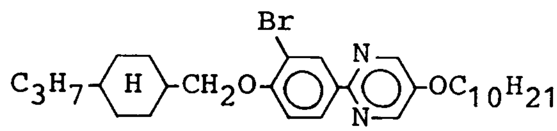 | 2 |
| 1-54 | 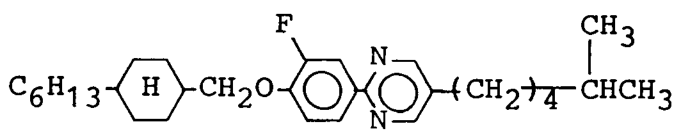 | 2 |
| 1-87 | 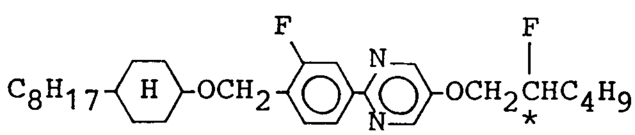 | 3 |
| | Composition H | 87 |

A ferroelectric liquid crystal device was prepared in the same manner as in Example 1 except for using the composition N. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15°C | 25°C | 35°C |
|---|---|---|---|
| Response time (μsec) | 364 | 244 | 186 |

Further, when the device was driven, a clear switching action was observed, and good bistability was shown after the termination of the voltage application.

As is understood from the comparison between Example 12 and Comparative Example 2, a ferroelectric liquid crystal device using the liquid crystal composition N containing both the compounds according to general formulas (I) and (III) showed an improved low-temperature operation characteristic, a high response

speed and a decreased temperature-dependence of the response speed.

Example 13

A liquid crystal composition O was prepared in the same manner as in Example 7 except that the following Example Compounds were used instead of Example Compounds Nos. 1-2, 1-16 and 1-34 prepared in Example 7 in respectively indicated proportions.

| Ex.Comp.No. | Structural formula | wt.parts |
|---|---|---|
| 1-49 | 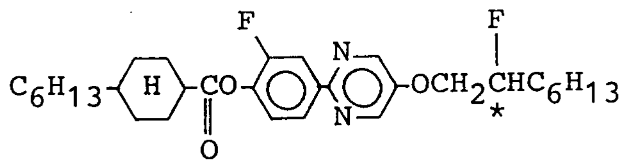 | 4 |
| 1-71 | 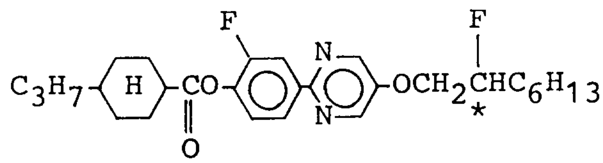 | 4 |
| | Composition H | 92 |

A ferroelectric liquid crystal device was prepared in the same manner as in Example 1 except for using the composition O. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15°C | 25°C | 35°C |
|---|---|---|---|
| Response time (μsec) | 315 | 203 | 152 |

Further, when the device was driven, a clear switching action was observed, and good bistability was shown after the termination of the voltage application.

As is understood from the comparison between Example 13 and Comparative Example 2, a ferroelectric liquid crystal device using the liquid crystal composition O containing both the compounds according to general formulas (I) and (III) showed an improved low-temperature operation characteristic, a high response speed and a decreased temperature-dependence of the response speed.

Example 14

A liquid crystal composition P was prepared in the same manner as in Example 7 except that the following Example Compounds were used instead of Example Compounds Nos. 1-2, 1-16 and 1-34 prepared in Example 7 in respectively indicated proportions.

| Ex.Comp.No. | Structural formula | wt.parts |
|---|---|---|
| 1-2 | 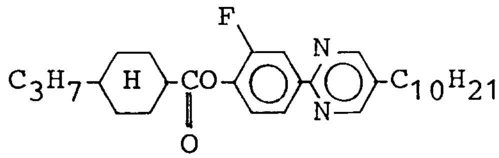 | 3 |
| 1-21 | 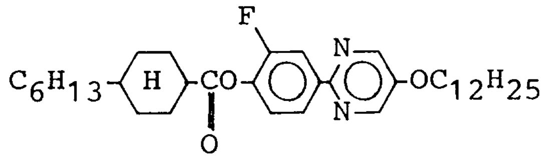 | 3 |
| 1-90 | 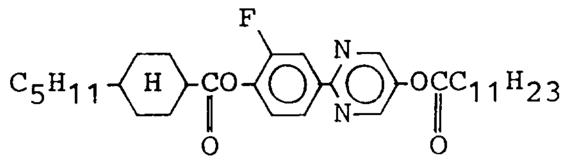 | 2 |
| 1-94 | 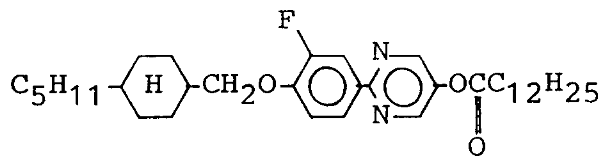 | 2 |
| | Composition H | 90 |

A ferroelectric liquid crystal device was prepared in the same manner as in Example 1 except for using the composition P. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 374 | 246 | 189 |

Further, when the device was driven, a clear switching action was observed, and good bistability was shown after the termination of the voltage application.

As is understood from the comparison between Example 14 and Comparative Example 2, a ferroelectric liquid crystal device using the liquid crystal composition P containing both the compounds according to general formulas (I) and (III) showed an improved low-temperature operation characteristic, a high response speed and a decreased temperature-dependence of the response speed.

Example

A liquid crystal composition Q was prepared in the same manner as in Example 7 except that the following Example Compounds were used instead of Example Compounds Nos. 1-2, 1-16 and 1-34 prepared in Example 7 in respectively indicated proportions.

| Ex.Comp.No. | Structural formula | wt.parts |
|---|---|---|
| 1-8 | 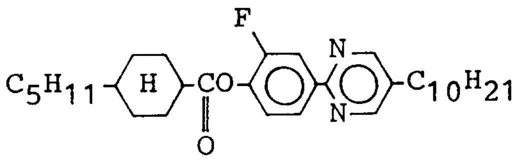 | 3 |
| 1-82 | 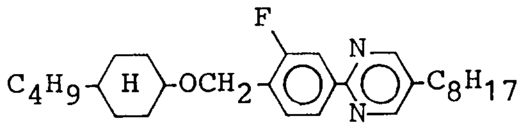 | 4 |
| 1-96 | 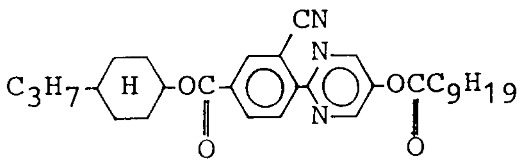 | 2 |
| 1-101 | 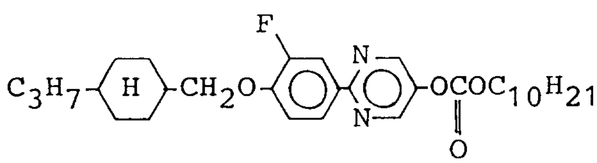 | 1 |
| | Composition H | 90 |

A ferroelectric liquid crystal device was prepared in the same manner as in Example 1 except for using the composition Q. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15°C | 25°C | 35°C |
|---|---|---|---|
| Response time (μsec) | 366 | 243 | 184 |

Further, when the device was driven, a clear switching action was observed, and good bistability was shown after the termination of the voltage application.

As is understood from the comparison between Example 15 and Comparative Example 2, a ferroelectric liquid crystal device using the liquid crystal composition Q containing both the compounds according to general formulas (I) and (III) showed an improved low-temperature operation characteristic, a high response speed and a decreased temperature-dependence of the response speed.

Example 16

A blank cell was prepared in the same manner as in Example 1 by using a 2 % aqueous solution of polyvinyl alcohol resin (PVA-117, available from Kuraray K.K.) instead of the 1.5 %-solution of polyimide resin precursor in dimethylacetoamide on each electrode plate. A ferroelectric liquid crystal device was prepared by filling the blank cell with the liquid crystal composition B prepared in Example 1. The liquid crystal device was subjected to measurement of optical response time in the same manner as in Example 1. The results are shown below.

| | 15 °C | 25 °C | 35 °C |
|---|---|---|---|
| Response time (μsec) | 128 | 85 | 70 |

Example 17

A blank cell was prepared in the same manner as in Example 3 except for omitting the $SiO_2$ layer to form an alignment control layer composed of the polyimide resin layer alone on each electrode plate. A ferroelectric liquid crystal device was prepared by filling the blank cell with the liquid crystal composition B prepared in Example 1. The liquid crystal device was subjected to measurement of optical response time in the same manner as in Example 1. The results are shown below.

| | 15 °C | 25 °C | 35 °C |
|---|---|---|---|
| Response time (μsec) | 125 | 83 | 68 |

As is apparent from the above Examples 16 and 17, also in the cases of different device structures, the devices containing the ferroelectric liquid crystal composition B according to the present invention respectively provided a remarkably improved operation characteristic at a lower temperature and also a decreased temperature-dependence of the response speed.

Synthesis Examples 6 - 8

2-fluoro-4-(5-octyl-2-pyrimidinyl)phenyl trans-4-n-propylcyclohexylcarboxylate (Synthesis Example 6, Example Compound No. 1-104), 2-fluoro-4-(5-undecyl-2-pyrimidinyl)phenyl trans-4-n-propylcyclohexylcarboxylate (Synthesis Example 7, Example Compound No. 1-3) and 2-fluoro-4-(5-undecyl-2-pyrimidinyl)phenyl trans-4-n-pentylcyclohexylcarboxylate (Synthesis Example 7, Example Compound No. 1-107) were synthesized similarly as in Synthesis Example 4 or Synthesis Example 5. Each Example Compound showed the following phase transition series, respectively.

Synthesis Example 6

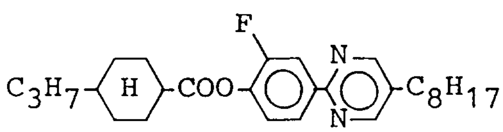

(Example Compound No. 1-4)

Phase transition temperature (°C)

$$\text{Cryst.}\ \underset{43.6}{\overset{65.0}{\rightleftharpoons}}\ \text{N}\ \underset{163.6}{\overset{164.6}{\rightleftharpoons}}\ \text{Iso.}$$

Synthesis Example 7

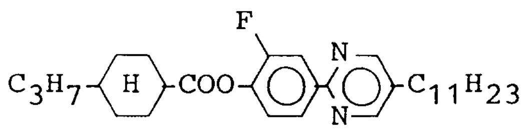

(Example Compound No. 1-3)

Phase transition temperature (°C)

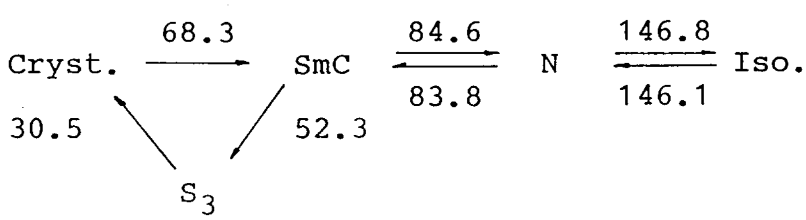


Synthesis Example 8

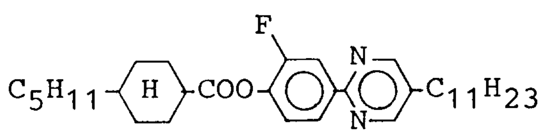

(Example Compound No. 1-107)

Phase transition temperature (°C)

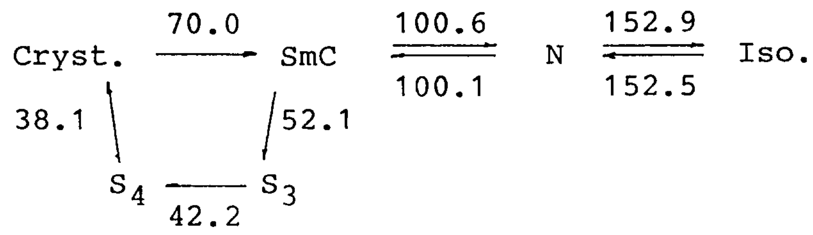


Example 18

A liquid crystal composition R was prepared by mixing the following compounds in respectively indicated proportions.

| Ex.Comp.No. | Structural formula | wt.parts |
|---|---|---|
| 20 | 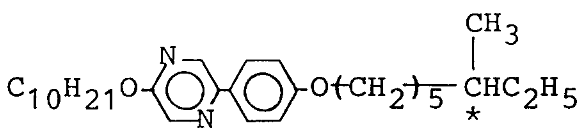 | 15 |
| 21 | 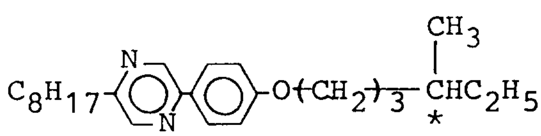 | 15 |
| 58 | 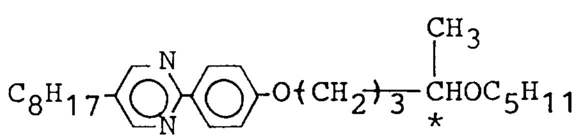 | 10 |
| 89 | 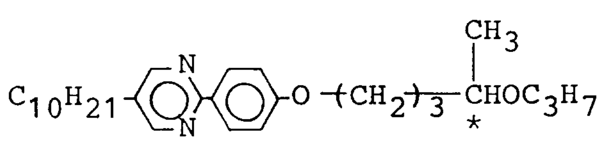 | 20 |
| 159 | 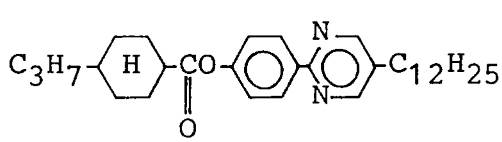 | 15 |
| 165 | 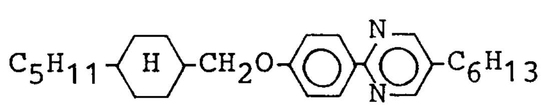 | 5 |

The liquid crystal composition R was further mixed with the following Example Compounds in the proportions indicated below to provide a liquid crystal composition 18-R.

| Ex.Comp.No. | Structural formula | wt.parts |
|---|---|---|
| 1-2 | 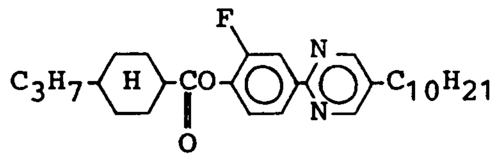 | 3.5 |
| 1-109 | 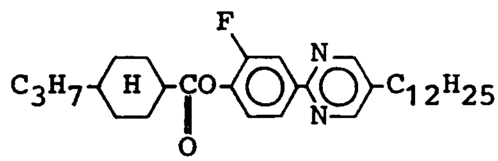 | 1.5 |
| 3-69 | 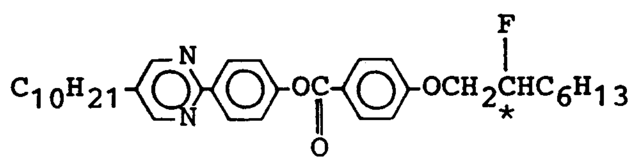 | 12.0 |
| 3-94 | 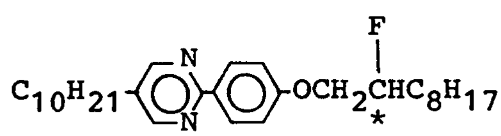 | 7.0 |
| 2-16 | 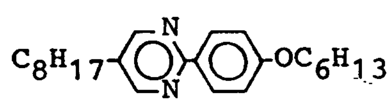 | 6.5 |
| 2-24 | 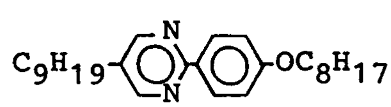 | 6.5 |
| 2-54 | 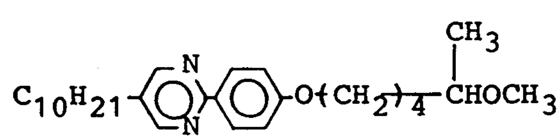 | 3.5 |
| 2-67 | 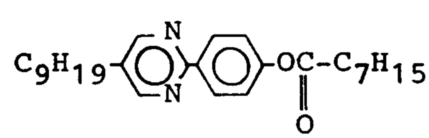 | 3.5 |
| | Composition R | 56.0 |

A ferroelectric liquid crystal device was prepared in the same manner as in Example 1 except for using the composition 18-R. In the ferroelectric liquid crystal device, a monodomain with a good and uniform

alignment characteristic was observed. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 115 | 80 | 68 |

Comparative Example 3

A liquid crystal composition 18-Rb was prepared by omitting only Example Compounds Nos. 1-2 and 1-109 from the liquid crystal composition 18-R prepared in Example 18, otherwise in the same manner as in Example 18.

A ferroelectric liquid crystal device was prepared in the same manner as in Example 1 except that the liquid crystal composition 18-Rb was injected into a cell. The measured values of the response time of the device were as follows.

| | 15 °C | 25 °C | 35 °C |
|---|---|---|---|
| Response time (μsec) | 132 | 87 | 71 |

As is understood from the comparison between Example 18 and Comparative Example 3, a ferroelectric liquid crystal device using the liquid crystal composition 18-R containing the mesomorphic compounds according to the present invention showed an improved low-temperature operation characteristic, a high response speed and a decreased temperature-dependence of the response speed.

Example 19

A liquid crystal composition 19-R was prepared in the same manner as in Example 18 except that the following Example Compounds were used instead of Example Compounds Nos. 1-2, 1-109, 2-16, 2-24, 2-54, 2-67, 3-69 and 3-94 prepared in Example 18 in respectively indicated proportions.

| Ex.Comp.No. | Structural formula | wt.parts |
|---|---|---|
| 1-11 | 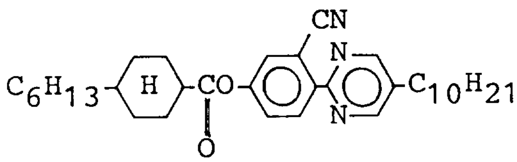 | 4 |
| 1-49 | 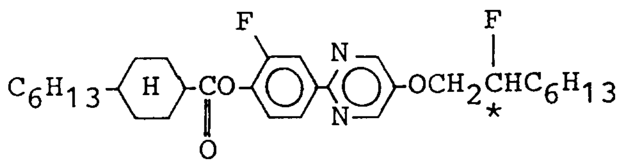 | 3 |
| 1-55 | 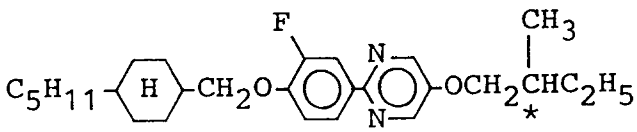 | 3 |
| 2-9 | 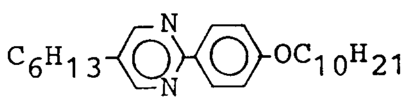 | 3 |
| 2-17 | 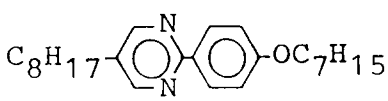 | 12 |

| | | |
|---|---|---|
| 2-48 | 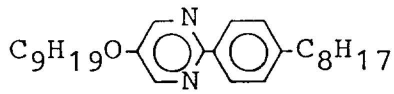 | 3 |
| 2-55 | 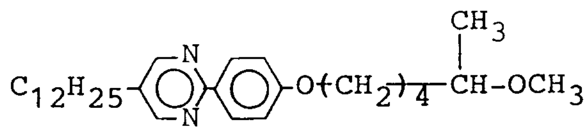 | 7 |
| 2-86 | 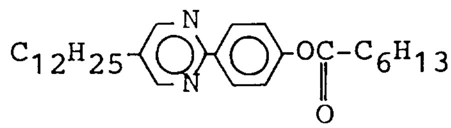 | 3 |
| 3-75 | 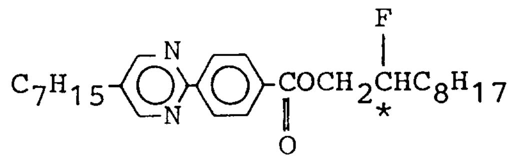 | 8.5 |
| 3-28 | 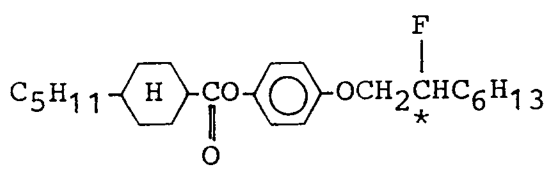 | 10.5 |
| | Composition R | 43 |

A ferroelectric liquid crystal device was prepared in the same manner as in Example 1 except for using the composition 19-R. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 100 | 72 | 58 |

Comparative Example 4

A liquid crystal composition 19-Rb was prepared by omitting only Example Compounds Nos. 1-11, 1-49 and 1-55 from the liquid crystal composition 19-R prepared in Example 19, otherwise in the same manner as in Example 19.

A ferroelectric liquid crystal device was prepared in the same manner as in Example 1 except that the liquid crystal composition 19-Rb was injected into a cell. The measured values of the response time of the device were as follows.

| | 15 °C | 25 °C | 35 °C |
|---|---|---|---|
| Response time (μsec) | 115 | 76 | 60 |

As is understood from the comparison between Example 19 and Comparative Example 4, a ferroelectric liquid crystal device using the liquid crystal composition 19-R containing the mesomorphic compounds

according to the present invention showed an improved low-temperature operation characteristic, a high response speed and a decreased temperature-dependence of the response speed.

Example 20

A liquid crystal composition 20-R was prepared in the same manner as in Example 18 except that the following Example Compounds were used instead of Example Compounds Nos. 1-2, 1-109, 2-16, 2-24, 2-54, 2-67, 3-69 and 3-94 prepared in Example 18 in respectively indicated proportions.

| Ex.Comp.No. | Structural formula | wt.parts |
|---|---|---|
| 1-20 | 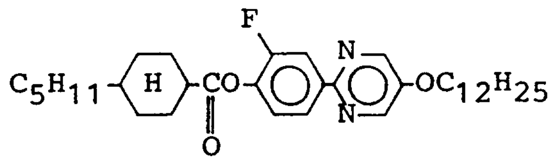 | 4 |
| 1-72 | 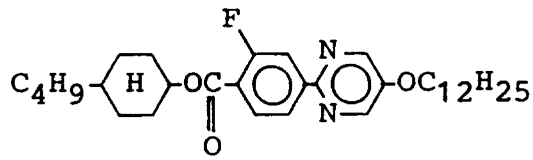 | 4 |
| 1-80 | 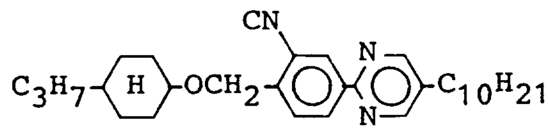 | 2 |
| 2-18 | 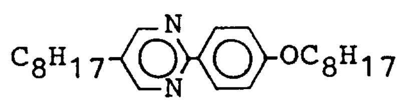 | 10 |
| 2-22 | 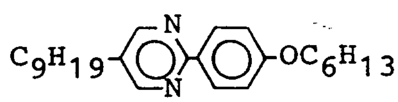 | 3 |
| 2-31 | 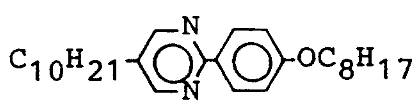 | 6 |
| 2-56 | 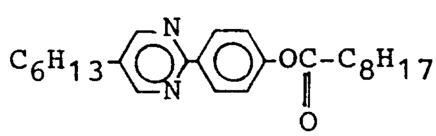 | 3 |
| 2-90 | 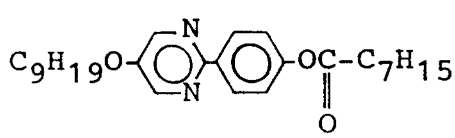 | 3 |

| | | |
|---|---|---|
| 3-48 | 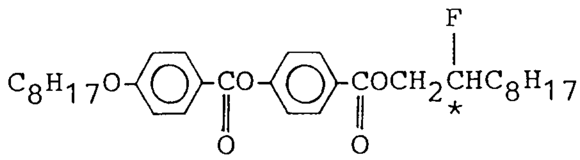 | 8 |
| 3-109 | 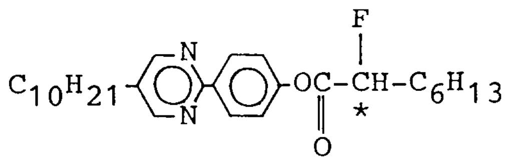 | 11 |
| | Composition R | 46 |

A ferroelectric liquid crystal device was prepared in the same manner as in Example 1 except for using the composition 20-R. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 112 | 76 | 65 |

Comparative Example 5

A liquid crystal composition 20-Rb was prepared by omitting only Example Compounds Nos. 1-20, 1-72 and 1-80 from the liquid crystal composition 20-R prepared in Example 20, otherwise in the same manner as in Example 20.

A ferroelectric liquid crystal device was prepared in the same manner as in Example 1 except that the liquid crystal composition 20-Rb was injected into a cell. The measured values of the response time of the device were as follows.

| | 15 °C | 25 °C | 35 °C |
|---|---|---|---|
| Response time (μsec) | 130 | 89 | 68 |

As is understood from the comparison between Example 20 and Comparative Example 5, a ferroelectric liquid crystal device using the liquid crystal composition 29-R containing the mesomorphic compound according to the present invention showed an improved low-temperature operation characteristic, a high response speed and a decreased temperature-dependence of the response speed.

Example 21

A liquid crystal composition S was prepared by mixing the following compounds in respectively indicated proportions.

| Ex.Comp.No. | Structural formula | wt.parts |
| --- | --- | --- |
| 8 | 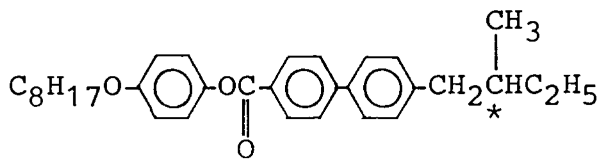 | 16 |
| 9 | 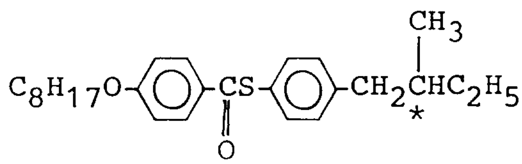 | 22.5 |
| 18 | 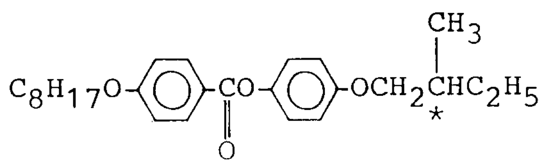 | 64 |
| 23 | 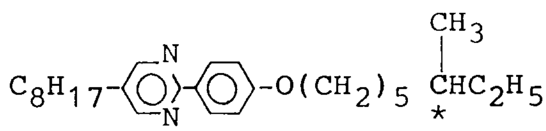 | 10 |
| 24 | 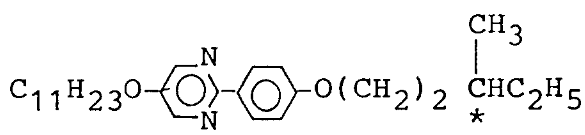 | 10 |
| 43 | 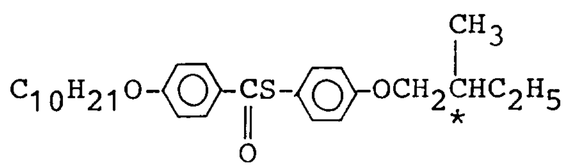 | 22.5 |
| 63 | 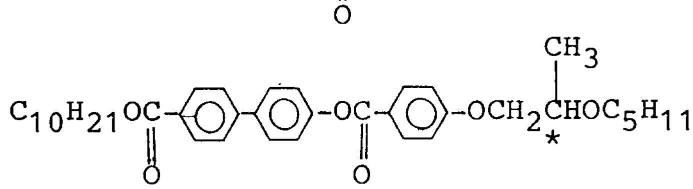 | 15 |
| 87 | 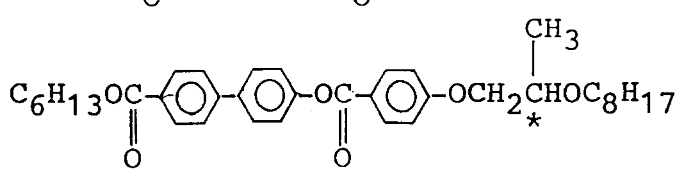 | 15 |
| 159 | 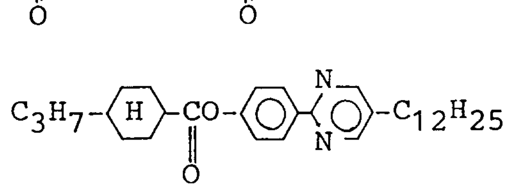 | 20 |

The liquid crystal composition S was further mixed with the following Example Compounds in the proportions indicated below to provide a liquid crystal composition 21-S.

| Ex.Comp.No. | Structural formula | wt.parts |
| --- | --- | --- |
| 1-2 | 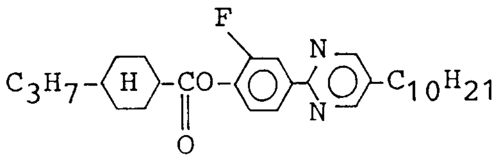 | 2 |
| 1-9 | 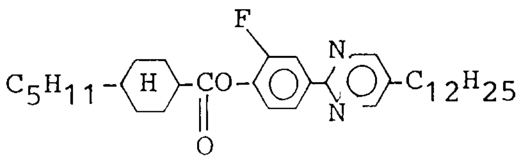 | 2 |

| | | |
|---|---|---|
| 1-16 | 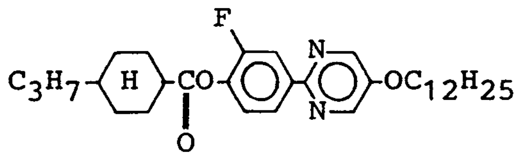 | 3 |
| 1-34 | 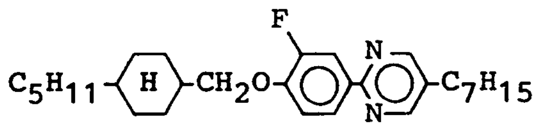 | 6.5 |
| 2-15 | 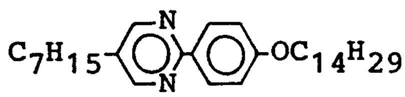 | 5 |
| 2-19 | 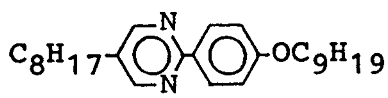 | 10 |
| 2-25 | 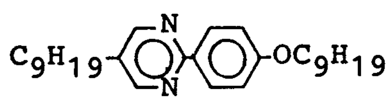 | 5 |
| 2-29 | 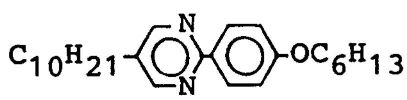 | 5 |
| 3-13 | 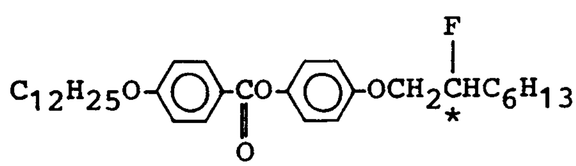 | 7 |
| 3-7 | 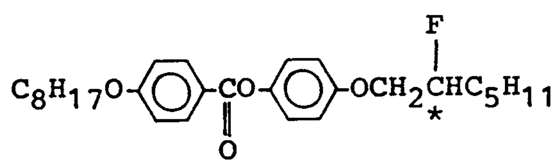 | 6 |
| 3-92 | 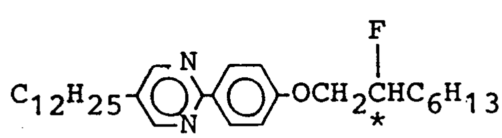 | 10 |
| | Composition S | 42 |

A ferroelectric liquid crystal device was prepared in the same manner as in Example 1 except for using the composition 21-S. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 335 | 215 | 178 |

Comparative Example 6

A liquid crystal composition 21-Sb was prepared by omitting only Example Compounds Nos. 1-2, 1-9, 1-16 and 1-34 from the liquid crystal composition 30-S prepared in Example 21, otherwise in the same manner as in Example 21.

A ferroelectric liquid crystal device was prepared in the same manner as in Example 1 except that the liquid crystal composition 21-Sb was injected into a cell. The measured values of the response time of the device were as follows.

| | 15 °C | 25 °C | 35 °C |
|---|---|---|---|
| Response time (μsec) | 385 | 231 | 180 |

As is understood from the comparison between Example 21 and Comparative Example 6, a ferroelectric liquid crystal device using the liquid crystal composition 30-S containing the mesomorphic compounds according to the present invention showed an improved low-temperature operation characteristic, a high response speed and a decreased temperature-dependence of the response speed.

Example 22

A liquid crystal composition 22-S was prepared in the same manner as in Example 21 except that the following Example Compounds were used instead of Example Compounds Nos. 1-2, 1-9, 1-16, 1-34, 2-15, 2-19, 2-25, 2-29, 3-7, 3-13 and 3-92 prepared in Example 21 in respectively indicated proportions.

| Ex.Comp.No. | Structural formula | wt.parts |
|---|---|---|
| 1-4 | 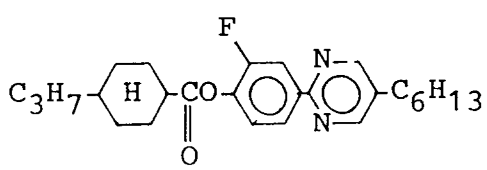 | 2 |
| 1-8 | 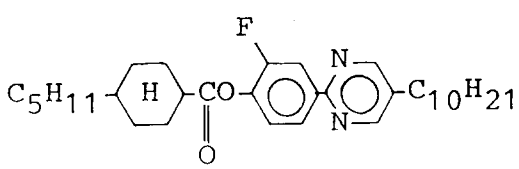 | 2 |

1-49 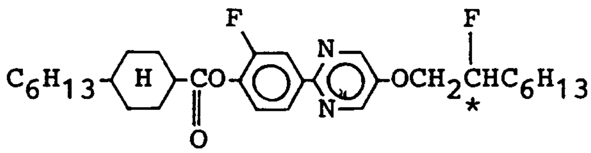 3

1-63 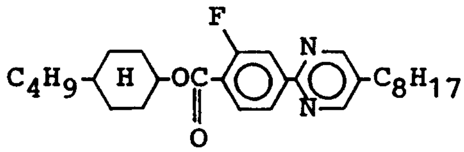 2

1-86 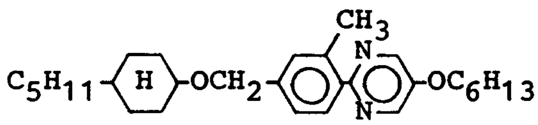 2

2-1 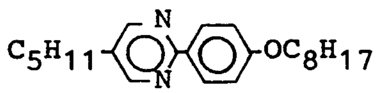 3

2-16 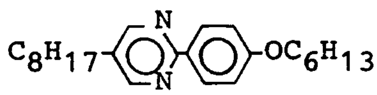 4

2-20 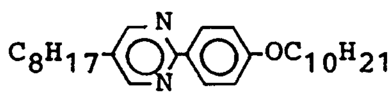 4

2-41 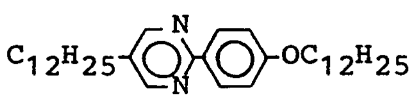 6

2-61 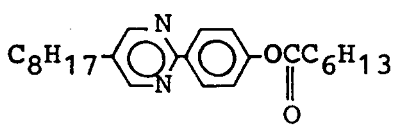 2

2-70 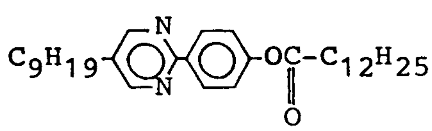 2

| | | |
|---|---|---|
| 2-92 | 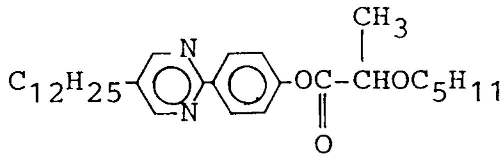 | 2 |
| 3-18 | 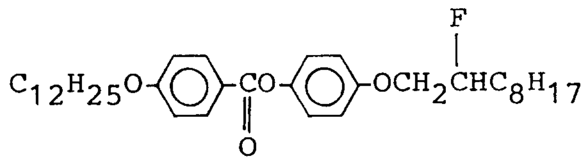 | 8 |
| 3-60 | 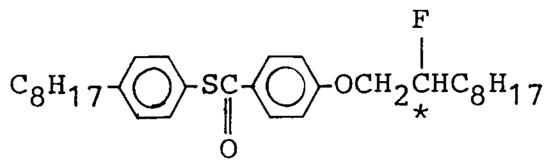 | 6 |
| 3-83 | 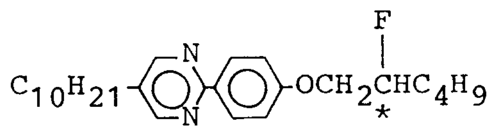 | 9 |
| | Composition S | 43 |

A ferroelectric liquid crystal device was prepared in the same manner as in Example 1 except for using the composition 22-S. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 313 | 205 | 172 |

Comparative Example 7

A liquid crystal composition 22-Sb was prepared by omitting only Example Compounds Nos. 1-4, 1-8, 1-49, 1-63 and 1-86 from the liquid crystal composition 22-S prepared in Example 22, otherwise in the same manner as in Example 22.

A ferroelectric liquid crystal device was prepared in the same manner as in Example 1 except that the liquid crystal composition 22-Sb was injected into a cell. The measured values of the response time of the device were as follows.

| | 15 °C | 25 °C | 35 °C |
|---|---|---|---|
| Response time (μsec) | 362 | 233 | 178 |

As is understood from the comparison between Example 22 and Comparative Example 7, a ferroelectric liquid crystal device using the liquid crystal composition 22-S containing the mesomorphic compounds according to the present invention showed an improved low-temperature operation characteristic, a high response speed and a decreased temperature-dependence of the response speed.

Example 23

A liquid crystal composition 23-S was prepared in the same manner as in Example 21 except that the following Example Compounds were used instead of Example Compounds Nos. 1-2, 1-9, 1-16, 1-34, 2-15, 2-19, 2-25, 2-29, 3-7, 3-13 and 3-92 prepared in Example 21 in respectively indicated proportions.

| Ex.Comp.No. | Structural formula | wt.parts |
|---|---|---|
| 1-49 | 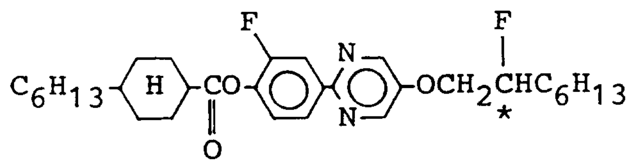 | 4 |
| 1-71 | 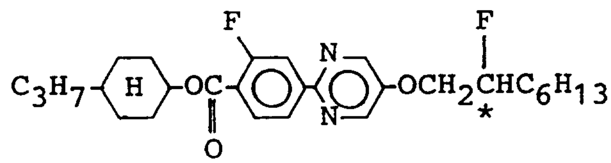 | 4 |
| 2-6 | 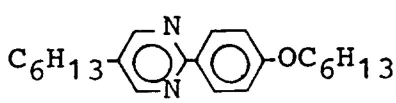 | 4 |
| 2-17 | 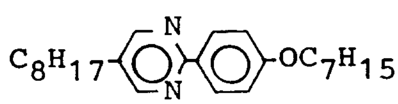 | 10 |
| 2-33 | 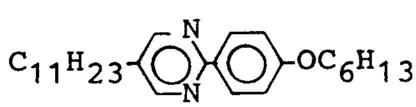 | 8 |
| 2-62 | 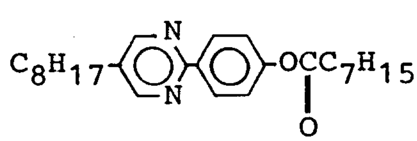 | 2 |
| 2-72 | 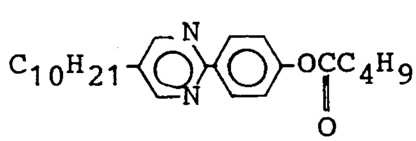 | 2 |
| 2-79 | 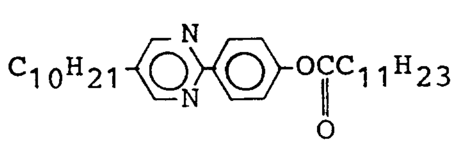 | 2 |

2-91 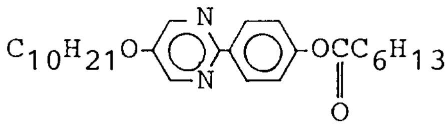 2

2-96 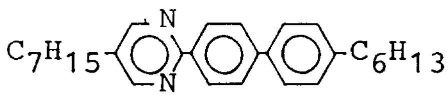 8

3-30 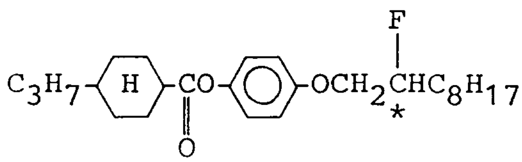 8

3-35 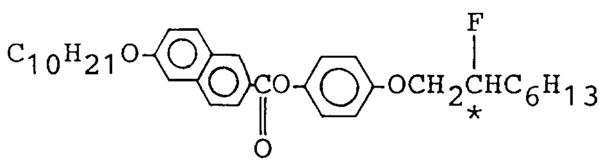 5

3-88 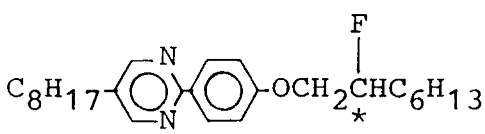 10

Composition S 31

A ferroelectric liquid crystal device was prepared in the same manner as in Example 1 except for using the composition 23-S. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 250 | 176 | 130 |

Comparative Example 8

A liquid crystal composition 23-Sb was prepared by omitting only Example Compounds Nos. 1-49 and 1-71 from the liquid crystal composition 23-S prepared in Example 23, otherwise in the same manner as in Example 23.

A ferroelectric liquid crystal device was prepared in the same manner as in Example 1 except that the liquid crystal composition 23-Sb was injected into a cell. The measured values of the response time of the device were as follows.

| | 15 °C | 25 °C | 35 °C |
|---|---|---|---|
| Response time (μsec) | 360 | 245 | 165 |

As is understood from the comparison between Example 23 and Comparative Example 8, a ferroelectric liquid crystal device using the liquid crystal composition 23-S containing the mesomorphic compounds according to the present invention showed an improved low-temperature operation characteristic, a high response speed and a decreased temperature-dependence of the response speed.

Example 24

A liquid crystal composition 24-S was prepared in the same manner as in Example 21 except that the following Example Compounds were used instead of Example Compounds Nos. 1-2, 1-9, 1-16, 1-34, 2-15, 2-19, 2-25, 2-29, 3-7, 3-13 and 3-92 prepared in Example 21 in respectively indicated proportions.

| Ex.Comp.No. | Structural formula | wt.parts |
|---|---|---|
| 1-2 | 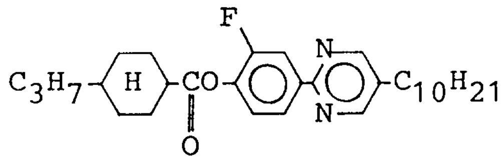 | 3 |
| 1-21 | 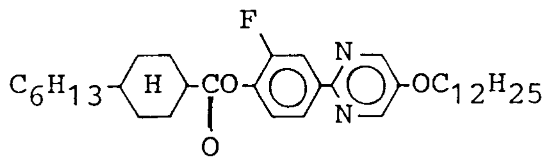 | 3 |
| 1-90 | 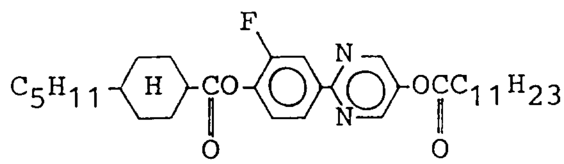 | 2 |
| 1-94 | 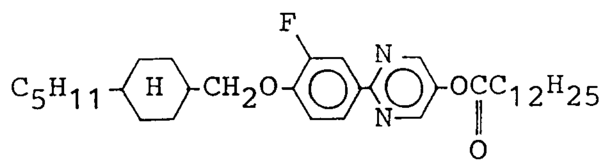 | 2 |
| 2-8 | 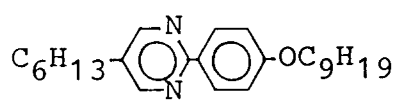 | 5 |
| 2-13 | 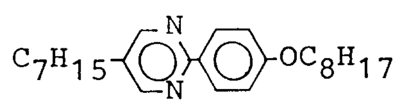 | 8 |
| 2-32 | 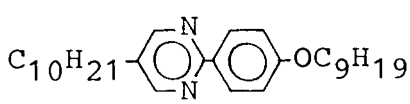 | 7 |
| 2-55 | 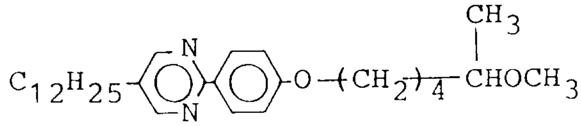 | 5 |

| | | |
|---|---|---|
| 2-74 | 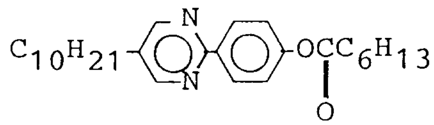 | 2 |
| 2-75 | 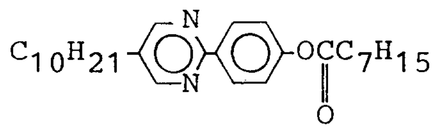 | 2 |
| 2-82 | 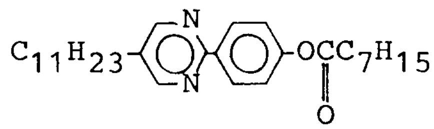 | 2 |
| 2-102 | 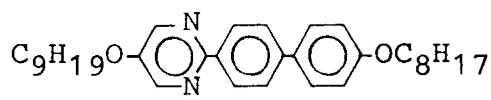 | 5 |
| 3-19 | 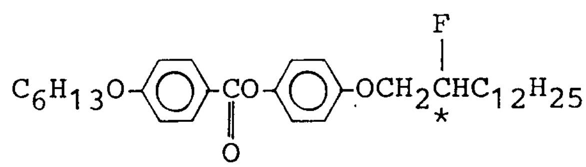 | 9 |
| 3-49 | 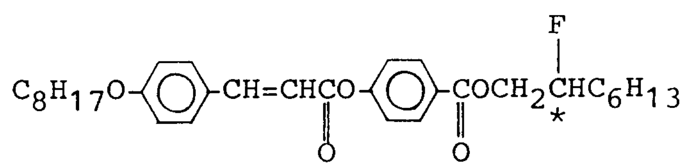 | 6 |
| 3-101 | 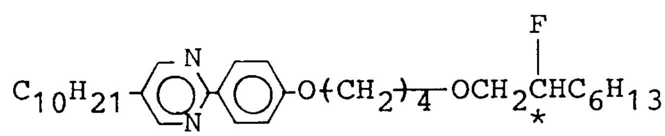 | 8 |
| | Composition S | 31 |

A ferroelectric liquid crystal device was prepared in the same manner as in Example 1 except for using the composition 24-S. In the ferroelectric liquid crystal device, a monodomain with a good and uniform alignment characteristic was observed. The ferroelectric liquid crystal device was subjected to measurement of response time and observation of a switching state, etc. in the same manner as in Example 1, whereby the following results were obtained.

| | 15 °C | 25 °C | 35 °C |
|---|---|---|---|
| Response time (μsec) | 321 | 207 | 175 |

Comparative Example 9

A liquid crystal composition 24-Sb was prepared by omitting only Example Compounds Nos. 1-2, 1-21, 1-90 and 1-94 from the liquid crystal composition 24-S prepared in Example 24, otherwise in the same

manner as in Example 24.

A ferroelectric liquid crystal device was prepared in the same manner as in Example 1 except that the liquid crystal composition 24-Sb was injected into a cell. The measured values of the response time of the device were as follows.

| | 15 °C | 25 °C | 35 °C |
|---|---|---|---|
| Response time (μsec) | 369 | 230 | 178 |

As is understood from the comparison between Example 24 and Comparative Example 9, a ferroelectric liquid crystal device using the liquid crystal composition 24-S containing the mesomorphic compounds according to the present invention showed an improved low-temperature operation characteristic, a high response speed and a decreased temperature-dependence of the response speed.

As described above, according to the present invention, there are provided a ferroelectric liquid crystal composition and a ferroelectric liquid crystal device containing the composition, which shows a good switching characteristic, an improved low-temperature operation characteristic and a decreased temperature-dependence of response speed.

## Claims

**1.** A liquid crystal composition, comprising:
at least one mesomorphic compound represented by the following formula (I):

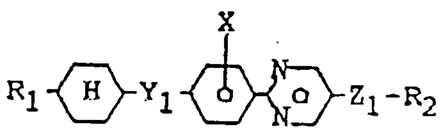

(I),

wherein $R_1$ denotes an n-alkyl group having 1-16 carbon atoms; $R_2$ denotes an optically active or inactive group selected from the following groups (i) to (iv):

(i) n-alkyl group having 1-16 carbon atoms;

(ii)

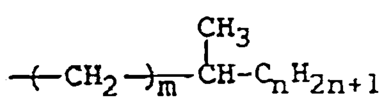

wherein m is 1-7 and n is 2-9 with proviso that $3 \leq m+n \leq 14$;

(iii)

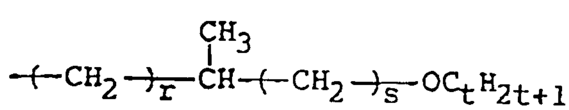

wherein r is 0-7 s is 0 or 1 and t is 1-14 with proviso that $1 \leq r+s+t \leq 14$; and

(iv)

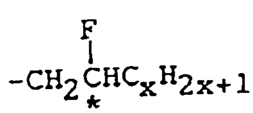

wherein x is 1-14 and * denotes an optical active center;

$Y_1$ denotes -COO-, -OCO-, $-CH_2O-$ or $-OCH_2-$; $Z_1$ denotes a single bond, -O-, -COO-, -OCO- or -OCOO-; and X denotes a halogen, cyano group or methyl group; and

at least one mesomorphic compound represented by the following formula (III):

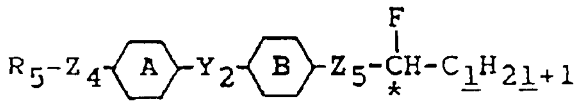
(III),

wherein $R_5$ denotes a linear or branched alkyl group having 1-18 carbon atoms; $y_2$ denotes a single bond, -COO-, -OCO-, -COS-, SCO-, $CH_2O$-, $-OCH_2$- or -CH=CH-COO-; $Z_4$ denotes a single bond, -O-, -COO- or -OCO-; $Z_5$ denotes $-OCH_2$-, $-COOCH_2$-, -OCO- or

$-O-(CH_2)_k-O-CH_2$; 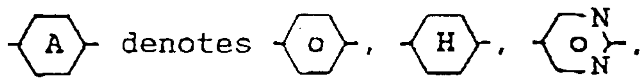
denotes , , ,

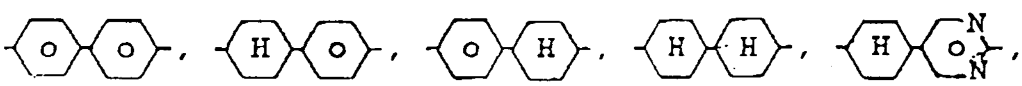
, , , , ,

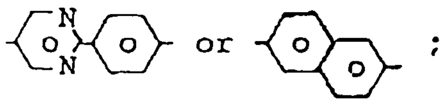
or ;

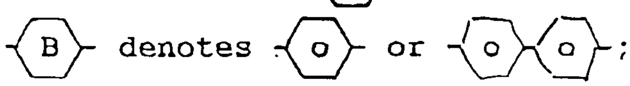
denotes or ;

$\underline{l}$ is 1 - 12 and k is 1 - 4.

**2.** A liquid crystal composition comprising at least one mesomorphic compound represented by the following formula (I):

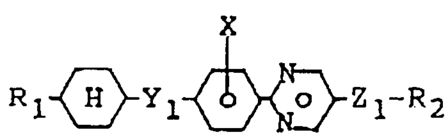
(I),

wherein $R_1$ denotes an n-alkyl group having 1-16 carbon atoms; $R_2$ denotes an optically active or inactive group selected from the following groups (i) to (iv):

(i) n-alkyl group having 1-16 carbon atoms;

(ii)

$$-(CH_2)_m-\underset{}{CH}(CH_3)-C_nH_{2n+1}$$

wherein m is 1-7 and n is 2-9 with proviso that 3 ≦ m+n ≦ 14;

(iii)

$$-(CH_2)_r-CH(CH_3)-(CH_2)_s-OC_tH_{2t+1}$$

wherein r is 0-7, s is 0 or 1 and t is 1-14 with proviso that 1 ≦ r+s+t ≦ 14; and

(iv)

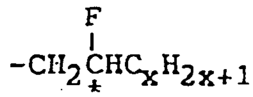

wherein x is 1-14; and * denotes an optically active center; $Y_1$ denotes -COO-, -OCO-, $-CH_2O-$ or $-OCH_2-$; $Z_1$ denotes a single bond, -O-, -COO-, -OCO- or -OCOO-; and X denotes a halogen, cyano group or methyl group;

at least one mesomorphic compound represented by the following formula (II):

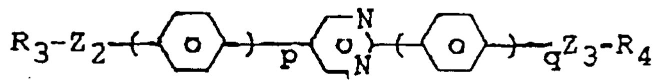

(II),

wherein $R_3$ and $R_4$ respectively denote a linear or branched optically inactive alkyl group having 1-18 carbon atoms capable of having $C_1$ - $C_{12}$ alkoxy group; $Z_2$ and $Z_3$ respectively denote a single bond, -O-, -OCO-, -COO- or -OCOO-; and p and q are respectively 0, 1 or 2 and (III):

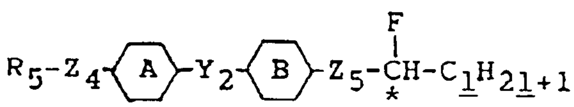

(III),

wherein $R_5$ denotes a linear or branched alkyl group having 1-18 carbon atoms; $y_2$ denotes a single bond, -COO-, -OCO-, -COS-, SCO-, $CH_2O-$, $-OCH_2-$ or - CH=CH-COO-; $Z_4$ denotes a single bond, -O-, -COO- or -OCO-; $Z_5$ denotes $-OCH_2-$, $-COOCH_2-$, -OCO- or

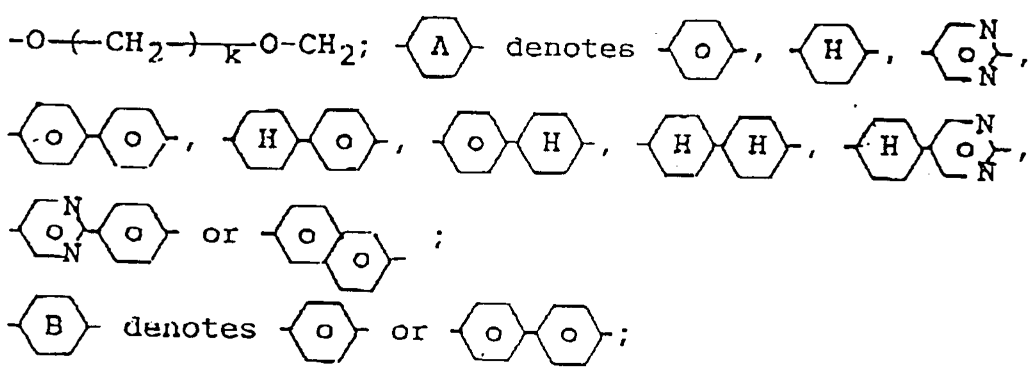

denotes , , ,

, , , , ,

or ;

denotes or ;

l is 1 - 12 and k is 1 - 4.

**3.** A liquid crystal composition according to anyone of the claims 1 and 2, wherein the mesomorphic compound according to formula (I) represents anyone of the following formulae (1-1) to (1-116)

1 − 1

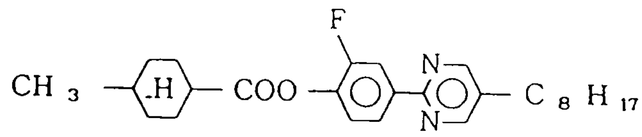

1 − 2

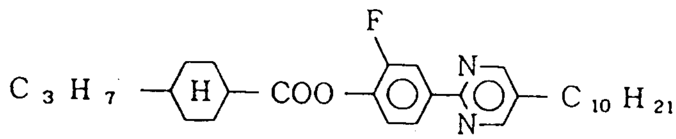

1 − 3

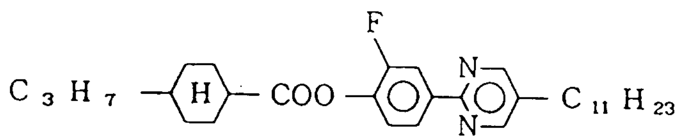

1 − 4

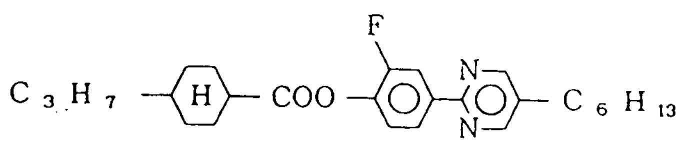

1 − 5

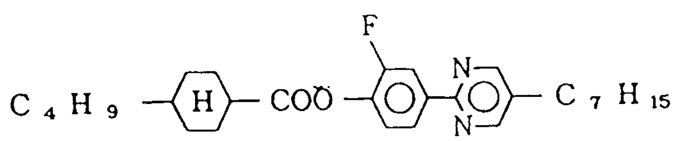

1 − 6

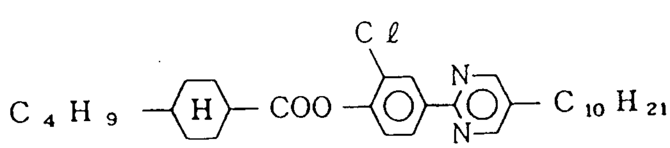

1 − 7

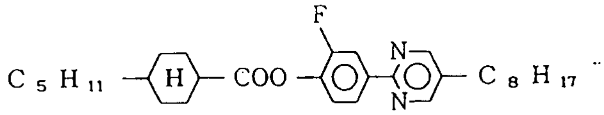

1 − 8

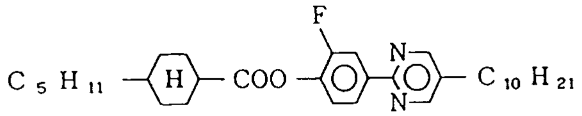

1 − 9

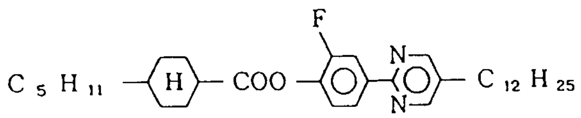

1 − 10

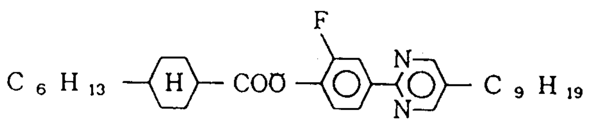

1 − 11

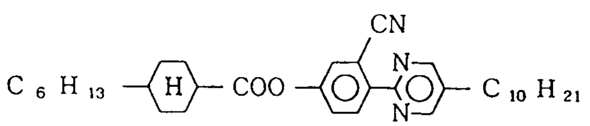

1 − 12

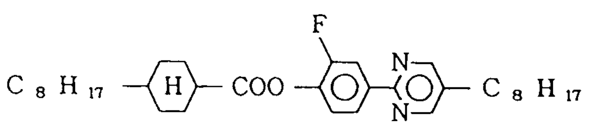

1 − 13

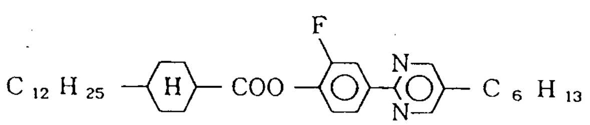

1 − 14

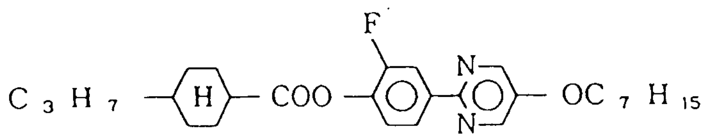

1 − 15

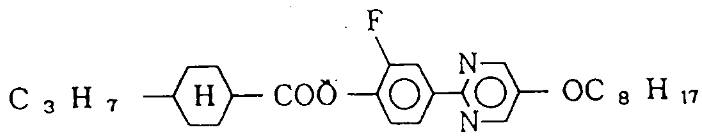

1 − 16

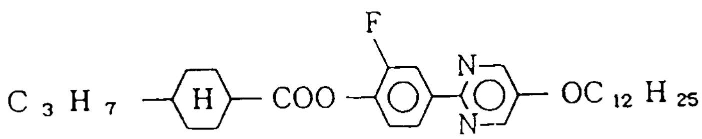

1 − 17

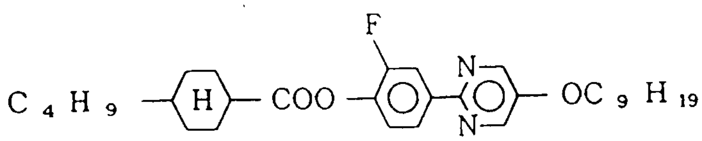

1 − 18

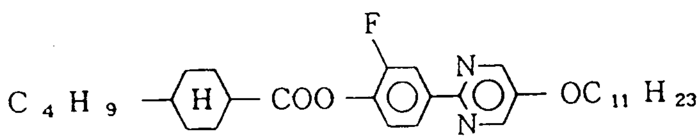

1 − 19

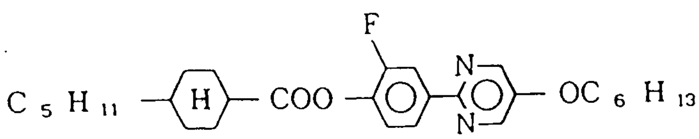

1 − 2 0

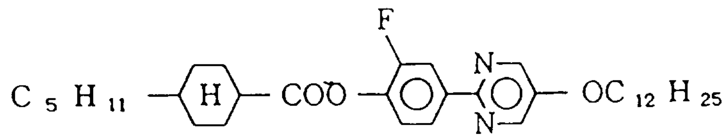

1 − 2 1

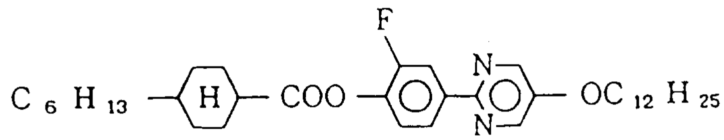

1 − 2 2

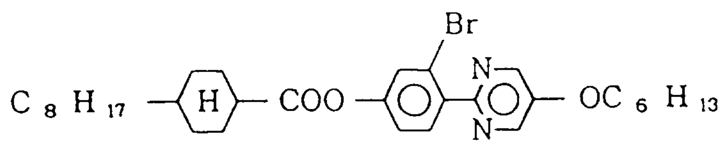

1 − 2 3

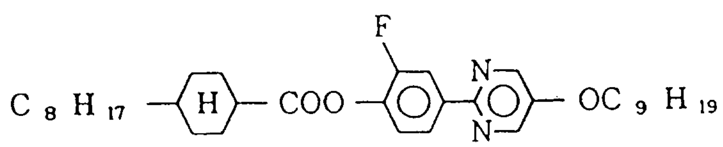

1 − 2 4

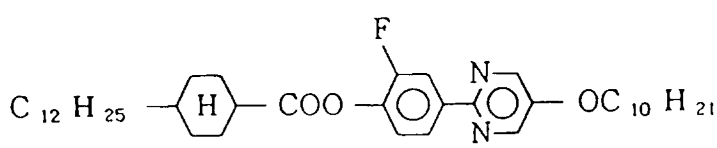

1 − 25

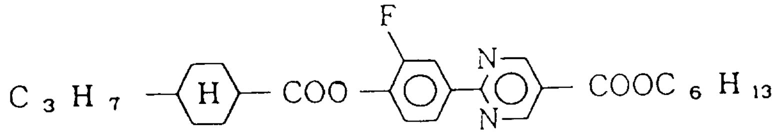

1 − 26

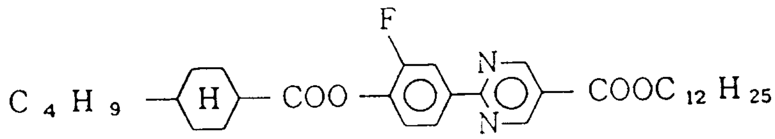

1 − 27

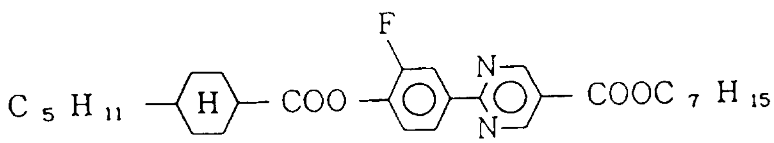

1 − 28

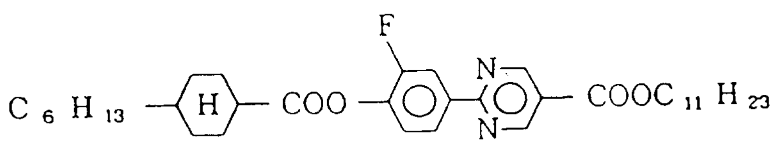

1 − 29

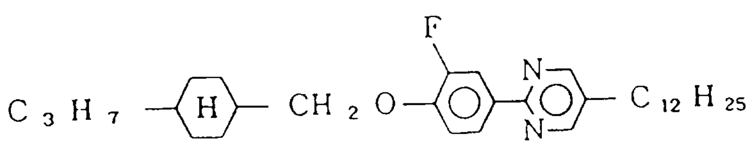

1 − 30

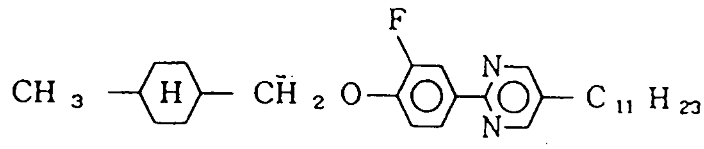

1 − 31

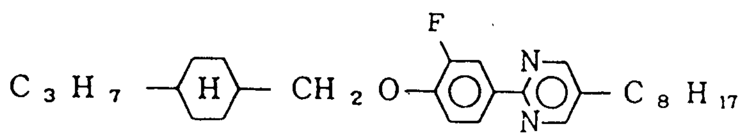

1 − 32

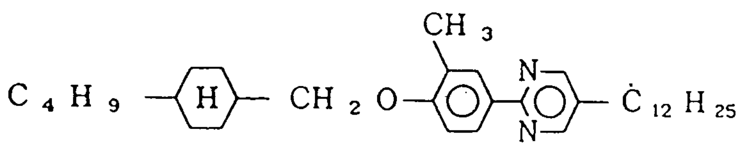

1 − 33

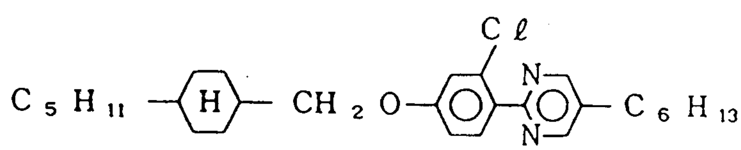

1 − 34

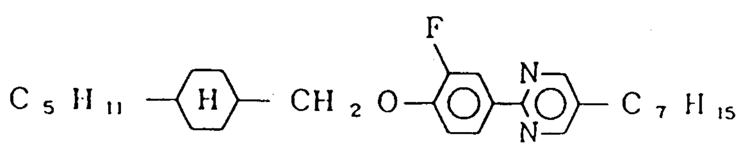

1 − 35

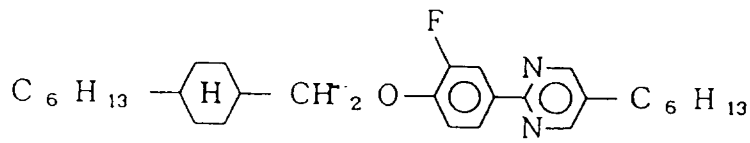

1 − 36

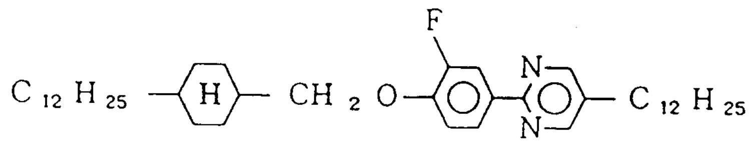

1 − 37

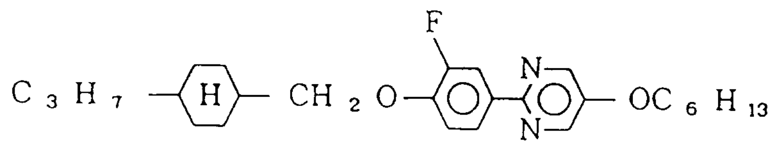

1 − 38

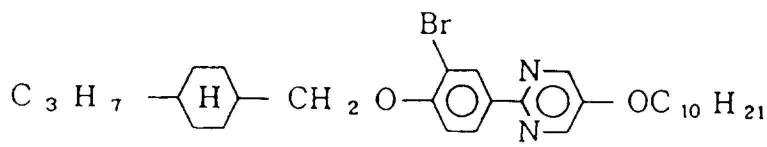

1 − 39

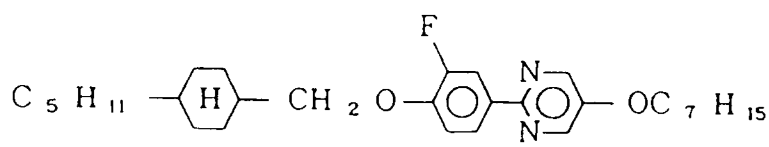

1 − 40

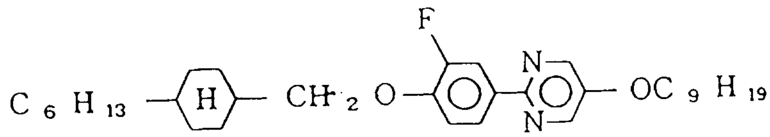

1 − 41

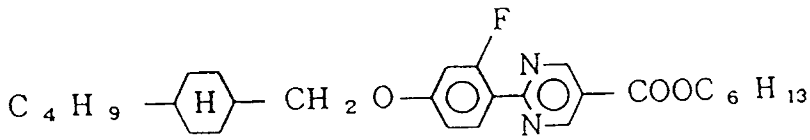

1 − 42

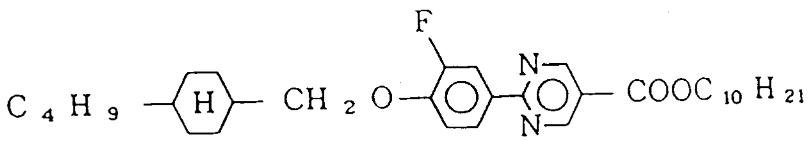

1 − 43

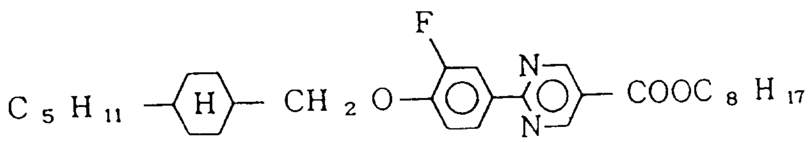

1 − 44

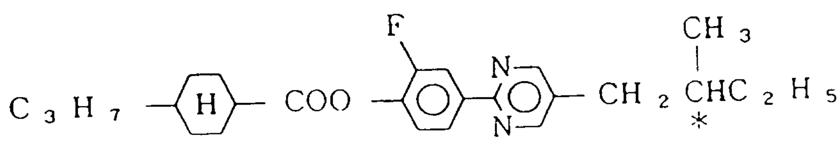

1 − 45

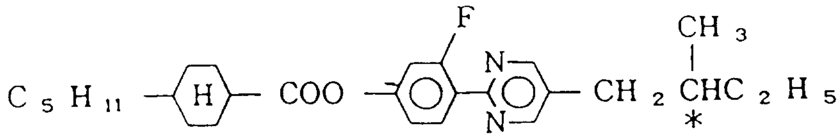

1 − 46

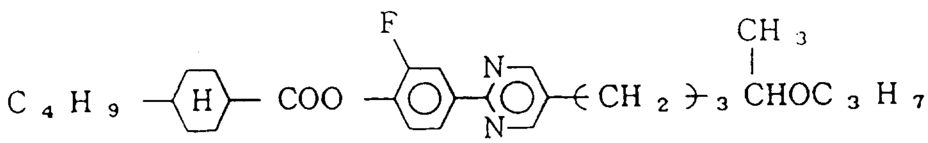

1 − 47

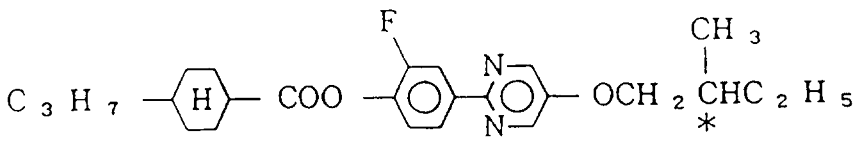

1 − 48

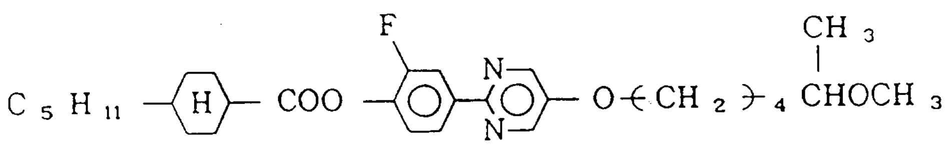

1 − 49

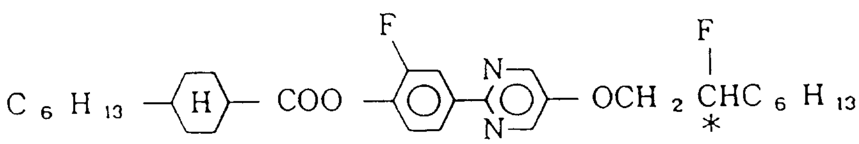

1 − 50

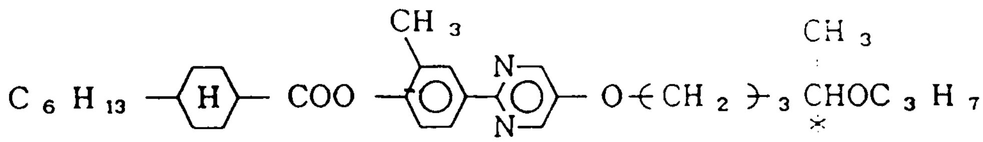

1 − 51

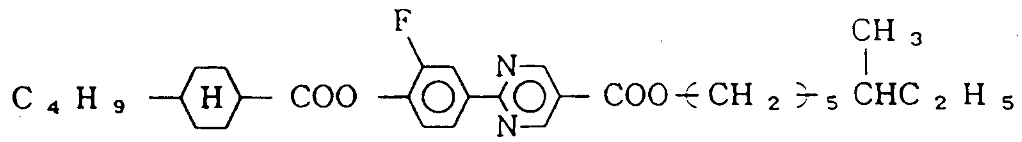

1 − 52

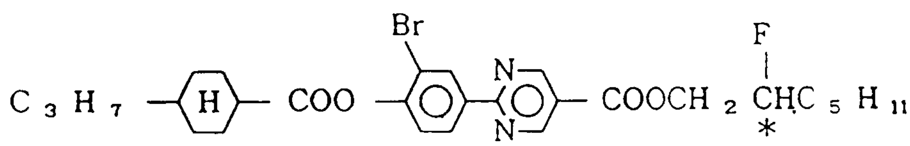

1 − 53

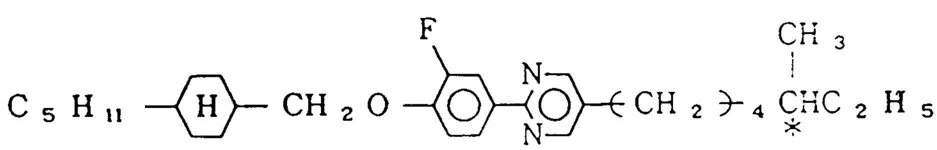

1 − 54

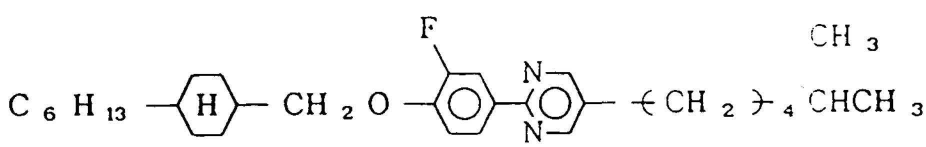

1 − 55

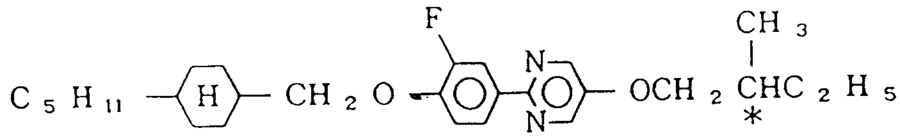

1 − 56

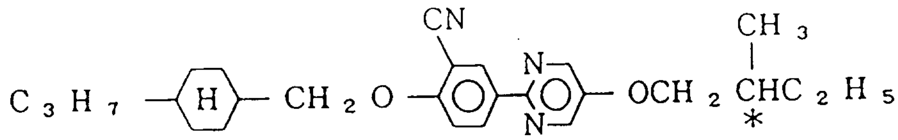

1 − 57

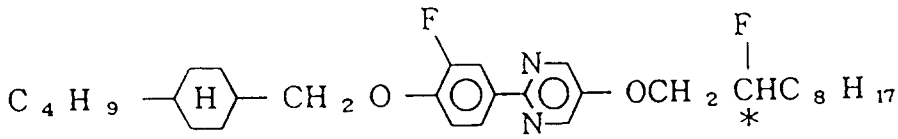

1 − 58

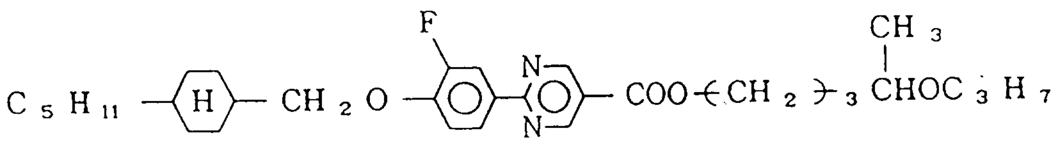

1 − 59

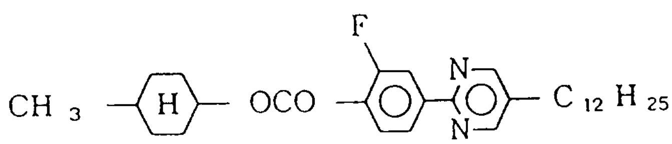

1 − 60

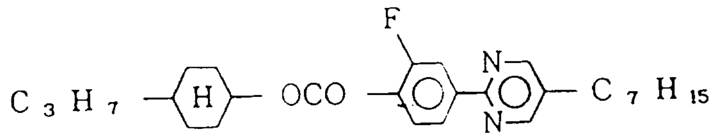

1 − 61

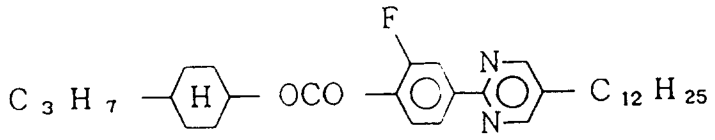

1 − 62

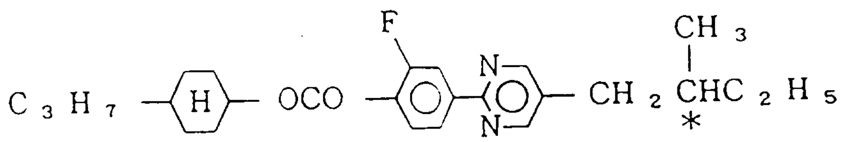

1 − 63

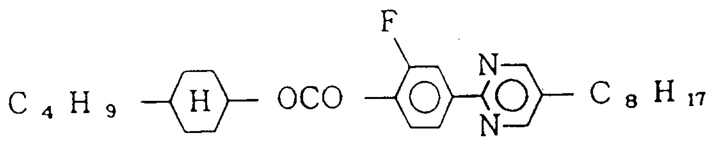

1 − 64

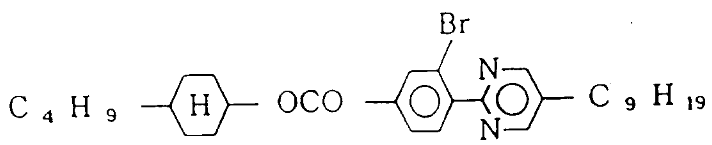

1 - 65

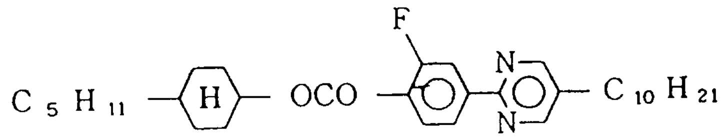

1 - 66

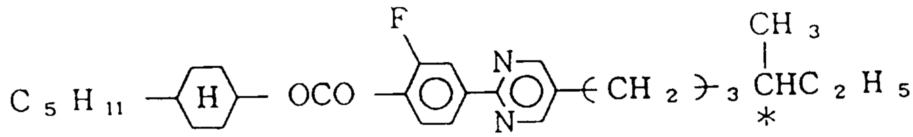

1 - 67

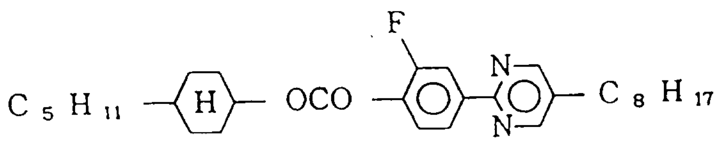

1 - 68

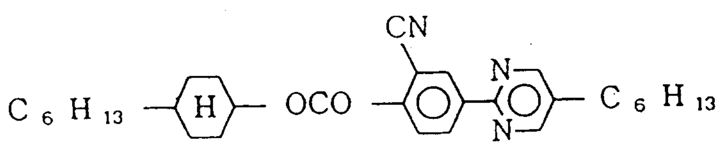

1 - 69

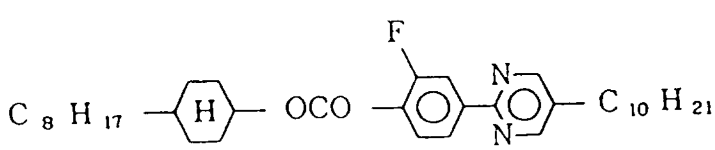

1 − 7 0

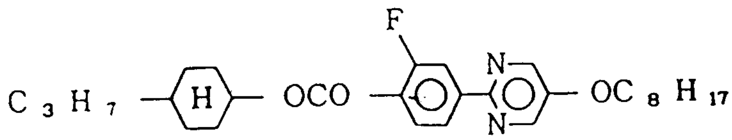

1 − 7 1

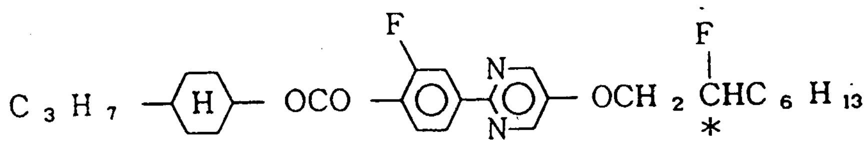

1 − 7 2

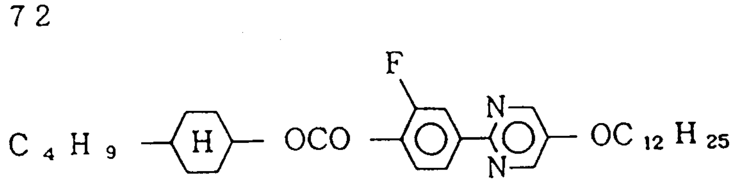

1 − 7 3

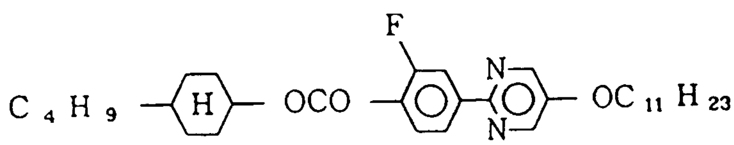

1 − 7 4

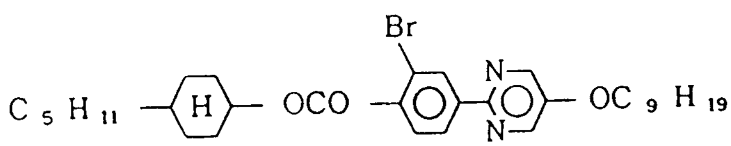

1 − 75

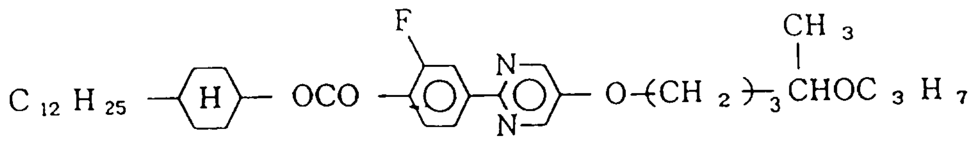

1 − 76

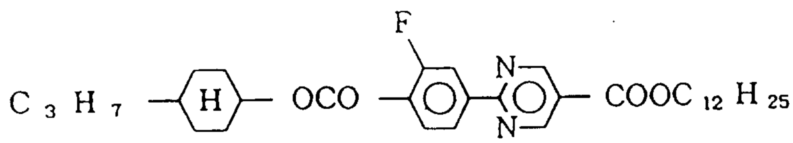

1 − 77

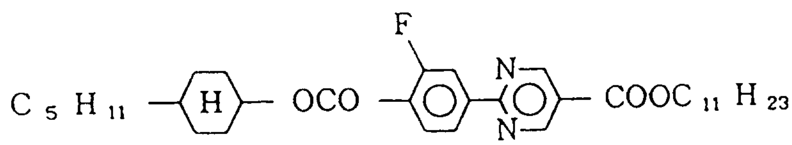

1 − 78

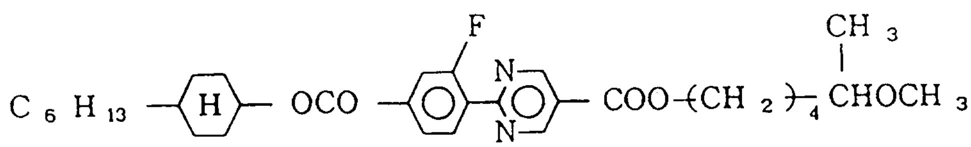

1 − 79

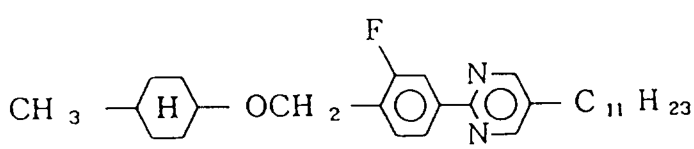

1－80

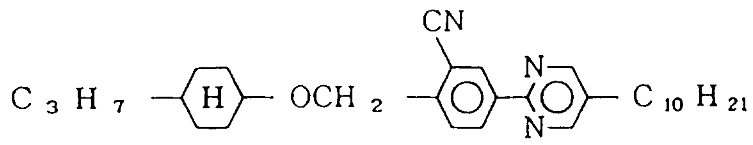

1－81

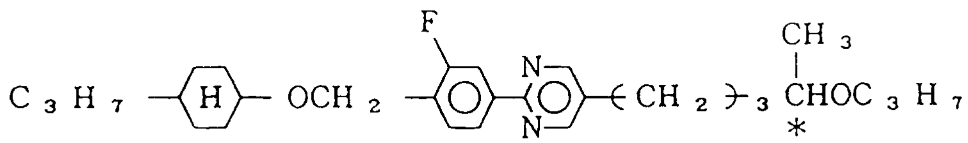

1－82

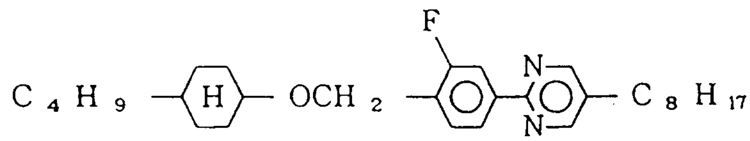

1－83

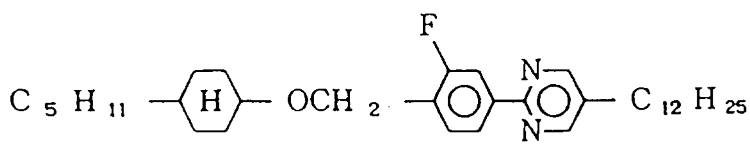

1－84

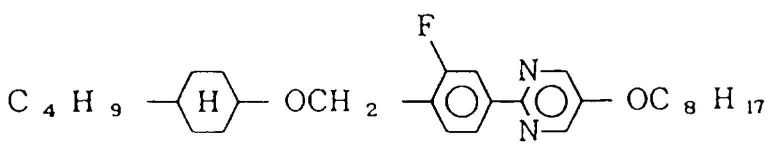

1 − 85

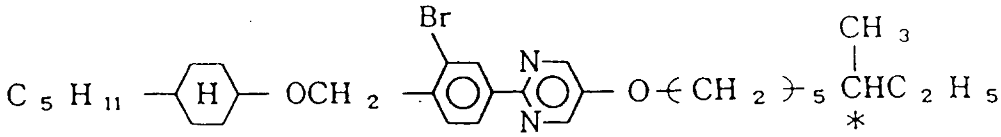

1 − 86

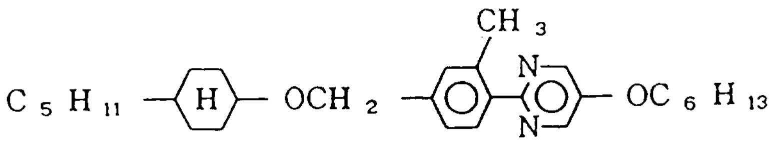

1 − 87

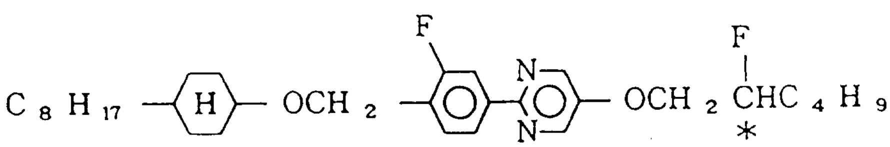

1 − 88

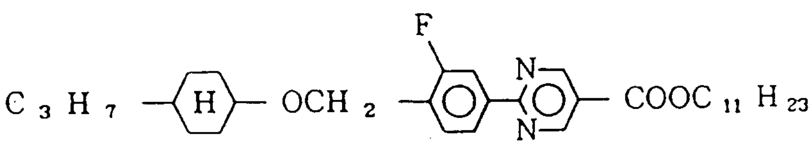

1 − 89

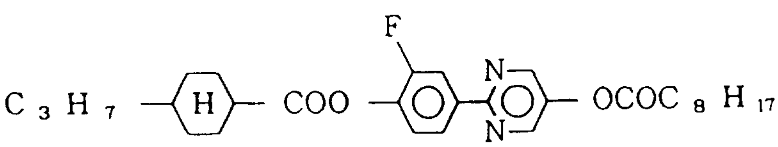

1 − 90

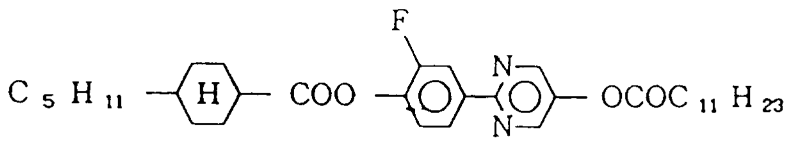

1 − 91

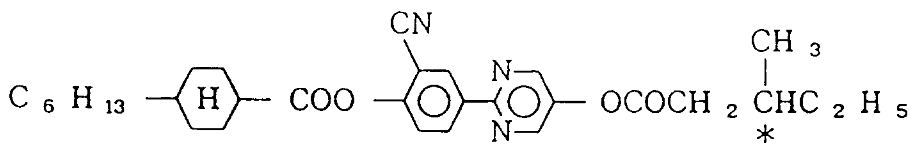

1 − 92

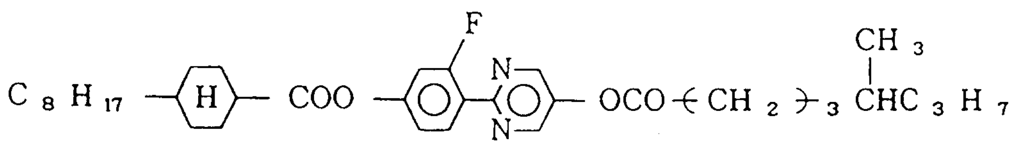

1 − 93

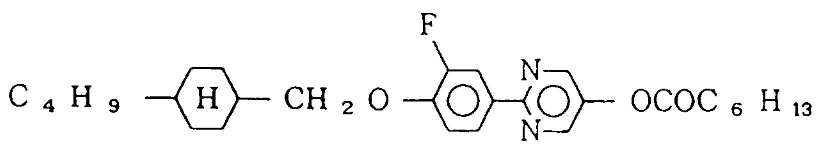

1 − 94

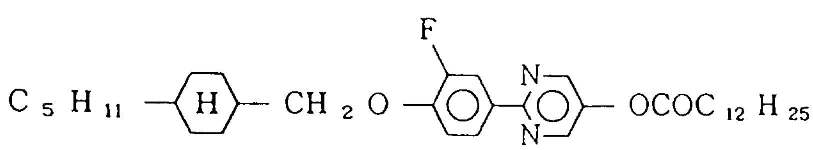

1 − 95

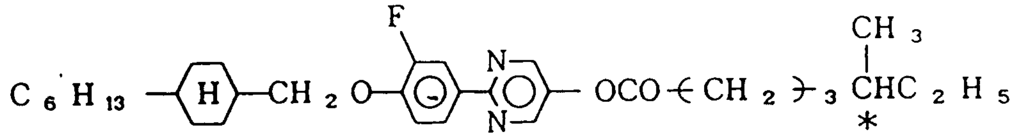

1 − 96

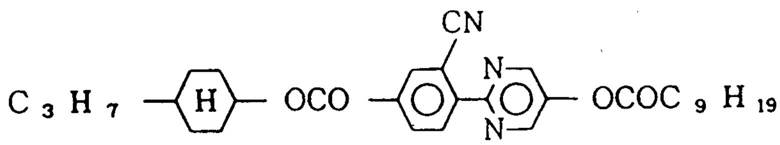

1 − 97

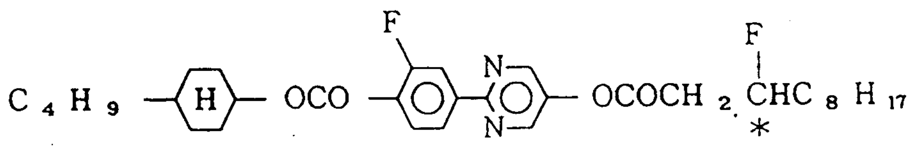

1 − 98

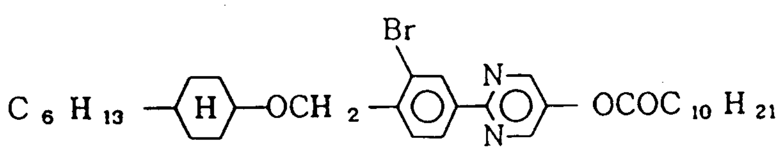

1 − 99

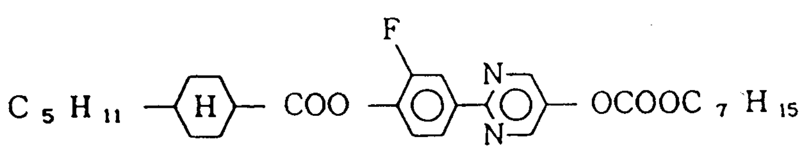

1 − 100

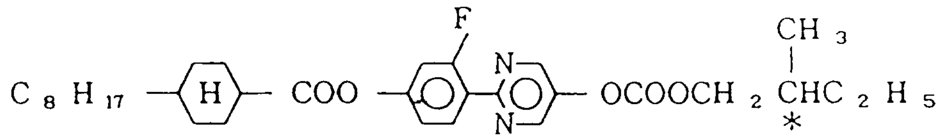

1 − 101

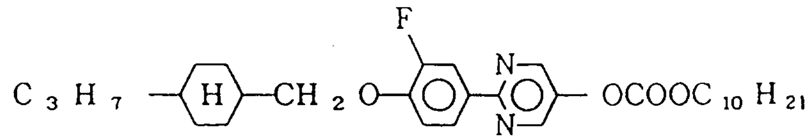

1 − 102

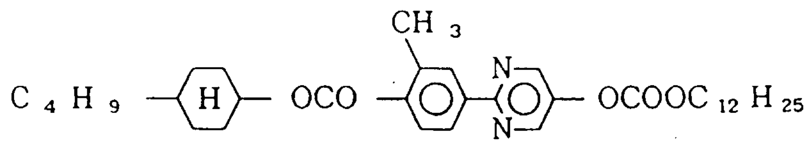

1 − 103

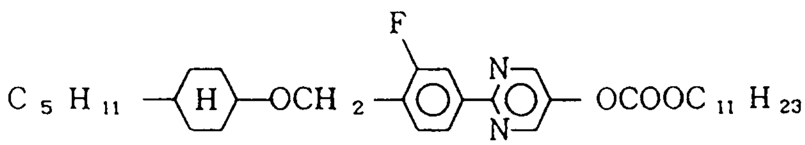

1 − 104

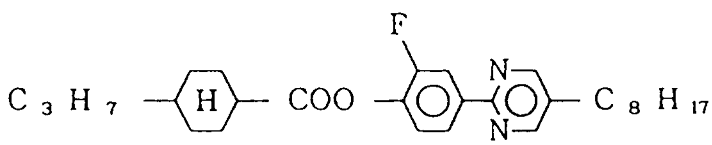

1 − 105

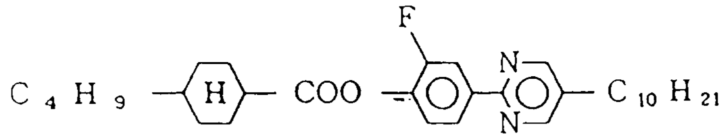

1 − 106

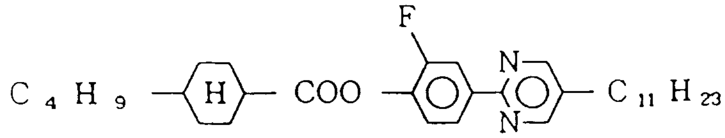

1 − 107

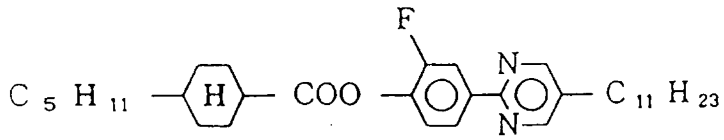

1 − 108

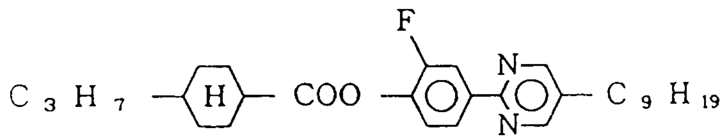

1 − 109

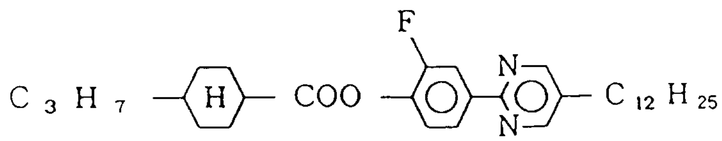

1 − 1 1 0

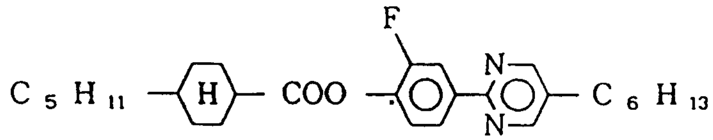

1 − 1 1 1

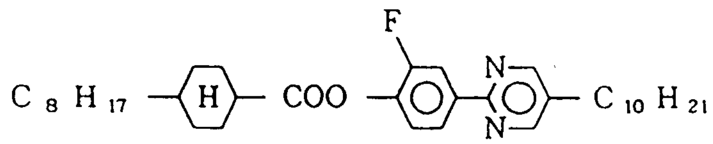

1 − 1 1 2

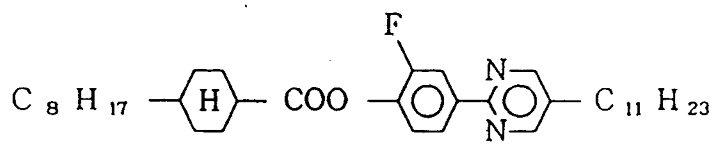

1 − 1 1 3

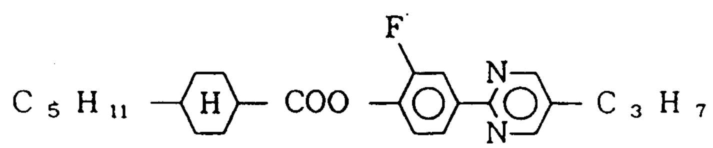

1 − 1 1 4

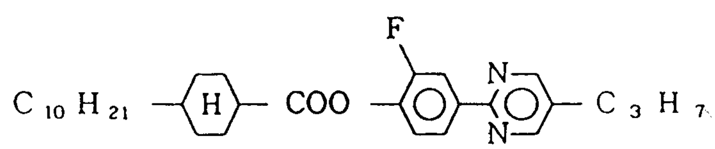

1 - 115

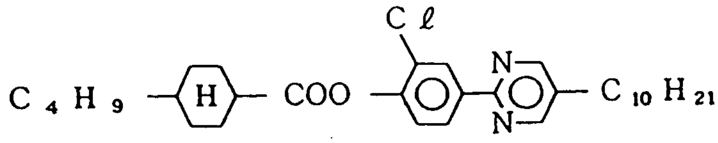

1 - 116

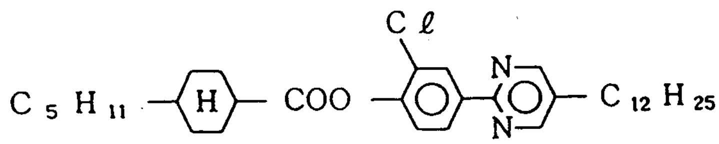

4. A liquid crystal composition according to claim 1, which comprises another mesomorphic compound other than those represented by the formulae (I) and (III) and comprises 1-300 wt. parts each of a compound of the formula (I) and a compound of the formula (III), per 100 wt. parts of said another mesomorphic compound.

5. A liquid crystal composition according to claim 1, which comprises another mesomorphic compound other than those represented by the formulae (I) and (III) and two or more species of compounds from each of at least one group of compounds of the formulae (I) and (III) in a total amount of 1-500 wt. parts per 100 wt. parts of said another mesomorphic compound.

6. A liquid crystal composition according to claim 2, which comprises another mesomorphic compound other than those represented by the formulae (I), (II) and (III) and comprises 1-300 wt. parts each of a compound of the formula (I), a compound of the formula (II) and a compound of the formula (III), per 100 wt. parts of said another mesomorphic compound.

7. A liquid crystal composition according to claim 1, which comprises another mesomorphic compound other than those represented by the formulae (I), (II) and (III) and two or more species of compounds from each of at least one group of compounds of the formulae (I), (II) and (III) in a total amount of 1-500 wt. parts per 100 wt. parts of said another mesomorphic compound.

8. A liquid crystal composition according to anyone of claims 1 to 7, which has a chiral smectic phase.

9. A liquid crystal device, comprising a pair of electrode plates and a liquid crystal composition according to anyone of claims 1 to 8 disposed between the electrode plates.

10. A liquid crystal device according to claim 9, which further comprises an alignment control layer on the electrode plates.

11. A liquid crystal device according to claim 10, wherein the alignment control layer has been subjected to rubbing.

12. A liquid crystal device according to claim 9, wherein the pair of electrode plates are disposed with a spacing therebetween sufficiently small to release the helical structure of the liquid crystal.

**Patentansprüche**

1. Flüssigkristallmischung, die
mindestens eine mesomorphe Verbindung, die durch die folgende Formel (I) wiedergegeben wird:

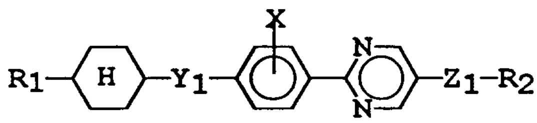 (I),

worin $R_1$ eine n-Alkylgruppe mit 1 bis 16 Kohlenstoffatomen bezeichnet; $R_2$ eine optisch aktive oder inaktive Gruppe bezeichnet, die aus den folgenden Gruppen (i) bis (iv) ausgewählt ist:

(i) n-Alkylgruppe mit 1 bis 16 Kohlenstoffatomen;

(ii)

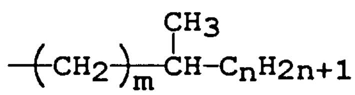 ,

worin m 1 bis 7 ist und n 2 bis 9 ist, wobei vorausgesetzt ist, daß $3 \leqq m + n \leqq 14$;

(iii)

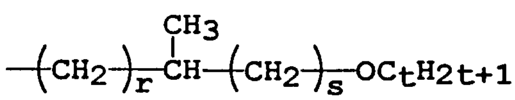 ,

worin r 0 bis 7 ist, s 0 oder 1 ist und t 1 bis 14 ist, wobei vorausgesetzt ist, daß $1 \leqq r + s + t \leqq 14$; und

(iv)

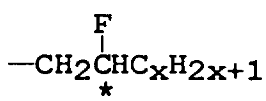 ,

worin x 1 bis 14 ist und * ein optisch aktives Zentrum bezeichnet;

$Y_1$ -COO-, -OCO-, $-CH_2O-$ oder- $-OCH_2-$ bezeichnet; $Z_1$ eine Einfachbindung, -O-, -COO-, -OCO- oder -OCOO- bezeichnet und X ein Halogen, eine Cyangruppe oder eine Methylgruppe bezeichnet; und mindestens eine mesomorphe Verbindung umfaßt, die durch die folgende Formel (III) wiedergegeben wird:

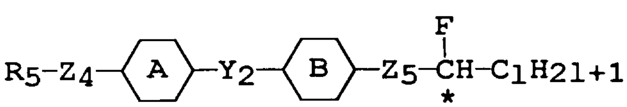 (III),

worin $R_5$ eine lineare oder verzweigte Alkylgruppe mit 1 bis 18 Kohlenstoffatomen bezeichnet; $Y_2$ eine Einfachbindung, -COO-, -OCO-, -COS-, -SCO-, $-CH_2O-$, $-OCH_2-$ oder -CH=CH-COO- bezeichnet; $Z_4$ eine Einfachbindung, -O-, -COO- oder -OCO- bezeichnet; $Z_5$ $-OCH_2-$, $-COOCH_2-$, -OCO- oder

$$-O-(CH_2)_k-O-CH_2-$$

bezeichnet;

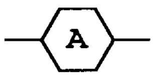 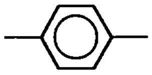, 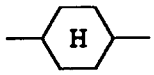, 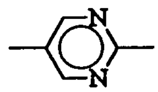, 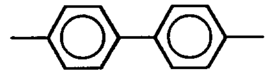,

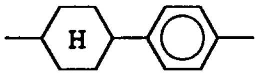, 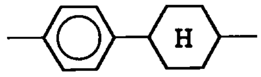, 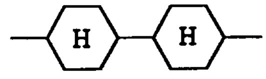,

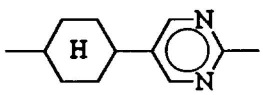, 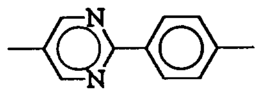 oder 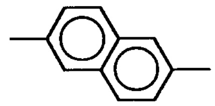 bezeichnet;

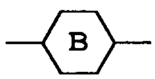 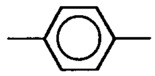 oder 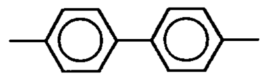 bezeichnet;

l 1 bis 12 ist und k 1 bis 4 ist.

**2.** Flüssigkristallmischung, die mindestens eine mesomorphe Verbindung, die durch die folgende Formel (I) wiedergegeben wird:

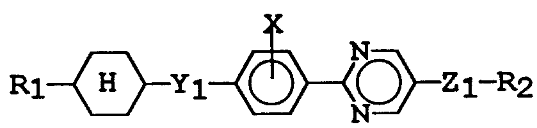 (I),

worin $R_1$ eine n-Alkylgruppe mit 1 bis 16 Kohlenstoffatomen bezeichnet; $R_2$ eine optisch aktive oder inaktive Gruppe bezeichnet, die aus den folgenden Gruppen (i) bis (iv) ausgewählt ist:

(i) n-Alkylgruppe mit 1 bis 16 Kohlenstoffatomen;

(ii)

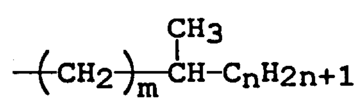 ,

worin m 1 bis 7 ist und n 2 bis 9 ist, wobei vorausgesetzt ist, daß $3 \leqq m + n \leqq 14$;

(iii)

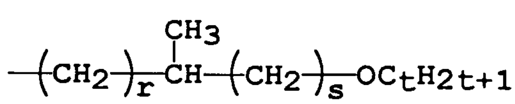 ,

worin r 0 bis 7 ist, s 0 oder 1 ist und t 1 bis 14 ist, wobei vorausgesetzt ist, daß $1 \leqq r + s + t \leqq 14$; und

(iv)

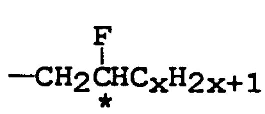 ,

worin x 1 bis 14 ist und * ein optisch aktives Zentrum bezeichnet;

$Y_1$ -COO-, -OCO-, $-CH_2O-$ oder $-OCH_2-$ bezeichnet; $Z_1$ eine Einfachbindung, -O-, -COO-, -OCO- oder -OCOO- bezeichnet und X ein Halogen, eine Cyangruppe oder eine Methylgruppe bezeichnet; mindestens eine mesomorphe Verbindung, die durch die folgende Formel (II) wiedergegeben wird:

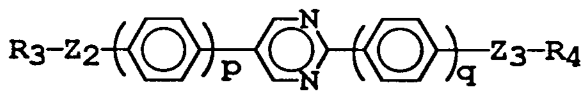

(II),

worin $R_3$ und $R_4$ je eine lineare oder verzweigte, optisch inaktive Alkylgruppe mit 1 bis 18 Kohlenstoffatomen, die eine $C_1$- bis $C_{12}$-Alkoxygruppe haben kann, bezeichnen; $Z_2$ und $Z_3$ je eine Einfachbindung, -O-, -OCO-, -COO- oder -OCOO- bezeichnen und p und q jeweils 0, 1 oder 2 bedeuten; und mindestens eine mesomorphe Verbindung umfaßt, die durch die folgende Formel (III) wiedergegeben wird:

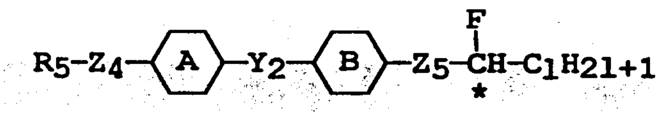

(III),

worin $R_5$ eine lineare oder verzweigte Alkylgruppe mit 1 bis 18 Kohlenstoffatomen bezeichnet; $Y_2$ eine Einfachbindung, -COO-, -OCO-, -COS-, -SCO-, $-CH_2O-$, $-OCH_2-$ oder -CH=CH-COO- bezeichnet; $Z_4$ eine Einfachbindung, -O-, -COO- oder -OCO- bezeichnet; $Z_5$ $-OCH_2-$, $-COOCH_2-$, -OCO- oder

$$-O-(CH_2)_k-O-CH_2-$$

bezeichnet;

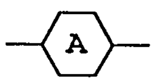 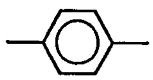, 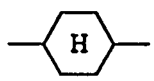, 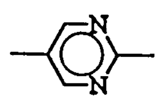, 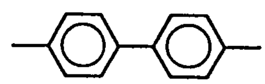,

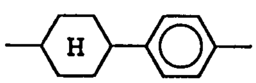, 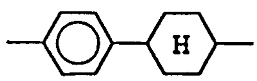, 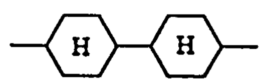,

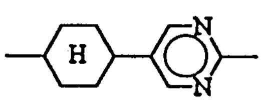, 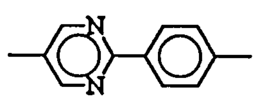 oder 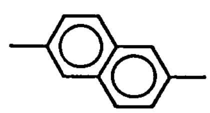 bezeichnet;

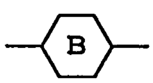 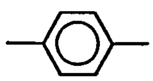 oder 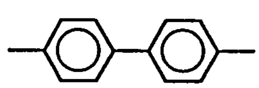 bezeichnet;

l 1 bis 12 ist und k 1 bis 4 ist.

**3.** Flüssigkristallmischung nach einem der Ansprüche 1 und 2, bei der die mesomorphe Verbindung gemäß Formel (I) durch irgendeine der folgenden Formeln (1-1) bis (1-116) wiedergegeben wird:

1 – 1

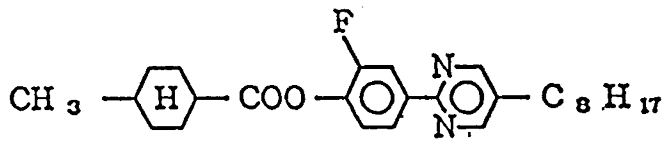

1 – 2

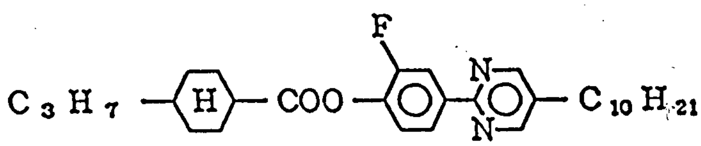

1 – 3

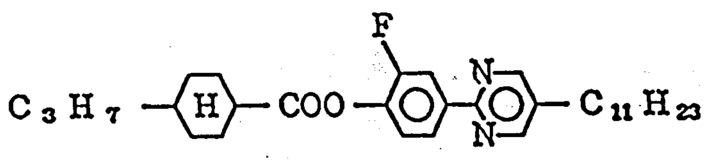

1 – 4

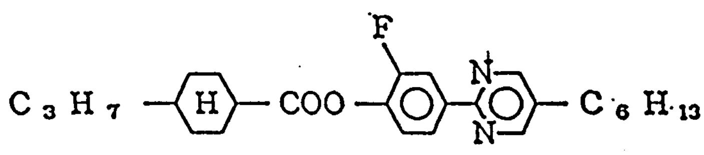

1 − 5

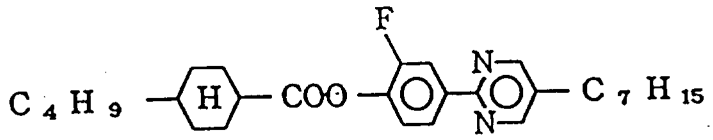

1 − 6

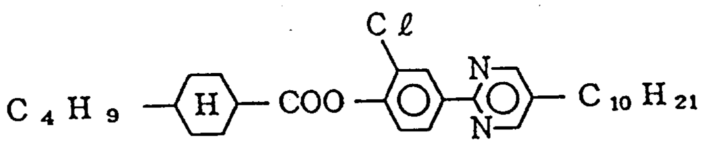

1 − 7

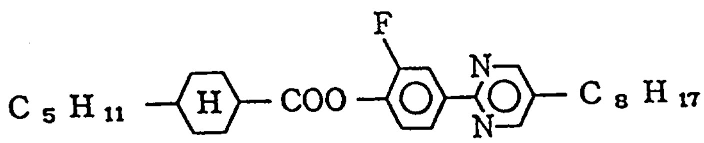

1 − 8

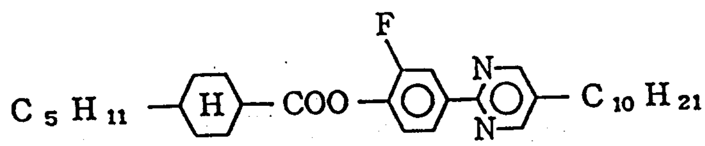

1 − 9

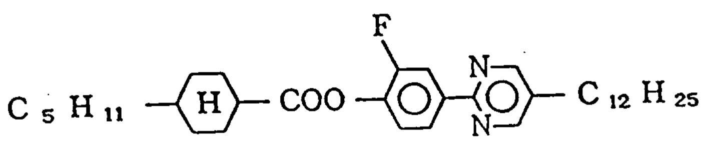

1 − 10

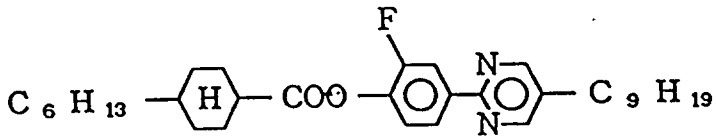

1 − 11

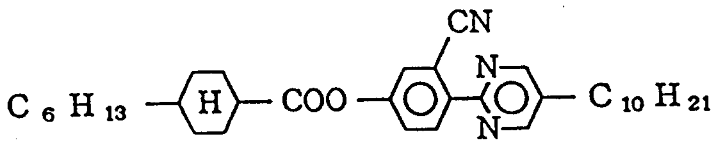

1 − 12

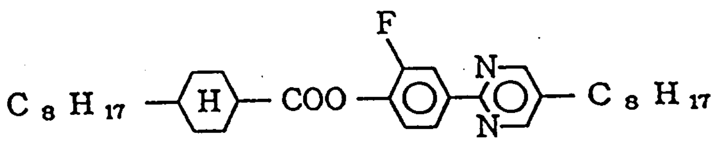

1 − 13

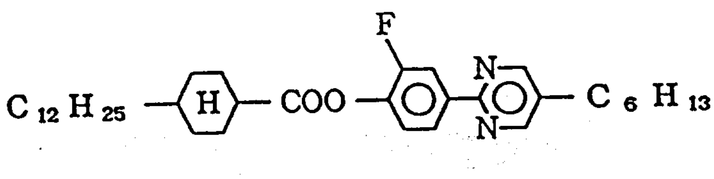

1 − 14

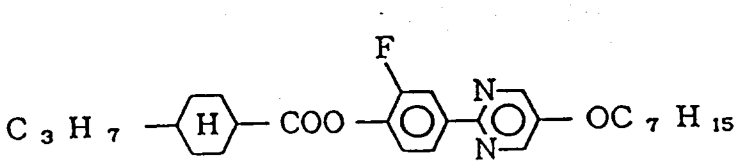

1 − 15

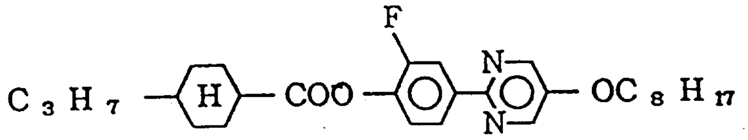

1 − 16

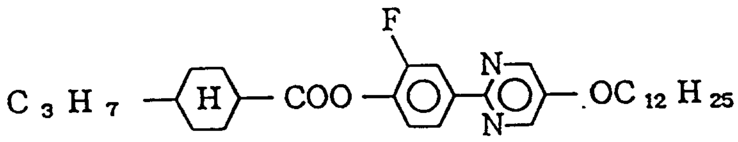

1 − 17

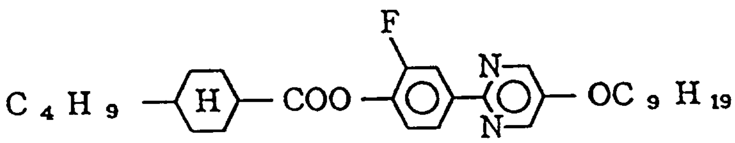

1 − 18

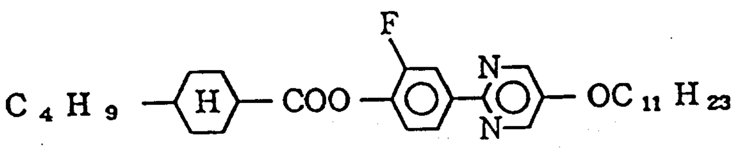

1 − 19

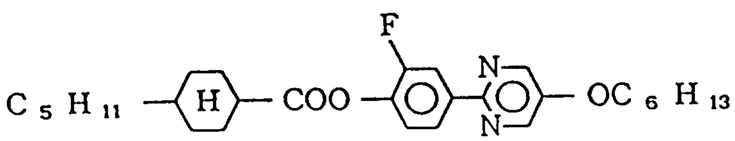

1 − 20

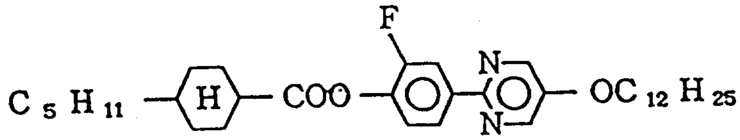

1 − 21

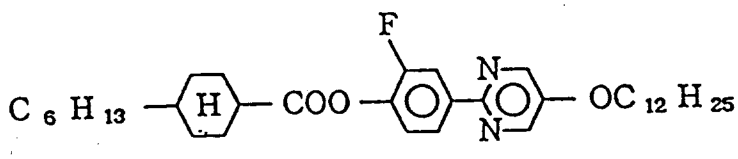

1 − 22

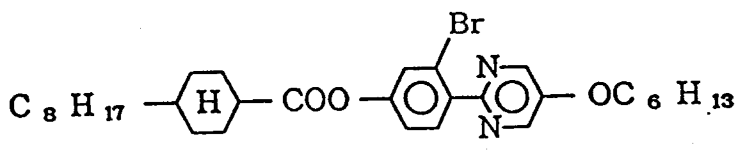

1 − 23

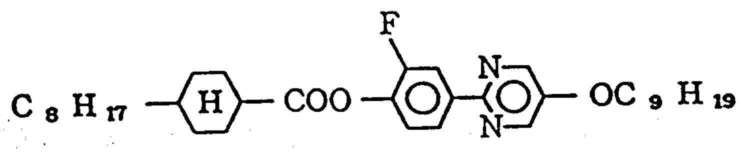

1 − 24

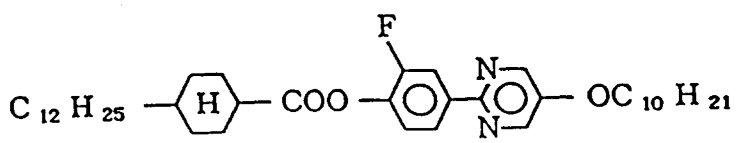

1 − 25

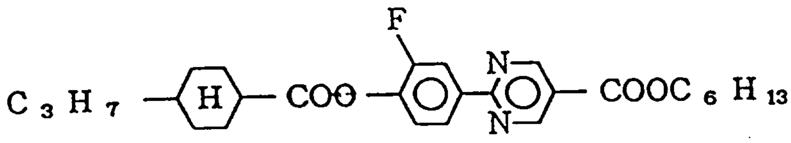

1 − 26

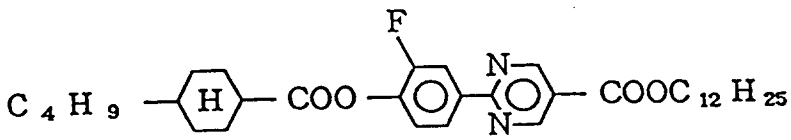

1 − 27

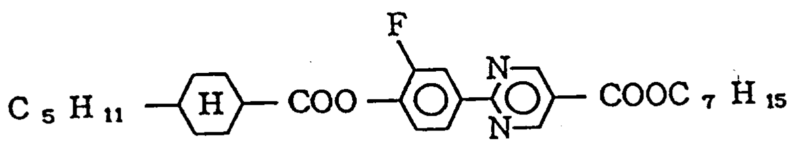

1 − 28

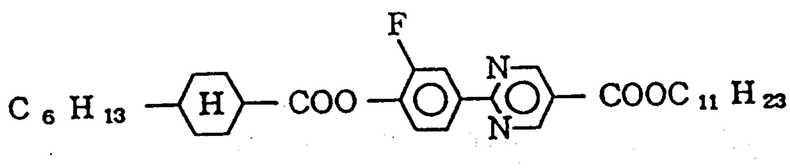

1 − 29

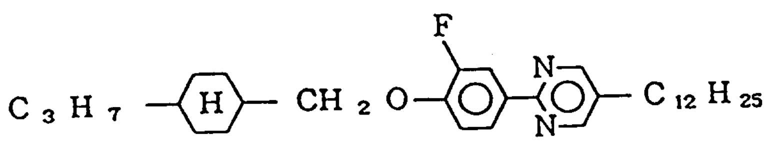

1 − 30

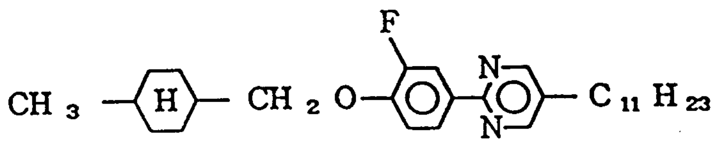

1 − 31

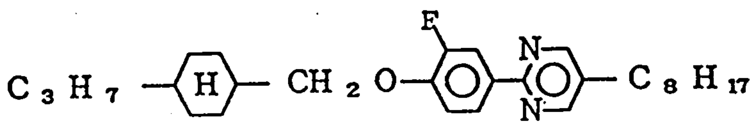

1 − 32

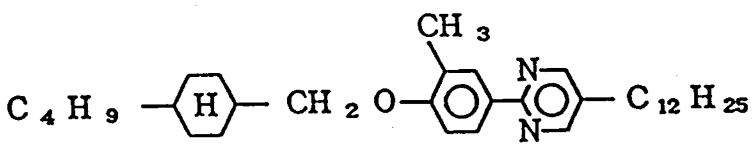

1 − 33

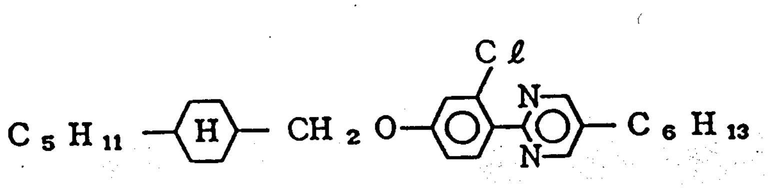

1 − 34

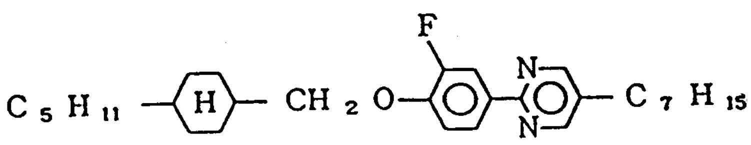

1 − 35

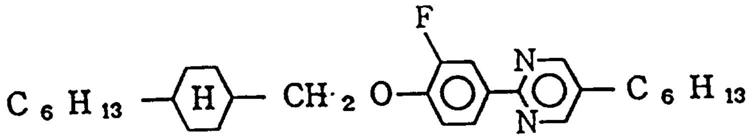

1 − 36

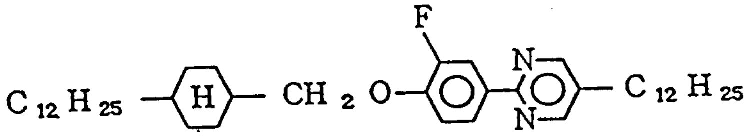

1 − 37

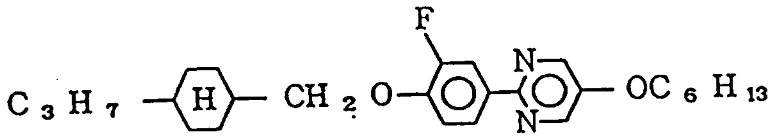

1 − 38

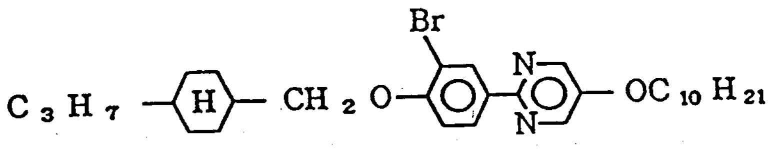

1 − 39

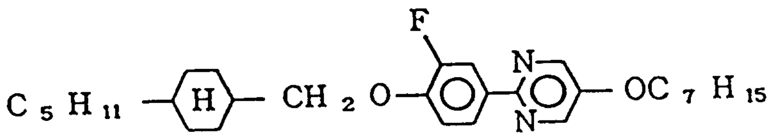

1 − 40

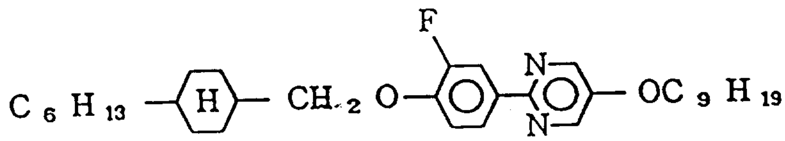

1 − 41

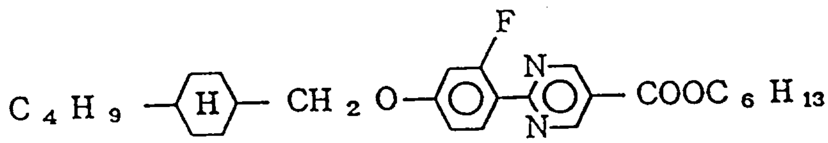

1 − 42

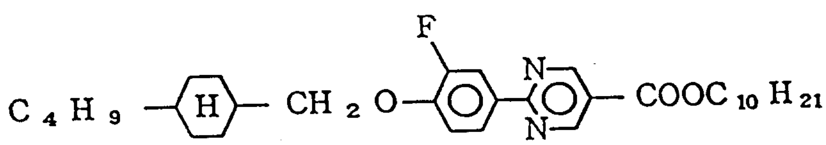

1 − 43

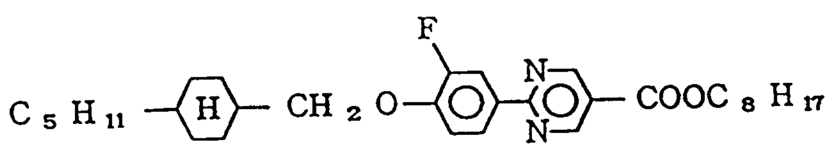

1 − 44

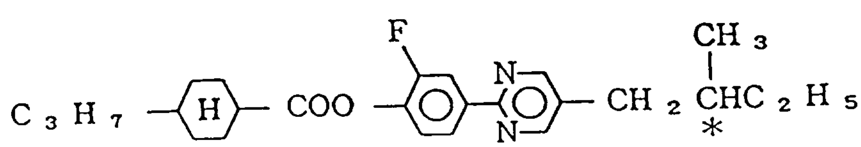

1 − 45

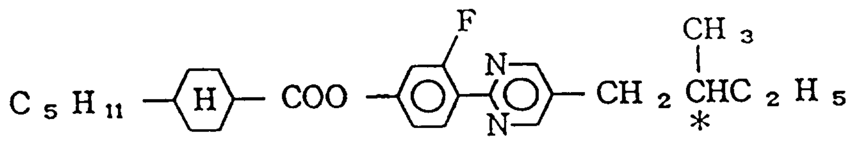

1 − 46

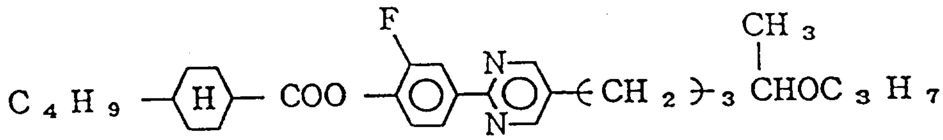

1 − 47

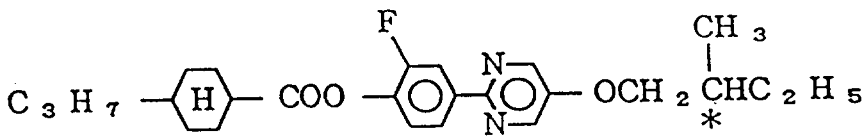

1 − 48

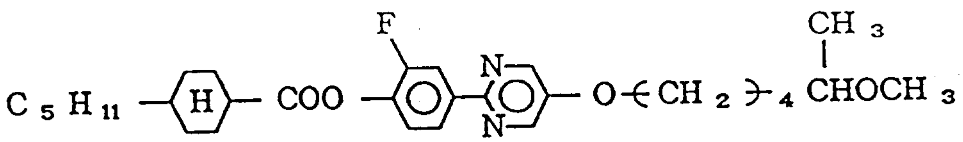

1 − 49

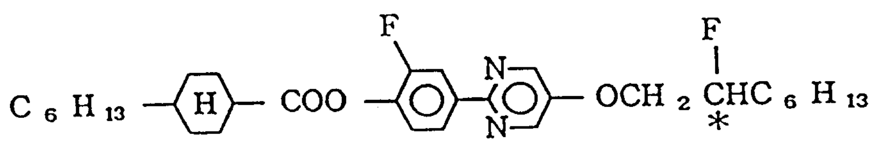

1－50

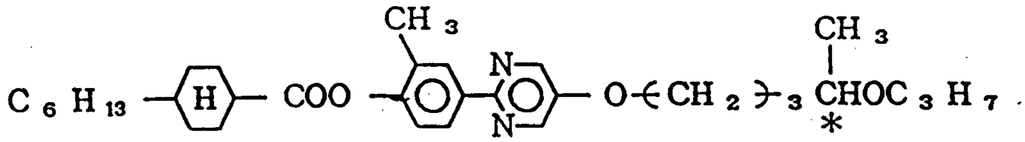

1－51

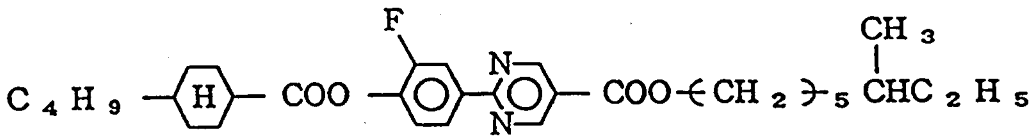

1－52

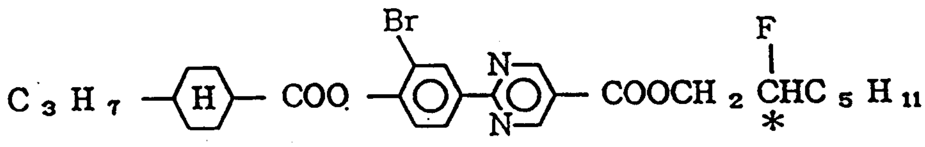

1－53

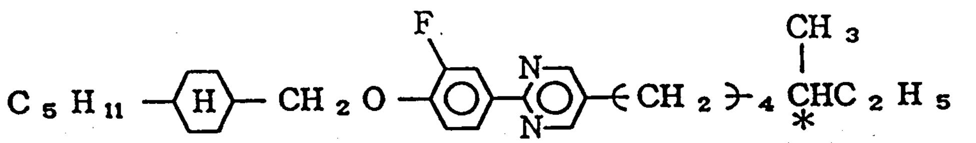

1－54

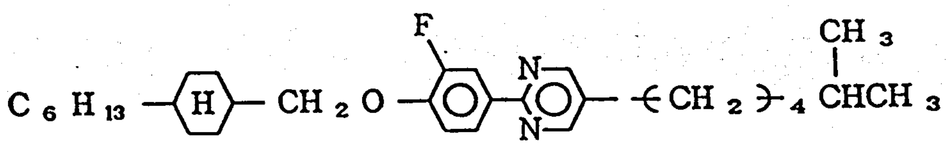

1－55

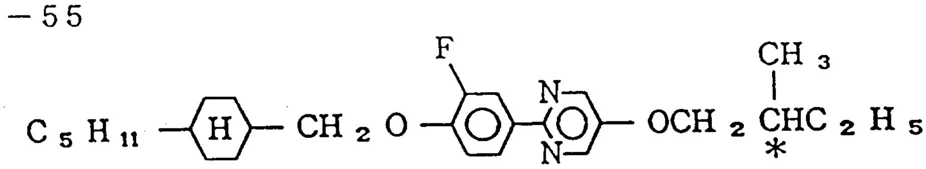

1－56

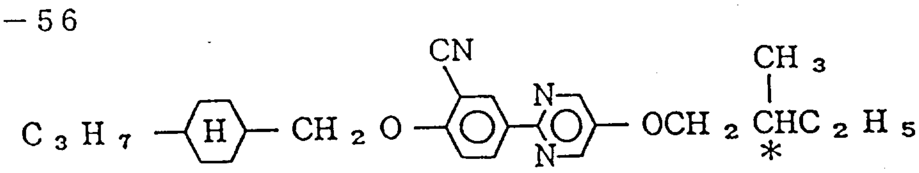

1－57

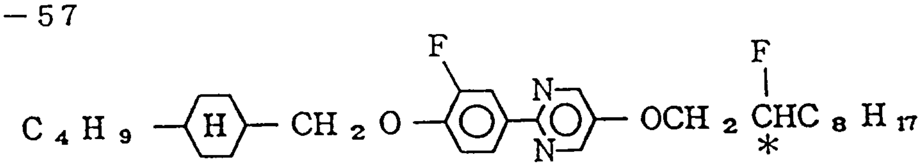

1－58

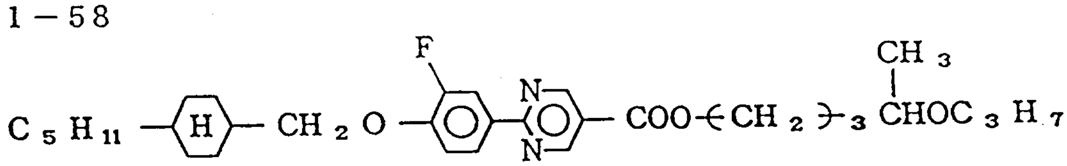

1－59

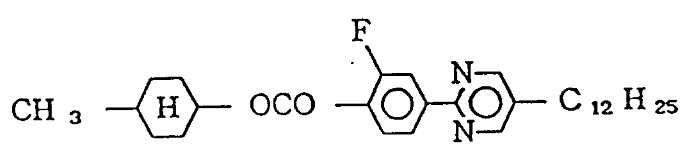

1 − 60

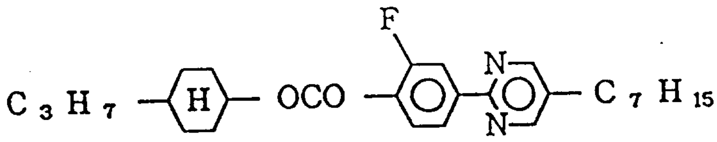

1 − 61

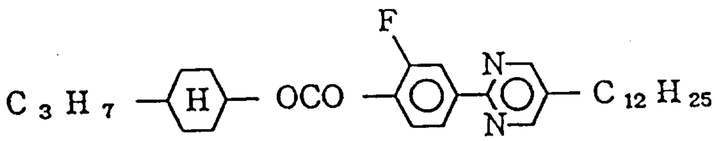

1 − 62

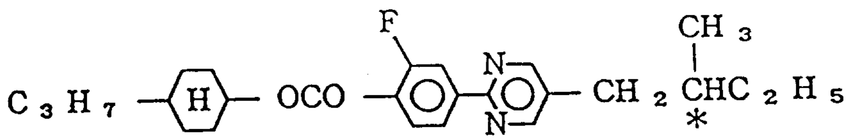

1 − 63

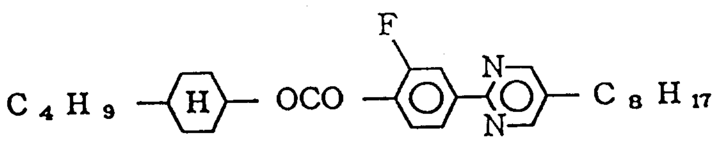

1 − 64

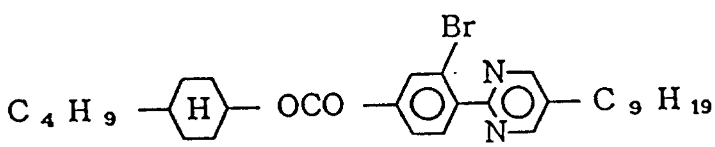

1 − 65

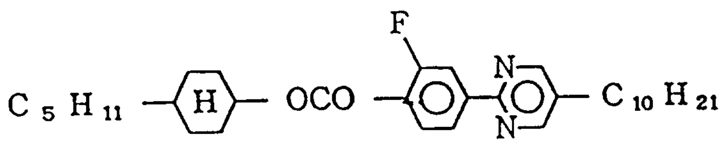

1 − 66

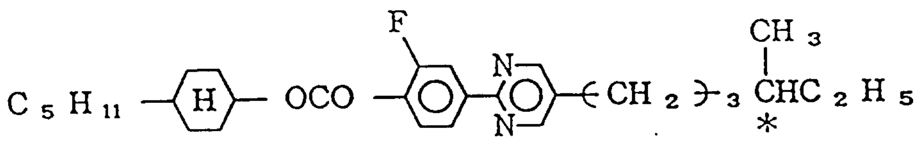

1 − 67

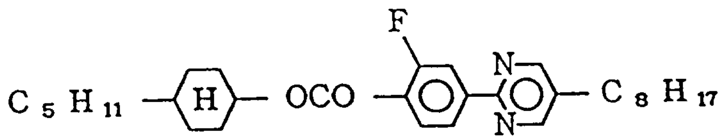

1 − 68

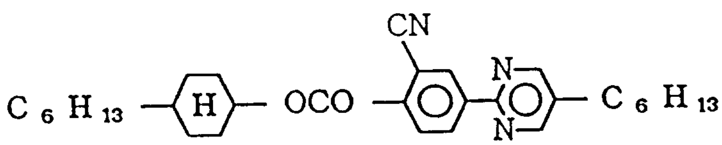

1 − 69

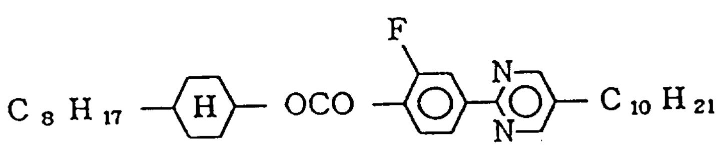

1 − 7 0

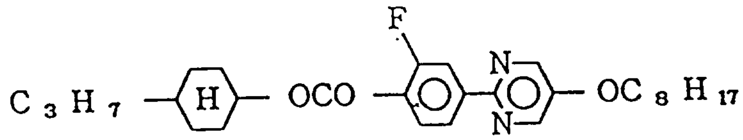

1 − 7 1

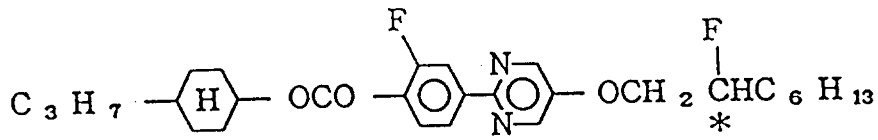

1 − 7 2

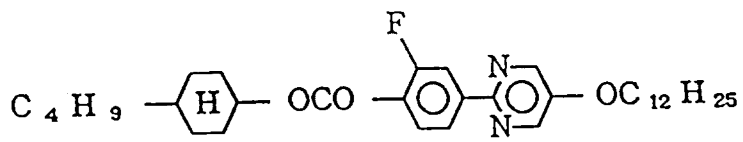

1 − 7 3

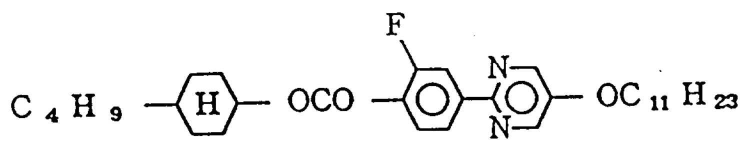

1 − 7 4

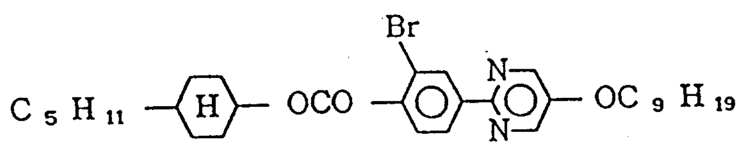

1－75

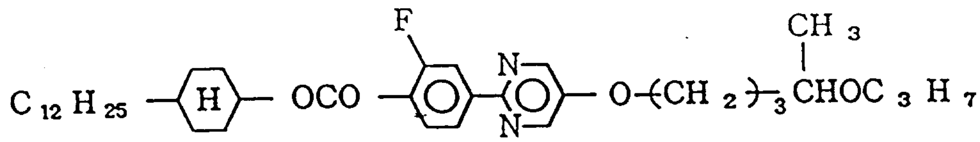

1－76

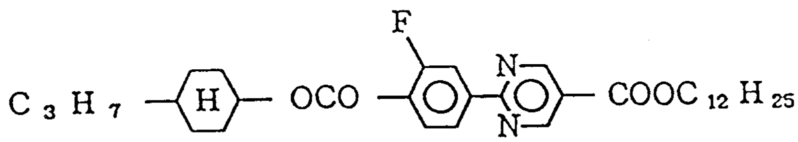

1－77

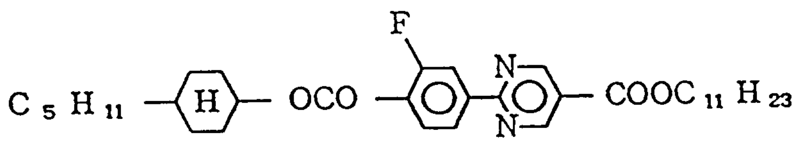

1－78

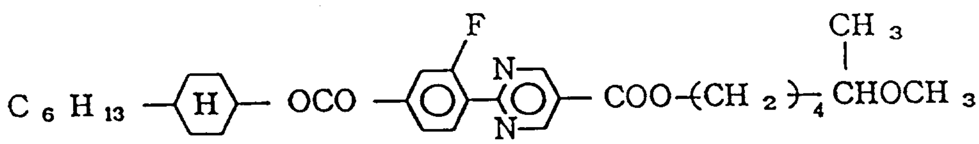

1－79

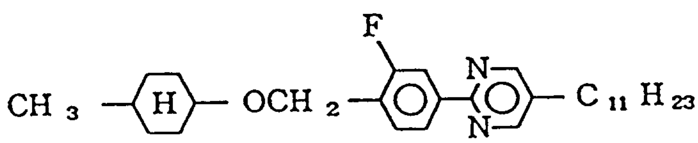

1 − 80

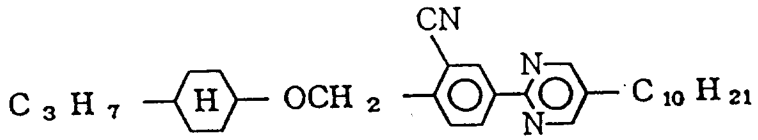

1 − 81

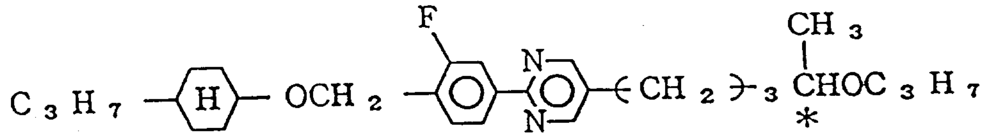

1 − 82

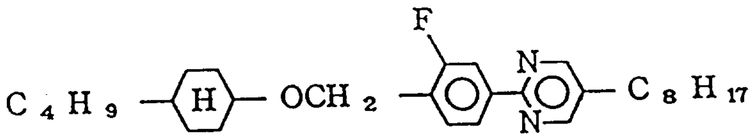

1 − 83

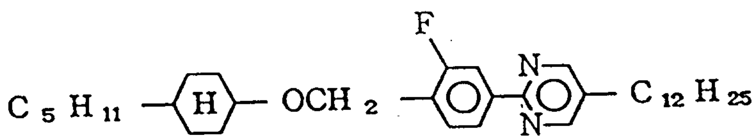

1 − 84

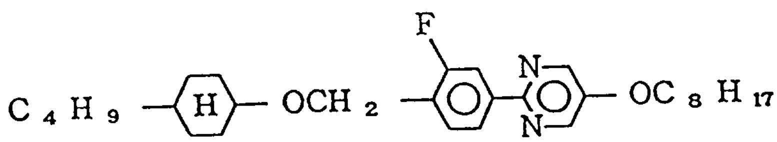

1 − 85

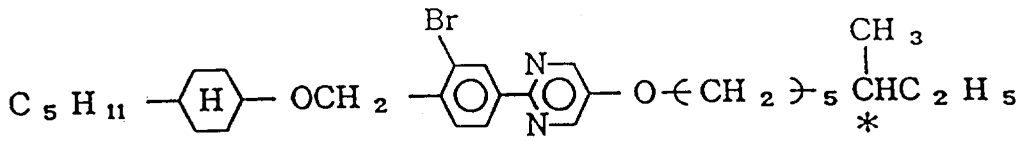

1 − 86

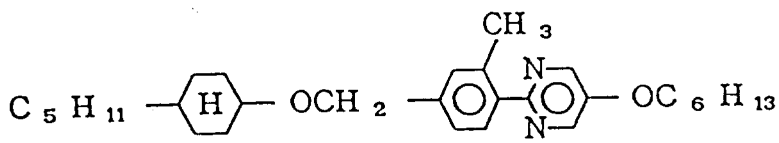

1 − 87

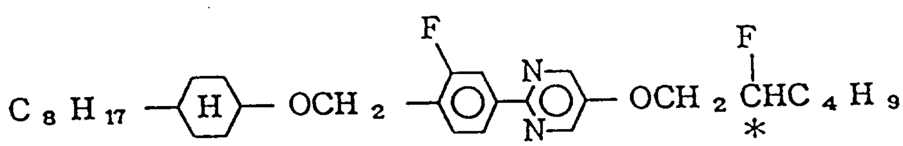

1 − 88

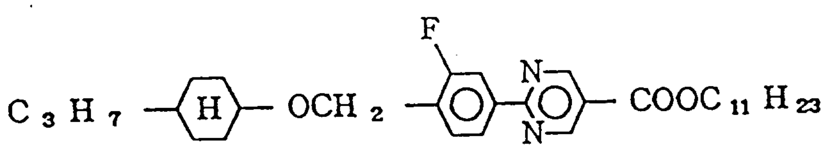

1 − 89

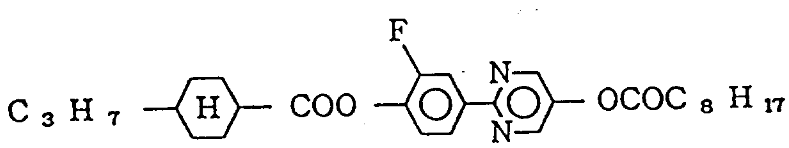

1－90

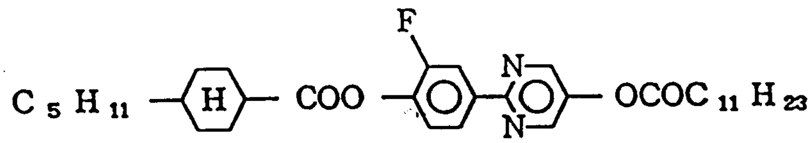

1－91

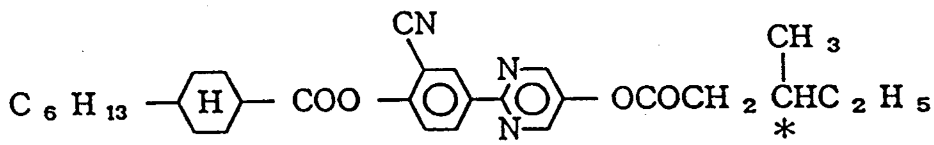

1－92

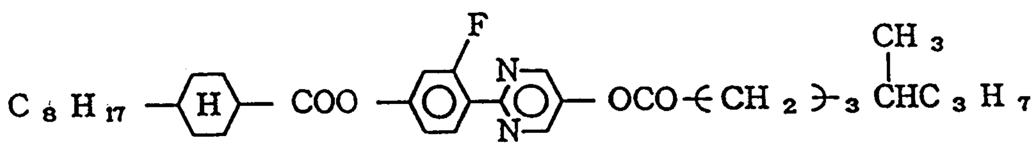

1－93

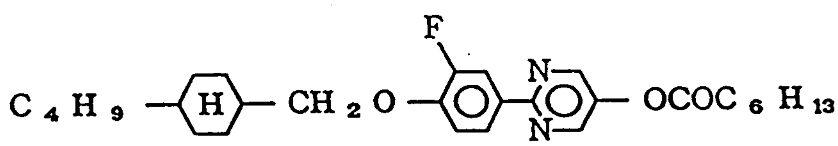

1－94

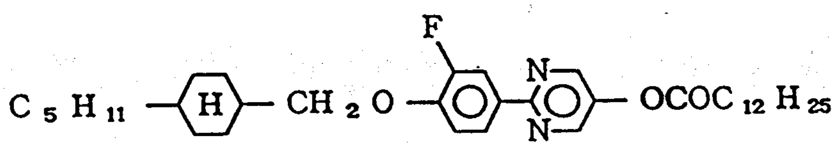

1 − 95

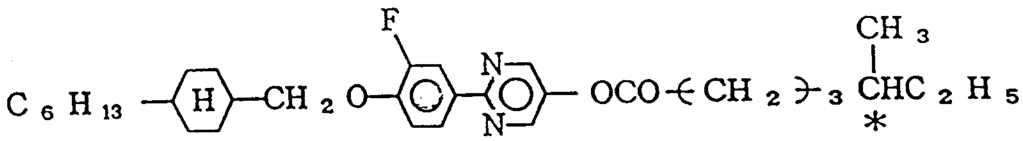

1 − 96

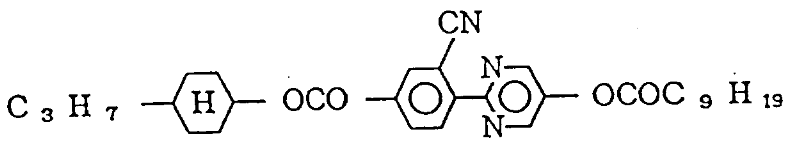

1 − 97

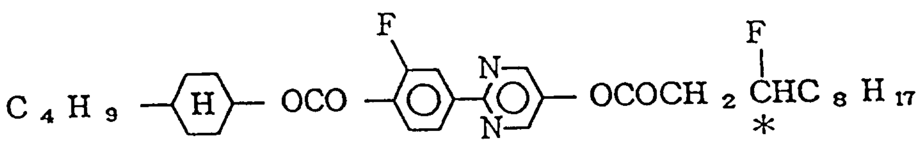

1 − 98

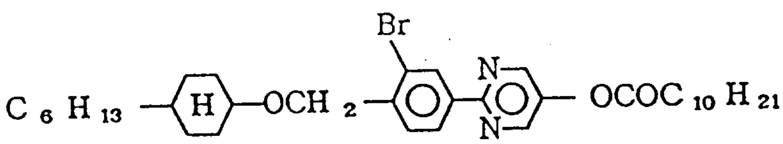

1 − 99

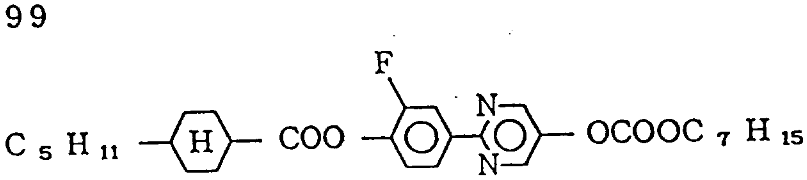

1－100

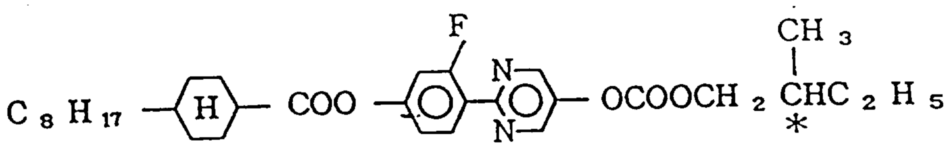

1－101

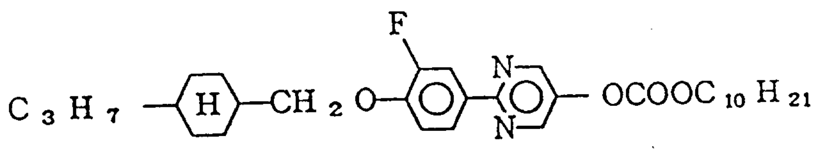

1－102

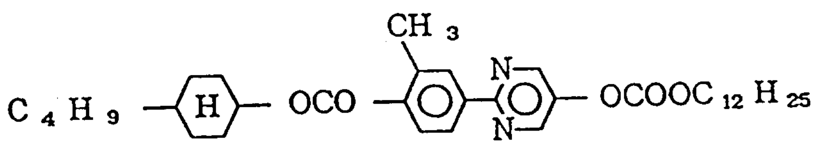

1－103

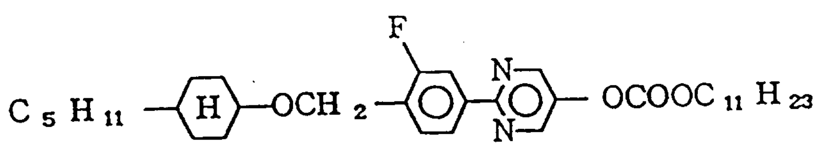

1－104

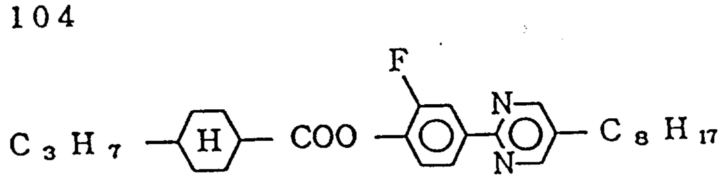

1 − 105

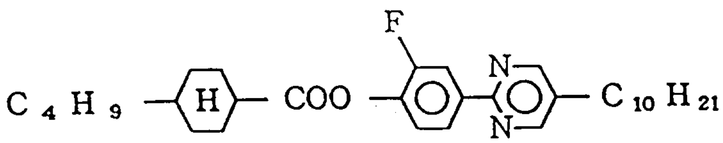

1 − 106

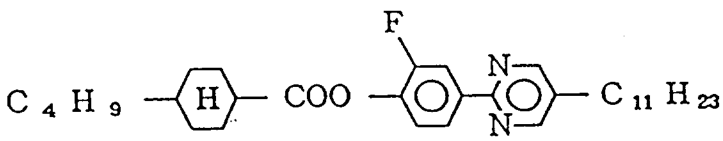

1 − 107

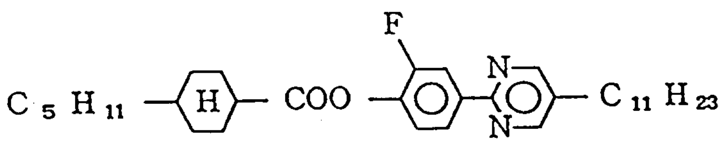

1 − 108

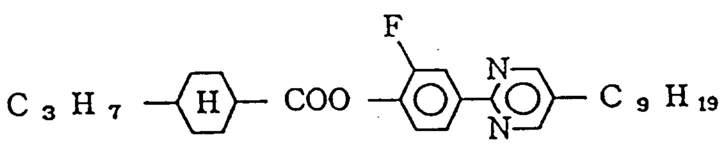

1 − 109

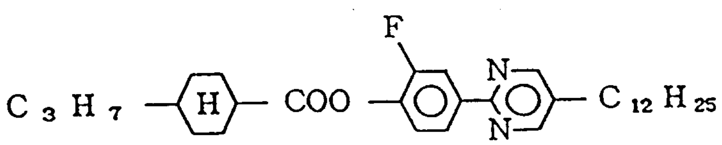

1 − 110

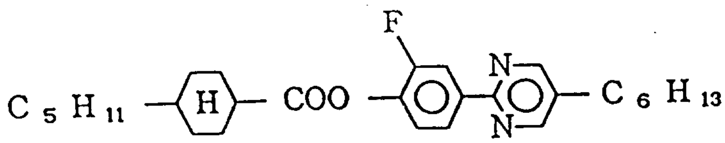

1 − 111

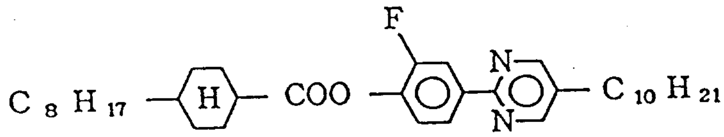

1 − 112

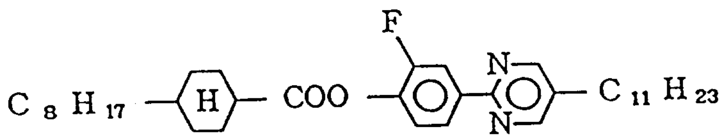

1 − 113

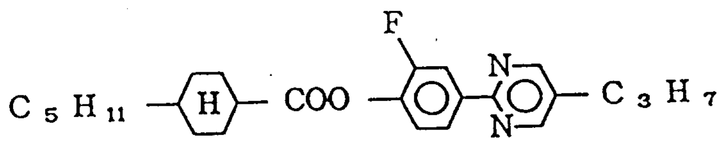

1 − 114

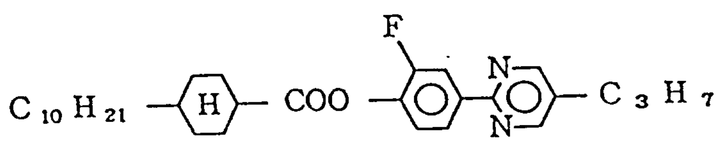

1 − 115

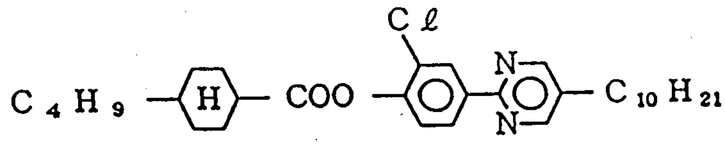

1 − 116

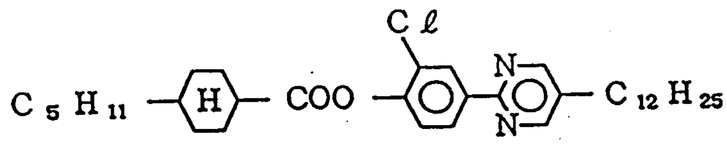

**4.** Flüssigkristallmischung nach Anspruch 1, die eine andere mesomorphe Verbindung umfaßt die von denen, die durch die Formein (I) und (III) wiedergegeben werden, verschieden ist, und pro 100 Masseteile der erwähnten anderen mesomorphen Verbindung je 1 bis 300 Masseteile einer Verbindung der Formel (I) und einer Verbindung der Formel (III) umfaßt.

**5.** Flüssigkristallmischung nach Anspruch 1, die eine andere mesomorphe Verbindung, die von denen, die durch die Formeln (I) und (III) wiedergegeben werden, verschieden ist, und zwei oder mehr Spezies von Verbindungen aus jeder von mindestens einer Gruppe von Verbindungen der Formeln (I) und (III) in einer Gesamtmenge von 1 bis 500 Masseteilen pro 100 Masseteile der erwähnten anderen mesomorphen Verbindung umfaßt.

**6.** Flüssigkristallmischung nach Anspruch 2, die eine andere mesomorphe Verbindung umfaßt, die von denen, die durch die Formein (I), (II) und (III) wiedergegeben werden, verschieden ist, und pro 100 Masseteile der erwähnten anderen mesomorphen Verbindung je 1 bis 300 Masseteile einer Verbindung der Formel (I), einer Verbindung der Formel (II) und einer Verbindung der Formel (III) umfaßt.

**7.** Flüssigkristallmischung nach Anspruch 2, die eine andere mesomorphe Verbindung, die von denen, die durch die Formeln (I), (II) und (III) wiedergegeben werden, verschieden ist, und zwei oder mehr Spezies von Verbindungen aus jeder von mindestens einer Gruppe von Verbindungen der Formeln (I), (II) und (III) in einer Gesamtmenge von 1 bis 500 Masseteilen pro 100 Masseteile der erwähnten anderen mesomorphen Verbindung umfaßt.

**8.** Flüssigkristallmischung nach einem der Ansprüche 1 bis 7, die eine chirale smektische Phase hat.

**9.** Flüssigkristallvorrichtung, die ein Paar Elektrodenplatten und eine Flüssigkristallmischung nach einem der Ansprüche 1 bis 8, die zwischen den Elektrodenplatten angeordnet ist, umfaßt.

**10.** Flüssigkristallvorrichtung nach Anspruch 9, die ferner auf den Elektrodenplatten eine Ausrichtungseinstellungsschicht aufweist.

**11.** Flüssigkristallvorrichtung nach Anspruch 10, bei der die Ausrichtungseinstellungsschicht einer Reibbehandlung unterzogen worden ist.

**12.** Flüssigkristallvorrichtung nach Anspruch 9, bei der das Paar Elektrodenplatten mit einem Abstand dazwischen angeordnet sind, der ausreichend gering ist, um die schraubenförmige Struktur des Flüssigkristalls zu lockern bzw. abzuwickeln.

## Revendications

**1.** Composition de cristal liquide, comprenant :

au moins un composé mésomorphe représenté par la formule (I) suivante :

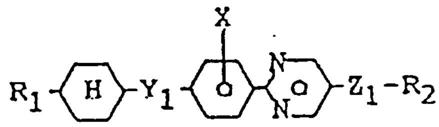

(I),

dans laquelle $R_1$ représente un groupe n-alkyle ayant 1 à 16 atomes de carbone ; $R_2$ représente un groupe optiquement actif ou inactif choisi entre les groupes (i) et (iv) suivants :

(i) un groupe n-alkyle ayant 1 à 16 atomes de carbone ;

(ii) un groupe

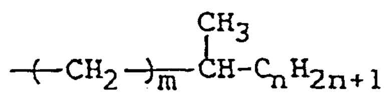

dans lequel m a une valeur de 1 à 7 et n a une valeur de 2 à 9, sous réserve que $3 \leqq m+n \leqq 14$ ;

(iii) un groupe

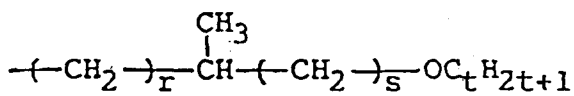

dans lequel R a une valeur de 0 à 7, s est égal à 0 ou 1 et t a une valeur de 1 à 14, sous réserve que $1 \leqq r+s+t \leqq 14$ ; et

(iv) un groupe

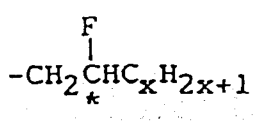

dans lequel X a une valeur de 1 à 14 et le signe * désigne un centre optiquement actif ;

$Y^1$ représente un groupe -COO-, -OCO-, $-CH_2O-$ ou $-OCH_2-$ ; $Z_1$ représente une liaison simple, -O-, un groupe -COO-, -OCO- ou -OCOO- ; et X représente un halogène, un groupe cyano ou un groupe méthyle ; et

au moins un composé mésomorphe représenté par la formule (III) suivante :

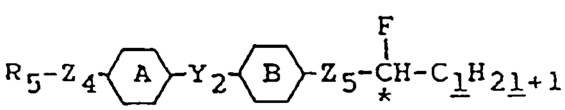

(III),

dans laquelle $R_5$ représente un groupe alkyle linéaire ou ramifié ayant 1 à 18 atomes de carbone ; $Y_2$ représente une liaison simple, un groupe -COO-, -OCO-, -COS-, SCO-, $CH_2O-$, $-OCH_2-$ ou =CH=CH-COO- ; $Z_4$ représente une liaison simple, -O-, un groupe -COO- ou -OCO- ; $Z_5$ représente un groupe $-OCH_2-$,$-COOCH_2-$, -OCO- ou

$$-O-(-CH_2-)_K-O-CH_2,$$

le groupe

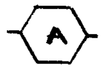

représente un groupe

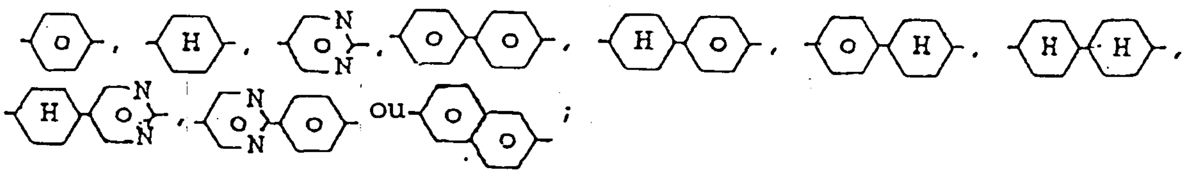

le groupe

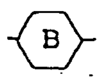

représente un groupe

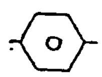 ou 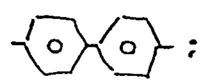

l a une valeur de 1 à 12 et k a une valeur de 1 à 4.

**2.** Composition de cristal liquide comprenant au moins un composé mésomorphe représenté par la formule (I) suivante :

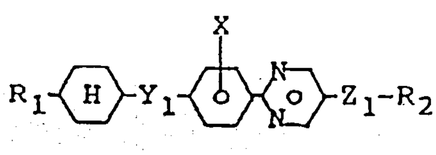 (I),

dans laquelle $R_1$ représente un groupe n-alkyle ayant 1 à 16 atomes de carbone ; $R_2$ représente un groupe optiquement actif ou inactif choisi entre les groupes (i) à (iv) suivants :

(i) un groupe n-alkyle ayant 1 à 16 atomes de carbone ;

(ii) un groupe

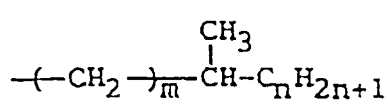

dans lequel m a une valeur de 1 à 7 et n a une valeur de 2 à 9, sous réserve que $3 \leqq m+n \leqq 14$ ;

(iii) un groupe

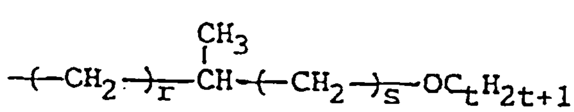

dans lequel r a une valeur de 0 à 7, s est égal à 0 ou 1 et t a une valeur de 1 à 14, sous réserve que $1 \leqq r+s+t \leqq 14$ ; et

(iv) un groupe

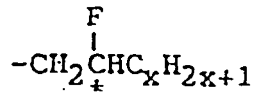

dans lequel x a une valeur de 1 à 14, et le signe * désigne un centre optiquement actif ;
$Y_1$ représente un groupe -COO-, -OCO-, -$CH_2O$- ou -$OCH_2$- ; $Z_1$ représente une liaison simple, -O-, un groupe -COO-, -OCO- ou -OCOO- ; X représente un halogène, un groupe cyano ou un groupe méthyle ;

au moins un composé mésomorphe représenté par la formule (II) suivante :

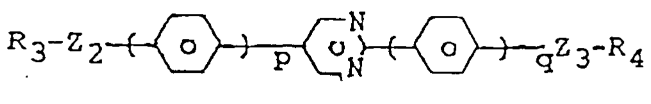 (II),

dans laquelle $R_3$ et $R_4$ représentent respectivement un groupe alkyle linéaire ou ramifié optiquement inactif ayant 1 à 18 atomes de carbone pouvant porter un groupe alkoxy en $C_1$ à $C_{12}$ ; $Z_2$ et $Z_3$ représentent respectivement une liaison simple, - O-, -OCO-, -COO- ou -OCOO- ; et p et q sont respectivement égaux à 0, 1 ou 2 ; et
au moins un composé mésomorphe représenté par la formule (III) suivante :

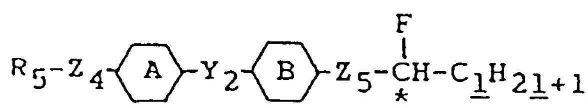 (III),

dans laquelle $R_5$ représente un groupe alkyle linéaire ou ramifié ayant 1 à 18 atomes de carbone ; $Y_2$ représente une liaison simple, un groupe -COO-, -OCO-, -COS-, SCO-, $CH_2O$-, -$OCH_2$- ou -CH=CH-COO- ; $Z_4$ représente une liaison simple, -O-, un groupe -COO- ou -OCO- ; $Z_5$ représente un groupe -$OCH_2$-, -$COOCH_2$-, -OCO- ou

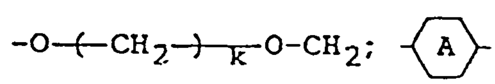

représente un groupe

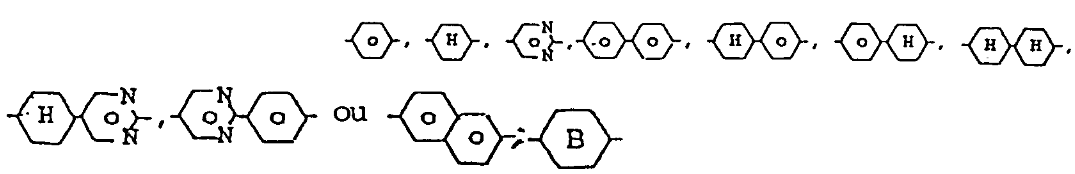

représente un groupe

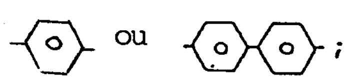

l a une valeur de 1 à 12 et k a une valeur de 1 à 4.

3. Composition de cristal liquide suivant l'une quelconque des revendications 1 et 2, dans laquelle le composé mésomorphe de formule (I) répond à l'une quelconque des formules (1-1) à (1-116) suivantes :

1 − 1

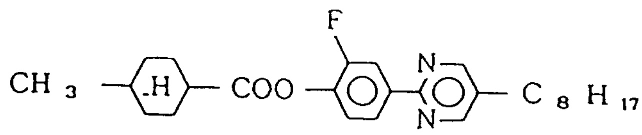

1 − 2

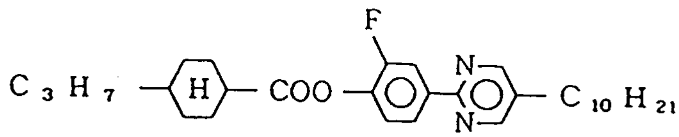

1 − 3

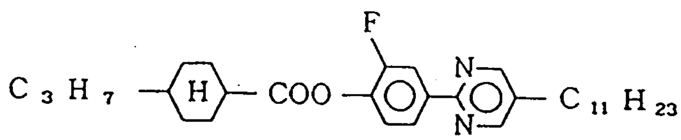

1 − 4

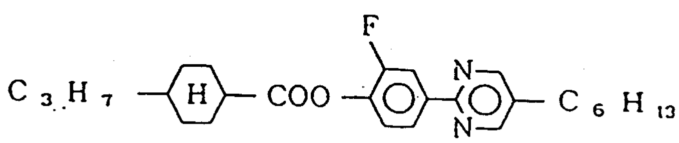

1 − 5

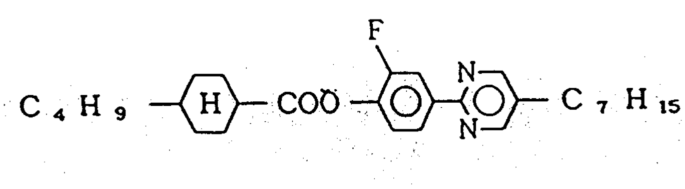

1 − 6

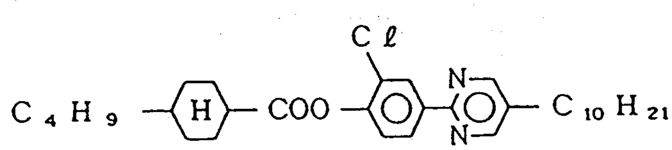

1 − 7

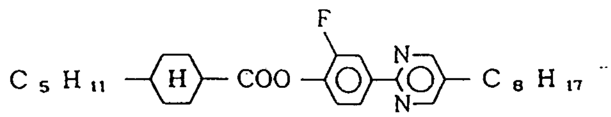

1 − 8

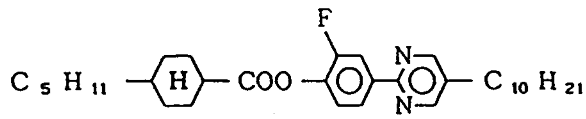

1 − 9

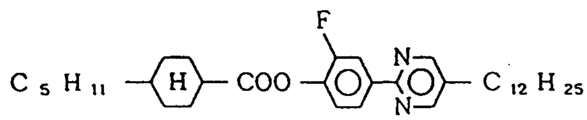

1 − 10

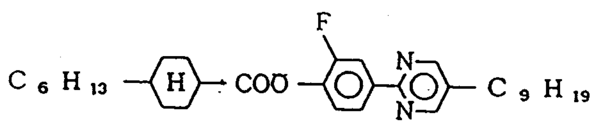

1 − 11

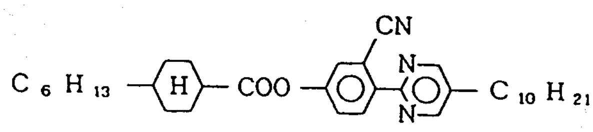

1 − 12

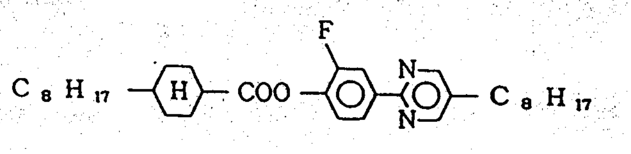

1 − 13

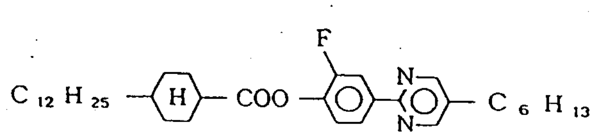

1 − 14

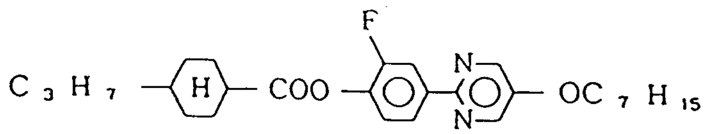

1 − 15

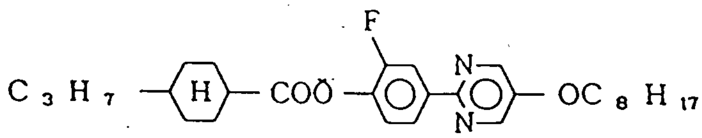

1 − 16

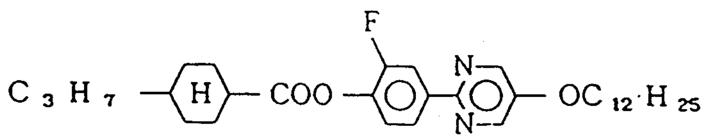

1 − 17

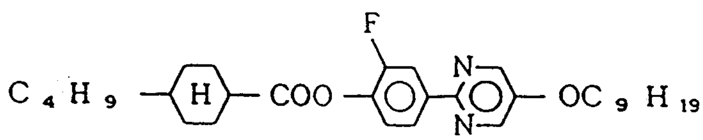

1 − 18

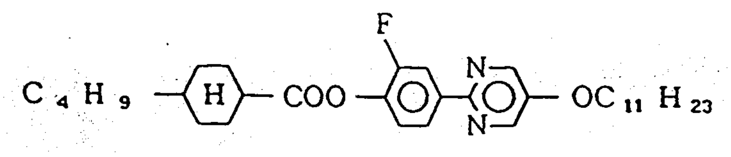

1 − 19

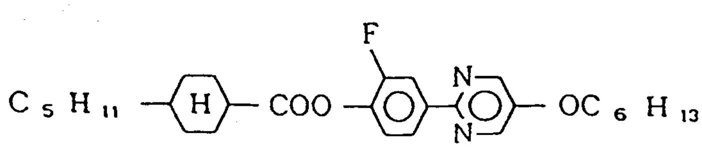

1 − 20

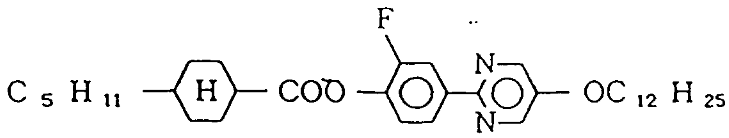

1 − 21

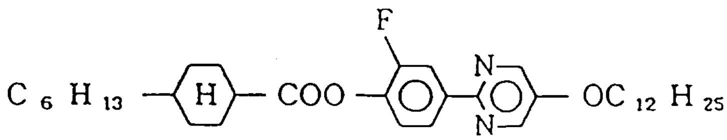

1 − 22

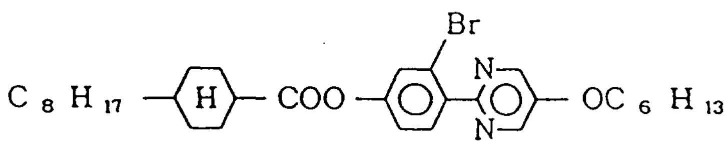

1 − 23

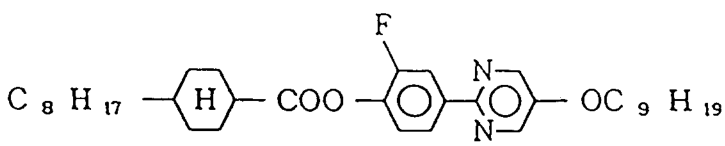

1 − 24

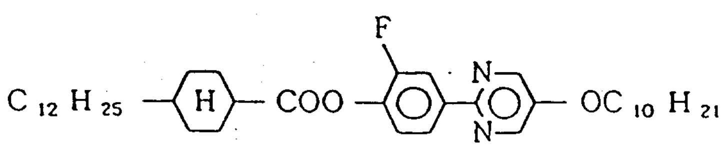

1 − 25

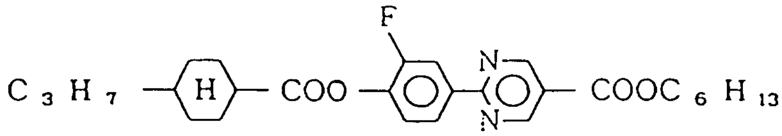

1 − 26

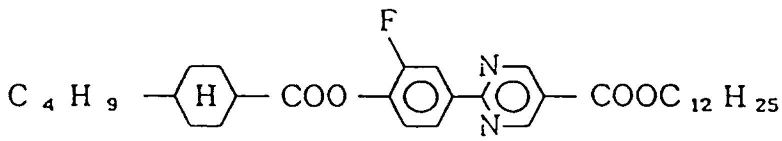

1 − 27

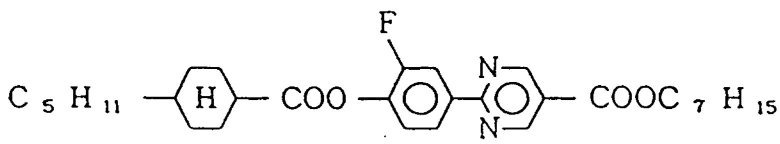

1 − 28

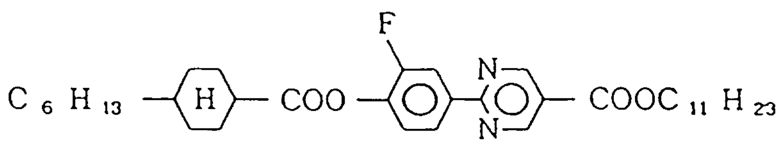

1 − 29

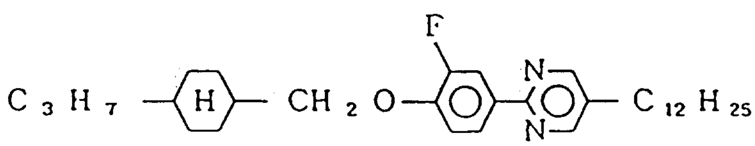

1 − 30

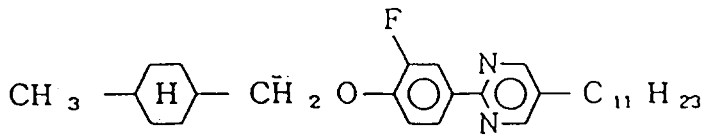

1 − 31

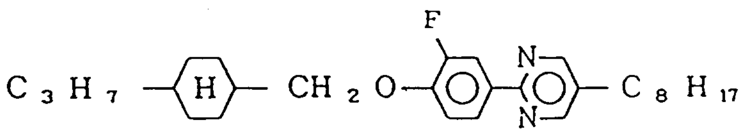

1 − 32

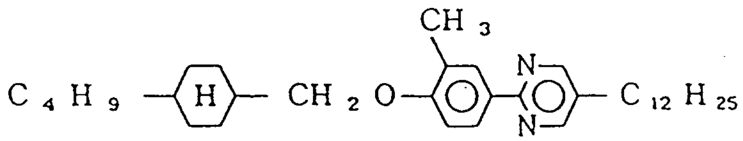

1 − 33

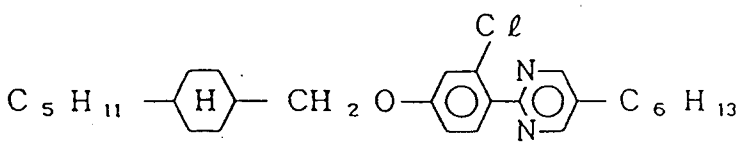

1 − 34

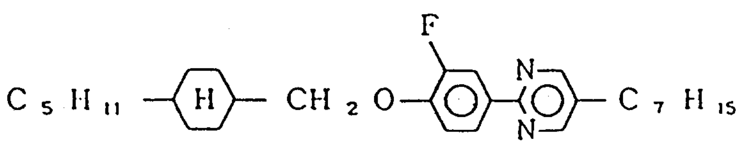

1 − 35

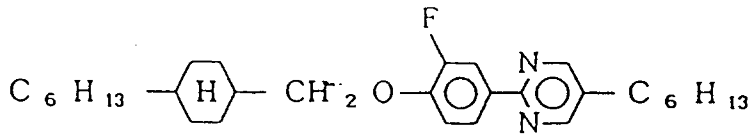

1 − 36

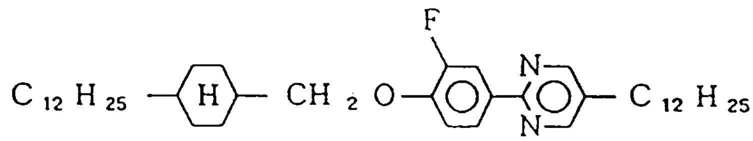

1 − 37

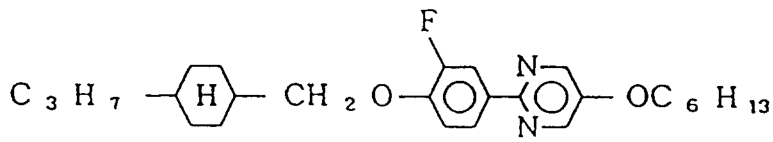

1 − 38

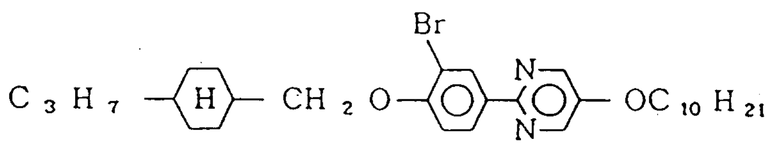

1 − 39

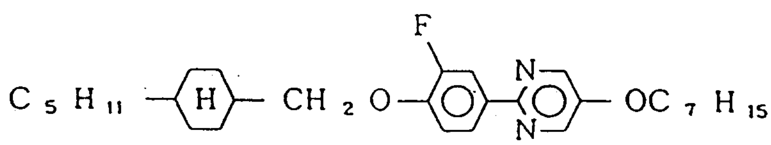

1 − 40

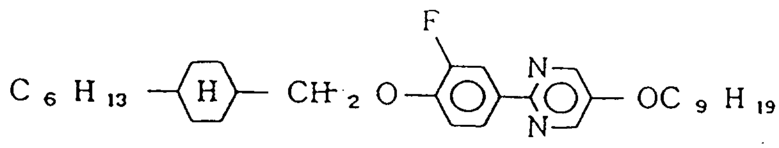

1 − 41

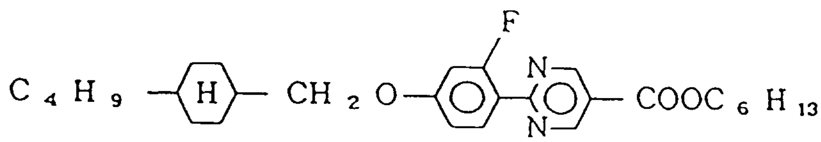

1 − 42

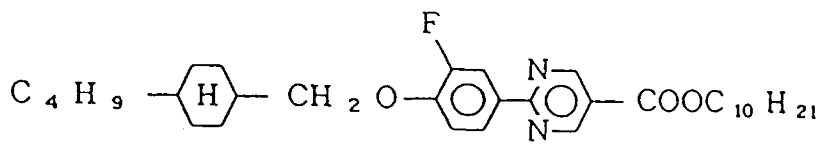

1 − 43

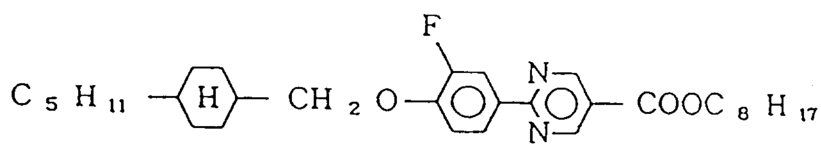

1 − 44

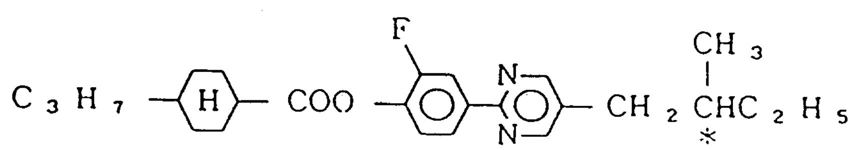

1 − 45

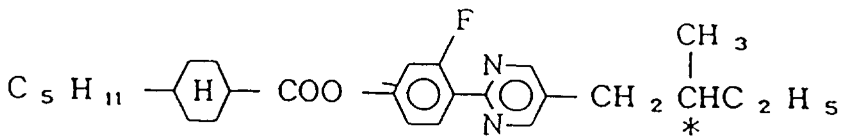

1 − 46

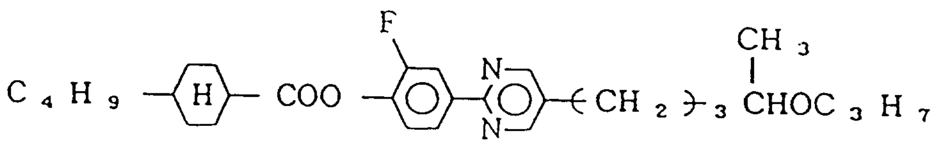

1 − 47

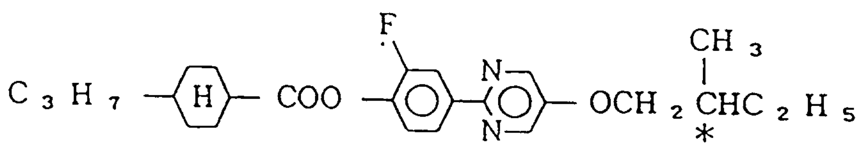

1 − 48

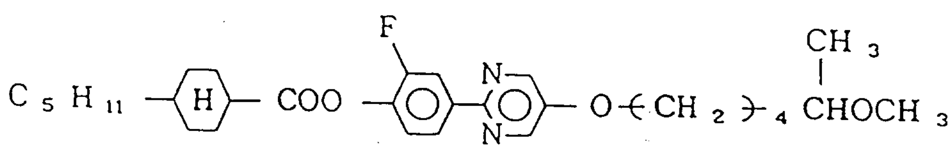

1 − 49

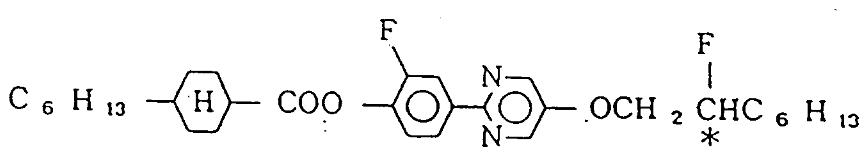

1 − 50

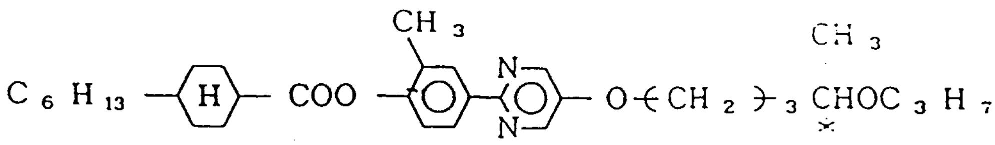

1 − 51

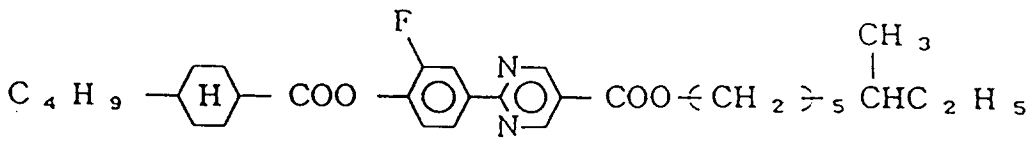

1 − 52

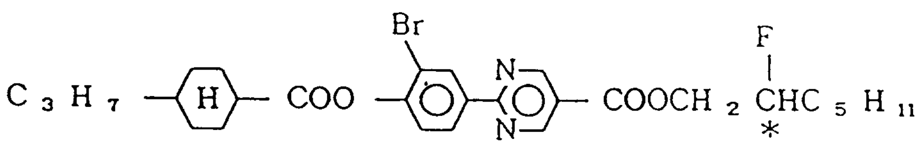

1 − 53

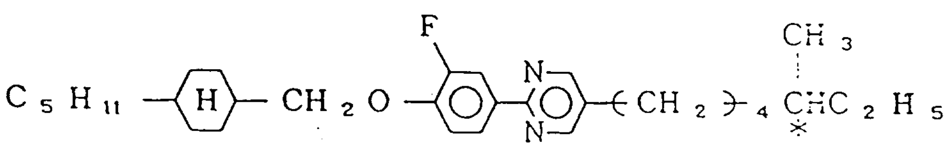

1 − 54

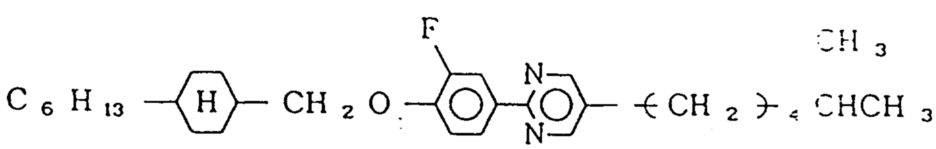

1 − 55

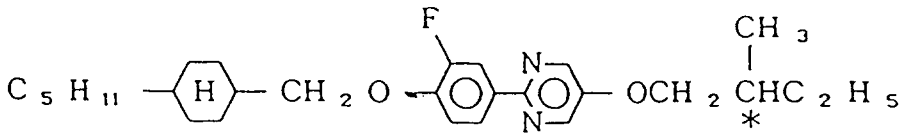

1 − 56

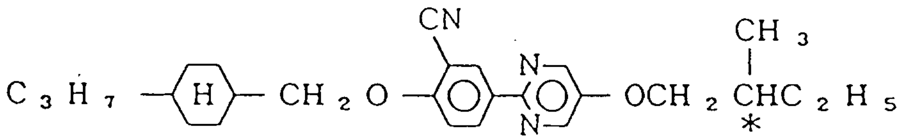

1 − 57

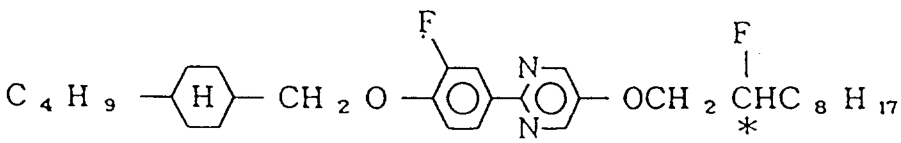

1 − 58

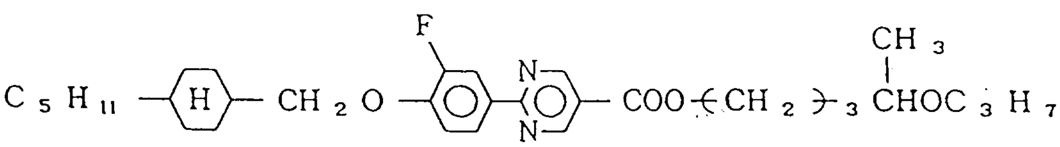

1 − 59

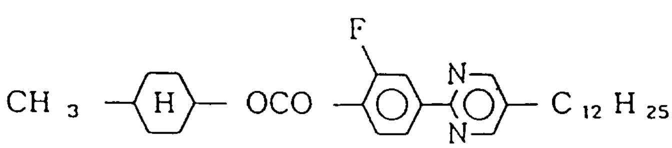

1 − 60

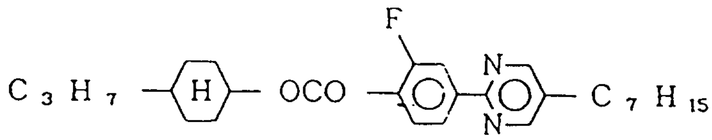

1 − 61

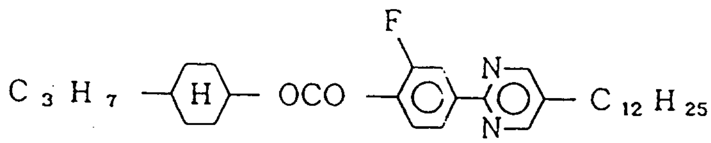

1 − 62

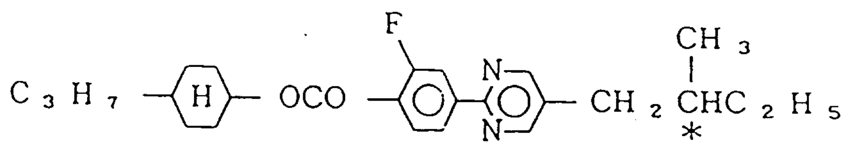

1 − 63

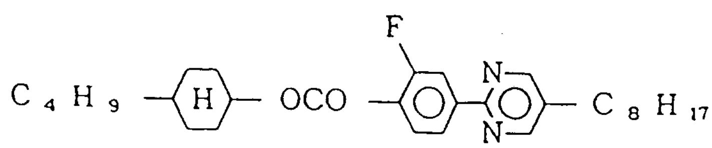

1 − 64

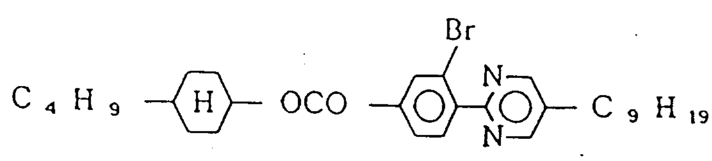

1 − 65

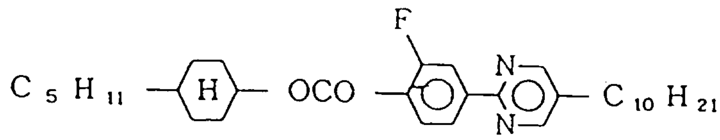

1 − 66

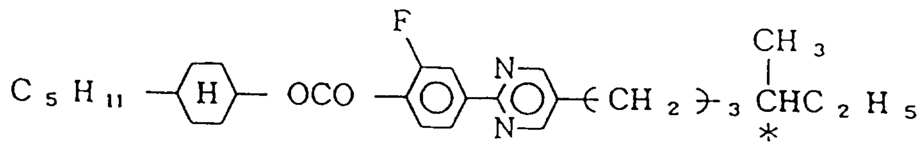

1 − 67

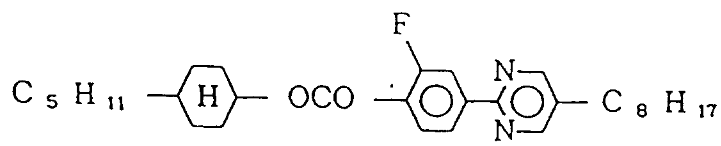

1 − 68

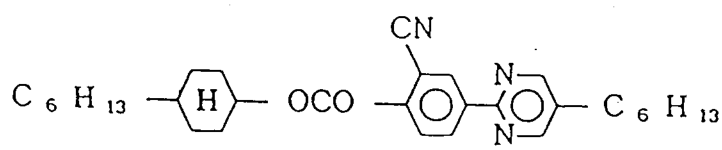

1 − 69

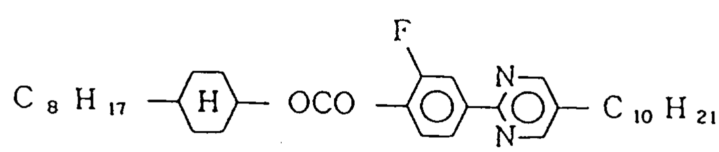

1－70

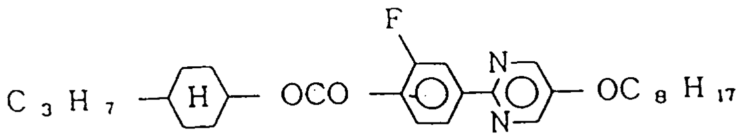

1－71

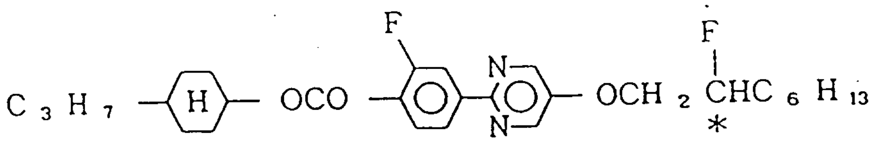

1－72

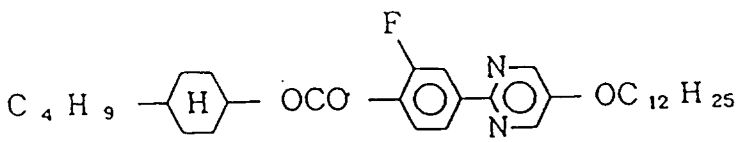

1－73

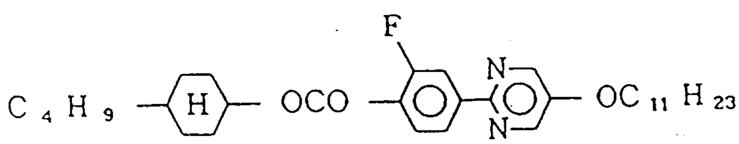

1－74

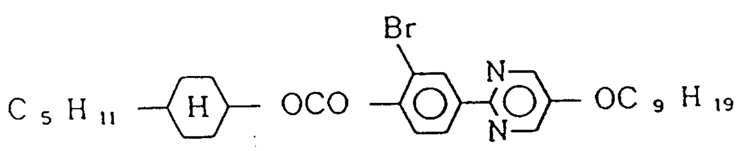

1 − 75

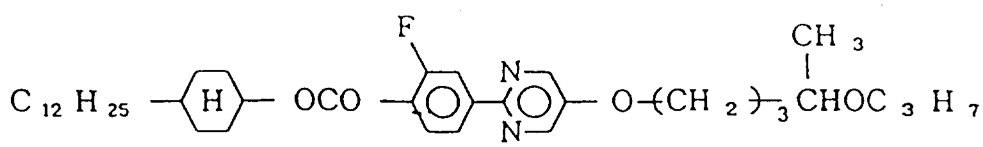

1 − 76

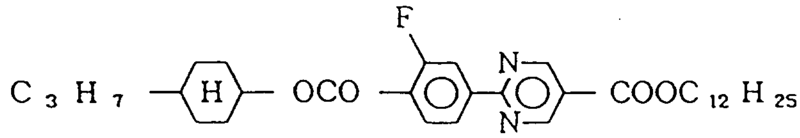

1 − 77

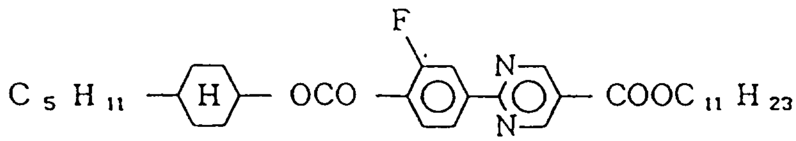

1 − 78

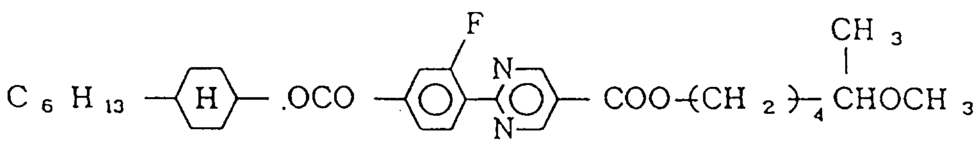

1 − 79

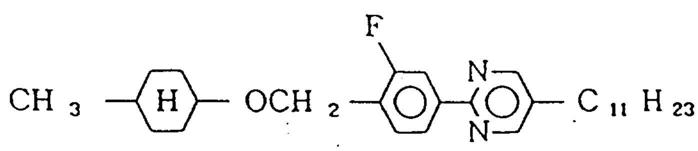

1 − 80

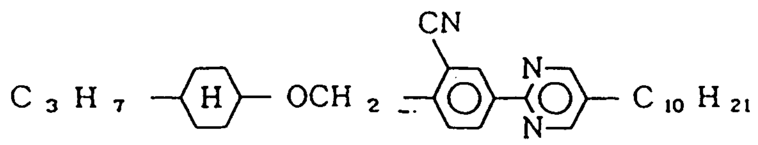

1 − 81

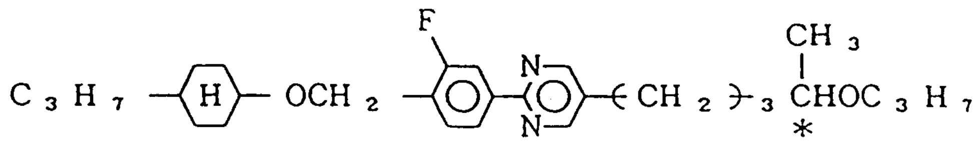

1 − 82

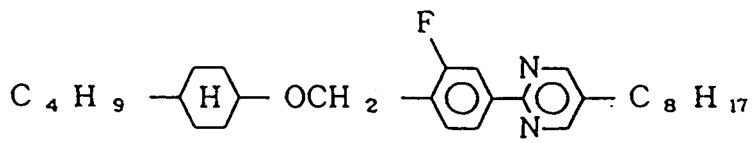

1 − 83

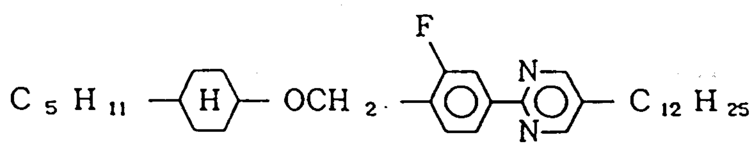

1 − 84

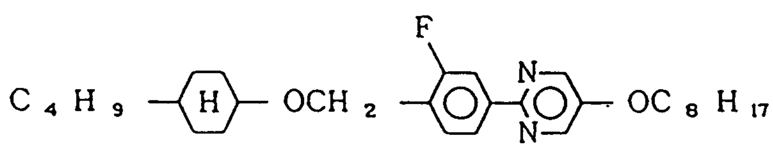

1 − 85

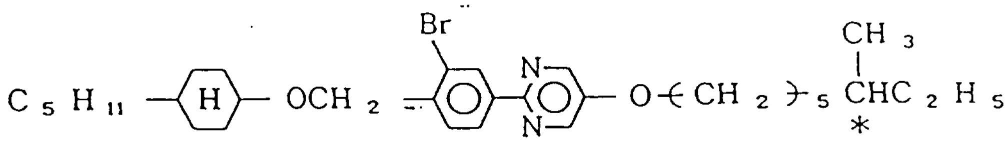

1 − 86

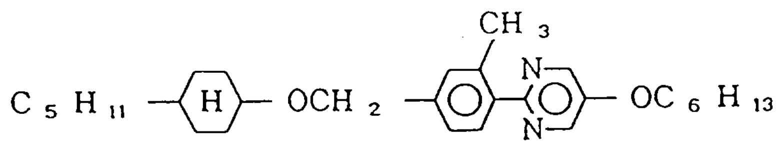

1 − 87

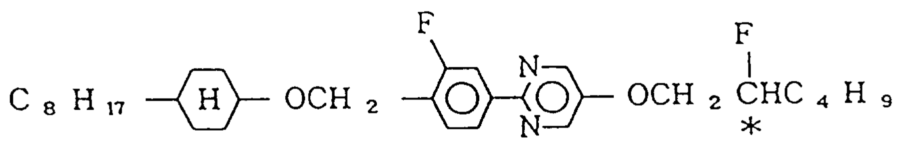

1 − 88

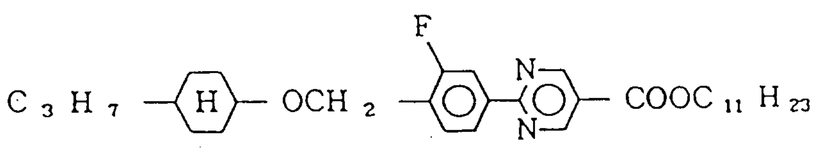

1 − 89

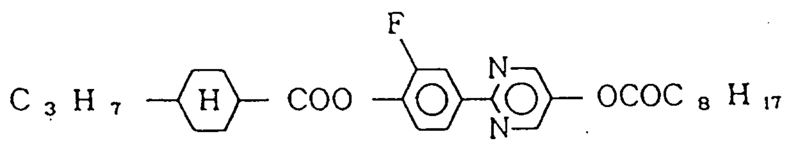

1 − 90

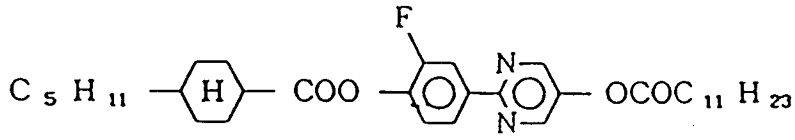

1 − 91

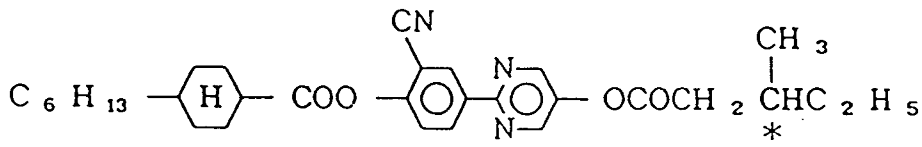

1 − 92

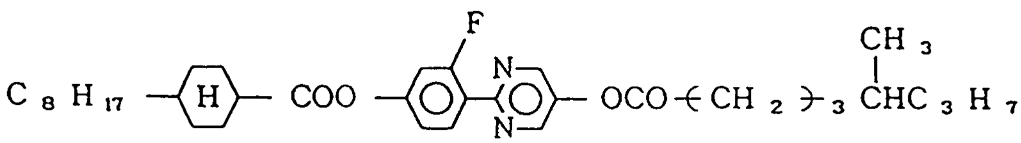

1 − 93

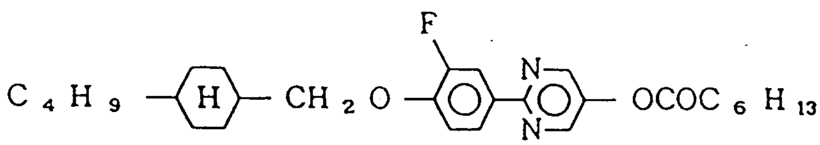

1 − 94

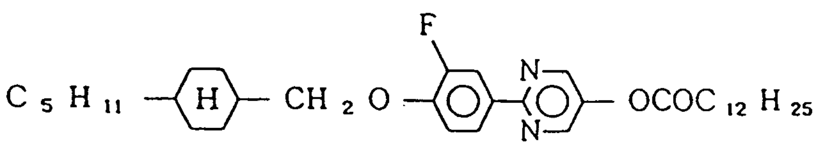

1 − 95

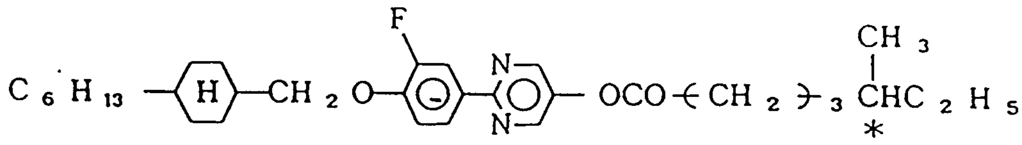

1 − 96

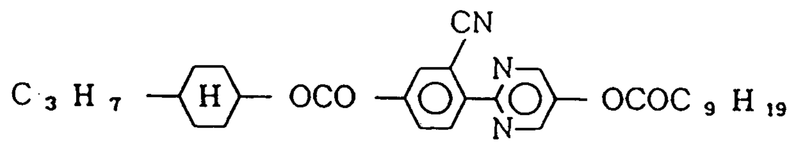

1 − 97

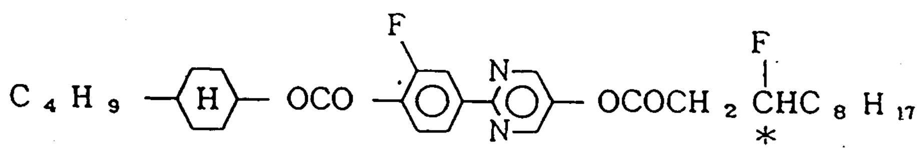

1 − 98

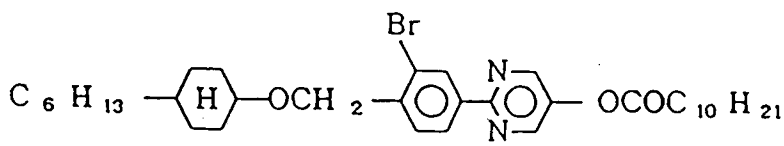

1 − 99

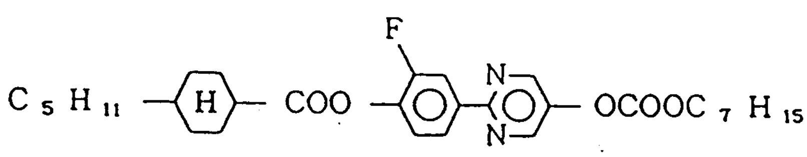

1 − 100

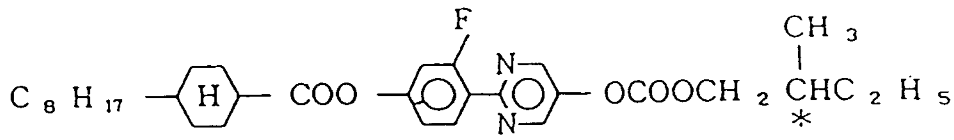

1 − 101

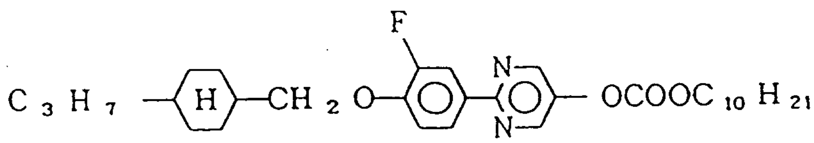

1 − 102

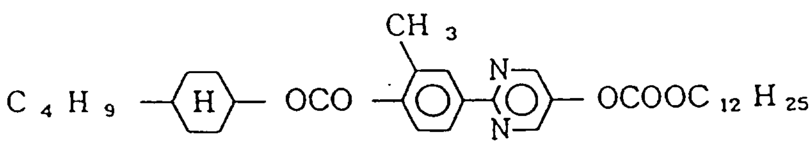

1 − 103

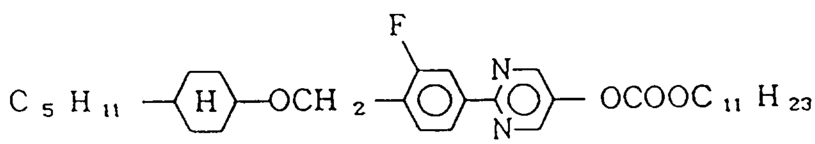

1 − 104

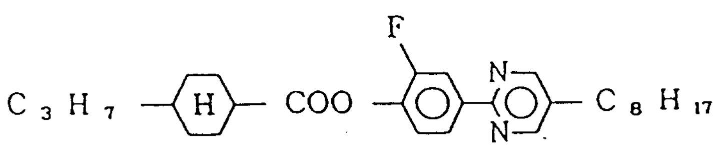

1 − 105

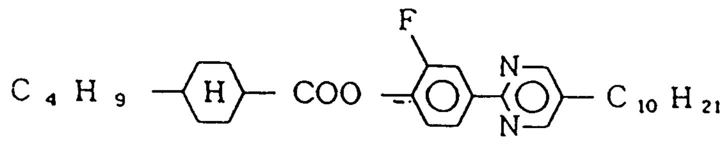

1 − 106

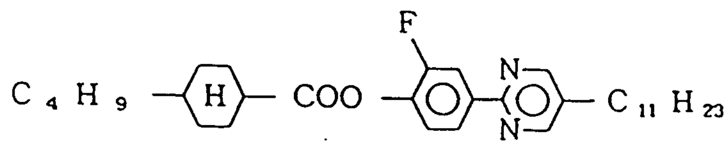

1 − 107

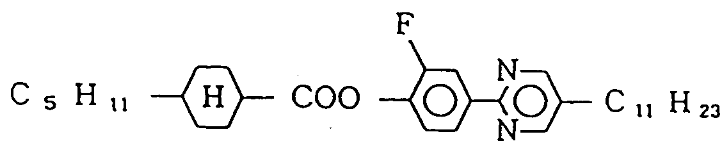

1 − 108

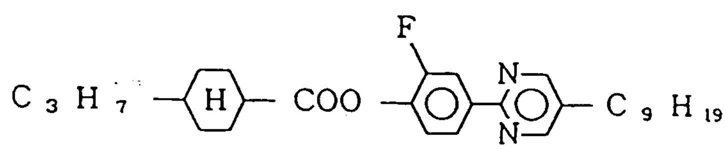

1 − 109

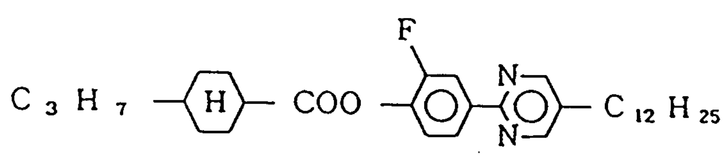

1 − 1 1 0

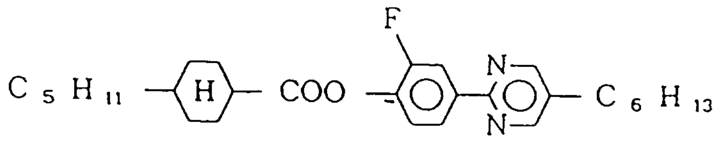

1 − 1 1 1

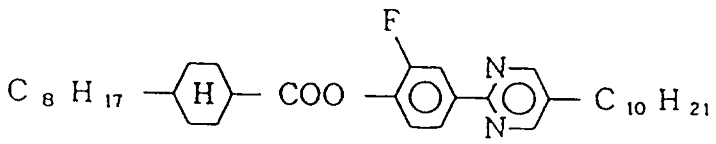

1 − 1 1 2

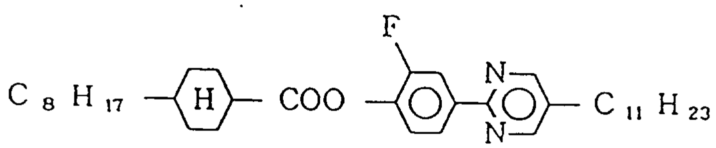

1 − 1 1 3

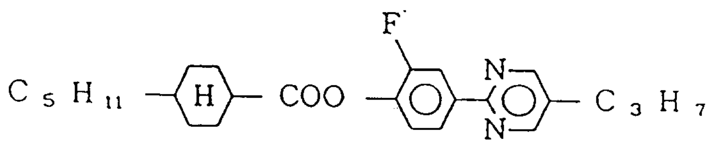

1 − 1 1 4

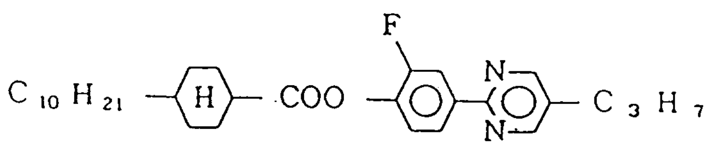

1 − 115

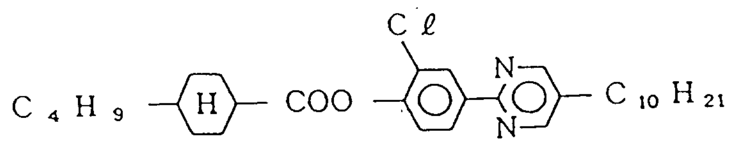

1 − 116

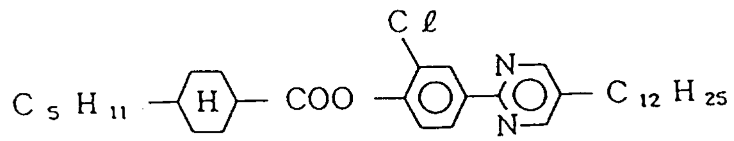

**4.** Composition de cristal liquide suivant la revendication 1, qui comprend un autre compose mésomorphe, autre que ceux représentés par les formules (I) et (III), et qui comprend un composé de formule (I) et un composé de formule (III) chacun en une quantité de 1 à 300 parties en poids, pour 100 parties en poids dudit autre composé mésomorphe.

**5.** Composition de cristal liquide suivant la revendication 1, qui comprend un autre composé mésomorphe autre que ceux représentés par les formules (I) et (III), et deux ou plus de deux types de composés de chacun d'au moins un groupe de composés de formule (I) et (III) en une quantité totale de 1 à 500 parties en poids pour 100 parties en poids dudit autre composé mésomorphe.

**6.** Composition de cristal liquide suivant la revendication 2, qui comprend un autre composé mésomorphe autre que ceux représentés par les formules (I), (II) et (III), et qui comprend un composé de formule (I), un composé de formule (II) et un composé de formule (III) chacun en une quantité de 1 à 300 parties en poids, pour 100 parties en poids dudit autre composé mésomorphe.

**7.** Composition de cristal liquide suivant la revendication 1, qui comprend un autre composé mésomorphe, autre que ceux représentés par les formules (I), (II) er (III), et deux ou plus de deux types de composés de chacun d'au moins un groupe de composés de formule (I), (II) et (III) en une quantité totale de 1 à 500 parties en poids pour 100 parties en poid dudit autre composé mésomorphe.

**8.** Composition de cristal liquide suivant l'une quelconque des revendications 1 à 7, qui comprend une phase smectique chirale.

**9.** Dispositif à cristaux liquides, comprenant une paire de plaques servant d'électrodes et une composition de cristal liquide suivant l'une quelconque des revendications 1 à 8 disposée entre les plaques servant d'électrodes.

**10.** Dispositif à cristaux liquides suivant la revendication 9, qui comprend en outre une couche d'ajustement d'alignement sur les plaque servant d'électrodes.

**11.** Dispositif à cristaux liquides suivant la revendication 10, dans lequel la couche d'ajustement d'alignement a été soumise à un frottement.

**12.** Dispositif à cristaux liquides suivant la revendication 9, dans lequel les deux plaques servant d'électrodes sont disposées avec entre elles un espace suffisamment petit pour libérer la structure hélicoïdale du cristal liquide.

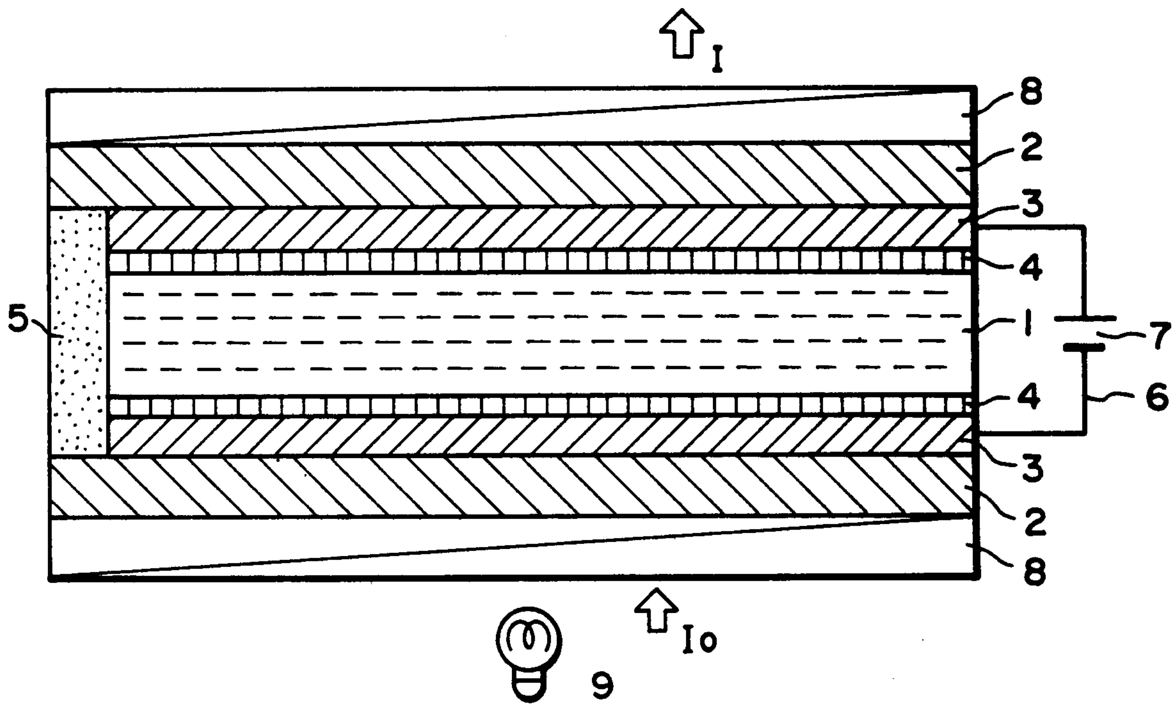


FIG. 1

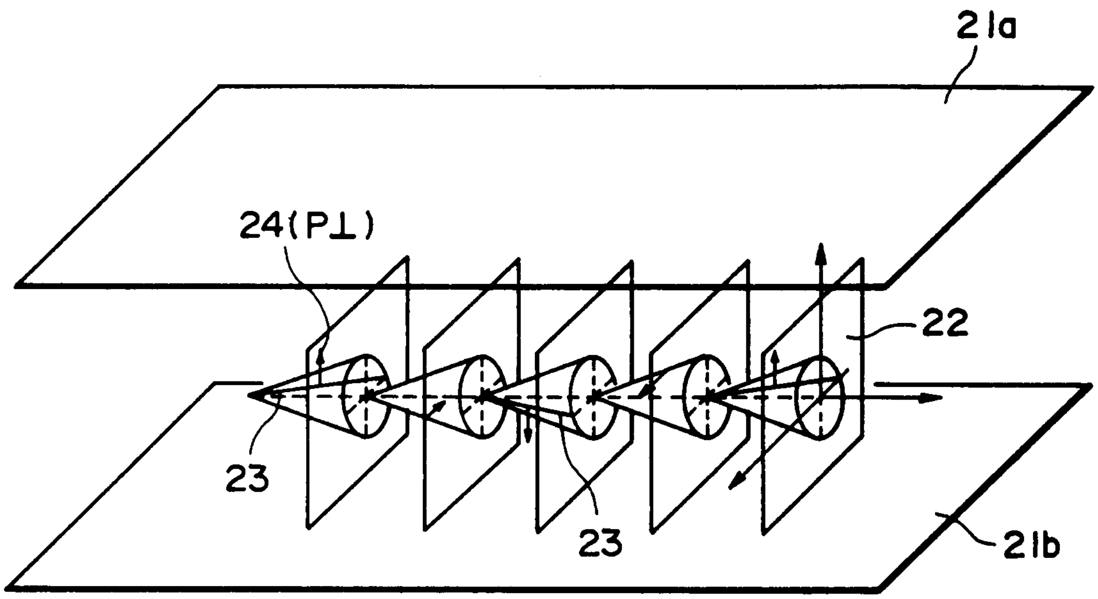


FIG. 2

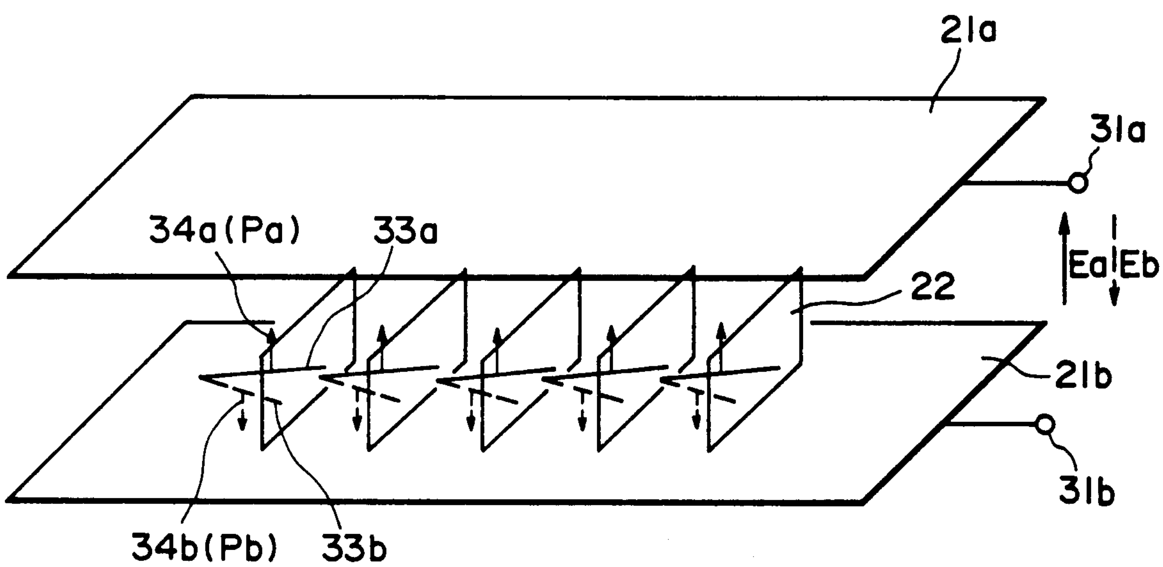


FIG. 3